# EUROPEAN PATENT APPLICATION

(11) **EP 4 417 602 A1**
(43) Date of publication of application: **21.08.2024**
(21) Application number: 23157025.0
(22) Date of filing: 16.02.2023
(51) Int. Cl.: C07D 401/06, A01N 43/56, C07D 403/06

(54) **SUBSTITUTED PYRAZOLE CARBOXAMIDES, SALTS THEREOF AND THEIR USE AS HERBICIDALLY ACTIVE SUBSTANCES**

(71) Applicant: Bayer AG, 51373 Leverkusen (DE)
(72) Inventor: FRACKENPOHL, Jens, 51373 Leverkusen (DE); KÖHN, Arnim, 51373 Leverkusen (DE); HEINEMANN, Ines, 51373 Leverkusen (DE); BROWN, Ronald, 51373 Leverkusen (DE); BARBER, David Michael, 51373 Leverkusen (DE); BRAUN, Ralf, 51373 Leverkusen (DE); MCLEOD, Michael Charles, 51373 Leverkusen (DE); BADART, Michael Peter, 51373 Leverkusen (DE); ALNAFTA, Neanne, 51373 Leverkusen (DE); MEISTER, Catherine Simone, 51373 Leverkusen (DE); REINGRUBER, Anna Maria, 51373 Leverkusen (DE); BOLLENBACH-WAHL, Birgit, 51373 Leverkusen (DE); GATZWEILER, Elmar, 51373 Leverkusen (DE)
(74) Representative: BIP Patents

(57) **Abstract**

The present invention relates to substituted pyrazole carboxamides of the general formula (I) or salts thereof, 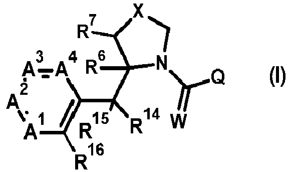 where the radicals in the general formula (I) correspond to the definitions given in the description, and to their use as herbicides, in particular for controlling broad-leaved weeds and/or weed grasses in crops of useful plants and/or as plant growth regulators for influencing the growth of crops of useful plants.

## Description

The invention relates to the technical field of crop protection agents, in particular that of herbicides for the selective control of broad-leaved weeds and weed grasses in crops of useful plants. Specifically, the present invention relates to substituted pyrazole carboxamides and salts thereof, to processes for their preparation and to their use as herbicides.

In their application, herbicides that are known to date for controlling harmful plants in crops of useful plants or herbicides for controlling unwanted vegetation sometimes have disadvantages, be it (a) that they have no or insufficient herbicidal activity against particularly harmful plants, (b) that the spectrum of harmful plants which can be controlled with an active compound is not broad enough, and/or (c) that the selectivity of the herbicides in and their compatibility with crop plants is too low, thereby causing unwanted damage and/or unwanted reduced harvest yields of the crops, and/or (d) that they have a toxicologically unfavorable profile.

Furthermore, some active compounds which can be used as plant growth regulators for a number of useful plants cause unwanted reduced harvest yields in other useful plants or are not compatible with the crop plant, or only within a narrow application rate range. Some of the known active compounds cannot be produced economically on an industrial scale owing to precursors and reagents which are difficult to obtain, or they have only insufficient chemical stabilities. In the case of other active compounds, the activity is too highly dependent on environmental conditions, such as weather and soil conditions.

Thus, there is still a need for alternative herbicides, in particular highly active herbicides that are useful at low application rates and/or having good compatibility with crop plants, for the selective application in plant crops or use on non-crop land. It is also desirable to provide alternative chemical active compounds which may be used in an advantageous manner as herbicides or plant growth regulators.

It is therefore an objective of the present invention to provide compounds having herbicidal activity which are highly effective against economically important harmful plants even at relatively low application rates and that can be used selectively in crop plants.

In FR2924931, certain heterocyclic 1-[(1H-pyrazol-4-yl)carbonyl] derivates intended for cosmetic use, are generically embraced in a narrow disclosure of compounds of the following formula: Wherein R₁ can represent a phenyl group or an alkyl CH₂-CH₃ group; R₂ represents a phenyl group or alkyl CH₂-CH₃ group or a CF₃ group, or a methoxymethyl group; amine R₃ and R₄ groups can represent one or two heteroatoms (N and/or O are mentioned as preferred) and can exist as the piperidine, *para-*methylpiperazine, azepane, and pyrrolidine rings. However, there is no disclosure or suggestion in this document of any such derivative bearing substituted arylhalide linked to the pyrrolidine moiety via a CH2-linker. Furthermore, there is no explicate disclosure or suggestion to select in this document of any such derivative wherein the substituted pyrazole ring bearing a methoxymethyl group in the 5^{th} position is described as having herbicidal properties.

In WO2009/153191, certain fungicidal proline amides are generically embraced in a broad disclosure of numerous compounds. However, there is no disclosure or suggestion in this document of any such derivative wherein A represents a methoxy methyl substituted pyrazole ring. Furthermore, there is no explicate disclosure or suggestion to select in this document of any such derivative where W represents a substituted phenyl ring bonded to central pyrrolidine core via an internal ether linker.

In WO2012/052491, certain fungicidal 1-(heterocyclic carbonyl)-2-substituted pyrrolidines and their thiocarbonyl derivatives are generically embraced in a broad disclosure of numerous compounds of the following formula: Wherein X₁ and X₂ can represent a halogen atom, T can represent an oxygen or a sulfur atom, n can represent 0, 1 or 2, Z₁ and Z₂ can represent various substituents including a hydrogen atom or a C₁-C₆ alkyl group, Q can represent a bond, an oxygen atom, an oxidized or unoxidized sulfur atom and Y can represent various rings among which is a phenyl ring. However, there is no explicit disclosure or suggestion to select in this document of any such derivative wherein n can represent 1, Q can represent a bond and z1 can be cyclized together with the Y ring.

In WO2015/052160, certain fungicidal 1-(heterylcarbonyl-2-(benzocycloalk-1-yl)piperidines or pyrrolidines are generically embraced in a broad disclosure of numerous compounds of the following formula: Wherein A represent a carbo-linked, unsaturated or partially saturated, 5-membered heterocyclyl group that can be substituted by up to four groups that can be the same or different, T represents an oxygen or sulfur atom, n represents 1 or 2, m represents 1, 2, or 3, while p represents 1,2,3 or 4. L₁ and L₂ independently represent CZ₄Z₅, NZ₆, an oxygen atom, a sulfur atom, a sulfide group, or a sulfone group. Z₁ can represent a hydrogen atom or a (un)substituted C₁-C₈ alkyl group, Z₂ can represents a hydrogen atom, a halogen atom, a hydroxyl group, a cyano group, an (un)substituted C₁-C₈ alkyl group, an (un)substituted C₁-C₈ alkoxy group, a C₁-C₈ halogenalkyl group, an amino group, or an Ci-Cs alkylamino. Z₃ can represent numerous substitutions patterns, including but not limited to the following; a hydrogen atom, a halogen atom, a nitro, cyano, isonitrile, hydroxy or amino sulfanyl group. However, there is no disclosure or suggestion in this document of any such derivative wherein A represents a methoxy methyl substituted pyrazole ring.

The aforementioned examples are distinct from the present invention, both in terms of their structure and uses. The substituted pyrazole carboxamides described herein do not contain alkyl or haloalkyl substituents attached to a carbon atom in the pyrazole moiety in all cases. More importantly, these are the first substituted pyrazole carboxamides bearing an alkoxyalkyl side chain to be described as having herbicidal properties.

It has now been surprisingly found that the substituted pyrazole carboxamides following general formula (I) or the salts thereof meet the said objectives and are particularly suitable as herbicides. Accordingly, the present invention provides substituted pyrazole carboxamides of the general formula (I) or salts thereof 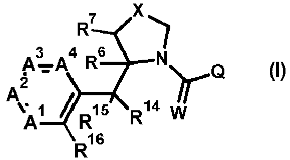 in which
Q represents a group Q-1 to Q-2 as outlined below 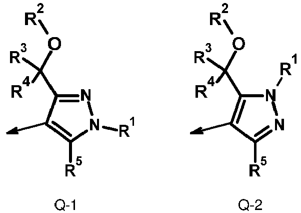
- A', A², A³ and A⁴: are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
- R¹⁷: in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- R¹⁷: in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- X: represents a direct bond or a group CR⁸R⁹, -CR⁸R⁹CH₂-, -CH₂CR⁸R⁹-,
- R¹: represents (C₁-C₈)-alkyl, (C₁-C₈)-haloalkyl, cyano-(C₁-C₈)-alkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, R¹²O-(C₁-C₈)-alkyl, R¹²O(R¹²O)CH-(C₁-C₈)-alkyl, tris[(C₁-C₈)-alkyl]silyl-O-(C₁-C₈)-alkyl, R¹⁰R¹¹N-(C₁-C₈)-alkyl, C(=O)R¹², C(=O)OR¹², C(=O)NR¹⁰R¹¹, R¹²O(O=)C-(C₁-C₈)-alkyl, R¹²(O=)C-(C₁-C₈)-alkyl, R¹⁰R¹¹N(O=)C-(C₁-C₈)-alkyl, S(O)ₚR¹³, R¹³S(O)ₚ-(C₁-C₈)-alkyl, (C₃-C₈)-cycloalkyl-(C₁-C₈)-alkyl, (C₃-C₈)-halocycloalkyl-(C₁-C₈)-alkyl, (C₄-C₈)-cycloalkenyl-(C₁-C₈)-alkyl, aryl, heteroaryl, heterocyclyl, aryl-(C₁-C₈)-alkyl, heteroaryl-(C₁-C₈)-alkyl, (C₃-C₈)-heterocyclyl-(C₁-C₈)-alkyl, 1,3-dioxolan-2-yl-(C₁-C₈)-alkyl, 4,5-dimethyl-1,3-dioxolan-2-yl-(C₁-C₈)-alkyl, 1,3-dioxan-2-yl-(C₁-C₈)-alkyl,
- R²: represents (C₁-C₈)-alkyl, (C₁-C₈)-haloalkyl, cyano-(C₁-C₈)-alkyl, (C₂-C₈)-alkenyl, (C₂-C₈)-alkynyl, aryl, heteroaryl, heterocyclyl, aryl-(C₁-C₈)-alkyl, heteroaryl-(C₁-C₈)-alkyl, (C₃-C₈)-heterocyclyl-(C₁-C₈)-alkyl, (C₃-C₈)-cycloalkyl, (C₃-C₈)-cycloalkyl-(C₁-C₈)-alkyl, (C₃-C₈)-halocycloalkyl, (C₃-C₈)-halocycloalkyl-(C₁-C₈)-alkyl, (C₁-C₈)-alkoxy-(C₁-C₈)-alkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-haloalkoxy-(C₁-C₈)-alkyl, R¹³S(O)ₚ-(C₁-C₈)-alkyl, R¹⁰R¹¹N-(C₁-C₈)-alkyl, R¹²O(O=)C-(C₁-C₈)-alkyl, R¹²(O=)C-(C₁-C₈)-alkyl, R¹⁰R¹¹N(O=)C-(C₁-C₈)-alkyl, tris[(C₁-C₈)-alkyl]silyl-O-(C₁-C₈)-alkyl, tris[(C₁-C₈)-alkyl]silyl-(C₁-C₈)-alkyl, tris[(C₁-C₈)-alkyl]silyl, bis-[(C₁-C₈)-alkyl](aryl)silyl, [(C₁-C₈)-alkyl]-bis-(aryl)silyl, bis-(C₁-C₈)-alkyl-C=N-, (C₁-C₈)-alkyl(aryl)-C=N-,
- R³ and R⁴: independently of one another represent hydrogen, (C₁-C₈)-alkyl, (C₁-C₈)-haloalkyl, (C₃-C₈)-cycloalkyl, (C₂-C₈)-alkenyl, R¹²O-(C₁-C₈)-alkyl, (C₁-C₈)-alkoxy,
- R⁵: represents hydrogen, halogen, (C₁-C₈)-alkyl, (C₁-C₈)-haloalkyl, (C₃-C₈)-cycloalkyl, (C₃-C₈)-halocycloalkyl,
- R⁶ and R⁷: independently of one another represent hydrogen, (C₁-C₈)-alkyl, (C₁-C₈)-haloalkyl, (C₃-C₈)-cycloalkyl, (C₂-C₈)-alkenyl,
- R⁸ and R⁹: independently of one another represent hydrogen, halogen, (C₁-C₈)-alkyl, (C₁-C₈)-haloalkyl, (C₃-C₈)-cycloalkyl, (C₂-C₈)-alkenyl, R¹²O-(C₁-C₈)-alkyl, OR¹², NR¹⁰R¹¹, SR¹³,
- R³ and R⁴: together with the carbon atom to which they are bonded form a fully saturated or partly saturated 3- to 10-membered monocyclic or bicyclic ring optionally interrupted by heteroatoms and optionally having further substitution,
- R³ and R⁴: together with the carbon atom to which they are bonded form a carbonyl group (C=O),
- R² and R³: together with the atoms to which they are bonded form a fully saturated or partly saturated 4-to 10-membered monocyclic or bicyclic ring optionally interrupted by heteroatoms and optionally having further substitution,
- R¹ and R⁵: together with the atoms to which they are bonded form a fully saturated or partly saturated 4-to 10-membered monocyclic or bicyclic ring optionally interrupted by heteroatoms and optionally having further substitution when Q represents a group Q-1,
- R¹ and R²: together with the atoms to which they are bonded form a fully saturated or partly saturated 4-to 10-membered monocyclic or bicyclic ring optionally interrupted by heteroatoms and optionally having further substitution when Q represents a group Q-2,
- p: is 0, 1, 2
- R¹⁰ and R¹¹: independently of one another represent hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-haloalkoxy-(C₁-C₈)-alkyl, (C₁-C₈)-alkylthio-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, COR¹², SO₂R¹³, heterocyclyl, (C₁-C₈)-alkoxycarbonyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkyl-aminocarbonyl-(C₁-C₈)-alkyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl, aryl-(C₁-C₈)-alkoxycarbonyl, heteroaryl-(C₁-C₈)-alkoxycarbonyl, (C₂-C₈)-alkenyloxycarbonyl, (C₂-C₈)-alkynyloxycarbonyl, heterocyclyl-(C₁-C₈)-alkyl, or
- R¹⁰ and R¹¹: together with the carbon atom to which they are bonded form a fully saturated or partly saturated 3- to 10-membered monocyclic or bicyclic ring optionally interrupted by heteroatoms and optionally having further substitution,
- R¹²: represents hydrogen, (C₁-C₈)-alkyl, (C₂-C₈)-alkenyl, (C₂-C₈)-alkinyl, (C₁-C₈)-cyanoalkyl, (C₁-C₁₀)-haloalkyl, (C₂-C₈)-haloalkenyl, (C₃-C₈)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-haloalkoxy-(C₁-C₈)-alkyl, (C₁-C₈)-alkoxy-(C₁-C₈)-haloalkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkoxy-(C₁-C₈)-alkyl, aryl, aryl-(C₁-C₈)-alkyl, aryl-(C₁-C₈)-alkoxy-(C₁-C₈)-alkyl, aryloxy-(C₁-C₈)-alkyl, heteroaryl-(C₁-C₈)-alkoxy-(C₁-C₈)-alkyl, heteroaryloxy-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkyl-aminocarbonyl-(C₁-C₈)-alkyl, bis-[(C₁-C₈)-alkyl]amino-(C₂-C₆)-alkyl, (C₁-C₈)-alkyl-amino-(C₂-C₆)-alkyl, aryl-(C₁-C₈)-alkylamino-(C₂-C₆)-alkyl, R¹³(O)ₚS-(C₁-C₈)-alkyl, hydroxycarbonyl-(C₁-C₈)-alkyl, heterocyclyl, heterocyclyl-(C₁-C₈)-alkyl, heterocyclyl-(C₁-C₈)-alkoxy-(C₁-C₈)-alkyl, heterocyclyloxy-(C₁-C₈)-alkyl, tris-[(C₁-C₈)-alkyl]silyl-(C₁-C₈)-alkyl, bis-[(C₁-C₈)-alkyl](aryl)silyl(C₁-C₈)-alkyl, [(C₁-C₈)-alkyl]-bis-(aryl)silyl-(C₁-C₈)-alkyl, (C₁-C₈)-alkylcarbonyloxy-(C₁-C₈)-alkyl, (C₃-C₈)-cycloalkylcarbonyloxy-(C₁-C₈)-alkyl, arylcarbonyloxy-(C₁-C₈)-alkyl, heteroarylcarbonyloxy-(C₁-C₈)-alkyl, Heterocyclylcarbonyloxy-(C₁-C₈)-alkyl, (C₁-C₈)-alkoxycarbonyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl,
- R¹³: represents hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, heterocyclyl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]amino, (C₁-C₈)-alkyl-amino, aryl-(C₁-C₈)-amino, aryl-(C₁-C₆)-alkyl-amino, aryl-[(C₁-C₈)-alkyl]amino; heteroaryl-(C₁-C₈)-amino, heteroaryl-(C₁-C₆)-alkyl-amino, heteroaryl-[(C₁-C₈)-alkyl]amino; Hetercyclyl-(C₁-C₈)-amino, heterocyclyl-(C₁-C₆)-alkyl-amino, heterocyclyl-[(C₁-C₈)-alkyl]amino; (C₃-C₈)-cycloalkyl-amino, (C₃-C₈)-cycloalkyl-[(C₁-C₈)-alkyl]amino, (C₁-C₈)-alkylcarbonylamino, arylcarbonylamino, hetarylcarbonylamino, N-azetidinyl, N-pyrrolidinyl, N-piperidinyl, N-morpholinyl,
- R¹⁴ and R¹⁵: independently of one another represent hydrogen, hydroxy, (C₁-C₈)-alkyl, (C₁-C₈)-haloalkyl, (C₃-C₈)-cycloalkyl, (C₂-C₈)-alkenyl, (C₁-C₈)-alkoxy, or
- R¹⁴ and R¹⁵: together with the carbon atom to which they are bonded form a fully saturated or partly saturated 3- to 10-membered monocyclic or bicyclic ring optionally interrupted by heteroatoms and optionally having further substitution, or
- R¹⁴ and R¹⁵: together with the carbon atom to which they are bonded form an ethylene group (C=CH₂) or a carbonyl group (C=O),
- R¹⁶: represents halogen, nitro, cyano, thiocyanato, (C₁-C₈)-alkyl, (C₁-C₈)-haloalkyl, cyano-(C₁-C₈)-alkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, OR¹², R¹²O-(C₁-C₈)-alkyl, R¹⁰R¹¹N-(C₁-C₈)-alkyl, NR¹⁰R¹¹, C(=O)R¹², C(=O)OR¹², C(=O)NR¹⁰R¹¹, R¹²O(O)C-(C₁-C₈)-alkyl, R¹²(O=)C-(C₁-C₈)-Alkyl, R¹⁰R¹¹N(O)C-(C₁-C₈)-alkyl, S(O)ₚR¹³, R¹³S(O)ₚ-(C₁-C₈)-alkyl, (C₃-C₈)-cycloalkyl-(C₁-C₈)-alkyl, aryl, heteroaryl, heterocyclyl, aryl-(C₁-C₈)-alkyl, heteroaryl-(C₁-C₈)-alkyl, (C₃-C₈)-heterocyclyl-(C₁-C₈)-alkyl,
- R¹⁷: represents hydrogen, halogen, nitro, cyano, thiocyanato, (C₁-C₈)-alkyl, (C₁-C₈)-haloalkyl, cyano-(C₁-C₈)-alkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, (C₄-C₈)-cycloalkenyl, (C₄-C₈)-halocycloalkenyl, OR¹², R¹²O-(C₁-C₈)-alkyl, R¹⁰R¹¹N-(C₁-C₈)-alkyl, NR¹⁰R¹¹, C(=O)R¹², C(=O)OR¹², C(=O)NR¹⁰R¹¹, R¹²O(O)C-(C₁-C₈)-alkyl, R¹²(O=)C-(C₁-C₈)-Alkyl, R¹⁰R¹¹N(O)C-(C₁-C₈)-alkyl, S(O)ₚR¹³, R¹³S(O)ₚ-(C₁-C₈)-alkyl, (C₃-C₈)-cycloalkyl-(C₁-C₈)-alkyl, (C₃-C₈)-halocycloalkyl-(C₁-C₈)-alkyl, (C₄-C₈)-cycloalkenyl-(C₁-C₈)-alkyl, (C₄-C₈)-halocycloalkenyl-(C₁-C₈)-alkyl, aryl, heterocyclyl, heteroaryl, aryl-(C₁-C₈)-alkyl, heteroaryl-(C₁-C₈)-alkyl, heterocyclyl-(C₁-C₈)-alkyl, tris[(C₁-C₈)alkyl]silyl, bis-[(C₁-C₈)-alkyl](aryl)silyl, (C₁-C₈)-alkyl-[bis-(aryl)]silyl, tris-[(C₁-C₈)-alkyl]silyl-(C₁-C₈)-alkyl, bis-[(C₁-C₈)-alkyl](aryl)silyl(C₁-C₈)-alkyl or (C₁-C₈)-alkyl-[bis-(aryl)]silyl-(C₁-C₈)-alkyl
and
- W: represents oxygen (O), sulfur (S).

The compounds of the general formula (I) can form salts by addition of a suitable inorganic or organic acid, for example mineral acids, for example HCl, HBr, H₂SO₄, H₃PO₄ or HNO₃, or organic acids, for example carboxylic acids such as formic acid, acetic acid, propionic acid, oxalic acid, lactic acid or salicylic acid or sulfonic acids, for example p-toluenesulfonic acid, onto a basic group, for example amino, alkylamino, dialkylamino, piperidino, morpholino or pyridino. In such a case, these salts will comprise the conjugated base of the acid as the anion. Suitable substituents in deprotonated form, for example sulfonic acids, particular sulfonamides or carboxylic acids, are capable of forming internal salts with groups, such as amino groups, which are themselves protonatable. Salts may also be formed by action of a base on compounds of the general formula (I). Suitable bases are, for example, organic amines such as trialkylamines, morpholine, piperidine and pyridine, and the hydroxides, carbonates and bicarbonates of ammonium, alkali metals or alkaline earth metals, especially sodium hydroxide, potassium hydroxide, sodium carbonate, potassium carbonate, sodium bicarbonate and potassium bicarbonate. These salts are compounds in which the acidic hydrogen is replaced by an agriculturally suitable cation, for example metal salts, especially alkali metal salts or alkaline earth metal salts, in particular sodium and potassium salts, or else ammonium salts, salts with organic amines or quaternary ammonium salts, for example with cations of the formula [NR^{a}R^{b}R^{c}R^{d}]⁺ in which R^{a} to R^{d} are each independently an organic radical, especially alkyl, aryl, arylalkyl or alkylaryl. Also suitable are alkylsulfonium and alkylsulfoxonium salts, such as (C₁-C₄)-trialkylsulfonium and (C₁-C₄)-trialkylsulfoxonium salts.

The substituted pyrazole carboxamides of the general formula (I) according to the invention can, depending on external conditions such as pH, solvent and temperature, be present in various tautomeric structures, all of which are embraced by the general formula (I).

The compounds of the formula (I) used in accordance with the invention and salts thereof are also referred to hereinafter as "compounds of the general formula (I)".

The invention preferably provides compounds of the general formula (I) in which
- Q: represents a group Q-1 to Q-2 as outlined below 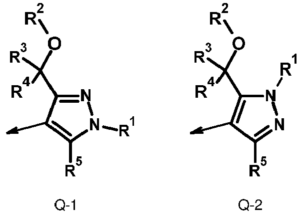
- A', A², A³ and A⁴: are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
- R¹⁷: in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- R¹⁷: in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- X: represents a direct bond or a group CR⁸R⁹, -CR⁸R⁹CH₂-, -CH₂CR⁸R⁹-,
- R¹: represents (C₁-C₇)-alkyl, (C₁-C₇)-haloalkyl, cyano-(C₁-C₇)-alkyl, (C₂-C₇)-alkenyl, (C₂-C₇)-haloalkenyl, (C₂-C₇)-alkynyl, (C₂-C₇)-haloalkynyl, (C₃-C₇)-cycloalkyl, (C₃-C₇)-halocycloalkyl, R¹²O-(C₁-C₇)-alkyl, R¹²O(R¹²O)CH-(C₁-C₇)-alkyl, tris[(C₁-C₇)-alkyl]silyl-O-(C₁-C₇)-alkyl, R¹⁰R¹¹N-(C₁-C₇)-alkyl, C(=O)R¹², C(=O)OR¹², C(=O)NR¹⁰R¹¹, R¹²O(O=)C-(C₁-C₇)-alkyl, R¹²(O=)C-(C₁-C₇)-alkyl, R¹⁰R¹¹N(O=)C-(C₁-C₇)-alkyl, S(O)ₚR¹³, R¹³S(O)ₚ-(C₁-C₇)-alkyl, (C₃-C₇)-cycloalkyl-(C₁-C₇)-alkyl, (C₃-C₇)-halocycloalkyl-(C₁-C₇)-alkyl, (C₄-C₇)-cycloalkenyl-(C₁-C₇)-alkyl, aryl, heteroaryl, heterocyclyl, aryl-(C₁-C₇)-alkyl, heteroaryl-(C₁-C₇)-alkyl, (C₃-C₇)-heterocyclyl-(C₁-C₇)-alkyl, 1,3-dioxolan-2-yl-(C₁-C₇)-alkyl, 4,5-dimethyl-1,3-dioxolan-2-yl-(C₁-C₇)-alkyl, 1,3-dioxan-2-yl-(C₁-C₇)-alkyl,
- R²: represents (C₁-C₇)-alkyl, (C₁-C₇)-haloalkyl, cyano-(C₁-C₇)-alkyl, (C₂-C₇)-alkenyl, (C₂-C₇)- (C₂-C₇)-alkynyl, aryl, heteroaryl, heterocyclyl, aryl-(C₁-C₇)-alkyl, heteroaryl-(C₁-C₇)-alkyl, (C₃-C₇)-heterocyclyl-(C₁-C₇)-alkyl, (C₃-C₇)-cycloalkyl, (C₃-C₇)-cycloalkyl-(C₁-C₇)-alkyl, (C₃-C₇)-halocycloalkyl, (C₃-C₇)-halocycloalkyl-(C₁-C₇)-alkyl, (C₁-C₇)-alkoxy-(C₁-C₇)-alkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-haloalkoxy-(C₁-C₇)-alkyl, R¹³S(O)ₚ-(C₁-C₇)-alkyl, R¹⁰R¹¹N-(C₁-C₇)-alkyl, R¹²O(O=)C-(C₁-C₇)-alkyl, R¹²(O=)C-(C₁-C₇)-alkyl, R¹⁰R¹¹N(O=)C-(C₁-C₇)-alkyl, tris[(C₁-C₇)-alkyl]silyl-O-(C₁-C₇)-alkyl, tris[(C₁-C₇)-alkyl]silyl-(C₁-C₇)-alkyl, tris[(C₁-C₇)-alkyl]silyl, bis-[(C₁-C₇)-alkyl](aryl)silyl, [(C₁-C₇)-alkyl]-bis-(aryl)silyl, bis-(C₁-C₇)-alkyl-C=N-, (C₁-C₇)-alkyl(aryl)-C=N-,
- R³ and R⁴: independently of one another represent hydrogen, (C₁-C₇)-alkyl, (C₁-C₇)-haloalkyl, (C₃-C₇)-cycloalkyl, (C₂-C₇)-alkenyl, R¹²O-(C₁-C₇)-alkyl, (C₁-C₇)-alkoxy,
- R⁵: represents hydrogen, halogen, (C₁-C₇)-alkyl, (C₁-C₇)-haloalkyl, (C₃-C₇)-cycloalkyl, (C₃-C₇)-halocycloalkyl,
- R⁶ and R⁷: independently of one another represent hydrogen, (C₁-C₇)-alkyl, (C₁-C₇)-haloalkyl, (C₃-C₇)-cycloalkyl, (C₂-C₇)-alkenyl,
- R⁸ and R⁹: independently of one another represent hydrogen, halogen, (C₁-C₇)-alkyl, (C₁-C₇)-haloalkyl, (C₃-C₇)-cycloalkyl, (C₂-C₇)-alkenyl, R¹²O-(C₁-C₇)-alkyl, OR¹², NR¹⁰R¹¹, SR¹³,
- R³ and R⁴: together with the carbon atom to which they are bonded form a fully saturated or partly saturated 3- to 10-membered monocyclic or bicyclic ring optionally interrupted by heteroatoms and optionally having further substitution, or
- R³ and R⁴: together with the carbon atom to which they are bonded form a carbonyl group (C=O),
- R² and R³: together with the atoms to which they are bonded form a fully saturated or partly saturated 4-to 10-membered monocyclic or bicyclic ring optionally interrupted by heteroatoms and optionally having further substitution,
- R¹ and R⁵: together with the atoms to which they are bonded form a fully saturated or partly saturated 4-to 10-membered monocyclic or bicyclic ring optionally interrupted by heteroatoms and optionally having further substitution when Q represents a group Q-1,
- R¹ and R²: together with the atoms to which they are bonded form a fully saturated or partly saturated 4-to 10-membered monocyclic or bicyclic ring optionally interrupted by heteroatoms and optionally having further substitution when Q represents a group Q-2,
- p: is 0, 1, 2
- R¹⁰ and R¹¹: independently of one another represent hydrogen, (C₁-C₇)-alkyl, (C₂-C₇)-alkenyl, (C₂-C₇)-alkynyl, (C₁-C₇)-cyanoalkyl, (C₁-C₁₀)-haloalkyl, (C₂-C₇)-haloalkenyl, (C₃-C₇)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-haloalkoxy-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, (C₃-C₇)-cycloalkyl-(C₁-C₇)-alkyl, (C₄-C₁₀)-cycloalkenyl-(C₁-C₇)-alkyl, COR¹², SO₂R¹³, heterocyclyl, (C₁-C₇)-alkoxycarbonyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkyl-aminocarbonyl-(C₁-C₇)-alkyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl, aryl-(C₁-C₇)-alkoxycarbonyl, heteroaryl-(C₁-C₇)-alkoxycarbonyl, (C₂-C₇)-alkenyloxycarbonyl, (C₂-C₇)-alkynyloxycarbonyl, heterocyclyl-(C₁-C₇)-alkyl, or
- R¹⁰ and R¹¹: together with the carbon atom to which they are bonded form a fully saturated or partly saturated 3- to 10-membered monocyclic or bicyclic ring optionally interrupted by heteroatoms and optionally having further substitution,
- R¹²: represents hydrogen, (C₁-C₇)-alkyl, (C₂-C₇)-alkenyl, (C₂-C₇)-alkinyl, (C₁-C₇)-cyanoalkyl, (C₁-C₁₀)-haloalkyl, (C₂-C₇)-haloalkenyl, (C₃-C₇)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-haloalkoxy-(C₁-C₇)-alkyl, (C₁-C₇)-alkoxy-(C₁-C₇)-haloalkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkoxy-(C₁-C₇)-alkyl, aryl, aryl-(C₁-C₇)-alkyl, aryl-(C₁-C₇)-alkoxy-(C₁-C₇)-alkyl, aryloxy-(C₁-C₇)-alkyl, heteroaryl-(C₁-C₇)-alkoxy-(C₁-C₇)-alkyl, heteroaryloxy-(C₁-C₇)-alkyl, heteroaryl, heteroaryl-(C₁-C₇)-alkyl, (C₃-C₇)-cycloalkyl-(C₁-C₇)-alkyl, (C₄-C₁₀)-cycloalkenyl-(C₁-C₇)-alkyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkyl-aminocarbonyl-(C₁-C₇)-alkyl, bis-[(C₁-C₇)-alkyl]amino-(C₂-C₆)-alkyl, (C₁-C₇)-alkyl-amino-(C₂-C₆)-alkyl, aryl-(C₁-C₇)-alkylamino-(C₂-C₆)-alkyl, R¹³(O)ₚS-(C₁-C₇)-alkyl, hydroxycarbonyl-(C₁-C₇)-alkyl, heterocyclyl, heterocyclyl-(C₁-C₇)-alkyl, heterocyclyl-(C₁-C₇)-alkoxy-(C₁-C₇)-alkyl, heterocyclyloxy-(C₁-C₇)-alkyl, tris-[(C₁-C₇)-alkyl]silyl-(C₁-C₇)-alkyl, bis-[(C₁-C₇)-alkyl](aryl)silyl(C₁-C₇)-alkyl, [(C₁-C₇)-alkyl]-bis-(aryl)silyl-(C₁-C₇)-alkyl, (C₁-C₇)-alkylcarbonyloxy-(C₁-C₇)-alkyl, (C₃-C₇)-cycloalkylcarbonyloxy-(C₁-C₇)-alkyl, arylcarbonyloxy-(C₁-C₇)-alkyl, heteroarylcarbonyloxy-(C₁-C₇)-alkyl, Heterocyclylcarbonyloxy-(C₁-C₇)-alkyl, (C₁-C₇)-alkoxycarbonyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl,
- R¹³: represents hydrogen, (C₁-C₇)-alkyl, (C₂-C₇)-alkenyl, (C₂-C₇)-alkynyl, (C₁-C₇)-cyanoalkyl, (C₁-C₁₀)-haloalkyl, (C₂-C₇)-haloalkenyl, (C₃-C₇)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, heterocyclyl-(C₁-C₇)-alkyl, (C₃-C₇)-cycloalkyl-(C₁-C₇)-alkyl, (C₄-C₁₀)-cycloalkenyl-(C₁-C₇)-alkyl, bis-[(C₁-C₇)-alkyl]amino, (C₁-C₇)-alkyl-amino, aryl-(C₁-C₇)-amino, aryl-(C₁-C₆)-alkyl-amino, aryl-[(C₁-C₇)-alkyl]amino; heteroaryl-(C₁-C₇)-amino, heteroaryl-(C₁-C₆)-alkyl-amino, heteroaryl-[(C₁-C₇)-alkyl]amino; Hetercyclyl-(C₁-C₇)-amino, heterocyclyl-(C₁-C₆)-alkyl-amino, heterocyclyl-[(C₁-C₇)-alkyl]amino; (C₃-C₇)-cycloalkyl-amino, (C₃-C₇)-cycloalkyl-[(C₁-C₇)-alkyl]amino, (C₁-C₇)-alkylcarbonylamino, arylcarbonylamino, hetarylcarbonylamino, N-azetidinyl, N-pyrrolidinyl, N-piperidinyl, N-morpholinyl,
- R¹⁴ and R¹⁵: independently of one another represent hydrogen, hydroxy, (C₁-C₇)-alkyl, (C₁-C₇)-haloalkyl, (C₃-C₇)-cycloalkyl, (C₂-C₇)-alkenyl, (C₁-C₇)-alkoxy, or
- R¹⁴ and R¹⁵: together with the carbon atom to which they are bonded form a fully saturated or partly saturated 3- to 10-membered monocyclic or bicyclic ring optionally interrupted by heteroatoms and optionally having further substitution, or
- R¹⁴ and R¹⁵: together with the carbon atom to which they are bonded form an ethylene group (C=CH₂) or a carbonyl group (C=O),
- R¹⁶: represents halogen, nitro, cyano, thiocyanato, (C₁-C₇)-alkyl, (C₁-C₇)-haloalkyl, cyano-(C₁-C₇)-alkyl, (C₂-C₇)-alkenyl, (C₂-C₇)-haloalkenyl, (C₂-C₇)-alkynyl, (C₂-C₇)-haloalkynyl, (C₃-C₇)-cycloalkyl, (C₃-C₇)-halocycloalkyl, OR¹², R¹²O-(C₁-C₇)-alkyl, R¹⁰R¹¹N-(C₁-C₇)-alkyl, NR¹⁰R¹¹, C(=O)R¹², C(=O)OR¹², C(=O)NR¹⁰R¹¹, R¹²O(O)C-(C₁-C₇)-alkyl, R¹²(O=)C-(C₁-C₇)-Alkyl, R¹¹R¹¹N(O)C-(C₁-C₇)-alkyl, S(O)ₚR¹³, R¹³S(O)ₚ-(C₁-C₇)-alkyl, (C₃-C₇)-cycloalkyl-(C₁-C₇)-alkyl, aryl, heteroaryl, heterocyclyl, aryl-(C₁-C₇)-alkyl, heteroaryl-(C₁-C₇)-alkyl, (C₃-C₇)-heterocyclyl-(C₁-C₇)-alkyl,
- R¹⁷: represents hydrogen, halogen, nitro, cyano, thiocyanato, (C₁-C₇)-alkyl, (C₁-C₇)-haloalkyl, cyano-(C₁-C₇)-alkyl, (C₂-C₇)-alkenyl, (C₂-C₇)-haloalkenyl, (C₂-C₇)-alkynyl, (C₂-C₇)-haloalkynyl, (C₃-C₇)-cycloalkyl, (C₃-C₇)-halocycloalkyl, (C₄-C₇)-cycloalkenyl, (C₄-C₇)-halocycloalkenyl, OR¹², R¹²O-(C₁-C₇)-alkyl, R¹⁰R¹¹N-(C₁-C₇)-alkyl, NR¹⁰R¹¹, C(=O)R¹², C(=O)OR¹², C(=O)NR¹⁰R¹¹, R¹²O(O)C-(C₁-C₇)-alkyl, R¹²(O=)C-(C₁-C₇)-Alkyl, R¹⁰R¹¹N(O)C-(C₁-C₇)-alkyl, S(O)ₚR¹³, R¹³S(O)ₚ-(C₁-C₇)-alkyl, (C₃-C₇)-cycloalkyl-(C₁-C₇)-alkyl, (C₃-C₇)-halocycloalkyl-(C₁-C₇)-alkyl, (C₄-C₇)-cycloalkenyl-(C₁-C₇)-alkyl, (C₄-C₇)-halocycloalkenyl-(C₁-C₇)-alkyl, aryl, heterocyclyl, heteroaryl, aryl-(C₁-C₇)-alkyl, heteroaryl-(C₁-C₇)-alkyl, heterocyclyl-(C₁-C₇)-alkyl, tris[(C₁-C₇)alkyl]silyl, bis-[(C₁-C₇)-alkyl](aryl)silyl, (C₁-C₇)-alkyl-[bis-(aryl)]silyl, tris-[(C₁-C₇)-alkyl]silyl-(C₁-C₇)-alkyl, bis-[(C₁-C₇)-alkyl](aryl)silyl(C₁-C₇)-alkyl or (C₁-C₇)-alkyl-[bis-(aryl)]silyl-(C₁-C₇)-alkyl
and
- W: represents oxygen (O) and sulfur (S), preferably oxygen (O).

The invention more preferably provides compounds of the general formula (I) in which
- Q: represents a group Q-1 to Q-2 as outlined below 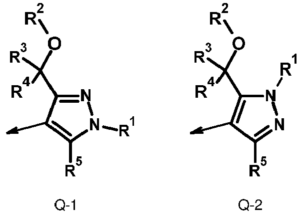
- A¹, A², A³ and A⁴: are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
- R¹⁷: in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- R¹⁷: in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- X: represents a direct bond or a group CR⁸R⁹, -CR⁸R⁹CH₂-, -CH₂CR⁸R⁹-,
- R¹: represents (C₁-C₆)-alkyl, (C₁-C₆)-haloalkyl, cyano-(C₁-C₆)-alkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, R¹²O-(C₁-C₆)-alkyl, R¹²O(R¹²O)CH-(C₁-C₆)-alkyl, tris[(C₁-C₆)-alkyl]silyl-O-(C₁-C₆)-alkyl, R¹⁰R¹¹N-(C₁-C₆)-alkyl, C(=O)R¹², C(=O)OR¹², C(=O)NR¹⁰R¹¹, R¹²O(O=)C-(C₁-C₆)-alkyl, R¹²(O=)C-(C₁-C₆)-alkyl, R¹⁰R¹¹N(O=)C-(C₁-C₆)-alkyl, S(O)ₚR¹³, R¹³S(O)ₚ-(C₁-C₆)-alkyl, (C₃-C₆)-cycloalkyl-(C₁-C₆)-alkyl, (C₃-C₆)-halocycloalkyl-(C₁-C₆)-alkyl, (C₄-C₆)-cycloalkenyl-(C₁-C₆)-alkyl, aryl, heteroaryl, heterocyclyl, aryl-(C₁-C₆)-alkyl, heteroaryl-(C₁-C₆)-alkyl, (C₃-C₆)-heterocyclyl-(C₁-C₆)-alkyl, 1,3-dioxolan-2-yl-(C₁-C₆)-alkyl, 4,5-dimethyl-1,3-dioxolan-2-yl-(C₁-C₆)-alkyl, 1,3-dioxan-2-yl-(C₁-C₆)-alkyl,
- R₂: represents (C₁-C₆)-alkyl, (C₁-C₆)-haloalkyl, cyano-(C₁-C₆)-alkyl, (C₂-C₆)-alkenyl, (C₂-C₆)- (C₂-C₆)-alkynyl, aryl, heteroaryl, heterocyclyl, aryl-(C₁-C₆)-alkyl, heteroaryl-(C₁-C₆)-alkyl, (C₃-C₆)-heterocyclyl-(C₁-C₆)-alkyl, (C₃-C₆)-cycloalkyl, (C₃-C₆)-cycloalkyl-(C₁-C₆)-alkyl, (C₃-C₆)-halocycloalkyl, (C₃-C₆)-halocycloalkyl-(C₁-C₆)-alkyl, (C₁-C₆)-alkoxy-(C₁-C₆)-alkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-haloalkoxy-(C₁-C₆)-alkyl, R¹³S(O)ₚ-(C₁-C₆)-alkyl, R¹⁰R¹¹N-(C₁-C₆)-alkyl, R¹²O(O=)C-(C₁-C₆)-alkyl, R¹²(O=)C-(C₁-C₆)-alkyl, R¹⁰R¹¹N(O=)C-(C₁-C₆)-alkyl, tris[(C₁-C₆)-alkyl]silyl-O-(C₁-C₆)-alkyl, tris[(C₁-C₆)-alkyl]silyl-(C₁-C₆)-alkyl, tris[(C₁-C₆)-alkyl]silyl, bis-[(C₁-C₆)-alkyl](aryl)silyl, [(C₁-C₆)-alkyl]-bis-(aryl)silyl, bis-(C₁-C₆)-alkyl-C=N-, (C₁-C₆)-alkyl(aryl)-C=N-,
- R³ and R⁴: independently of one another represent hydrogen, (C₁-C₆)-alkyl, (C₁-C₆)-haloalkyl, (C₃-C₆)-cycloalkyl, (C₂-C₆)-alkenyl, R¹²O-(C₁-C₆)-alkyl, (C₁-C₆)-alkoxy,
- R⁵: represents hydrogen, halogen, (C₁-C₆)-alkyl, (C₁-C₆)-haloalkyl, (C₃-C₆)-cycloalkyl, (C₃-C₆)-halocycloalkyl,
- R⁶ and R⁷: independently of one another represent hydrogen, (C₁-C₆)-alkyl, (C₁-C₆)-haloalkyl, (C₃-C₆)-cycloalkyl, (C₂-C₆)-alkenyl,
- R⁸ and R⁹: independently of one another represent hydrogen, halogen, (C₁-C₆)-alkyl, (C₁-C₆)-haloalkyl, (C₃-C₆)-cycloalkyl, (C₂-C₆)-alkenyl, R¹²O-(C₁-C₆)-alkyl, OR¹², NR¹⁰R¹¹, SR¹³,
- R³ and R⁴: together with the carbon atom to which they are bonded form a fully saturated or partly saturated 3- to 10-membered monocyclic or bicyclic ring optionally interrupted by heteroatoms and optionally having further substitution, or
- R³ and R⁴: together with the carbon atom to which they are bonded form a carbonyl group (C=O),
- R² and R³: together with the atoms to which they are bonded form a fully saturated or partly saturated 4-to 10-membered monocyclic or bicyclic ring optionally interrupted by heteroatoms and optionally having further substitution,
- R¹ and R⁵: together with the atoms to which they are bonded form a fully saturated or partly saturated 4-to 10-membered monocyclic or bicyclic ring optionally interrupted by heteroatoms and optionally having further substitution when Q represents a group Q-1,
- R¹ and R²: together with the atoms to which they are bonded form a fully saturated or partly saturated 4-to 10-membered monocyclic or bicyclic ring optionally interrupted by heteroatoms and optionally having further substitution when Q represents a group Q-2,
- p: is 0, 1, 2
- R¹⁰ and R¹¹: independently of one another represent hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₃-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-haloalkoxy-(C₁-C₆)-alkyl, (C₁-C₆)-alkylthio-(C₁-C₆)-alkyl, (C₁-C₆)-haloalkylthio-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, COR¹², SO₂R¹³, heterocyclyl, (C₁-C₆)-alkoxycarbonyl, bis-[(C₁-C₆)-alkyl]aminocarbonyl-(C₁-C₆)-alkyl, (C₁-C₆)-alkyl-aminocarbonyl-(C₁-C₆)-alkyl, aryl-(C₁-C₆)-alkyl-aminocarbonyl-(C₁-C₆)-alkyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl, aryl-(C₁-C₆)-alkoxycarbonyl, heteroaryl-(C₁-C₆)-alkoxycarbonyl, (C₂-C₆)-alkenyloxycarbonyl, (C₂-C₆)-alkynyloxycarbonyl, heterocyclyl-(C₁-C₆)-alkyl, or
- R¹⁰ and R¹¹: together with the carbon atom to which they are bonded form a fully saturated or partly saturated 3- to 10-membered monocyclic or bicyclic ring optionally interrupted by heteroatoms and optionally having further substitution,
- R¹²: represents hydrogen, (C₁-C₆)-alkyl, (C₂-C₆)-alkenyl, (C₂-C₆)-alkinyl, (C₁-C₆)-cyanoalkyl, (C₁-C₁₀)-haloalkyl, (C₂-C₆)-haloalkenyl, (C₃-C₆)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-haloalkoxy-(C₁-C₆)-alkyl, (C₁-C₆)-alkoxy-(C₁-C₆)-haloalkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkoxy-(C₁-C₆)-alkyl, aryl, aryl-(C₁-C₆)-alkyl, aryl-(C₁-C₆)-alkoxy-(C₁-C₆)-alkyl, aryloxy-(C₁-C₆)-alkyl, heteroaryl-(C₁-C₆)-alkoxy-(C₁-C₆)-alkyl, heteroaryloxy-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, bis-[(C₁-C₆)-alkyl]aminocarbonyl-(C₁-C₆)-alkyl, (C₁-C₆)-alkyl-aminocarbonyl-(C₁-C₆)-alkyl, aryl-(C₁-C₆)-alkyl-aminocarbonyl-(C₁-C₆)-alkyl, bis-[(C₁-C₆)-alkyl]amino-(C₂-C₆)-alkyl, (C₁-C₆)-alkyl-amino-(C₂-C₆)-alkyl, aryl-(C₁-C₆)-alkylamino-(C₂-C₆)-alkyl, R¹³(O)ₚS-(C₁-C₆)-alkyl, hydroxycarbonyl-(C₁-C₆)-alkyl, heterocyclyl, heterocyclyl-(C₁-C₆)-alkyl, heterocyclyl-(C₁-C₆)-alkoxy-(C₁-C₆)-alkyl, heterocyclyloxy-(C₁-C₆)-alkyl, tris-[(C₁-C₆)-alkyl]silyl-(C₁-C₆)-alkyl, bis-[(C₁-C₆)-alkyl](aryl)silyl(C₁-C₆)-alkyl, [(C₁-C₆)-alkyl]-bis-(aryl)silyl-(C₁-C₆)-alkyl, (C₁-C₆)-alkylcarbonyloxy-(C₁-C₆)-alkyl, (C₃-C₆)-cycloalkylcarbonyloxy-(C₁-C₆)-alkyl, arylcarbonyloxy-(C₁-C₆)-alkyl, heteroarylcarbonyloxy-(C₁-C₆)-alkyl, Heterocyclylcarbonyloxy-(C₁-C₆)-alkyl, (C₁-C₆)-alkoxycarbonyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl,
- R¹³: represents hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₃-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, heterocyclyl-(C₁-C₆)-alkyl, (C₃-C₆)-cycloalkyl-(C₁-C₆)-alkyl, (C₄-C₁₀)-cycloalkenyl-(C₁-C₆)-alkyl, bis-[(C₁-C₆)-alkyl]amino, (C₁-C₆)-alkyl-amino, aryl-(C₁-C₆)-amino, aryl-(C₁-C₆)-alkyl-amino, aryl-[(C₁-C₆)-alkyl]amino; heteroaryl-(C₁-C₆)-amino, heteroaryl-(C₁-C₆)-alkyl-amino, heteroaryl-[(C₁-C₆)-alkyl]amino; Hetercyclyl-(C₁-C₆)-amino, heterocyclyl-(C₁-C₆)-alkyl-amino, heterocyclyl-[(C₁-C₆)-alkyl]amino; (C₃-C₆)-cycloalkyl-amino, (C₃-C₆)-cycloalkyl-[(C₁-C₆)-alkyl]amino, (C₁-C₆)-alkylcarbonylamino, arylcarbonylamino, hetarylcarbonylamino, N-azetidinyl, N-pyrrolidinyl, N-piperidinyl, N-morpholinyl,
- R¹⁴ and R¹⁵: independently of one another represent hydrogen, hydroxy, (C₁-C₆)-alkyl, (C₁-C₆)-haloalkyl, (C₃-C₆)-cycloalkyl, (C₂-C₆)-alkenyl, (C₁-C₆)-alkoxy, or
- R¹⁴ and R¹⁵: together with the carbon atom to which they are bonded form a fully saturated or partly saturated 3- to 10-membered monocyclic or bicyclic ring optionally interrupted by heteroatoms and optionally having further substitution, or
- R¹⁴ and R¹⁵: together with the carbon atom to which they are bonded form an ethylene group (C=CH₂) or a carbonyl group (C=O),
- R¹⁶: represents halogen, nitro, cyano, thiocyanato, (C₁-C₆)-alkyl, (C₁-C₆)-haloalkyl, cyano-(C₁-C₆)-alkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, OR¹², R¹²O-(C₁-C₆)-alkyl, R¹⁰R¹¹N-(C₁-C₆)-alkyl, NR¹⁰R¹¹, C(=O)R¹², C(=O)OR¹², C(=O)NR¹⁰R¹¹, R¹²O(O)C-(C₁-C₆)-alkyl, R¹²(O=)C-(C₁-C₆)-Alkyl, R¹⁰R¹¹N(O)C-(C₁-C₆)-alkyl, S(O)ₚR¹³, R¹³S(O)ₚ-(C₁-C₆)-alkyl, (C₃-C₆)-cycloalkyl-(C₁-C₆)-alkyl, aryl, heteroaryl, heterocyclyl, aryl-(C₁-C₆)-alkyl, heteroaryl-(C₁-C₆)-alkyl, (C₃-C₆)-heterocyclyl-(C₁-C₆)-alkyl,
- R¹⁷: represents hydrogen, halogen, nitro, cyano, thiocyanato, (C₁-C₆)-alkyl, (C₁-C₆)-haloalkyl, cyano-(C₁-C₆)-alkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, (C₄-C₆)-cycloalkenyl, (C₄-C₆)-halocycloalkenyl, OR¹², R¹²O-(C₁-C₆)-alkyl, R¹⁰R¹¹N-(C₁-C₆)-alkyl, NR¹⁰R¹¹, C(=O)R¹², C(=O)OR¹², C(=O)NR¹⁰R¹¹, R¹²O(O)C-(C₁-C₆)-alkyl, R¹²(O=)C-(C₁-C₆)-Alkyl, R¹⁰R¹¹N(O)C-(C₁-C₆)-alkyl, S(O)ₚR¹³, R¹³S(O)ₚ-(C₁-C₆)-alkyl, (C₃-C₆)-cycloalkyl-(C₁-C₆)-alkyl, (C₃-C₆)-halocycloalkyl-(C₁-C₆)-alkyl, (C₄-C₆)-cycloalkenyl-(C₁-C₆)-alkyl, (C₄-C₆)-halocycloalkenyl-(C₁-C₆)-alkyl, aryl, heterocyclyl, heteroaryl, aryl-(C₁-C₆)-alkyl, heteroaryl-(C₁-C₆)-alkyl, heterocyclyl-(C₁-C₆)-alkyl, tris[(C₁-C₆)alkyl]silyl, bis-[(C₁-C₆)-alkyl](aryl)silyl, (C₁-C₆)-alkyl-[bis-(aryl)]silyl, tris-[(C₁-C₆)-alkyl]silyl-(C₁-C₆)-alkyl, bis-[(C₁-C₆)-alkyl](aryl)silyl(C₁-C₆)-alkyl or (C₁-C₆)-alkyl-[bis-(aryl)]silyl-(C₁-C₆)-alkyl
and
- W: represents oxygen (O).

The invention particularly provides compounds of the general formula (I) in which
- Q: represents a group Q-1 to Q-2 as outlined below 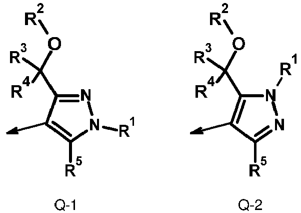
- A¹, A², A³ and A⁴: are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
- R¹⁷: in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- R¹⁷: in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- X: represents a direct bond or a group CR⁸R⁹, -CR⁸R⁹CH₂-, -CH₂CR⁸R⁹-,
- R¹: represents methyl, ethyl, prop-1-yl, 1-methylethyl, but-1-yl, 1-methylprop-1-yl, 2-methylprop-1-yl, 1,1-dimethylethyl, pent-1-yl, 1-methylbut-1-yl, 2-methylbut-1-yl, 3-methylbut-1-yl, 1,1-dimethylprop-1-yl, 1,2-dimethylprop-1-yl, 2,2-dimethylprop-1-yl, 1-ethylprop-1-yl, hex-1-yl, prop-2-en-1-yl, but-2-en-1-yl, but-3-en-1-yl, pent-2-en-1-yl, pent-3-en-1-yl, pent-4-en-1-yl, 1-methylprop-2-en-1-yl, 2-methylprop-2-en-1-yl, 1-methylbut-2-en-1-yl, 2-methylbut-2-en-1-yl, 3-methylbut-2-en-1-yl, 1-methylbut-3-en-1-yl, 2-methylbut-3-en-1-yl, 3-methylbut-3-en-1-yl, 1,1-dimethylprop-2-en-1-yl, 1,2-dimethylprop-2-en-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methyl-buty-2-n-1-yl, 1-methylbut-3-yn-1-yl, 2-methylbut-3-yn-1-yl, cyclobutyl, cyclopentyl, cyclo-hexyl, cyclopropylmethyl, cyclobutylmethyl, cyclopentylmethyl, cyclohexylmethyl, 2-cyclo-propyleth-1-yl, 1-cyclopropyleth-1-yl, cyanomethyl, 2-cyanoeth-1-yl, 1-cyanoeth-1-yl, 3-cyano-prop-1-yl, 2-cyano-prop-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methylbut-2-yn-1-yl, 1-methylbut-3-yn-1-yl, 1,1-dimethylprop-2-yn-1-yl, 1-ethylprop-2-yn-1-yl, hex-2-yn-1-yl, hex-3-yn-1-yl, hex-4-yn-1-yl, hex-5-yn-1-yl, 1,3-dioxolan-2-ylmethyl, 4,5-dimethyl-1,3-dioxolan-2-ylmethyl, 1,3-dioxan-2-ylmethyl, trifluoromethyl, pentafluoroethyl, 1,1,2,2-tetrafluoroethyl, heptafluoropropyl, nonafluorobutyl, 1-fluoroeth-1-yl, 2-fluoroeth-1-yl, difluoromethyl, 2,2-difluoroeth-1-yl, 2,2,2-trifluoroeth-1-yl, 3,3,3-trifluoroprop-1-yl, methoxymethyl, ethoxy-methyl, 2-ethoxyeth-1-yl, 1-ethoxyeth-1-yl, 2-methoxyeth-1-yl, 1-methoxyeth-1-yl, 3-methoxyprop-1-yl, 3-ethoxyprop-1-yl, 4-methoxybut-1-yl, 3-methoxyprop-2-yl, 2,2-dimethoxyeth-1-yl, 3,3-dimethoxyprop-1-yl, trifluoromethoxymethyl, benzyl, 4-fluorobenzyl, 3-fluorobenzyl, 2-fluorobenzyl, 4-chlorobenzyl, 3-chlorobenzyl, 2-chlorobenzyl, phenyl, 4-fluorophenyl, 3-fluorophenyl, 2-fluorophenyl, 4-chlorophenyl, 3-chlorophenyl, 2-chloro-phenyl, methylsulfanylmethyl, ethylsulfanylmethyl, 2-(methylsulfanyl)eth-1-yl, 2-(ethyl-sulfanyl)eth-1-yl, trifluoromethylsulfanylmethyl, pyridin-2-yl, pyridin-3-yl, pyridin-4-yl, pyridin-2-ylmethyl, pyridin-3-ylmethyl, pyridin-4-ylmethyl, 2-(2-methoxyethoxy)eth-1-yl, 2-(2-ethoxyethoxy)eth-1-yl, methylaminocarbonylmethyl, aminocarbonylmethyl, ethylamino-carbonylmethyl, dimethylaminocarbonylmethyl, diethylaminocarbonylmethyl, methyl(ethyl)-aminocarbonylmethyl, hydroxycarbonylmethyl, methoxycarbonylmethyl, ethoxy-carbonylmethyl, 1-methyleth-1-yloxycarbonylmethyl, 1,1-dimethyleth-1-yloxycarbonyl-methyl, 2-hydroxycarbonyleth-1-yl, 2-methoxycarbonyleth-1-yl, 2-ethoxycarbonyleth-1-yl, 2-(1-methyleth-1-yloxycarbonyl)eth-1-yl, 2-(1,1-dimethyleth-1-yloxycarbonyl)eth-1-yl, 2-(benzyloxycarbonyl)eth-1-yl, 1-hydroxycarbonyleth-1-yl, 1-methoxycarbonyleth-1-yl, 1-ethoxycarbonyleth-1-yl, 1-(1-methyleth-1-yloxycarbonyl)eth-1-yl, 1-(1,1-dimethyleth-1-yloxycarbonyl)eth-1-yl, 1-(benzyloxycarbonyl)eth-1-yl, 1-hydroxycarbonyl-1-methyleth-1-yl, 1-methoxycarbonyl-1-methyleth-1-yl, 1-ethoxycarbonyl-1-methyleth-1-yl, 1-(1-methyleth-1-yloxycarbonyl)-1-methyleth-1-yl, 1-(1,1-dimethyleth-1-yloxycarbonyl) 1-methyleth-1-yl, 1-(benzyloxycarbonyl)1-methyleth-1-yl, methylaminocarbonyl, dimethylaminocarbonyl, diethylaminocarbonyl, methyl(ethyl)aminocarbonyl, 1-methyleth-1-ylaminocarbonyl, cyclopropylaminocarbonyl, cyclobutylaminocarbonyl, cyclopentylaminocarbonyl, cyclo-hexylaminocarbonyl, phenylaminocarbonyl, benzylaminocarbonyl, methoxycarbonyl, ethoxycarbonyl, 1-methyleth-1-yloxycarbonyl, 1,1-dimethyleth-1-yloxycarbonyl, benzyl-oxycarbonyl, 2-(methylamino)eth-1-yl, 2-(dimethylamino)eth-1-yl, 2-(ethylamino)eth-1-yl, 2-(diethylamino)eth-1-yl, 2-(methylsulfonylamino)eth-1-yl, 2-(ethylsulfonylamino)eth-1-yl, 2-(benzylsulfonylamino)eth-1-yl, 2-(phenylsulfonylamino)eth-1-yl, 2-(methylcarbonyl-aminosulfonylamino)eth-1-yl, 2-(ethylcarbonylaminosulfonylamino)eth-1-yl, methyl-sulfonylmethyl, 2-(methylsulfonyl)eth-1-yl, ethylsulfonylmethyl, 2-(ethylsulfonyl)eth-1-yl, (2-trismethylsilyleth-1-yloxy)methyl, (2-trismethylsilyleth-1-yloxy)eth-1-yl, methyl-carbonylaminosulfonylmethyl, ethylcarbonylaminosulfonylmethyl, 2-(methylcarbonyl-aminosulfonyl)eth-1-yl, tetrahydrofuran-2-ylmethyl, tetrahydrofuran-3-ylmethyl, oxetan-3-ylmethyl, tetrahydropyran-2-ylmethyl, tetrahydropyran-3-ylmethyl, tetrahydropyran-4-yl-methyl, oxetan-3-yl, tetrahydrofuran-3-yl,
- R²: represents methyl, ethyl, prop-1-yl, 1-methylethyl, but-1-yl, 1-methylprop-1-yl, 2-methylprop-1-yl, 1,1-dimethylethyl, pent-1-yl, 1-methylbut-1-yl, 2-methylbut-1-yl, 3-methylbut-1-yl, 1,1-dimethylprop-1-yl, 1,2-dimethylprop-1-yl, 2,2-dimethylprop-1-yl, 1-ethylprop-1-yl, hex-1-yl, prop-2-en-1-yl, but-2-en-1-yl, but-3-en-1-yl, pent-2-en-1-yl, pent-3-en-1-yl, pent-4-en-1-yl, 1-methylprop-2-en-1-yl, 2-methylprop-2-en-1-yl, 1-methylbut-2-en-1-yl, 2-methylbut-2-en-1-yl, 3-methylbut-2-en-1-yl, 1-methylbut-3-en-1-yl, 2-methylbut-3-en-1-yl, 3-methylbut-3-en-1-yl, 1,1-dimethylprop-2-en-1-yl, 1,2-dimethylprop-2-en-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methyl-buty-2-n-1-yl, 1-methylbut-3-yn-1-yl, 2-methylbut-3-yn-1-yl, cyclobutyl, cyclopentyl, cyclo-hexyl, cyclopropylmethyl, cyclobutylmethyl, cyclopentylmethyl, cyclohexylmethyl, 3,3-difluorocyclobutyl, 3,3-difluorocyclobutylmethyl, (1-fluorocycloprop-1-yl)methyl), (1-chloro-cycloprop-1-yl)methyl), (1-cyanocycloprop-1-yl)methyl), (1-methylcycloprop-1-yl)methyl), 2-cyclopropyleth-1-yl, 1-cyclopropyleth-1-yl, cyanomethyl, 2-cyanoeth-1-yl, 1-cyanoeth-1-yl, 3-cyano-prop-1-yl, 2-cyano-prop-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methylbut-2-yn-1-yl, 1-methylbut-3-yn-1-yl, 1,1-dimethylprop-2-yn-1-yl, 1-ethylprop-2-yn-1-yl, hex-2-yn-1-yl, hex-3-yn-1-yl, hex-4-yn-1-yl, hex-5-yn-1-yl, benzyl, 4-fluorobenzyl, 3-fluorobenzyl, 2-fluoro-benzyl, 4-chlorobenzyl, 3-chlorobenzyl, 2-chlorobenzyl, phenyl, 4-fluorophenyl, 3-fluoro-phenyl, 2-fluorophenyl, 4-chlorophenyl, 3-chlorophenyl, 2-chlorophenyl, 4-methylphenyl, trifluoromethyl, pentafluoroethyl, 1,1,2,2-tetrafluoroethyl, heptafluoropropyl, nonafluoro-butyl, 1-fluoroeth-1-yl, 2-fluoroeth-1-yl, difluoromethyl, 2,2-difluoroeth-1-yl, 2,2,2-tri-fluoroeth-1-yl, 3,3,3-trifluoroprop-1-yl, 2,2-difluoroprop-1-yl, 3,3,3,2,2-pentafluoroprop-1-yl, 3,3,2,2-tetrafluoroprop-1-yl, 2-chloro-2,2-difluoroeth-1-yl, 2,2,2-trifluoro-1-methyleth-1-yl, 2-ethoxyeth-1-yl, 1-ethoxyeth-1-yl, 2-methoxyeth-1-yl, 1-methoxyeth-1-yl, 3-methoxyprop-1-yl, 3-ethoxyprop-1-yl, 4-methoxybut-1-yl, 1-(methoxymethyl)eth-1-yl, 2-(2-methoxy-ethoxy)eth-1-yl, 2-(2-ethoxyethoxy)eth-1-yl, 2-(2-methoxyethoxyethoxy)eth-1-yl, 2-(2-ethoxyethoxyethoxy)eth-1-yl, 2-(2-methoxyethoxyethoxyethoxy)eth-1-yl, 2-(2-ethoxyethoxy-ethoxyethoxy)eth-1-yl, tetrahydrofuran-2-ylmethyl, tetrahydrofuran-3-ylmethyl, oxetan-3-ylmethyl, tetrahydropyran-2-ylmethyl, tetrahydropyran-3-ylmethyl, tetrahydropyran-4-yl-methyl, oxetan-3-yl, tetrahydrofuran-3-yl, dimethylimino, diethylamino,
- R³ and R⁴: independently of one another represent hydrogen, methyl, ethyl, prop-1-yl, 1-methyleth-1-yl, 1,1-dimethyleth-1-yl, or R³ and R⁴ together with the carbon atom to which they are bonded form a carbonyl group (C=O),
- R⁵: represents hydrogen, fluorine, chlorine, bromine, iodine, methyl, ethyl, 1-methyleth-1-yl, 1,1-dimethyleth-1-yl, prop-1-yl, difluoromethyl, trifluoromethyl, cyclopropyl, cyclobutyl, cyclopentyl, cyclohexyl,
- R⁶ and R⁷: independently of one another represent hydrogen, methyl, ethyl, 1-methyleth-1-yl, 1,1-dimethyleth-1-yl, prop-1-yl, difluoromethyl, trifluoromethyl, cyclopropyl, cyclobutyl, cyclopentyl, cyclohexyl,
- R⁸ and R⁹: independently of one another represent hydrogen, fluoro, chloro, bromo, methyl, ethyl, prop-1-yl, 1-methyleth-1-yl, 1,1-dimethyleth-1-yl, trifluoromethyl, cyclopropyl, cyclobutyl, cyclopentyl, methoxymethyl, methoxy, hydroxy, dimethylamino, methylsulfanyl,
- R² and R³: together with the atoms to which they are bonded form a fully saturated or partly saturated 4-to 10-membered monocyclic or bicyclic ring optionally interrupted by heteroatoms and optionally having further substitution,
- R¹ and R⁵: together with the atoms to which they are bonded form a fully saturated or partly saturated 4-to 10-membered monocyclic or bicyclic ring optionally interrupted by heteroatoms and optionally having further substitution when Q represents a group Q-1,
- R¹ and R²: together with the atoms to which they are bonded form a fully saturated or partly saturated 4-to 10-membered monocyclic or bicyclic ring optionally interrupted by heteroatoms and optionally having further substitution when Q represents a group Q-2,
- R¹⁴ and R¹⁵: independently of one another represent hydrogen, hydroxy, methyl, ethyl, prop-1-yl, 1-methyl-eth-1-yl, trifluoromethyl, difluoromethyl, 2,2-difluoroeth-1-yl, cyclopropyl, cyclobutyl, cyclopentyl, cyclohexyl, prop-2-en-1-yl, methoxy
- R¹⁴ and R¹⁵: together with the carbon atom to which they are bonded form a fully saturated or partly saturated 3- to 10-membered monocyclic or bicyclic ring optionally interrupted by heteroatoms and optionally having further substitution,
- R¹⁴ and R¹⁵: together with the carbon atom to which they are bonded form an ethylene group (C=CH₂) or a carbonyl group (C=O),
- R¹⁶: represents fluoro, chloro, bromo, iodo, nitro, cyano, thiocyanato, methyl, ethyl, 1-methyleth-1-yl, 1,1-dimethyleth-1-yl, prop-1-yl, cyclopropyl, cyclobutyl, cyclopentyl, cyclohexyl, ethenyl, ethynyl, difluoromethyl, trifluoromethyl, chlorodifluoromethyl, 2,2-difluoroeth-1-yl, 3,3,3-trifluoroeth-1-yl, methoxy, ethoxy, 1-methyleth-1-yloxy, methylsulfanyl, ethylsulfanyl, trifluoromethoxy, difluoromethoxy, trifluoromethylsulfanyl, methylsulfonyl,
- R¹⁷: represents hydrogen, fluoro, chloro, bromo, iodo, nitro, cyano, methyl, ethyl, difluoromethyl, trifluoromethyl, methoxy, trifluoromethoxy, difluoromethoxy
and
- W: represents oxygen (O).

The invention more particularly provides compounds of the general formula (I) in which
Q represents a group Q-1.1 to Q-2.35 as outlined below

| | | | | |
|---|---|---|---|---|
| 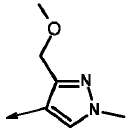 | 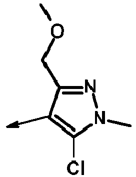 | 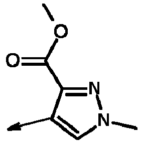 | 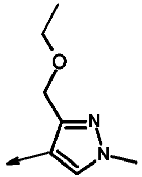 | 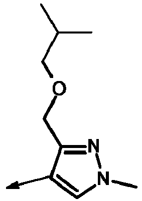 |
| Q-1.1 | Q-1.2 | Q-1.3 | Q-1.4 | Q-1.5 |

| | | | | |
|---|---|---|---|---|
| 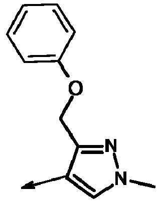 | 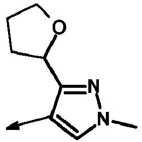 | 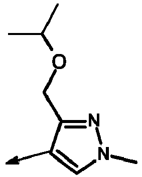 | 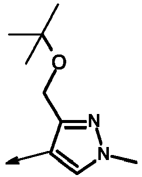 | 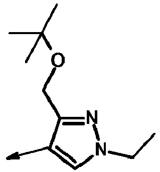 |
| Q-1.6 | Q-1.7 | Q-1.8 | Q-1.9 | Q-1.10 |

| | | | | |
|---|---|---|---|---|
| 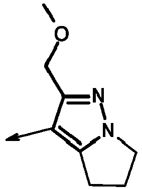 | 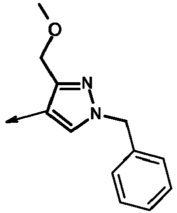 | 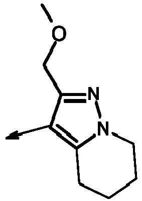 | 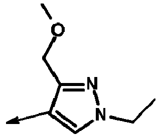 | 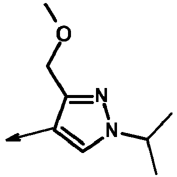 |
| Q-1.11 | Q-1.12 | Q-1.13 | Q-1.14 | Q-1.15 |

| | | | | |
|---|---|---|---|---|
| 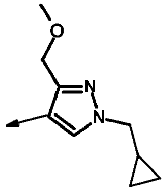 | 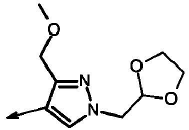 | 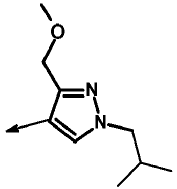 | 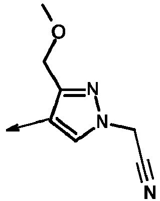 | 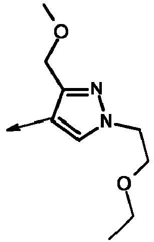 |
| Q-1.16 | Q-1.17 | Q-1.18 | Q-1.19 | Q-1.20 |

| | | | | |
|---|---|---|---|---|
| 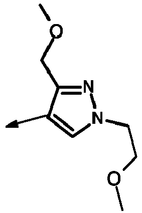 | 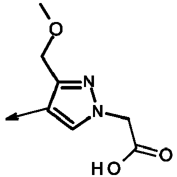 | 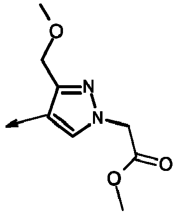 | 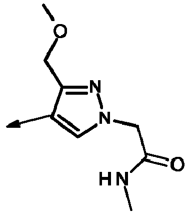 | 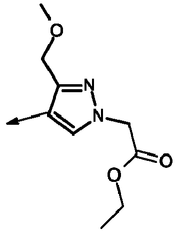 |
| Q-1.21 | Q-1.22 | Q-1.23 | Q-1.24 | Q-1.25 |

| | | | | |
|---|---|---|---|---|
| 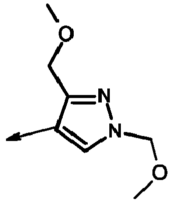 | 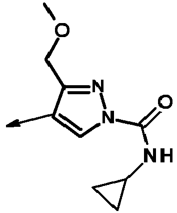 | 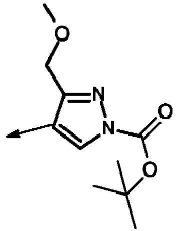 | 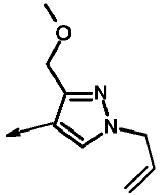 | 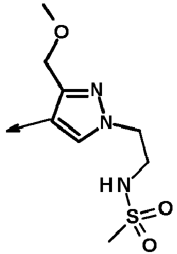 |
| Q-1.26 | Q-1.27 | Q-1.28 | Q-1.29 | Q-1.30 |

| | | | | |
|---|---|---|---|---|
| 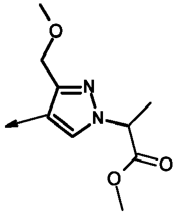 | 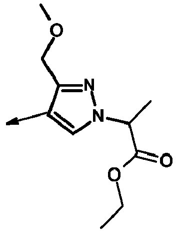 | 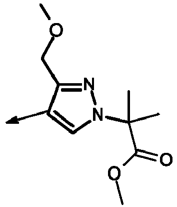 | 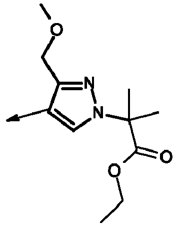 | 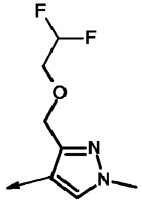 |
| Q-1.31 | Q-1.32 | Q-1.33 | Q-1.34 | Q-1.35 |

| | | | | |
|---|---|---|---|---|
| 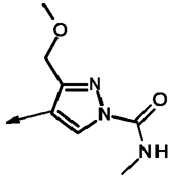 | 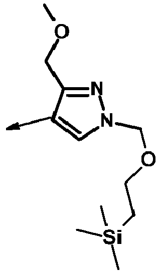 | 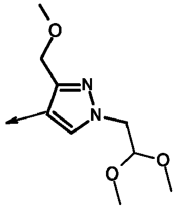 | 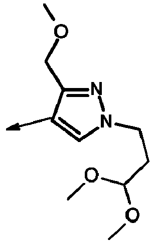 | 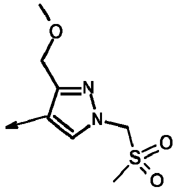 |
| Q-1.36 | Q-1.37 | Q-1.38 | Q-1.39 | Q-1.40 |

| | | | | |
|---|---|---|---|---|
| 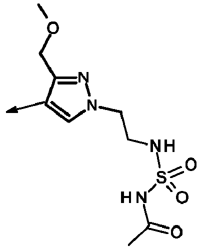 | 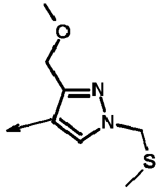 | 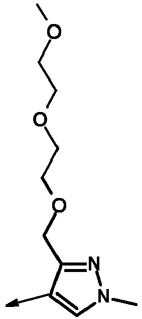 | 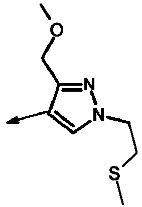 | 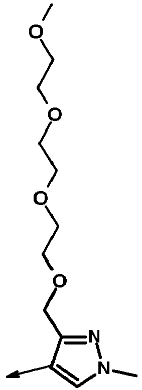 |
| Q-1.41 | Q-1.42 | Q-1.43 | Q-1.44 | Q-1.45 |

| | | | | |
|---|---|---|---|---|
| 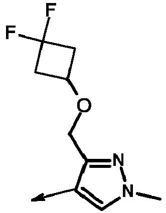 | 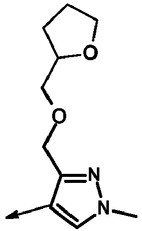 | 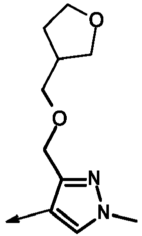 | 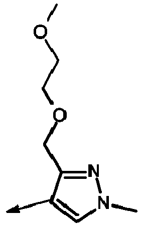 | 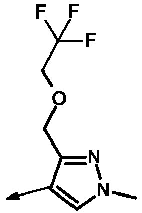 |
| Q-1.46 | Q-1.47 | Q-1.48 | Q-1.49 | Q-1.50 |

| | | | | |
|---|---|---|---|---|
| 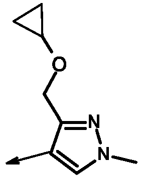 | 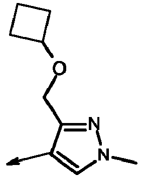 | 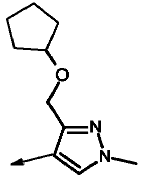 | 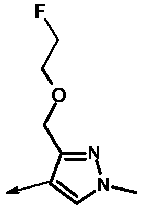 | 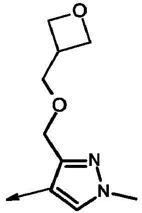 |
| Q-1.51 | Q-1.52 | Q-1.53 | Q-1.54 | Q-1.55 |

| | | | | |
|---|---|---|---|---|
| 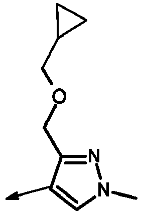 | 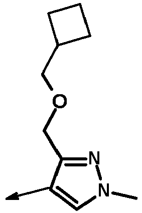 | 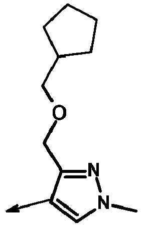 | 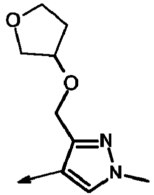 | 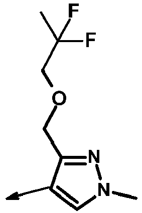 |
| Q-1.56 | Q-1.57 | Q-1.58 | Q-1.59 | Q-1.60 |

| | | | | |
|---|---|---|---|---|
| 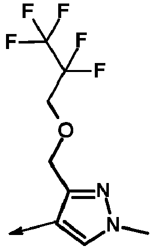 | 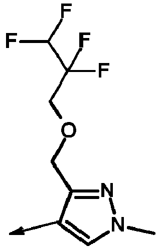 | 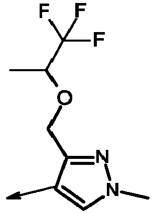 | 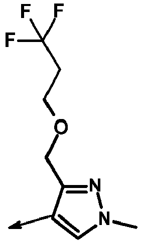 | 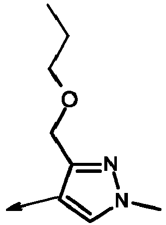 |
| Q-1.61 | Q-1.62 | Q-1.63 | Q-1.64 | Q-1.65 |

| | | | | |
|---|---|---|---|---|
| 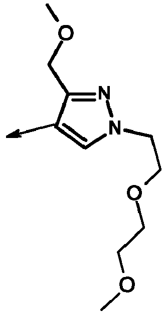 | 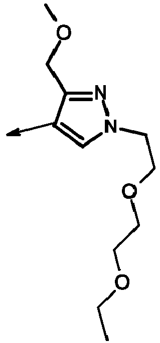 | 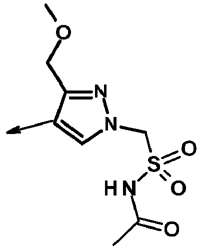 | 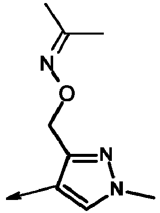 | 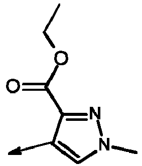 |
| Q-1.66 | Q-1.67 | Q-1.68 | Q-1.69 | Q-1.70 |

| | | | |
|---|---|---|---|
| 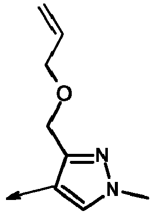 | 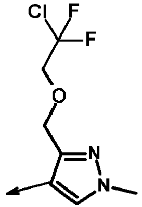 | 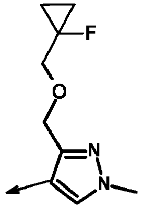 | 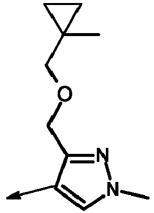 |
| Q-1.71 | Q-1.72 | Q-1.73 | Q-1.74 |

| | | | | |
|---|---|---|---|---|
| 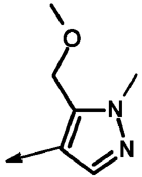 | 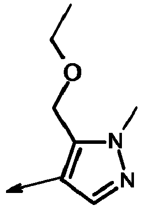 | 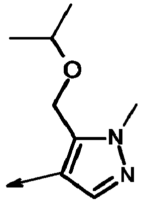 | 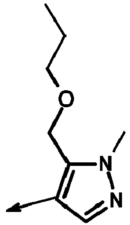 | 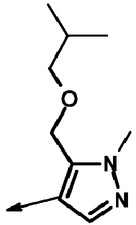 |
| Q-2.1 | Q-2.2 | Q-2.3 | Q-2.4 | Q-2.5 |

| | | | | |
|---|---|---|---|---|
| 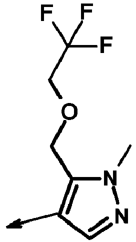 | 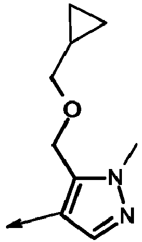 | 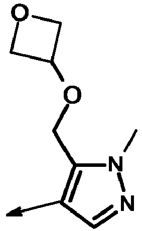 | 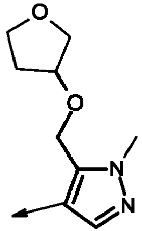 | 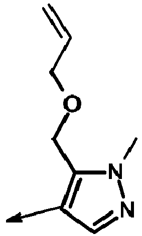 |
| Q-2.6 | Q-2.7 | Q-2.8 | Q-2.9 | Q-2.10 |

| | | | | |
|---|---|---|---|---|
| 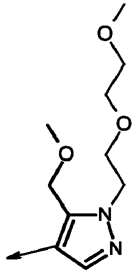 | 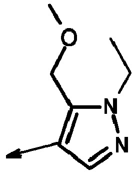 | 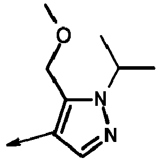 | 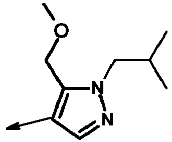 | 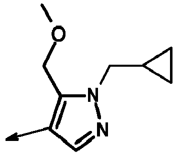 |
| Q-2.11 | Q-2.12 | Q-2.13 | Q-2.14 | Q-2.15 |

| | | | | |
|---|---|---|---|---|
| 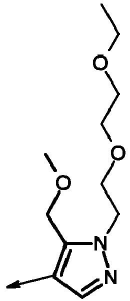 | 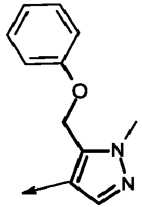 | 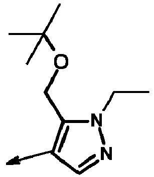 | 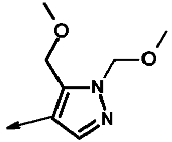 | 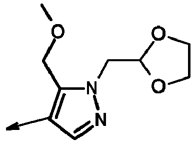 |
| Q-2.16 | Q-2.17 | Q-2.18 | Q-2.19 | Q-2.20 |

| | | | | |
|---|---|---|---|---|
| 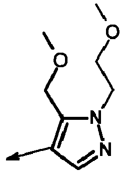 | 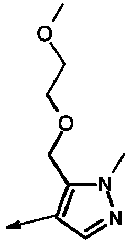 | 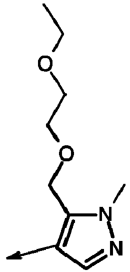 | 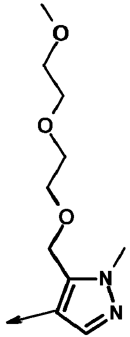 | 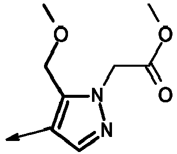 |
| Q-2.21 | Q-2.22 | Q-2.23 | Q-2.24 | Q-2.25 |

| | | | | |
|---|---|---|---|---|
| 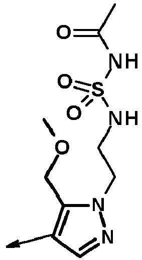 | 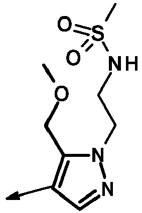 | 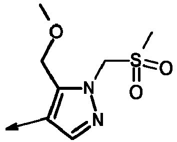 | 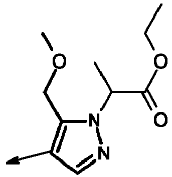 | 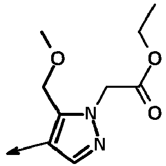 |
| Q-2.26 | Q-2.27 | Q-2.28 | Q-2.29 | Q-2.30 |

| | | | | |
|---|---|---|---|---|
| 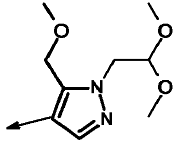 | 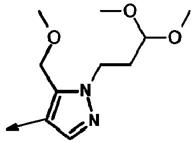 | 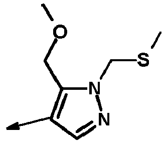 | 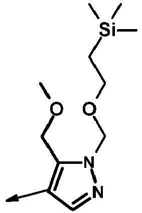 | 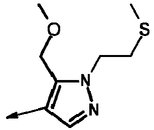 |
| Q-2.31 | Q-2.32 | Q-2.33 | Q-2.34 | Q-2.35 |

| |
|---|
| 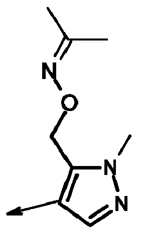 |
| Q-2.36 |

- A¹, A², A³ and A⁴: are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
- R¹⁷: in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- R¹⁷: in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
- X: represents a direct bond or a group CR⁸R⁹, -CR⁸R⁹CH₂-, -CH₂CR⁸R⁹-,
- R⁶ and R⁷: independently of one another represent hydrogen, methyl,
- R⁸ and R⁹: independently of one another represent hydrogen, fluoro, methyl,
- R¹⁴ and R¹⁵: independently of one another represent hydrogen, hydroxy, methyl, ethyl, difluoromethyl, 2,2-difluoroeth-1-yl, or
- R¹⁴ and R¹⁵: together with the carbon atom to which they are bonded form a fully saturated or partly saturated 3- to 10-membered monocyclic or bicyclic ring optionally interrupted by heteroatoms and optionally having further substitution, or
- R¹⁴ and R¹⁵: together with the carbon atom to which they are bonded form an ethylene group (C=CH₂) or a carbonyl group (C=O),
- R¹⁶: represents fluoro, chloro, bromo, iodo, methyl, methoxy,
- R¹⁷: represents hydrogen, fluoro, chloro, bromo, methyl
and
- W: represents oxygen (O).

The invention most particularly provides compounds of the general formula (I) in which
- Q: represents a group Q-1.1, Q-1.2, Q-1.3, Q-1.4, Q-1.5, Q-1.6, Q-1.7, Q-1.8, Q-1.9, Q-1.10, Q-1.11, Q-1.12, Q-1.13, Q-1.14, Q-1.15, Q-1.16, Q-1.17, Q-1.19, Q-1.21, Q-1.26, Q-1.27, Q-1.28, Q-1.29, Q-1.30, Q-1.36, Q-1.37, Q-1.38, Q-1.39, Q-1.42, Q-1.50, Q-1.52, Q-1.56, Q-1.57, Q-1.59, Q-1.60, Q-1.61, Q-1.62, Q-1.63, Q-1.64, Q-1.65, Q-1.66, Q-1.72, Q-1.73, Q-2.1, Q-2.2, Q-2.3, Q-2.4, Q-2.5, Q-2.6, Q-2.7, Q-2.8, Q-2.9, Q-2.10,Q-2.12, Q-2.13, Q-2.14, Q-2.15, Q-2.21, Q-2.28, Q-2.31, Q-2.32, Q-2.34, Q-2.35, Q-2.36 as outlined above,
- A¹, A², A³ and A⁴: are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
- X: represents a direct bond or a group CR⁸R⁹,
- R⁶ and R⁷: independently of one another represent hydrogen, methyl,
- R⁸ and R⁹: represent hydrogen,
- R¹⁴ and R¹⁵: independently of one another represent hydrogen, hydroxy, methyl, ethyl, difluoromethyl, 2,2-difluoroeth-1-yl,
- R¹⁴ and R¹⁵: together with the carbon atom to which they are bonded form a fully saturated 3-membered monocyclic ring optionally having further substitution, or
- R¹⁴ and R¹⁵: together with the carbon atom to which they are bonded form an ethylene group (C=CH₂) or a carbonyl group (C=O),
- R¹⁶: represents fluoro, chloro, bromo, methyl, methoxy,
- R¹⁷: represents hydrogen, fluoro, chloro
and
- W: represents oxygen (O).

The definitions of radicals listed above in general terms or within areas of preference apply both to the end products of the general formula (I) and correspondingly to the starting materials or intermediates required for preparation in each case. These radical definitions can be combined with one another as desired, i.e. including combinations between the given preferred ranges. Primarily for reasons of higher herbicidal activity, better selectivity and/or better producibility, compounds of the abovementioned general formula (I) according to the invention or their salts or their use according to the invention are of particular interest in which individual radicals have one of the preferred meanings already specified or specified below, or in particular those in which one or more of the preferred meanings already specified or specified below occur in combination. With regard to the compounds according to the invention, the terms used above and further below will be elucidated. These are familiar to the person skilled in the art and especially have the definitions elucidated hereinafter.

Unless defined differently, names of chemical groups are generally to be understood such that attachment to the skeleton or the remainder of the molecule is via the structural element mentioned last, i.e. for example in the case of (C₂-C₈)-alkenyloxy via the oxygen atom and in the case of (C₁-C₈)-alkoxy-(C₁-C₄)-alkyl or (C₁-C₈)-alkoxycarbonyl-(C₁-C₈)-alkyl, in each case via the carbon atom of the alkyl group. In the case of specified groups, such as OR¹⁰, NR⁸R⁹, S(O)ₚR¹¹, C(=O)R¹⁰, C(=O)OR¹⁰, or C(=O)NR⁸R⁹ the attachment to the skeleton is via the structural element mentioned first, i.e. for example in the case of OR¹⁰ via the oxgyen atom and in the case of C(=O)OR¹⁰ via the carbonyl atom.

According to the invention, "alkylsulfonyl" - alone or as part of a chemical group - refers to straight-chain or branched alkylsulfonyl, preferably having 1 to 8 or 1 to 6 carbon atoms, for example (but not limited to) (C₁-C₆)-alkylsulfonyl such as methylsulfonyl, ethylsulfonyl, propylsulfonyl, 1-methylethyl-sulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl and 1-ethyl-2-methylpropylsulfonyl.

According to the invention, "alkylthio" or "alkylsulfanyl" - alone or as part of a chemical group - denotes straight-chain or branched S-alkyl, preferably having 1 to 8 or 1 to 6 carbon atoms, such as (Ci-C₁₀)-, (C₁-C₆)- or (C₁-C₄)-alkylthio, for example (but not limited to) (C₁-C₆)-alkylthio or (C₁-C₆)-alkylsulfanyl such as methylthio, ethylthio, propylthio, 1-methylethylthio, butylthio, 1-methylpropylthio, 2-methylpropylthio, 1,1-dimethylethylthio, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethyl-propylthio, hexylthio, 1-methylpentylthio, 2-methylpentylthio, 3-methylpentylthio, 4-methylpentyl-thio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and 1-ethyl-2-methylpropylthio or the corresponding names with -sulfanyl instead of -thio.

According to the invention, "alkylsulfinyl (alkyl-S(=O)-)", unless defined differently elsewhere, denotes alkyl radicals which are attached to the skeleton via -S(=O)-, such as (C₁-C₁₀)-, (C₁-C₆)- or (C₁-C₄)-alkylsulfinyl, for example (but not limited to) (C₁-C₆)-alkylsulfinyl such as methylsulfinyl, ethylsulfinyl, propylsulfinyl, 1-methylethylsulfinyl, butylsulfinyl, 1-methylpropylsulfinyl, 2-methyl-propylsulfinyl, 1,1-dimethylethylsulfinyl, pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutyl-sulfinyl, 1-ethylbutylsulfinyl, 2-ethylbutylsulfinyl, 1,1,2-trimethylpropylsulfinyl, 1,2,2-trimethyl-propylsulfinyl, 1-ethyl-1-methylpropylsulfinyl and 1-ethyl-2-methylpropylsulfinyl.

According to the invention, "Alkoxy" denotes an alkyl radical bonded via an oxygen atom, for example (but not limited to) (C₁-C₆)-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methyl-butoxy, 3-methylbutoxy, 1,1-dimethylpropoxy, 1,2-dimethylpropoxy, 2,2-dimethylpropoxy, 1-ethyl-propoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy.

According to the invention, "Haloalkoxy" is, for example, OCF₃, OCHF₂, OCH₂F, OCF₂CF₃, OCH₂CF₃ and OCH₂CH₂Cl; this applies correspondingly to haloalkenyloxy, haloalkynyloxy and other halogen-substituted radicals. The term "C₁-C₆-haloalkoxy" as mentioned herein by way of example thus refers to a C₁-C₆-alkoxy group as defined above in which one or more hydrogen atoms are replaced with halogen atoms that may be the same or different. Examples of C₁-C₆-haloalkoxy include but are not limited to chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy and 1,1,1-trifluoroprop-2-oxy.

Likewise, "Alkenyloxy" denotes an alkenyl radical attached via an oxygen atom, and alkynyloxy denotes an alkynyl radical attached via an oxygen atom, such as (C₂-C₁₀)-, (C₂-C₆)- or (C₂-C₄)-alkenoxy and (C₃-C₁₀)-, (C₃-C₆)- or (C₃-C₄)-alkynyloxy.

The term "C₂-C₆-alkenyloxy" mentioned here by way of example refers to a formula (C₂-C₆-alkenyl)-O-, in which the term "C₂-C₆-alkenyl" group is which the as defined herein. Examples of C₂-C₆-alkenyloxy include but are not limited to ethenyloxy (or "vinyloxy"), prop-2-en-1-yloxy (or "allyloxy"), prop-1-en-1-yloxy, prop-1-en-2-yloxy (or "isopropenyloxy"), but-3-enyloxy, but-2-enyloxy, but-1-enyloxy, 2-methylprop-2-enyloxy, 1-methylprop-2-enyloxy, 2-methylprop-1-enyloxy and 1-methylprop-1-enyloxy.

According to the invention, "Cycloalkoxy" denotes a cycloalkyl radical attached via an oxygen atom and cycloalkenyloxy denotes a cycloalkenyl radical attached via an oxygen atom.

According to the invention, "alkylcarbonyl" (alkyl-C(=O)-), unless defined differently elsewhere, represents alkyl radicals attached to the skeleton via -C(=O)-, such as (C₁-C₁₀)-, (C₁-C₆)- or (C₁-C₄)-alkylcarbonyl. Here, the number of the carbon atoms refers to the alkyl radical in the alkylcarbonyl group.

Analogously, "alkenylcarbonyl" and "alkynylcarbonyl", unless defined differently elsewhere, in accordance with the invention, respectively represent alkenyl and alkynyl radicals attached to the skeleton via -C(=O)-, such as (C₂-C₁₀)-, (C₂-C₆)- or (C₂-C₄)-alkenylcarbonyl and (C₂-C₁₀)-, (C₂-C₆)- or (C₂-C₄)-alkynylcarbonyl. Here, the number of the carbon atoms refers to the alkenyl or alkynyl radical in the alkenyl or alkynyl group.

"Alkoxycarbonyl (alkyl-O-C(=O)-)," unless defined differently elsewhere: alkyl radicals attached to the skeleton via -O-C(=O)-, such as (C₁-C₁₀)-, (C₁-C₆)- or (C₁-C₄)-alkoxycarbonyl. Here, the number of the carbon atoms refers to the alkyl radical in the alkoxycarbonyl group.

Analogously, "alkenyloxycarbonyl" and "alkynyloxycarbonyl", unless defined differently elsewhere, in accordance with the invention, respectively represent alkenyl and alkynyl radicals attached to the skeleton via -O-C(=O)-, such as (C₂-C₁₀)-, (C₂-C₆)- or (C₂-C₄)-alkenyloxycarbonyl and (C₃-C₁₀)-, (C₃-C₆)- or (C₃-C₄)-alkynyloxycarbonyl. Here, the number of the carbon atoms refers to the alkenyl or alkynyl radical in the alkenyloxycarbonyl or alkynyloxycarbonyl group.

The term "aryl" denotes an optionally substituted mono-, bi- or polycyclic aromatic system having preferably 6 to 14, especially 6 to 10, ring carbon atoms, for example phenyl, naphthyl, anthryl, phenanthrenyl and the like, preferably phenyl.

The term "optionally substituted aryl" also embraces polycyclic systems, such as tetrahydronaphthyl, indenyl, indanyl, fluorenyl, biphenylyl, where the bonding site is on the aromatic system. In systematic terms, "aryl" is generally also encompassed by the term "optionally substituted phenyl". Preferred aryl substituents here are, for example, hydrogen, halogen, alkyl, cycloalkyl, cycloalkyl-alkyl, cycloalkenyl, halocycloalkyl, alkenyl, alkynyl, aryl, arylalkyl, arylalkenyl, heteroaryl, heteroarylalkyl, heterocyclyl, heterocyclylalkyl, alkoxyalkyl, alkylthio, haloalkylthio, haloalkyl, alkoxy, haloalkoxy, cycloalkoxy, cycloalkylalkoxy, aryloxy, heteroraryloxy, alkoxyalkoxy, alkynylalkoxy, alkenyloxy, dialkylamino-alkoxy, tris-[alkyl]silyl, di-[alkyl]arylsilyl, di-[alkyl]alkylsilyl, tris-[alkyl]silylalkynyl, arylalkynyl, heteroarylalkynyl, alkylalkynyl, cycloalkyl-alkynyl, haloalkylalkynyl, heterocyclyl-N-alkoxy, nitro, cyano, amino, alkylamino, dialkylamino, alkylcarbonylamino, cycloalkylcarbonylamino, arylcarbonylamino, alkoxycarbonylamino, alkoxy-carbonylalkylamino, arylalkoxycarbonylalkylamino, hydroxycarbonyl, alkoxycarbonyl, amino-carbonyl, alkylaminocarbonyl, cycloalkylaminocarbonyl, dialkylaminocarbonyl, heteroarylalkoxy, arylalkoxy.

A heterocyclic radical (heterocyclyl) contains at least one heterocyclic ring (=carbocyclic ring in which at least one carbon atom has been replaced by a heteroatom, preferably by a heteroatom from the group of N, O, S, P) which is saturated, unsaturated, partially saturated or heteroaromatic and may be unsubstituted or substituted, in which case the bonding site is localized on a ring atom. If the heterocyclyl radical or the heterocyclic ring is optionally substituted, it may be fused to other carbocyclic or heterocyclic rings. In the case of optionally substituted heterocyclyl, polycyclic systems are also included, for example 8-azabicyclo[3.2.1]octanyl, 8-azabicyclo[2.2.2]octanyl or 1-aza-bicyclo[2.2.1]heptyl. Optionally substituted heterocyclyl also includes spirocyclic systems, such as, for example, 1-oxa-5-aza-spiro[2.3]hexyl. Unless defined otherwise, the heterocyclic ring preferably contains 3 to 9 ring atoms, in particular 3 to 6 ring atoms, and one or more, preferably 1 to 4, in particular 1, 2 or 3 heteroatoms in the heterocyclic ring, preferably from the group N, O and S, where, however, two oxygen atoms must not be directly adjacent to one another, for example having one heteroatom from the group consisting of N, O and S 1- or 2- or 3-pyrrolidinyl, 3,4-dihydro-2H-pyrrol-2- or -3-yl, 2,3-dihydro-1H-pyrrol-1- or -2- or -3- or -4- or -5-yl; 2,5-dihydro-1H-pyrrol-1- or -2- or -3-yl, 1- or 2- or 3- or 4-piperidinyl; 2,3,4,5-tetrahydropyridin-2- or -3- or -4- or -5-yl or -6-yl; 1,2,3,6-tetrahydropyridin-1- or -2- or -3- or -4- or -5- or -6-yl; 1,2,3,4-tetrahydropyridin-1- or -2- or -3- or -4- or -5- or -6-yl; 1,4-dihydropyridin-1- or -2- or -3- or -4-yl; 2,3-dihydropyridin-2- or -3- or -4- or -5- or -6-yl; 2,5-dihydropyridin-2- or -3- or -4- or -5- or -6-yl, 1- or 2- or 3- or 4-azepanyl; 2,3,4,5-tetra-hydro-1H-azepin-1- or -2- or -3- or -4- or -5- or -6- or -7-yl; 2,3,4,7-tetrahydro-1H-azepin-1- or -2- or -3- or -4- or -5- or -6- or -7-yl; 2,3,6,7-tetrahydro-1H-azepin-1- or -2- or -3- or -4-yl; 3,4,5,6-tetra-hydro-2H-azepin-2- or -3- or -4- or -5- or -6- or -7-yl; 4,5-dihydro-1H-azepin-1- or -2- or -3- or -4-yl; 2,5-dihydro-1H-azepin-1- or -2- or -3- or -4- or -5- or -6- or -7-yl; 2,7-dihydro-1H-azepin-1- or -2- or -3- or -4-yl; 2,3-dihydro-1H-azepin-1- or -2- or -3- or -4- or -5- or -6- or -7-yl; 3,4-dihydro-2H-azepin-2- or -3- or - 4- or -5- or -6- or -7-yl; 3,6-dihydro-2H-azepin-2- or -3- or -4- or -5- or -6- or -7-yl; 5,6-dihydro-2H-azepin-2- or -3- or -4- or -5- or -6- or -7-yl; 4,5-dihydro-3H-azepin-2- or -3- or -4- or -5- or -6- or -7-yl; 1H-azepin-1- or -2- or -3- or -4- or -5- or -6- or -7-yl; 2H-azepin-2- or -3- or -4- or -5- or -6- or -7-yl; 3H-azepin-2- or -3- or -4- or -5- or -6- or -7-yl; 4H-azepin-2- or -3- or -4- or -5- or -6- or -7-yl, 2- or 3-oxolanyl (= 2- or 3-tetrahydrofuranyl); 2,3-dihydrofuran-2- or -3- or -4- or -5-yl; 2,5-dihydrofuran-2- or -3-yl, 2- or 3- or 4-oxanyl (= 2- or 3- or 4-tetrahydropyranyl); 3,4-dihydro-2H-pyran-2- or -3- or -4- or - 5- or -6-yl; 3,6-dihydro-2H-pyran-2- or -3-or -4- or -5- or -6-yl; 2H-pyran-2- or -3- or -4- or -5- or -6-yl; 4H-pyran-2- or -3- or -4-yl, 2- or -3- or -4-oxepanyl; 2,3,4,5-tetrahydro-oxepin-2- or -3- or -4- or -5- or - 6- or -7-yl; 2,3,4,7-tetrahydrooxepin-2- or -3- or -4- or -5- or -6- or -7-yl; 2,3,6,7-tetrahydrooxepin-2- or -3- or -4-yl; 2,3-dihydrooxepin-2- or -3- or -4- or -5- or -6- or -7-yl; 4,5-dihydrooxepin-2- or -3- or -4-yl; 2,5-dihydrooxepin-2- or -3- or -4- or -5- or -6- or -7-yl; oxepin-2- or -3- or -4- or -5- or -6- or -7-yl; 2- or 3-tetrahydrothiophenyl; 2,3-dihydrothiophen-2- or -3- or -4- or -5-yl; 2,5-dihydrothiophen-2- or -3-yl; tetrahydro-2H-thiopyran-2- or -3- or -4-yl; 3,4-dihydro-2H-thiopyran-2- or -3- or -4- or -5- or -6-yl; 3,6-dihydro-2H-thiopyran-2- or -3- or -4- or -5- or -6-yl; 2H-thiopyran-2- or -3- or -4- or -5- or -6-yl; 4H-thiopyran-2- or -3- or -4-yl. Preferred 3-membered and 4-membered heterocycles are, for example, 1- or 2-aziridinyl, oxiranyl, thiiranyl, 1- or 2- or 3-azetidinyl, 2- or 3-oxetanyl, 2- or 3-thietanyl, 1,3-dioxetan-2-yl. Further examples of "heterocyclyl" are a partially or fully hydrogenated heterocyclic radical having two heteroatoms from the group of N, O and S, for example 1- or 2- or 3- or 4-pyrazolidinyl; 4,5-dihydro-3H-pyrazol-3- or -4- or -5-yl; 4,5-dihydro-1H-pyrazol-1- or -3- or -4- or -5-yl; 2,3-dihydro-1H-pyrazol-1- or -2- or -3- or -4- or -5-yl; 1- or -2- or -3- or -4-imidazolidinyl; 2,3-dihydro-1H-imidazol-1- or -2- or -3- or -4-yl; 2,5-dihydro-1H-imidazol-1- or -2- or -4- or -5-yl; 4,5-dihydro-1H-imidazol-1- or - 2- or -4- or -5-yl; hexahydropyridazin-1- or -2- or -3- or -4-yl; 1,2,3,4-tetrahydropyridazin-1- or -2- or - 3- or -4- or -5- or -6-yl; 1,2,3,6-tetrahydropyridazin-1- or -2- or -3- or -4- or -5- or -6-yl; 1,4,5,6-tetrahydropyridazin-1- or -3- or -4- or -5- or -6-yl; 3,4,5,6-tetrahydropyridazin-3- or -4- or -5-yl; 4,5-dihydropyridazin-3- or -4-yl; 3,4-dihydropyridazin-3- or -4- or -5- or -6-yl; 3,6-dihydropyridazin-3- or - 4-yl; 1,6-dihydro-pyridazin-1- or -3- or -4- or -5- or -6-yl; hexahydropyrimidin-1- or -2- or -3- or -4-yl; 1,4,5,6-tetrahydropyrimidin-1- or -2- or -4- or -5- or -6-yl; 1,2,5,6-tetrahydropyrimidin-1- or -2- or -4- or -5- or -6-yl; 1,2,3,4-tetrahydropyrimidin-1- or -2- or -3- or -4- or -5- or -6-yl; 1,6-dihydropyrimidin-1- or -2- or -4- or -5- or -6-yl; 1,2-dihydropyrimidin-1- or -2- or -4- or -5- or -6-yl; 2,5-dihydropyrimidin-2- or -4- or -5-yl; 4,5-dihydropyrimidin- 4- or -5- or -6-yl; 1,4-dihydropyrimidin-1- or -2- or -4- or -5- or -6-yl; 1- or -2- or -3-piperazinyl; 1,2,3,6-tetrahydropyrazin-1- or -2- or -3- or -5- or -6-yl; 1,2,3,4-tetrahydropyrazin-1- or -2- or -3- or -4- or -5- or -6-yl; 1,2-dihydropyrazin-1- or -2- or -3- or -5- or -6-yl; 1,4-dihydropyrazin-1- or -2- or -3-yl; 2,3-dihydropyrazin-2- or -3- or -5- or -6-yl; 2,5-dihydro-pyrazin-2- or -3-yl; 1,3-dioxolan-2- or -4- or -5-yl; 1,3-dioxol-2- or -4-yl; 1,3-dioxan-2- or -4- or -5-yl; 4H-1,3-dioxin-2- or -4- or -5- or -6-yl; 1,4-dioxan-2- or -3- or -5- or -6-yl; 2,3-dihydro-1,4-dioxin-2- or -3- or - 5- or -6-yl; 1,4-dioxin-2- or -3-yl; 1,2-dithiolan-3- or -4-yl; 3H-1,2-dithiol-3- or -4- or -5-yl; 1,3-dithiolan-2- or -4-yl; 1,3-dithiol-2- or -4-yl; 1,2-dithian-3- or -4-yl; 3,4-dihydro-1,2-dithiin-3- or -4- or - 5- or -6-yl; 3,6-dihydro-1,2-dithiin-3- or -4-yl; 1,2-dithiin-3- or -4-yl; 1,3-dithian-2- or -4- or -5-yl; 4H-1,3-dithiin-2- or -4- or -5- or -6-yl; isoxazolidin-2- or -3- or -4- or -5-yl; 2,3-dihydroisoxazol-2- or -3- or -4- or -5-yl; 2,5-dihydroisoxazol-2- or -3- or -4- or -5-yl; 4,5-dihydroisoxazol-3- or -4- or -5-yl; 1,3-oxazolidin-2- or -3- or -4- or -5-yl; 2,3-dihydro-1,3-oxazol-2- or -3- or -4- or -5-yl; 2,5-dihydro-1,3-oxazol-2- or -4- or -5-yl; 4,5-dihydro-1,3-oxazol-2- or -4- or -5-yl; 1,2-oxazinan-2- or -3- or -4- or -5- or -6-yl; 3,4-dihydro-2H-1,2-oxazin-2- or -3- or -4- or -5- or -6-yl; 3,6-dihydro-2H-1,2-oxazin-2- or -3- or - 4- or -5- or -6-yl; 5,6-dihydro-2H-1,2-oxazin-2- or -3- or -4- or -5- or -6-yl; 5,6-dihydro-4H-1,2-oxazin-3- or -4- or -5- or -6-yl; 2H-1,2-oxazin-2- or -3- or -4- or -5- or -6-yl; 6H-1,2-oxazin-3- or -4- or -5- or - 6-yl; 4H-1,2-oxazin-3- or -4- or -5- or -6-yl; 1,3-oxazinan-2- or -3- or -4- or -5- or -6-yl; 3,4-dihydro-2H-1,3-oxazin-2- or -3- or -4- or -5- or -6-yl; 3,6-dihydro-2H-1,3-oxazin-2- or -3- or -4- or -5- or -6-yl; 5,6-dihydro-2H-1,3-oxazin-2- or -4- or -5- or -6-yl; 5,6-dihydro-4H-1,3-oxazin-2- or -4- or -5- or -6-yl; 2H-1,3-oxazin-2- or -4- or -5- or -6-yl; 6H-1,3-oxazin-2- or -4- or -5- or -6-yl; 4H-1,3-oxazin-2- or -4- or -5- or -6-yl; morpholin-2- or -3- or -4-yl; 3,4-dihydro-2H-1,4-oxazin-2- or -3- or -4- or -5- or -6-yl; 3,6-dihydro-2H-1,4-oxazin-2- or -3- or -5- or -6-yl; 2H-1,4-oxazin-2- or -3- or -5- or -6-yl; 4H-1,4-oxazin-2- or -3-yl; 1,2-oxazepan-2- or -3- or -4- or -5- or -6- or -7-yl; 2,3,4,5-tetrahydro-1,2-oxazepin-2- or -3- or -4- or -5- or -6- or -7-yl; 2,3,4,7-tetrahydro-1,2-oxazepin-2- or -3- or -4- or -5- or -6- or -7-yl; 2,3,6,7-tetrahydro-1,2-oxazepin-2- or -3- or -4- or -5- or -6- or -7-yl; 2,5,6,7-tetrahydro-1,2-oxazepin-2- or -3- or -4- or -5- or -6- or -7-yl; 4,5,6,7-tetrahydro-1,2-oxazepin-3- or -4- or -5- or -6- or -7-yl; 2,3-dihydro-1,2-oxazepin-2- or -3- or -4- or -5- or -6- or -7-yl; 2,5-dihydro-1,2-oxazepin-2- or -3- or -4- or - 5- or -6- or -7-yl; 2,7-dihydro-1,2-oxazepin-2- or -3- or -4- or -5- or -6- or -7-yl; 4,5-dihydro-1,2-oxazepin-3- or -4- or -5- or -6- or -7-yl; 4,7-dihydro-1,2-oxazepin-3- or -4- or -5- or -6- or -7-yl; 6,7-dihydro-1,2-oxazepin-3- or -4- or -5- or -6- or -7-yl; 1,2-oxazepin-3- or -4- or -5- or -6- or -7-yl; 1,3-oxazepan-2- or -3- or -4- or -5- or -6- or -7-yl; 2,3,4,5-tetrahydro-1,3-oxazepin-2- or -3- or -4- or -5- or - 6- or -7-yl; 2,3,4,7-tetrahydro-1,3-oxazepin-2- or -3- or -4- or -5- or -6- or -7-yl; 2,3,6,7-tetrahydro-1,3-oxazepin-2- or -3- or -4- or -5- or -6- or -7-yl; 2,5,6,7-tetrahydro-1,3-oxazepin-2- or -4- or -5- or -6- or - 7-yl; 4,5,6,7-tetrahydro-1,3-oxazepin-2- or -4- or -5- or -6- or -7-yl; 2,3-dihydro-1,3-oxazepin-2- or -3- or -4- or -5- or -6- or -7-yl; 2,5-dihydro-1,3-oxazepin-2- or -4- or -5- or -6- or -7-yl; 2,7-dihydro-1,3-oxazepin-2- or -4- or -5- or -6- or -7-yl; 4,5-dihydro-1,3-oxazepin-2- or -4- or -5- or -6- or -7-yl; 4,7-dihydro-1,3-oxazepin-2- or -4- or -5- or -6- or -7-yl; 6,7-dihydro-1,3-oxazepin-2- or -4- or -5- or -6- or - 7-yl; 1,3-oxazepin-2- or -4- or -5- or -6- or -7-yl; 1,4-oxazepan-2- or -3- or -5- or -6- or -7-yl; 2,3,4,5-tetrahydro-1,4-oxazepin-2- or -3- or -4- or -5- or -6- or -7-yl; 2,3,4,7-tetrahydro-1,4-oxazepin-2- or -3- or -4- or -5- or -6- or -7-yl; 2,3,6,7-tetrahydro-1,4-oxazepin-2- or -3- or -5- or -6- or -7-yl; 2,5,6,7-tetrahydro-1,4-oxazepin-2- or -3- or -5- or -6- or -7-yl; 4,5,6,7-tetrahydro-1,4-oxazepin-2- or -3- or -4- or -5- or -6- or -7-yl; 2,3-dihydro-1,4-oxazepin-2- or -3- or -5- or -6- or -7-yl; 2,5-dihydro-1,4-oxazepin-2- or -3- or -5- or -6- or -7-yl; 2,7-dihydro-1,4-oxazepin-2- or -3- or -5- or -6- or -7-yl; 4,5-dihydro-1,4-oxazepin-2- or -3- or -4- or -5- or -6- or -7-yl; 4,7-dihydro-1,4-oxazepin-2- or -3- or -4- or -5- or -6- or - 7-yl; 6,7-dihydro-1,4-oxazepin-2- or -3- or -5- or -6- or -7-yl; 1,4-oxazepin-2- or -3- or -5- or -6- or -7-yl; isothiazolidin-2- or -3- or -4- or -5-yl; 2,3-dihydroisothiazol-2- or -3- or -4- or -5-yl; 2,5-dihydroisothiazol-2- or -3- or -4- or -5-yl; 4,5-dihydroisothiazol-3- or -4- or -5-yl; 1,3-thiazolidin-2- or - 3- or -4- or -5-yl; 2,3-dihydro-1,3-thiazol-2- or -3- or -4- or -5-yl; 2,5-dihydro-1,3-thiazol-2- or -4- or -5-yl; 4,5-dihydro-1,3-thiazol-2- or -4- or -5-yl; 1,3-thiazinan-2- or -3- or -4- or -5- or -6-yl; 3,4-dihydro-2H-1,3-thiazin-2- or -3- or -4- or -5- or -6-yl; 3,6-dihydro-2H-1,3-thiazin-2- or -3- or -4- or -5- or -6-yl; 5,6-dihydro-2H-1,3-thiazin-2- or -4- or -5- or -6-yl; 5,6-dihydro-4H-1,3-thiazin-2- or -4- or -5- or -6-yl; 2H-1,3-thiazin-2- or -4- or -5- or -6-yl; 6H-1,3-thiazin-2- or -4- or -5- or -6-yl; 4H-1,3-thiazin-2- or -4- or -5- or -6-yl. Further examples of "heterocyclyl" are a partially or fully hydrogenated heterocyclic radical having 3 hetero-atoms from the group of N, O and S, for example 1,4,2-dioxazolidin-2- or -3- or -5-yl; 1,4,2-dioxazol-3- or -5-yl; 1,4,2-dioxazinan-2- or -3- or -5- or -6-yl; 5,6-dihydro-1,4,2-dioxazin-3- or -5- or -6-yl; 1,4,2-dioxazin-3- or -5- or -6-yl; 1,4,2-dioxazepan-2- or -3- or -5- or -6- or -7-yl; 6,7-dihydro-5H-1,4,2-dioxazepin-3- or -5- or -6- or -7-yl; 2,3-dihydro-7H-1,4,2-dioxazepin-2- or -3- or -5- or -6- or -7-yl; 2,3-dihydro-5H-1,4,2-dioxazepin-2- or -3- or -5- or -6- or -7-yl; 5H-1,4,2-dioxazepin-3- or -5- or -6- or -7-yl; 7H-1,4,2-dioxazepin-3- or -5- or -6- or -7-yl. Structural examples of heterocycles which are optionally substituted further are also listed below:

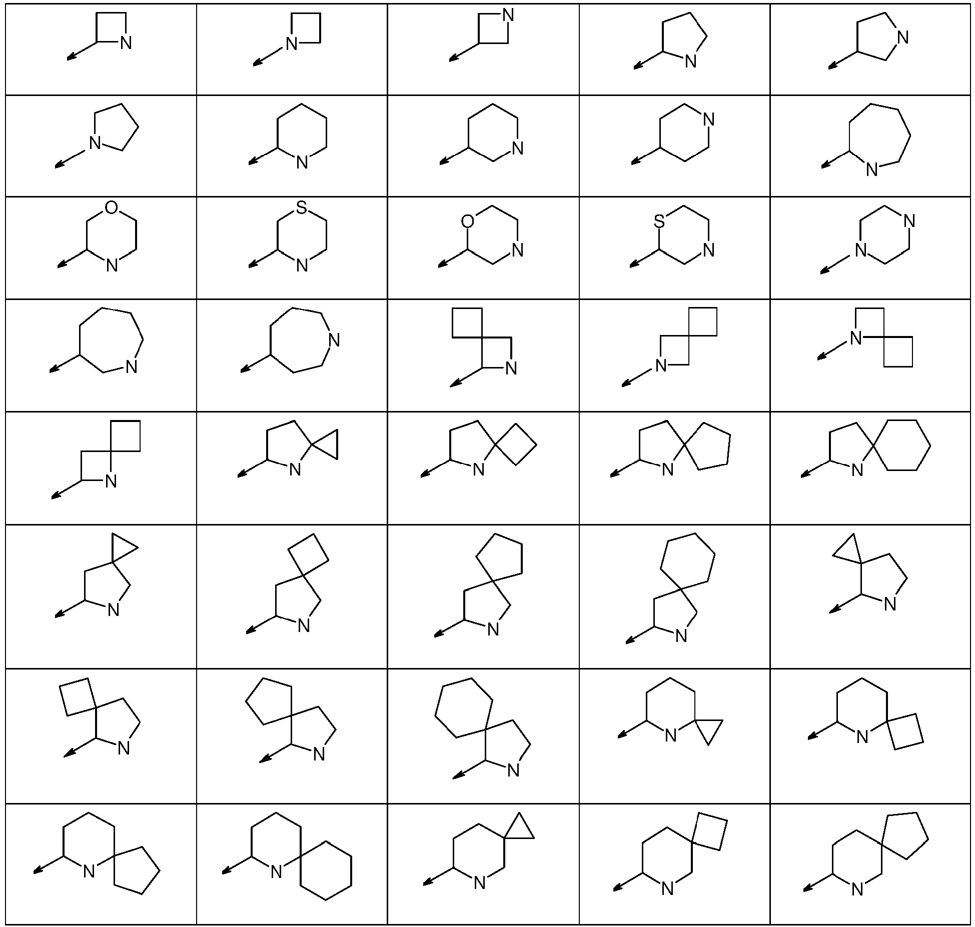

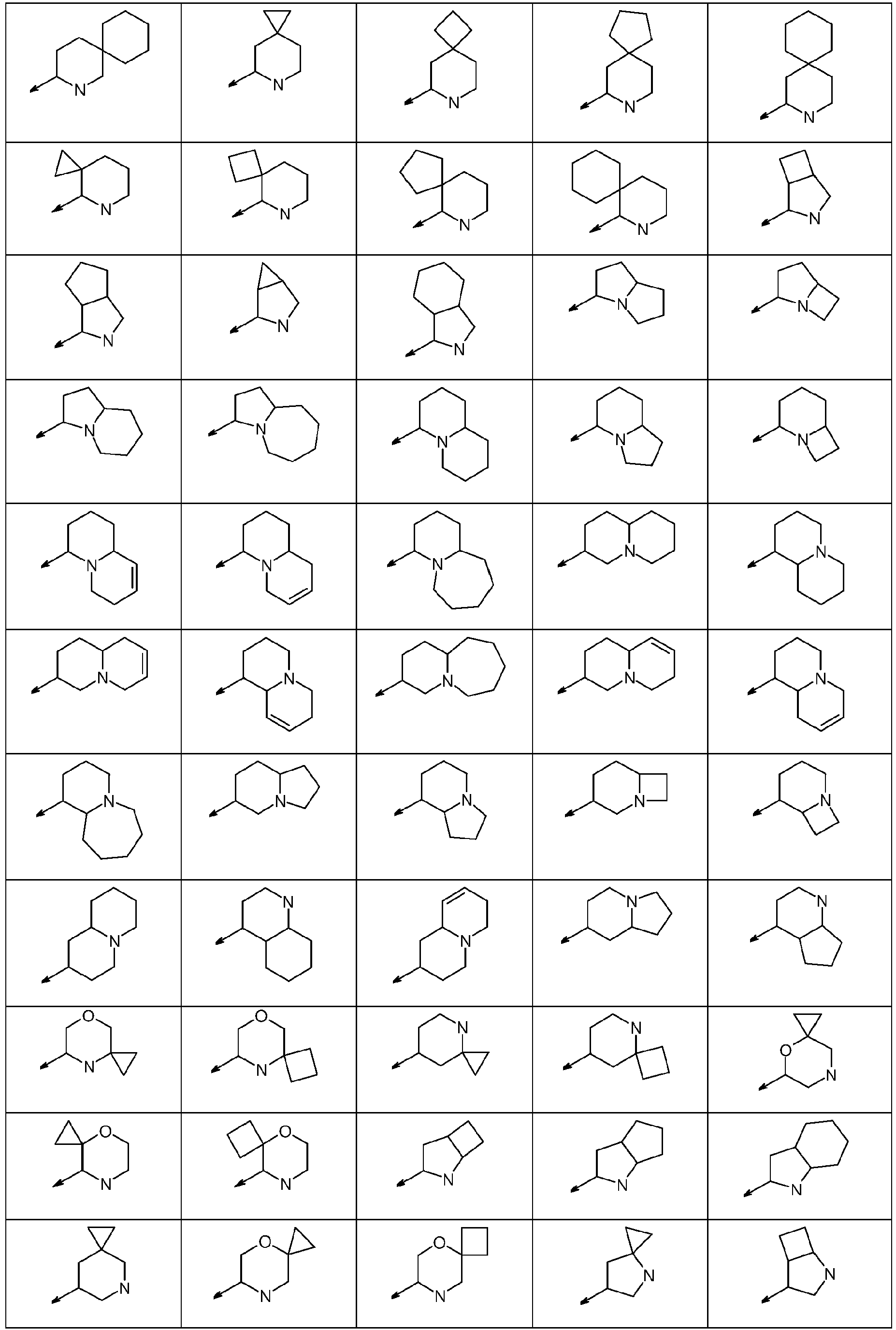

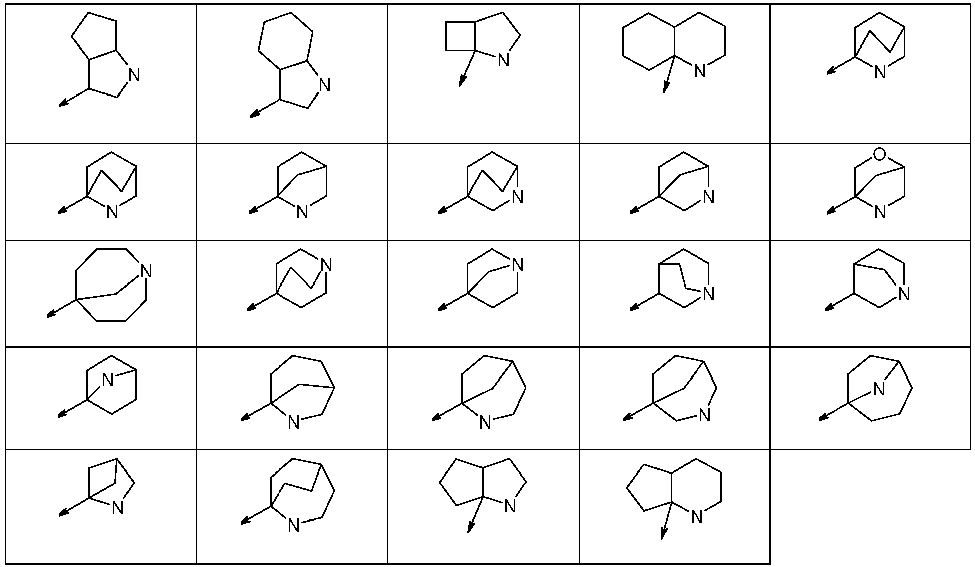

The heterocycles listed above are preferably substituted, for example, by hydrogen, halogen, alkyl, haloalkyl, hydroxyl, alkoxy, cycloalkoxy, aryloxy, alkoxyalkyl, alkoxyalkoxy, cycloalkyl, halocycloalkyl, aryl, arylalkyl, heteroaryl, heterocyclyl, alkenyl, alkylcarbonyl, cycloalkylcarbonyl, aryl-carbonyl, heteroarylcarbonyl, alkoxycarbonyl, hydroxycarbonyl, cycloalkoxycarbonyl, cycloalkyl-alkoxycarbonyl, alkoxycarbonylalkyl, arylalkoxycarbonyl, arylalkoxycarbonylalkyl, alkynyl, alkynyl-alkyl, alkylalkynyl, trisalkylsilylalkynyl, nitro, amino, cyano, haloalkoxy, haloalkylthio, alkylthio, hydrothio, hydroxyalkyl, oxo, heteroarylalkoxy, arylalkoxy, heterocyclylalkoxy, heterocyclylalkylthio, heterocyclyloxy, heterocyclylthio, heteroaryloxy, dialkylamino, alkylamino, cycloalkylamino, hydroxy-carbonylalkylamino, alkoxycarbonylalkylamino, arylalkoxycarbonylalkylamino, alkoxycarbonylalkyl(alkyl)amino, aminocarbonyl, alkylaminocarbonyl, dialkylaminocarbonyl, cycloalkylaminocarbonyl, hydroxycarbonylalkyl-aminocarbonyl, alkoxycarbonylalkylaminocarbonyl, aryl-alkoxycarbonylalkylaminocarbonyl.

When a base structure is substituted "by one or more substituents" from a list of radicals (= group) or a generically defined group of radicals, this in each case includes simultaneous substitution by a plurality of identical and/or structurally different radicals. In the case of a partially or fully saturated nitrogen heterocycle, this may be joined to the remainder of the molecule either via carbon or via the nitrogen. Suitable substituents for a substituted heterocyclic radical are the substituents specified further down, and additionally also oxo and thioxo. The oxo group as a substituent on a ring carbon atom is then, for example, a carbonyl group in the heterocyclic ring. As a result, lactones and lactams are preferably also included. The oxo group may also occur on the ring heteroatoms, which may exist in different oxidation states, for example in the case of N and S, and in that case form, for example, the divalent -N(O)-,-S(O)- (also SO for short) and -S(O)₂- (also SO₂ for short) groups in the heterocyclic ring. In the case of - N(O)- and -S(O)- groups, both enantiomers in each case are included.

According to the invention, the expression "heteroaryl" refers to heteroaromatic compounds, i.e. fully unsaturated aromatic heterocyclic compounds, preferably 5- to 7-membered rings having 1 to 4, preferably 1 or 2, identical or different heteroatoms, preferably O, S or N. Inventive heteroaryls are, for example, 1H-pyrrol-1-yl; 1H-pyrrol-2-yl; 1H-pyrrol-3-yl; furan-2-yl; furan-3-yl; thien-2-yl; thien-3-yl, 1H-imidazol-1-yl; 1H-imidazol-2-yl; 1H-imidazol-4-yl; 1H-imidazol-5-yl; 1H-pyrazol-1-yl; 1H-pyrazol-3-yl; 1H-pyrazol-4-yl; 1H-pyrazol-5-yl, 1H-1,2,3-triazol-1-yl, 1H-1,2,3-triazol-4-yl, 1H-1,2,3-triazol-5-yl, 2H-1,2,3-triazol-2-yl, 2H-1,2,3-triazol-4-yl, 1H-1,2,4-triazol-1-yl, 1H-1,2,4-triazol-3-yl, 4H-1,2,4-triazol-4-yl, 1,2,4-oxadiazol-3-yl, 1,2,4-oxadiazol-5-yl, 1,3,4-oxadiazol-2-yl, 1,2,3-oxa-diazol-4-yl, 1,2,3-oxadiazol-5-yl, 1,2,5-oxadiazol-3-yl, azepinyl, pyridin-2-yl, pyridin-3-yl, pyridin-4-yl, pyrazin-2-yl, pyrazin-3-yl, pyrimidin-2-yl, pyrimidin-4-yl, pyrimidin-5-yl, pyridazin-3-yl, pyridazin-4-yl, 1,3,5-triazin-2-yl, 1,2,4-triazin-3-yl, 1,2,4-triazin-5-yl, 1,2,4-triazin-6-yl, 1,2,3-triazin-4-yl, 1,2,3-triazin-5-yl, 1,2,4-, 1,3,2-, 1,3,6- and 1,2,6-oxazinyl, isoxazol-3-yl, isoxazol-4-yl, isoxazol-5-yl, 1,3-oxazol-2-yl, 1,3-oxazol-4-yl, 1,3-oxazol-5-yl, isothiazol-3-yl, isothiazol-4-yl, isothiazol-5-yl, 1,3-thiazol-2-yl, 1,3-thiazol-4-yl, 1,3-thiazol-5-yl, oxepinyl, thiepinyl, 1,2,4-triazolonyl and 1,2,4-diazepinyl, 2H-1,2,3,4-tetrazol-5-yl, 1H-1,2,3,4-tetrazol-5-yl, 1,2,3,4-oxatriazol-5-yl, 1,2,3,4-thia-triazol-5-yl, 1,2,3,5-oxatriazol-4-yl, 1,2,3,5-thiatriazol-4-yl. The heteroaryl groups according to the invention may also be substituted by one or more identical or different radicals. If two adjacent carbon atoms are part of a further aromatic ring, the systems are fused heteroaromatic systems, such as benzofused or polyannealed heteroaromatics. Preferred examples are quinolines (e.g. quinolin-2-yl, quinolin-3-yl, quinolin-4-yl, quinolin-5-yl, quinolin-6-yl, quinolin-7-yl, quinolin-8-yl); isoquinolines (e.g. isoquinolin-1-yl, isoquinolin-3-yl, isoquinolin-4-yl, isoquinolin-5-yl, isoquinolin-6-yl, isoquinolin-7-yl, isoquinolin-8-yl); quinoxaline; quinazoline; cinnoline; 1,5-naphthyridine; 1,6-naphthyridine; 1,7-naphthyridine; 1,8-naphthyridine; 2,6-naphthyridine; 2,7-naphthyridine; phthalazine; pyridopyrazines; pyridopyrimidines; pyridopyridazines; pteridines; pyrimidopyrimidines. Examples of heteroaryl are also 5- or 6-membered benzofused rings from the group of 1H-indol-1-yl, 1H-indol-2-yl, 1H-indol-3-yl, 1H-indol-4-yl, 1H-indol-5-yl, 1H-indol-6-yl, 1H-indol-7-yl,
1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzo-thiophen-5-yl, 1-benzothiophen-6-yl, 1-benzothiophen-7-yl, 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 2H-indazol-3-yl, 2H-indazol-4-yl, 2H-indazol-5-yl, 2H-indazol-6-yl, 2H-indazol-7-yl, 2H-isoindol-2-yl, 2H-isoindol-1-yl, 2H-isoindol-3-yl, 2H-isoindol-4-yl, 2H-isoindol-5-yl, 2H-isoindol-6-yl; 2H-isoindol-7-yl, 1H-benzimidazol-1-yl, 1H-benzimidazol-2-yl, 1H-benzimidazol-4-yl, 1H-benzimidazol-5-yl, 1H-benzimidazol-6-yl, 1H-benzimidazol-7-yl, 1,3-benzoxazol-2-yl, 1,3-benzoxazol-4-yl, 1,3-benzoxazol-5-yl, 1,3-benzoxazol-6-yl, 1,3-benzoxazol-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,2-benzisoxazol-3-yl, 1,2-benzisoxazol-4-yl, 1,2-benzisoxazol-5-yl, 1,2-benzisoxazol-6-yl, 1,2-benzisoxazol-7-yl, 1,2-benzisothiazol-3-yl, 1,2-benzisothiazol-4-yl, 1,2-benzisothiazol-5-yl, 1,2-benzisothiazol-6-yl, 1,2-benzisothiazol-7-yl.

The term "halogen" denotes, for example, fluorine, chlorine, bromine or iodine. If the term is used for a radical, "halogen" denotes, for example, a fluorine, chlorine, bromine or iodine atom.

According to the invention, "alkyl" denotes a straight-chain or branched open-chain, saturated hydrocarbon radical which is optionally mono- or polysubstituted, and in the latter case is referred to as "substituted alkyl". Preferred substituents are halogen atoms, alkoxy, haloalkoxy, cyano, alkylthio, haloalkylthio, amino or nitro groups, particular preference being given to methoxy, methyl, fluoroalkyl, cyano, nitro, fluorine, chlorine, bromine or iodine.

The prefix "di" includes the combination of equal or different alkyl radicals, e.g. dimethyl or methyl(ethyl) or ethyl(methyl).

According to the invention, "Haloalkyl", "-alkenyl" and "-alkynyl" respectively denote alkyl, alkenyl and alkynyl partially or fully substituted by identical or different halogen atoms, for example monohaloalkyl such as CH₂CH₂Cl, CH₂CH₂Br, CHClCH₃, CH₂Cl, CH₂F; perhaloalkyl such as CCl₃, CClF₂, CFCl₂, CF₂CClF₂, CF₂CClFCF₃; polyhaloalkyl such as CH₂CHFCl, CF₂CClFH, CF₂CBrFH, CH₂CF₃; the term perhaloalkyl also encompasses the term perfluoroalkyl.

The term "C₂-C₆-haloalkenyl" as mentioned herein by way of example refers to a C₂-C₆-alkenyl group as defined above in which one or more hydrogen atoms are replaced with one or more halogen atoms that may be the same or different. Typically, C₂-C₆-haloalkenyl comprises up to 9 halogen atoms that can be the same or different.

The term "C₂-C₆-haloalkynyl" as mentioned herein by way of example refers to a C₂-C₆-alkynyl group as defined above in which one or more hydrogen atoms are replaced with one or more halogen atoms that may be the same or different. Typically, C₂-C₆-haloalkynyl comprises up to 9 halogen atoms that can be the same or different.

According to the invention, the expression "(C₁-C₄)-alkyl" mentioned here by way of example is a brief notation for straight-chain or branched alkyl having one to 4 carbon atoms according to the range stated for carbon atoms, i.e. encompasses the methyl, ethyl, 1-propyl, 2-propyl, 1-butyl, 2-butyl, 2-methylpropyl or tert-butyl radicals. General alkyl radicals with a larger specified range of carbon atoms, e.g. "(C₁-C₆)-alkyl", correspondingly also encompass straight-chain or branched alkyl radicals with a greater number of carbon atoms, i.e. according to the example also the alkyl radicals having 5 and 6 carbon atoms. Unless stated specifically, preference is given to the lower carbon skeletons, for example having from 1 to 6 carbon atoms, or having from 2 to 6 carbon atoms in the case of unsaturated groups, in the case of the hydrocarbyl radicals such as alkyl, alkenyl and alkynyl radicals, including in composite radicals. Alkyl radicals, including in composite radicals such as alkoxy, haloalkyl, etc., are, for example, methyl, ethyl, n-propyl or i-propyl, n-, i-, t- or 2-butyl, pentyls, hexyls such as n-hexyl, i-hexyl and 1,3-dimethylbutyl, heptyls such as n-heptyl, 1-methylhexyl and 1,4-dimethylpentyl; alkenyl and alkynyl radicals are defined as the possible unsaturated radicals corresponding to the alkyl radicals, where at least one double bond or triple bond is present. Preference is given to radicals having one double bond or triple bond.

According to the invention, the term "alkenyl" also includes, in particular, straight-chain or branched open-chain hydrocarbon radicals having more than one double bond, such as 1,3-butadienyl and 1,4-pentadienyl, but also allenyl or cumulenyl radicals having one or more cumulated double bonds, for example allenyl (1,2-propadienyl), 1,2-butadienyl and 1,2,3-pentatrienyl. Alkenyl denotes, for example, vinyl which may optionally be substituted by further alkyl radicals, for example (but not limited thereto) (C₂-C₆)-alkenyl such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

According to the invention, the term "alkynyl" also includes, in particular, straight-chain or branched open-chain hydrocarbon radicals having more than one triple bond, or else having one or more triple bonds and one or more double bonds, for example 1,3-butatrienyl or 3-penten-1-yn-1-yl. (C₂-C₆)-Alkynyl denotes, for example, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl.

According to the invention, the term "cycloalkyl" denotes a carbocyclic saturated ring system having preferably 3-8 ring carbon atoms, for example cyclopropyl, cyclobutyl, cyclopentyl or cyclohexyl, which optionally has further substitution, preferably by hydrogen, alkyl, alkoxy, cyano, nitro, alkylthio, haloalkylthio, halogen, alkenyl, alkynyl, haloalkyl, amino, alkylamino, dialkylamino, alkoxycarbonyl, hydroxycarbonyl, arylalkoxycarbonyl, aminocarbonyl, alkylaminocarbonyl, cycloalkylaminocarbonyl or an oxo group. In the case of optionally substituted cycloalkyl, cyclic systems with substituents are included, also including substituents with a double bond on the cycloalkyl radical, for example an alkylidene group such as methylidene or an oxo group. In the case of optionally substituted cycloalkyl, polycyclic aliphatic systems are also included, for example bicyclo[1.1.0]butan-1-yl, bicyclo[1.1.0]butan-2-yl, bicyclo[2.1.0]pentan-1-yl, bicyclo[1.1.1]pentan-1-yl, bicyclo[2.1.0]pentan-2-yl, bicyclo[2.1.0]pentan-5-yl, bicyclo[2.1.1]hexyl, bicyclo[2.2.1]hept-2-yl, bicyclo[2.2.2]octan-2-yl, bicyclo[3.2.1]octan-2-yl, bicyclo[3.2.2]nonan-2-yl, adamantan-1-yl and adamantan-2-yl, but also systems such as 1,1'-bi(cyclopropyl)-1-yl, 1,1'-bi(cyclopropyl)-2-yl, for example. The term "(C₃-C₇)-cycloalkyl" is a brief notation for cycloalkyl having three to 7 carbon atoms, corresponding to the range specified for carbon atoms. In the case of substituted cycloalkyl, spirocyclic aliphatic systems are also included, for example spiro[2.2]pent-1-yl, spiro[2.3]hex-1-yl, spiro[2.3]hex-4-yl, 3-spiro[2.3]hex-5-yl, spiro[3.3]hept-1-yl, spiro[3.3]hept-2-yl.

"Cycloalkenyl" denotes a carbocyclic, nonaromatic, partially unsaturated ring system having preferably 4-8 carbon atoms, e.g. 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl, also including substituents with a double bond on the cycloalkenyl radical, for example an alkylidene group such as methylidene. In the case of optionally substituted cycloalkenyl, the elucidations for substituted cycloalkyl apply correspondingly.

According to the invention, "haloalkylthio" or "haloalkylsulfanyl" - on its own or as constituent part of a chemical group - represents straight-chain or branched S-haloalkyl, preferably having 1 to 8, or having 1 to 6 carbon atoms, such as (C₁-C₈)-, (C₁-C₆)- or (C₁-C₄)-haloalkylthio, for example (but not limited thereto) trifluoromethylthio, pentafluoroethylthio, difluoromethylthio, 2,2-difluoroeth-1-ylthio, 2,2,2-difluoroeth-1-ylthio, 3,3,3-prop-1-ylthio or the corresponding name with -sulfanyl.

"Halocycloalkyl" and "halocycloalkenyl" denote cycloalkyl and cycloalkenyl, respectively, which are partially or fully substituted by identical or different halogen atoms, such as F, Cl and Br, or by haloalkyl, such as trifluoromethyl or difluoromethyl, for example 1-fluorocycloprop-1-yl, 2-fluoro-cycloprop-1-yl, 2,2-difluorocycloprop-1-yl, 1-fluorocyclobut-1-yl, 1-trifluoromethylcycloprop-1-yl, 2-trifluoromethylcycloprop-1-yl, 1-chlorocycloprop-1-yl, 2-chlorocycloprop-1-yl, 2,2-dichloro-cyclo-prop-1-yl, 3,3-difluorocyclobutyl.

According to the invention, "trialkylsilyl" - on its own or as constituent part of a chemical group - represents straight-chain or branched Si-alkyl, preferably having 1 to 8, or having 1 to 6 carbon atoms, such as tri[(C₁-C₈)-, (C₁-C₆)- or (C₁-C₄)-alkyl]silyl, for example (but not limited thereto) trimethylsilyl, triethylsilyl, tri(n-propyl)silyl, tri(isopropyl)silyl, tri(n-butyl)silyl, tri(1-methylprop-1-yl)silyl, tri(2-methylprop-1-yl)silyl, tri(1,1-dimethyleth-1-yl)silyl, tri(2,2-dimethyleth-1-yl)silyl.

The term "oxo" as used herein refers to an oxygen atom which is bound to a carbon atom or sulfur atom via a double bound.

The term "methylidene" as used herein refers to a CH₂ group connected to a carbon atom via a double bond.

The term "halomethylidene" as used herein refers to a CX₂ group connected to a carbon atom via a double bond, wherein X is halogen.

If the compounds can form, through a hydrogen shift, tautomers whose structure is not formally covered by the general formula (I), these tautomers are nevertheless covered by the definition of the inventive compounds of the general formula (I), unless a particular tautomer is under consideration. For example, many carbonyl compounds may be present both in the keto form and in the enol form, both forms being encompassed by the definition of the compound of the general formula (I).

Depending on the nature of the substituents and the manner in which they are attached, the compounds of the general formula (I) may be present as stereoisomers. The general formula (I) embraces all possible stereoisomers defined by the specific three-dimensional form thereof, such as atropisomers, enantiomers, diastereomers, Z and E isomers. If, for example, one or more alkenyl groups are present, diastereomers (Z and E isomers) may occur. If, for example, one or more asymmetric carbon atoms are present, atropisomers, enantiomers and diastereomers may occur. Stereoisomers can be obtained from the mixtures obtained in the preparation by customary separation methods. The chromatographic separation can be affected either on the analytical scale to find the enantiomeric excess or the diastereomeric excess, or else on the preparative scale to produce test specimens for biological testing. It is likewise possible to selectively prepare stereoisomers by using stereoselective reactions with use of optically active starting materials and/or auxiliaries. The invention thus also relates to all stereoisomers which are embraced by the general formula (I), but are not shown in their specific stereomeric form, unless specified in the experimental section, and to mixtures thereof. Differences in stereochemistry may have an impact on biological effects.

If the compounds are obtained as solids, the purification can also be carried out by recrystallization or digestion. If individual compounds of general formula (I) cannot be obtained in a satisfactory manner by the routes described below, they can be prepared by derivatization of other compounds of general formula (I).

Suitable isolation methods, purification methods and methods for separating stereoisomers or atropisomers of compounds of the general formula (I) are methods generally known to the person skilled in the art from analogous cases, for example by physical processes such as crystallization, chromatographic methods, in particular column chromatography and HPLC (high pressure liquid chromatography), distillation, optionally under reduced pressure, extraction and other methods, any mixtures that remain can generally be separated by chromatographic separation, for example on chiral solid phases. Suitable for preparative amounts or on an industrial scale are processes such as crystallization, for example of diastereomeric salts which can be obtained from the diastereomer mixtures using optically active acids and, if appropriate, provided that acidic groups are present, using optically active bases.

Synthesis of substituted pyrazole carboxamides of the general formula (I):
The substituted pyrazole carboxamides of the general formula (I) according to the invention can be prepared using known processes. The synthesis routes used and examined proceed from commercially available or easily synthesised substituted pyrazolyl carboxylic acids which are converted into substituted pyrazole carboxamides of the general formula (I) using an amide-forming reaction with suitably substituted amines. In the following schemes, the groups Q, X, A¹, A², A³, A⁴, R¹, R², R³, R⁴, R⁵, R⁶, R⁷, R¹⁴, R¹⁵, R¹⁶ and R¹⁷ of the general formula (I) have the meanings defined above, unless exemplary, but not limiting definitions are given.

The key azetidine intermediates (IIa) can be prepared using a variety of synthetic methods (WO2016/030310; Angew. Chem. Int. Ed. 2010, 49, 2900; WO2009/076404). The first synthesis route for substituted azetidines (IIa) proceeds via acyl protection of the amino group in azetidine with a suitable acyl chloride, e.g. pivaloyl chloride, followed by conversion into the corresponding thio- amide (III) using a suitable reagent, e.g. Lawesson's reagent or phosphorous pentasulfide (Scheme 1). Subsequent alkylation using sec-BuLi and TMEDA in a polar-aprotic solvent (e.g. tetrahydrofuran) affords the desired azetidine (IV) which is deprotected using methyl lithium and HCl in a suitable polar-aprotic solvent (e.g. tetrahydrofuran). The synthesis of substituted azetidines (IIa) can also be accomplished starting from N-protected azetidinyl carboxylic acids (Scheme 1). Using this method boc-protected azetidinyl carboxylic acid (V) is converted into its corresponding Weinreb amide (VI) using methyl(methoxy)amine together with suitable amide coupling reagents (e.g. 2,4,6-tripropyl-1,3,5,2,4,6-trioxatriphosphorinan-2,4,6-trioxide with N,N-diisopropylethyl amine in a polar-aprotic solvent such as dichloromethane) and then coupled to a suitable substituted phenyl or heteroaryl bromide using n-butyl lithium in abs. tetrahydrofuran to afford ketone (VII). Thereafter, the carbonyl group is reduced to an alcohol group using a suitable hydride-based reducing agent (e.g. sodium borohydride) in a suitable solvent (e.g. methanol), and the free hydroxy group is then reacted with 1,1'-thiocarbonyldiimidazole in the presence of a suitable base (e.g. DMAP = 4-(*N,N*-dimethyl-amino)pyridine) to afford thiocarbamate (VIII). Removal of the thiocarbamoyl group is accomplished in the presence of a suitable reagent system (e.g. tributyltin hydride and AIBN = Azobisiso-butyronitrile) followed by cleavage of the boc-protecting group to afford desired azetidine intermediate (II). Reagents that can accomplish the boc deprotection include TFA = trifluoroacetic acid in a suitable polar-aprotic solvent such as dichloromethane and HCl in dioxan. Lastly, substituted azetidines (IIa) can also be prepared in 6-step sequence starting from substituted N-boc protected beta-amino acids (IX) via reduction of the acid moiety, subsequent change of protecting group from boc to pmb (p-methoxybenzyl, also abbreviated as PMB), transformation of the alcohol (X) into chloride (XI), cyclisation to form the pmb-protected azetidine (XII). Removal of the pmb-protecting group completes this alternative synthesis route to the key azetidine intermediates (IIa) which can finally be converted into substituted pyrazole carboxamides of the general formula (I) using an amide-forming reagent system (e.g. T3P = 2,4,6-tripropyl-1,3,5,2,4,6-trioxatriphosphorinan-2,4,6-trioxide with DIPEA = N,N-diisopropylethyl amine or HOBt = 1-Hydroxybenzotriazole and EDC = 1-Ethyl-3-(3-dimethylaminopropyl)carbodiimide together with DIPEA in a polar-aprotic solvent such as dichloromethane). In scheme 1 below the groups A¹, A², A³, A⁴, R¹, R², R³, R⁴, and R⁵, have the meanings defined above, whilst Q by way of example, but not by limitation represents Q-1, R⁶, R⁷, R¹⁴ and R¹⁵, by way of example, but not by limitation represent hydrogen and X by way of example, but not by limitation represents a direct bond.

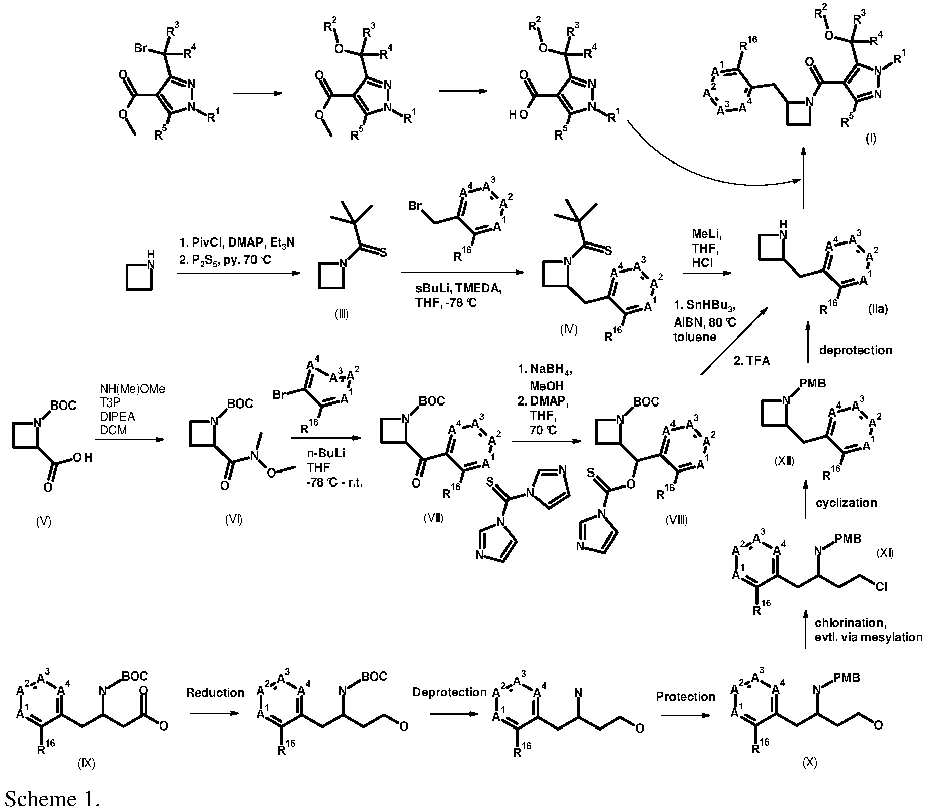

In regards to the preparation of the pyrrolidine coupling partner (IIb), synthesis began with a based-catalyzed condensation of commercially available compounds, 1-vinylpyrrolidin-2-one (XIII) and a suitable ethyl ester, namely ethyl (2-fluorophenyl)acetate (XIV) (Scheme 2). The afforded β-οxο lactam (XV) was achieved via treatment with sodium hydride in abs THF with heating at elevated temperature. Subsequent acid-mediated transformation of the lactam ring - using concentrated hydrochloric acid (HCl), heated at reflux in ethanol, followed by treatment with 50% aqueous sodium hydroxide at 0 °C - converted compound (XV) into pyrroline (XVI). In turn, compound (XVI) was reduced using sodium borohydride in methanol, to furnish the desired pyrrolidine (IIb). Synthesis of the pyrroline (XVI) from 1-vinylpyrrolidin-2-one (XV) was inspired by literature references (WO 2009/153191; Padwa, A et. al., "Science of Synthesis 27: Category 4. Compounds with Two Carbon Heteroatom Bonds", 2005, 268-269), and a routine procedure was employed to carry out the final reductive step. That is, the use of a suitable hydride-based reducing agent (e.g. sodium borohydride) in a suitable solvent (e.g. methanol). With the pyrrolidine coupling partner (IIb) in hand, conversion into the substituted pyrazole carboxamides of the general formula (I) was successfully achieved using the amide-forming reagent system mentioned above (that is, T3P = 2,4,6-tripropyl-1,3,5,2,4,6-trioxa-triphosphorinan-2,4,6-trioxide with DIPEA = N,N-diisopropylethyl amine or HOBt = 1-Hydroxy-benzotriazole and EDC = 1-Ethyl-3-(3-dimethylaminopropyl)carbodiimide together with DIPEA in a polar-aprotic solvent such as dichloromethane). For the aforementioned cases where an enantiomer has been described, preparation was achieved using high-pressure liquid chromatography of the racemic mixture obtained following the amide coupling step, as summarised in Scheme 2 below.

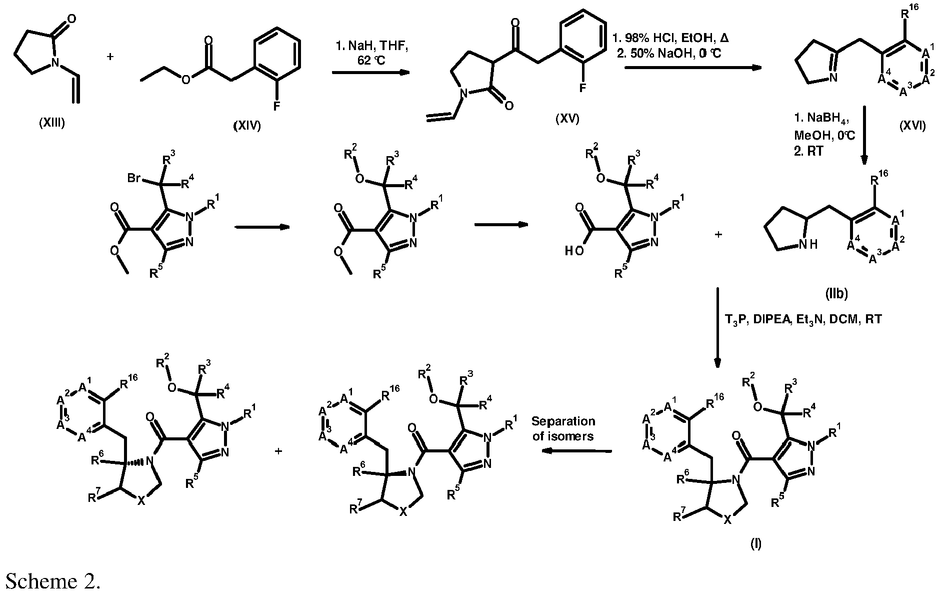

In Scheme 2 above the groups A¹, A², A³, A⁴, R¹, R², R³, R⁴, and R⁵, have the meanings defined above, whilst Q by way of example, but not by limitation represents Q-2, R⁶, R⁷, R¹⁴ and R¹⁵, by way of example, but not by limitation represent hydrogen and X by way of example, but not by limitation represents a CH₂ group.

Selected detailed synthesis examples for the compounds of the general formula (I) according to the invention are given below. The example numbers mentioned correspond to the numbering scheme in Schemes 1 and 2 and Table 1. The ¹H-NMR spectroscopy data reported for the chemical examples described in the sections that follow were obtained on Bruker instruments at 600 MHz, 400 MHz or 300 MHz using CDCl₃ or d₆-DMSO as the solvent with tetramethylsilane (δ = 0.00 ppm) as the internal standard. The signals listed have the meanings given below: br = broad; s = singlet, d = doublet, t = triplet, dd = doublet of doublets, ddd = doublet of a doublet of doublets, m = multiplet, q = quartet, qu = quintet, sext = sextet, sept = septet, dq = doublet of quartets, dt = doublet of triplets.

### No. I-062: [2-(2-fluorobenzyl)pyrrolidin-1-yl][2-(methoxymethyl)-4,5,6,7-tetrahydropyrazolo[1,5-a]pyridin-3-yl]methanone

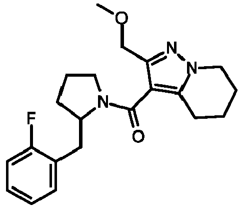

A mixture of THF and distilled water (4 mL, 3:1) was added to methyl 2-(methoxymethyl)-4,5,6,7-tetrahydropyrazolo[1,5-a]pyridine-3-carboxylate (0.15 g, 0.67 mmol), followed by lithium hydroxide (56 mg, 1.3 mmol) at room temperature. Following overnight stirring, the reaction mixture was acidified using aqueous HCl (1 M), and the aqueous layer extracted with EtOAc. The combined organics were dried with Na₂SO₄, and concentrated in vacuo to give the desired 2-(methoxymethyl)-4,5,6,7-tetrahydropyrazolo[1,5-a]pyridine-3-carboxylic acid (0.13 g, 88% of theory) as a white solid, which was subsequently used without further purification. 1H-NMR (400.0 MHz, CDCl3): δ_{H} = 4.71 (2 H, s), 4.13 (2 H, t, J 8.0 Hz), 3.51 (3 H, s), 3.10 (2 H, t, J 8.0 Hz), 2.05-2.03 (2 H, m), 1.90-1.87 (2 H, m). To a stirring solution of the 2-(methoxymethyl)-4,5,6,7-tetrahydropyrazolo[1,5-a]pyridine-3-carboxylic acid (0.13 g, 0.68 mmol) and 2-(2-fluorobenzyl)pyrrolidine (0.12 g, 0.68 mmol) in DCM (3 mL) was added the dropwise triethylamine (0.26 mL, 1.9 mmol). The reaction mixture was then flushed with argon and propanephosphonic acid anhydride (0.55 mL, 0.93 mmol) was then added under inert conditions. After 2 hours, water (0.5 mL) was then added, and the organic phase was extracted with DCM (3 mL). The organic phase was washed with aqueous NaHCO3 (0.5 mL, 5%), and then concentrated in vacuo, to give the desired [2-(2-fluorobenzyl)pyrrolidin-1-yl][2-(methoxymethyl)-4,5,6,7-tetrahydropyrazolo[1,5-a]pyridin-3-yl]methanone I-062 as a yellow oil (0.22 g, 85% of theory).

¹H-NMR (400.0 MHz, CDCl₃): δ_{H} = 7.21-7.18 (2H, m), 7.10-7.00 (2H, m), 4.50 (2H, d, J 12.0 Hz), 4.42 (2H, d, J 12.0 Hz), 4.19-4.08 (2H, m), 3.32 (3H, s), 2.08-2.00 (2H, m), 1.93-1.87 (2H, m), 1.83-1.80 (2H, m), 1.78-1.72 (2H, m), 1.05-0.99 (2H, m), 0.88-0.84 (2H, m).

### No. I-093: [2-(2-Fluorobenzyl)pyrrolidin-1-yl] { 1-methyl-5-[(tetrahydrofuran-3-yloxy)methyl]-1H-pyrazol-4-yl}methanone

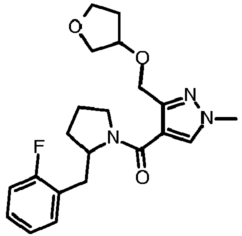

To a stirring solution of 1-methyl-5-[(tetrahydrofuran-3-yloxy)methyl]-1H-pyrazole-4-carboxylic acid (10 mg, 0.044 mmol) and 2-(2-fluorobenzyl)pyrrolidine (8.7 mg, 0.049 mmol) in DCM (6 mL) was added the dropwise triethylamine (18 µL, 0.13 mmol). The reaction mixture was then flushed with argon and propanephosphonic acid anhydride (39 µL, 0.066 mmol) was then added under inert conditions. After 2 hours, water (0.5 mL) was then added, and the organic phase was extracted with DCM (5 mL). The organic phase was washed with aqueous NaHCO₃ (0.5 mL, 5%), and then concentrated in vacuo, to give the desired compound [2-(2-fluorobenzyl)pyrrolidin-1-yl] { 1-methyl-5-[(tetrahydrofuran-3-yloxy)methyl]-1H-pyrazol-4-yl}methanone I-093 (20 mg, 90% of theory), as an oil.

¹H-NMR (400.0 MHz, CDCl₃): δ_{H} = 7.56 (1H, s), 7.32-7.19 (2H, m), 7.10-7.00 (2H, m), 4.90-4.81 (2H, m), 4.53 (2H, br s), 4.28 (1 H, s), 3.93 (3 H, s), 3.91-3.77 (2 H, m), 3.49-3.40 (2H, m), 3.23-3.20 (2H, m), 2.98-2.93 (2H, m), 2.08-1.66 (3H, m), 1.25-1.23 (2H, br s).

### No. I-094: [2-(2-fluorobenzyl)pyrrolidin-1-yl] { 1-methyl-3-[(2,2-difluoropropoxy)methyl]-1H-pyrazol-4-yl} methanone

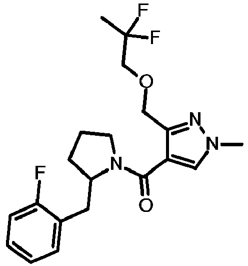

To a stirring solution of ethyl 3-(bromomethyl)-1-methyl-1H-pyrazole-4-carboxylate (0.1 g, 0.41 mmol) and 2,2-difluoropropan-1-ol (43 mg, 0.45 mmol) in THF (0.6 mL) at 0 °C was added potassium *tert-*butoxide (0.45 mL, 0.45 mmol). After stirring for 10 mins, the reaction mixture was acidified using 1M HCl at 0 °C. The aqueous layer was extracted using EtOAc (3 mL) and then concentrated *in vacuo.* The residual was purified using column chromatography (heptane:acetone, 100:0 to 0:100), to give the desired ethyl 1-methyl-3-[(2,2,3,3-tetrafluoropropoxy)methyl]-1H-pyrazole-4-carboxylate (87 mg, 72%), which was subsequently used without further purification. A mixture of THF and distilled water (4 mL, 3:1) was added to ethyl 1-methyl-3-[(2,2-difluoropropoxy)methyl]-1H-pyrazole-4-carboxylate (87 mg, 0.29 mmol), followed by lithium hydroxide (31 mg, 0.73 mmol). The reaction mixture was heated at 65 °C for a few hours, then stirred at room temperature overnight. The reaction mixture was acidified using 1 M HCl, and the aqueous layer extracted with DCM. The combined organics were dried with Na₂SO₄ and concentrated *in vacuo* to give the desired 3-[(2,2-difluoropropoxy)methyl]-1-methyl-1H-pyrazole-4-carboxylic acid (62 mg, 79% of theory), which was subsequently used without further purification. To a stirring solution of 3-[(2,2-difluoropropoxy)methyl]-1-methyl-1H-pyrazole-4-carboxylic acid (76 mg, 0.33 mmol) and 2-(2-fluorobenzyl)pyrrolidine (64 mg, 0.36 mmol) in DCM (2 mL) was added dropwise triethylamine (0.14 mL, 0.97 mmol). The reaction mixture was then flushed with argon and propanephosphonic acid anhydride (0.43 mL, 0.49 mmol) was then added under inert conditions. After overnight, water (0.5 mL) was then added, and the organic phase was extracted with DCM (5 mL). The organic phase was washed with aqueous NaHCO₃ (0.5 mL, 5%), and then concentrated *in vacuo,* to give the desired 1[2-(2-fluorobenzyl)pyrrolidin-1-yl]{ 1-methyl-3-[(2,2-difluoropropoxy)methyl]-1H-pyrazol-4-yl}methanone I-094 (91 mg, 64% of theory) as a colourless oil. ¹H-NMR (400.0 MHz, CDCl₃): δ_{H} = 7.52-7.49 (1H, br s), 7.33-7.17 (2H, m), 7.09-7.00 (2H, m), 4.88 - 4.86 (2H, m), 4.80-4.77 (2H, m), 3.90 (3H, s), 3.71 (1H, t, J 12.0 Hz), 1.92-1.43 (11H, m).

### No. I-100: [2-(2-fluorobenzyl)pyrrolidin-1-yl] { 1-methyl-3-[(2,2,3,3-tetrafluoropropoxy)methyl]-1H-pyrazol-4-yl}methanone

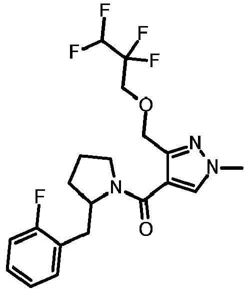

To a stirring solution of ethyl 3-(bromomethyl)-1-methyl-1H-pyrazole-4-carboxylate (0.1 g, 0.41 mmol) and 2,2,3,3-tetrafluoropropan-1-ol (59 mg, 0.45 mmol) in THF (0.6 mL) at 0 °C was added potassium tert-butoxide (0.45 mL, 0.45 mmol). After stirring for 10 mins, the reaction mixture was acidified using 1M HCl at 0 °C. The aqueous layer was extracted using EtOAc (3 mL) and then concentrated in vacuo. The residual was purified using column chromatography (heptane:acetone, 100:0 to 0:100), to give the desired ethyl 1-methyl-3-[(2,2,3,3-tetrafluoropropoxy)methyl]-1H-pyrazole-4-carboxylate (87 mg, 72% of theory), which was subsequently used without further purification. A mixture of THF and distilled water (4 mL, 3:1) was added to ethyl 1-methyl-3-[(2,2,3,3-tetrafluoropropoxy)methyl]-1H-pyrazole-4-carboxylate (87 mg, 0.29 mmol), followed by lithium hydroxide (31 mg, 0.73 mmol). The reaction mixture was heated at 65 °C for a few hours, then stirred at room temperature overnight. The reaction mixture was acidified using 1 M HCl, and the aqueous layer extracted with DCM. The combined organics were dried with Na₂SO₄ and concentrated in vacuo to give the desired 1-methyl-3-[(2,2,3,3-tetrafluoropropoxy)methyl]-1H-pyrazole-4-carboxylic acid (62 mg, 79% of theory), which was subsequently used without further purification. To a stirring solution of 1-methyl-3-[(2,2,3,3-tetrafluoropropoxy)methyl]-1H-pyrazole-4-carboxylic acid (62 mg, 0.23 mmol) and 2-(2-fluorobenzyl)pyrrolidine (45 mg, 0.25 mmol) in DCM (2 mL) was added dropwise triethylamine (96 µL, 0.69 mmol). The reaction mixture was then flushed with argon and propanephosphonic acid anhydride (302 µL, 0.344 mmol) was then added under inert conditions. After 2 hours, water (0.5 mL) was then added, and the organic phase was extracted with DCM (5 mL). The organic phase was washed with aqueous NaHCO₃ (0.5 mL, 5%), and then concentrated in vacuo, to give the desired [2-(2-fluorobenzyl)pyrrolidin-1-yl] {1-methyl-3-[(2,2,3,3-tetrafluoropropoxy)methyl]-1H-pyrazol-4-yl}methanone I-100 (85 mg, 77%) as an oil.

¹H-NMR (400.0 MHz, CDCl₃): δ_{H} = 7.52-7.50 (1H, br s), 7.29-7.18 (2H, m), 7.09-7.00 (2H, m), 6.00 (1H, m), 5.00-4.80 (2H, m), 4.50 (1H, br s), 3.91 (3H, s), 3.60-3.20 (6H, m), 1.88-1.65 (4H, m).

### No. I-196: [2-(2-fluorobenzyl)azetidin-1-yl][5-(methoxymethyl)-1-methyl-1H-pyrazol-4-yl]methanone

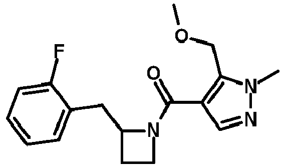

1-[(tert-butoxy) carbonyl] azetidine-2-carboxylic acid (300 g, 1.491 mol], methoxy(methyl)amine (109.33 g,1.789 mol) and 2,4,6-tripropyl-1,3,5,2,4,6-trioxatriphosphorinan-2,4,6-trioxide (1423.23 g, 4.473 mol) were added successively to a solution of N,N-Diisopropylethylamine (963.48 g, 2.4857.455 mol) in dichloromethane (3 L). The resulting reaction mixture was stirred under nitrogen at 25°C for 16h and was quenched thereafter with aqueous NH₄Cl solution. The aqueous layer was extracted with ethyl acetate (800 mL X 3), and then the combined organic layer was washed with brine, dried over Na₂SO₄, and concentrated under reduced pressure to obtain desired tert-butyl 2-[methoxy(methyl)carbamoyl] azetidine-1-carboxylate as a yellow oil (330 g, 91 % of theory). To a flame-dried round-bottomed flask equipped with a magnetic stir bar and addition funnel under nitrogen atmosphere was added 1-bromo-2-fluorobenzene (60 g, 0.2857 mol) and dry tetrahydrofuran (600 mL). The resulting solution was cooled to -78°C, and n-BuLi (157 mL, 0.3142 mol) was added dropwise. The reaction mixture was stirred for 2h, followed by addition of tert-butyl 2-[methoxy(methyl)carbamoyl] azetidine-1-carboxylate (83.75 g, 0.3428 mol). The reaction mixture was allowed to warm to ambient temperature. Stirring was continued for 16h, and the reaction mixture was quenched with aqueous NH₄Cl solution. The aqueous layer was then extracted thoroughly with ethyl acetate (300 mL X 3), and the combined organic layer was washed with brine, dried over sodium sulfate, and concentrated under reduced pressure. The remaining residue was purified by column chromatography (PE:ethyl acetate = 100:0 to 90:10) to afford tert-butyl 2-[(2-fluorophenyl) carbonyl] azetidine-1-carboxylate (37 g, 59 % of theory). A solution of tert-butyl 2-[(2-fluorophenyl) carbonyl] azetidine-1-carboxylate (30 g, 0.1074 mol) in dry MeOH (300 mL) was cooled to 0°C, and NaBH₄ (4.86 g, 0.1289 mol) was added carefully under nitrogen atmosphere. The resulting reaction mixture was stirred for 1 h and quenched with aqueous NH₄Cl solution. The aqueous layer was then extracted with ethyl acetate (300 mL X 3), and the combined organic layer was washed with brine, dried over Na₂SO₄, and concentrated under reduced pressure to obtain tert-butyl 2-[1-(2-fluorophenyl)-1-hydroxymethyl]azetidine-1-carboxylate as a yellow oil (20 g, 67% of theory). A mixture of tert-butyl 2-[1-(2-fluorophenyl)-1-hydroxymethyl]azetidine-1-carboxylate (20 g, 71.09 mmol), 1,1'-thiocarbonyldiimidazole (19 g, 106.64 mmol) and DMAP (869 mg, 7.11 mmol) in tetrahydrofuran (200 mL) was stirred at 70°C for 16 h. Thereafter, the reaction mixture was cooled to room temperature, quenched with water, and extracted with ethyl acetate. The combined organic layer was concentrated under vacuum and purified by column chromatography to give the desired product (22 g, 56.2 mmol, 79% of theory), which was dissolved in toluene (100 mL) under nitrogen atmosphere. SnHBu₃ (49.07 g, 168.60 mmol) and AIBN (1.85 g, 11.24 mmol) were added, and the resulting reaction mixture was stirred at 80° C for 5 h. After cooling to room temperature, water was added, and the aqueous layer was extracted thoroughly with ethyl acetate. The combined organic layer was concentrated under vacuum and purified by column chromatography to give tert-butyl 2-[(2-fluorophenyl)methyl]azetidine-1-carboxylate (12 g, 80% of theory). To a solution of tert-butyl 2-[(2-fluorophenyl)methyl]azetidine-1-carboxylate (12 g, 45.23 mmol) in dichloromethane (100 mL) was added trifluoroacetic acid (25 mL). The reaction mixture was stirred at room temperature for 16 h, concentrated under vacuum, and purified by column chromatography to give 2-[(2-fluorophenyl)-methyl]azetidine as a white solid (6.8 g, 91% of theory). 2-[(2-fluorophenyl)methyl]azetidine (50 mg, 0.25 mmol) was dissolved in dry dichloromethane (5 mL) under nitrogen atmosphere. After stirring for 5 min at room temperature [5-(methoxymethyl)-1-methyl-1H-pyrazol-4-yl]carboxylic acid (42 mg, 0.25 mmol), triethyl amine (0.14 mL, 0.99 mmol) and 2,4,6-tripropyl-1,3,5,2,4,6-trioxatri-phosphorinan-2,4,6-trioxide (1.5 equiv, 0.37 mmol) were added. The resulting reaction mixture was stirred at room temperature for 2 d, quenched with water and filtered via a phase separator. The organic layer was evaporated under reduced pressure, and the remaining residue was purified via prep. HPLC to afford [2-(2-fluorobenzyl)azetidin-1-yl] [5-(methoxymethyl)-1-methyl-1H-pyrazol-4-yl]methanone I-196 (30 mg, 36 % of theory) as colourless solid. ¹H-NMR (400.0 MHz, CDCl₃): δ_{H} = 7.53-7.48 (m, 1H), 7.25-7.19 (m, 2H), 7.09-7.05 (m, 2H), 4.95-4.93 (d, 1H), 4.88-4.82 (m, 1H), 4.82-4.80 (d, 1H), 4.12-4.06 (m, 2H), 3.92 (s, 3H), 3.41-3.33 (br. m, 1H), 3.40 (s, 3H), 3.21-3.11 (br. m, 1H), 2.38-2.30 (m, 1H), 2.09-2.01 (m, 1H).

### No. I-199: [2-(2-Fluorobenzyl)azetidin-1-yl][5-(2-methylprop-1-yloxymethyl)-1-methyl-1H-pyrazol-4-yl]methanone

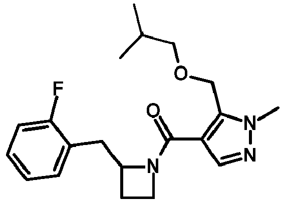

2-[(2-Fluorophenyl)methyl]azetidine (50 mg, 0.25 mmol) was dissolved in dry dichloromethane (5 mL) under nitrogen atmosphere. After stirring for 5 min at room temperature [5-(2-methylprop-1-ylmethyl)-1-methyl-1H-pyrazol-4-yl]carboxylic acid (53 mg, 0.25 mmol), triethyl amine (0.14 mL, 0.99 mmol) and 2,4,6-tripropyl-1,3,5,2,4,6-trioxatriphosphorinan-2,4,6-trioxide (1.5 equiv, 0.37 mmol) were added.

The resulting reaction mixture was stirred at room temperature for 5 h, quenched with water and filtered via a phase separator. The organic layer was evaporated under reduced pressure, and the remaining residue was purified via prep. HPLC to afford [2-(2-fluorobenzyl)azetidin-1-yl][5-(2-methylprop-1-yloxymethyl)-1-methyl-1H-pyrazol-4-yl]methanone I-199 (37 mg, 39 % of theory) as colourless solid. ¹H-NMR (400.0 MHz, CDCl₃): δ_{H} = 7.53-7.48 (m, 1H), 7.25-7.18 (m, 2H), 7.09-7.03 (m, 2H), 5.01-4.97 (d, 1H), 4.88-4.84 (d, 1H), 4.86-4.78 (m, 1H), 4.12-4.06 (m, 2H), 3.93 (s, 3H), 3.41-3.34 (br. m, 1H), 3.31-3.26 (m, 2H), 3.21-3.11 (br. m, 1H), 2.37-2.28 (m, 1H), 2.09-1.99 (m, 1H), 1.93-1.84 (sept, 1H), 0.91-0.88 (d, 6H).

In analogy to the preparation examples cited above and recited at the appropriate point and taking account of the general details relating to the preparation of substituted pyrazole carboxamides of the general formula (I), the compounds cited below are obtained.

Table 1: Examples of preferred compounds of the general formula (I). The respective stereochemistry at stereocentre C-2 is described by "Rac", representing a racemic mixture of both enantiomers, by "Ent-1" representing the enantiomer with the lower retention time in the chiral HPLC method used herein, or by "Ent-2" representing the enantiomer with the higher retention time in the chiral HPLC method used herein. Within the definitions for A¹, A², A³, A⁴, G, Q, R⁶, R⁷, R¹⁴, R¹⁵, R¹⁶, R¹⁷ and X given below, an arrow represents a bond to the carbon bearing G, R¹⁴ and R¹⁵ as substituents. For the sake of clarity and by way of example, but not by limitation, G represents the substituted aryl or heteroaryl motif shown below comprising A¹, A², A³, A⁴ and R¹⁶.

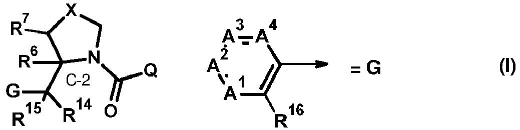

| Ex. No. | G | Q | Conf. C-2 | R⁶ | R⁷ | R¹⁴ | R¹⁵ | X |
|---|---|---|---|---|---|---|---|---|
| I-001 | 2,5-difluorophenyl | Q-1.1 | Rac | H | H | H | H | -CH₂- |
| I-002 | 2-fluorophenyl | Q-1.1 | Rac | H | H | H | H | -CH₂- |
| I-003 | 2-fluorophenyl | Q-1.1 | Ent-1 | H | H | H | H | -CH₂- |
| I-004 | 2-chloro-6-fluorophenyl | Q-1.3 | Rac | H | H | H | H | -CH₂- |
| I-005 | 2-fluorophenyl | Q-1.5 | Rac | H | H | H | H | -CH₂- |
| I-006 | 2-fluorophenyl | Q-1.6 | Rac | H | H | H | H | -CH₂- |
| I-007 | 2,4-difluorophenyl | Q-1.5 | Rac | H | H | H | H | -CH₂- |
| I-008 | 2,4-difluorophenyl | Q-1.6 | Rac | H | H | H | H | -CH₂- |
| I-009 | 2,5-difluorophenyl | Q-1.5 | Rac | H | H | H | H | -CH₂- |
| I-010 | 2,5-difluorophenyl | Q-1.6 | Rac | H | H | H | H | -CH₂- |
| I-011 | 2-fluorophenyl | Q-1.7 | Rac | H | H | H | H | -CH₂- |
| I-012 | 2-fluorophenyl | Q-1.1 | Ent-2 | H | H | H | H | -CH₂- |
| I-013 | 2-fluorophenyl | Q-1.10 | Rac | H | H | H | H | -CH₂- |
| I-014 | 2-methoxyphenyl | Q-1.1 | Rac | H | H | H | H | -CH₂- |
| I-015 | 3-fluoropyridin-2-yl | Q-1.1 | Rac | H | H | H | H | -CH₂- |
| I-016 | 2,4-dichlorophenyl | Q-1.1 | Rac | H | H | H | H | -CH₂- |
| I-017 | 2-chlorophenyl | Q-1.1 | Rac | H | H | H | H | -CH₂- |
| I-018 | 2-bromophenyl | Q-1.1 | Rac | H | H | H | H | -CH₂- |
| I-019 | 2-fluorophenyl | Q-1.1 | Rac | H | H | =O | | -CH₂- |
| I-020 | 2-fluorophenyl | Q-1.1 | Rac | H | H | 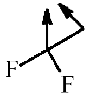 | | -CH₂- |
| I-021 | 2-fluorophenyl | Q-1.1 | Rac | H | H | CH₃ | H | -CH₂- |
| I-022 | 2-fluorophenyl | Q-1.11 | Rac | H | H | H | H | -CH₂- |
| I-023 | 2-fluorophenyl | Q-1.1 | Rac | H | H | =CH₂ | | -CH₂- |
| I-024 | 2-fluorophenyl | Q-1.12 | Rac | H | H | H | H | -CH₂- |
| I-025 | 2-fluorophenyl | Q-1.15 | Rac | H | H | H | H | -CH₂- |
| I-026 | 2-fluorophenyl | Q-1.16 | Rac | H | H | H | H | -CH₂- |
| I-027 | 2-fluorophenyl | Q-1.14 | Rac | H | H | H | H | -CH₂- |
| I-028 | 2-fluorophenyl | Q-1.17 | Rac | H | H | H | H | -CH₂- |
| I-029 | 2-fluorophenyl | Q-1.19 | Rac | H | H | H | H | -CH₂- |
| I-030 | 2-fluorophenyl | Q-1.66 | Rac | H | H | H | H | -CH₂- |
| I-031 | 2-fluorophenyl | Q-1.24 | Rac | H | H | H | H | -CH₂- |
| I-032 | 2-fluorophenyl | Q-1.21 | Rac | H | H | H | H | -CH₂- |
| I-033 | 2-fluorophenyl | Q-1.22 | Rac | H | H | H | H | -CH₂- |
| I-034 | 2-fluorophenyl | Q-1.23 | Rac | H | H | H | H | -CH₂- |
| I-035 | 2-fluorophenyl | Q-1.26 | Rac | H | H | H | H | -CH₂- |
| I-036 | 2-fluorophenyl | Q-1.27 | Rac | H | H | H | H | -CH₂- |
| I-037 | 2-fluorophenyl | Q-1.41 | Rac | H | H | H | H | -CH₂- |
| I-038 | 2-fluorophenyl | Q-1.30 | Rac | H | H | H | H | -CH₂- |
| I-039 | 2-fluorophenyl | Q-1.29 | Rac | H | H | H | H | -CH₂- |
| I-040 | 2-fluorophenyl | Q-1.28 | Rac | H | H | H | H | -CH₂- |
| I-041 | 2-fluorophenyl | Q-1.34 | Rac | H | H | H | H | -CH₂- |
| I-042 | 2-chlorophenyl | Q-2.1 | Ent-1 | H | H | H | H | -CH₂- |
| I-043 | 2-chlorophenyl | Q-2.1 | Ent-2 | H | H | H | H | -CH₂- |
| I-044 | 2-fluorophenyl | Q-1.35 | Rac | H | H | H | H | -CH₂- |
| I-045 | 2-fluorophenyl | Q-1.32 | Rac | H | H | H | H | -CH₂- |
| I-046 | 2-fluorophenyl | Q-1.40 | Rac | H | H | H | H | -CH₂- |
| I-047 | 2-fluorophenyl | Q-1.38 | Rac | H | H | H | H | -CH₂- |
| I-048 | 2-fluorophenyl | Q-1.39 | Rac | H | H | H | H | -CH₂- |
| I-049 | 2-fluorophenyl | Q-1.37 | Rac | H | H | H | H | -CH₂- |
| I-050 | 2-fluorophenyl | Q-1.42 | Rac | H | H | H | H | -CH₂- |
| I-051 | 2-fluorophenyl | Q-1.36 | Rac | H | H | H | H | -CH₂- |
| I-052 | 2-fluorophenyl | Q-1.68 | Rac | H | H | H | H | -CH₂- |
| I-053 | 2-fluorophenyl | Q-1.1 | Ent-1 | H | H | OH | H | -CH₂- |
| I-054 | 2-fluorophenyl | Q-1.1 | Ent-2 | H | H | OH | H | -CH₂- |
| I-055 | 2,6-difluorophenyl | Q-1.37 | Rac | H | H | H | H | -CH₂- |
| I-056 | 2-fluorophenyl | Q-1.2 | Rac | H | H | H | H | -CH₂- |
| I-057 | 2-fluorophenyl | Q-1.2 | Ent-1 | H | H | H | H | -CH₂- |
| I-058 | 2-fluorophenyl | Q-1.2 | Ent-2 | H | H | H | H | -CH₂- |
| I-059 | 2-fluorophenyl | Q-1.21 | Ent-1 | H | H | OH | H | -CH₂- |
| I-060 | 2-fluorophenyl | Q-1.21 | Ent-1 | H | H | H | H | -CH₂- |
| I-061 | 2-fluorophenyl | Q-1.45 | Rac | H | H | H | H | -CH₂- |
| I-062 | 2-fluorophenyl | Q-1.13 | Rac | H | H | H | H | -CH₂- |
| I-063 | 2-fluorophenyl | Q-1.46 | Rac | H | H | H | H | -CH₂- |
| I-064 | 2-fluorophenyl | Q-1.47 | Rac | H | H | H | H | -CH₂- |
| I-065 | 2-fluorophenyl | Q-1.48 | Rac | H | H | H | H | -CH₂- |
| I-066 | 2-fluorophenyl | Q-2.1 | Ent-1 | CH₃ | H | H | H | -CH₂- |
| I-067 | 2-fluorophenyl | Q-1.49 | Rac | H | H | H | H | -CH₂- |
| I-068 | 2,4-difluorophenyl | Q-1.1 | Rac | H | H | H | H | -CH₂- |
| I-069 | 2,5-difluorophenyl | Q-1.1 | Rac | H | H | H | H | -CH₂- |
| I-070 | 2-chlorophenyl | Q-1.4 | Rac | H | H | H | H | -CH₂- |
| I-071 | 2,5-difluorophenyl | Q-1.1 | Ent-1 | H | H | H | H | -CH₂- |
| I-072 | 2,5-difluorophenyl | Q-1.1 | Ent-2 | H | H | H | H | -CH₂- |
| I-073 | 2-chloro-6-fluorophenyl | Q-1.1 | Rac | H | H | H | H | -CH₂- |
| I-074 | 2-fluorophenyl | Q-1.52 | Rac | H | H | H | H | -CH₂- |
| I-075 | 2-fluorophenyl | Q-1.51 | Rac | H | H | H | H | -CH₂- |
| I-076 | 2-fluorophenyl | Q-1.50 | Rac | H | H | H | H | -CH₂- |
| I-077 | 2,3-difluorophenyl | Q-1.1 | Rac | H | H | H | H | -CH₂- |
| I-078 | 2-methylphenyl | Q-1.1 | Rac | H | H | H | H | -CH₂- |
| I-079 | 2,4-difluorophenyl | Q-1.1 | Ent-1 | H | H | H | H | -CH₂- |
| I-080 | 2,4-difluorophenyl | Q-1.1 | Ent-2 | H | H | H | H | -CH₂- |
| I-081 | 2,6-difluorophenyl | Q-1.1 | Rac | H | H | H | H | -CH₂- |
| I-082 | 2,6-difluorophenyl | Q-1.1 | Ent-1 | H | H | H | H | -CH₂- |
| I-083 | 2,6-difluorophenyl | Q-1.1 | Ent-2 | H | H | H | H | -CH₂- |
| I-084 | 2-methylphenyl | Q-1.1 | Ent-1 | H | H | H | H | -CH₂- |
| I-085 | 2-methylphenyl | Q-1.1 | Ent-2 | H | H | H | H | -CH₂- |
| I-086 | 2-chloro-6-fluorophenyl | Q-1.1 | Ent-1 | H | H | H | H | -CH₂- |
| I-087 | 2-chloro-6-fluorophenyl | Q-1.1 | Ent-2 | H | H | H | H | -CH₂- |
| I-088 | 2,3-difluorophenyl | Q-1.1 | Ent-1 | H | H | H | H | -CH₂- |
| I-089 | 2,3-difluorophenyl | Q-1.1 | Ent-2 | H | H | H | H | -CH₂- |
| I-090 | 2-fluorophenyl | Q-1.54 | Rac | H | H | H | H | -CH₂- |
| I-091 | 2-fluorophenyl | Q-1.55 | Rac | H | H | H | H | -CH₂- |
| I-092 | 2-fluorophenyl | Q-1.56 | Rac | H | H | H | H | -CH₂- |
| I-093 | 2-fluorophenyl | Q-1.59 | Rac | H | H | H | H | -CH₂- |
| I-094 | 2-fluorophenyl | Q-1.60 | Rac | H | H | H | H | -CH₂- |
| I-095 | 2-fluorophenyl | Q-1.61 | Rac | H | H | H | H | -CH₂- |
| I-096 | 2,4-difluorophenyl | Q-1.3 | Rac | H | H | H | H | -CH₂- |
| I-097 | 2,5-difluorophenyl | Q-1.3 | Rac | H | H | H | H | -CH₂- |
| I-098 | 2-fluorophenyl | Q-1.71 | Rac | H | H | H | H | -CH₂- |
| I-099 | 2-fluorophenyl | Q-1.65 | Rac | H | H | H | H | -CH₂- |
| I-100 | 2-fluorophenyl | Q-1.62 | Rac | H | H | H | H | -CH₂- |
| I-101 | 2-fluorophenyl | Q-1.72 | Rac | H | H | H | H | -CH₂- |
| I-102 | 2-fluorophenyl | Q-1.73 | Rac | H | H | H | H | -CH₂- |
| I-103 | 2-fluorophenyl | Q-1.63 | Rac | H | H | H | H | -CH₂- |
| I-104 | 2-fluorophenyl | Q-1.8 | Rac | H | H | H | H | -CH₂- |
| I-105 | 2-fluorophenyl | Q-1.4 | Rac | H | H | H | H | direct bond |
| I-106 | 2-chlorophenyl | Q-1.4 | Rac | H | H | H | H | direct bond |
| I-107 | 2-chloro-6-fluorophenyl | Q-1.35 | Rac | H | H | H | H | direct bond |
| I-108 | 2-fluorophenyl | Q-1.35 | Rac | H | H | H | H | direct bond |
| I-109 | 2-chlorophenyl | Q-1.35 | Rac | H | H | H | H | direct bond |
| I-110 | 2-chloro-6-fluorophenyl | Q-1.4 | Rac | H | H | H | H | direct bond |
| I-111 | 2-fluorophenyl | Q-1.2 | Rac | H | H | H | H | direct bond |
| I-112 | 2-fluorophenyl | Q-1.1 | Rac | H | H | H | H | direct bond |
| I-113 | 2-chlorophenyl | Q-1.1 | Rac | H | H | H | H | direct bond |
| I-114 | 2-chloro-6-fluorophenyl | Q-1.1 | Rac | H | H | H | H | direct bond |
| I-115 | 2-chloro-6-fluorophenyl | Q-1.70 | Rac | H | H | H | H | direct bond |
| I-116 | 2-chloro-6-fluorophenyl | Q-1.10 | Rac | H | H | H | H | direct bond |
| I-117 | 2-fluorophenyl | Q-1.10 | Rac | H | H | H | H | direct bond |
| I-118 | 2-fluorophenyl | Q-1.70 | Rac | H | H | H | H | direct bond |
| I-119 | 2,4-difluorophenyl | Q-2.1 | Ent-2 | H | H | H | H | -CH₂- |
| I-120 | 2-fluorophenyl | Q-2.2 | Rac | H | H | H | H | -CH₂- |
| I-121 | 2-fluorophenyl | Q-2.6 | Ent-1 | H | H | H | H | -CH₂- |
| I-122 | 2-fluorophenyl | Q-2.6 | Ent-2 | H | H | H | H | -CH₂- |
| I-123 | 2-fluorophenyl | Q-2.7 | Rac | H | H | H | H | -CH₂- |
| I-124 | 2,6-difluorophenyl | Q-2.1 | Rac | H | H | H | H | -CH₂- |
| I-125 | 2,6-difluorophenyl | Q-2.1 | Ent-1 | H | H | H | H | -CH₂- |
| I-126 | 2,6-difluorophenyl | Q-2.1 | Ent-2 | H | H | H | H | -CH₂- |
| I-127 | 2-fluorophenyl | Q-2.36 | Ent-1 | H | H | H | H | -CH₂- |
| I-128 | 2-fluorophenyl | Q-2.36 | Ent-2 | H | H | H | H | -CH₂- |
| I-129 | 2-fluorophenyl | Q-2.36 | Rac | H | H | H | H | -CH₂- |
| I-130 | 2-fluorophenyl | Q-2.9 | Rac | H | H | H | H | -CH₂- |
| I-131 | 2-fluorophenyl | Q-2.8 | Ent-1 | H | H | H | H | -CH₂- |
| I-132 | 2-fluorophenyl | Q-2.8 | Ent-2 | H | H | H | H | -CH₂- |
| I-133 | 2-fluorophenyl | Q-2.8 | Rac | H | H | H | H | -CH₂- |
| I-134 | 2-fluorophenyl | Q-2.10 | Rac | H | H | H | H | -CH₂- |
| I-135 | 2,3-difluorophenyl | Q-2.1 | Rac | H | H | H | H | -CH₂- |
| I-136 | 2,3-difluorophenyl | Q-2.1 | Ent-1 | H | H | H | H | -CH₂- |
| I-137 | 2,3-difluorophenyl | Q-2.1 | Ent-2 | H | H | H | H | -CH₂- |
| I-138 | 2-methylphenyl | Q-2.1 | Rac | H | H | H | H | -CH₂- |
| I-139 | 2-methylphenyl | Q-2.1 | Ent-1 | H | H | H | H | -CH₂- |
| I-140 | 2-methylphenyl | Q-2.1 | Ent-2 | H | H | H | H | -CH₂- |
| I-141 | 2-fluorophenyl | Q-2.11 | Rac | H | H | H | H | -CH₂- |
| I-142 | 2-fluorophenyl | Q-2.2 | Ent-2 | H | H | H | H | -CH₂- |
| I-143 | 2-fluorophenyl | Q-2.2 | Ent-1 | H | H | H | H | -CH₂- |
| I-144 | 2-fluorophenyl | Q-2.10 | Ent-2 | H | H | H | H | -CH₂- |
| I-145 | 2-fluorophenyl | Q-2.12 | Rac | H | H | H | H | -CH₂- |
| I-146 | 2,5-difluorophenyl | Q-2.13 | Rac | H | H | H | H | -CH₂- |
| I-147 | 2,4-difluorophenyl | Q-2.13 | Rac | H | H | H | H | -CH₂- |
| I-148 | 2,4-difluorophenyl | Q-2.15 | Rac | H | H | H | H | -CH₂- |
| I-149 | 2-fluorophenyl | Q-2.13 | Rac | H | H | H | H | -CH₂- |
| I-150 | 2-fluorophenyl | Q-2.15 | Rac | H | H | H | H | -CH₂- |
| I-151 | 2-chloro-6-fluorophenyl | Q-2.1 | Rac | H | H | H | H | -CH₂- |
| I-152 | 2,6-dichlorophenyl | Q-2.1 | Rac | H | H | H | H | -CH₂- |
| I-153 | 2-chloro-6-fluorophenyl | Q-2.12 | Rac | H | H | H | H | -CH₂- |
| I-154 | 2-chloro-6-fluorophenyl | Q-2.13 | Rac | H | H | H | H | -CH₂- |
| I-155 | 2-chloro-6-fluorophenyl | Q-2.15 | Rac | H | H | H | H | -CH₂- |
| I-156 | 2-fluorophenyl | Q-2.1 | Ent-1 | H | H | H | H | -CH₂- |
| I-157 | 2-fluorophenyl | Q-2.1 | Ent-2 | H | H | H | H | -CH₂- |
| I-158 | 2,4-difluorophenyl | Q-2.1 | Rac | H | H | H | H | -CH₂- |
| I-159 | 2-fluoro-4-chlorophenyl | Q-2.1 | Rac | H | H | H | H | -CH₂- |
| I-160 | 2-fluorophenyl | Q-2.5 | Rac | H | H | H | H | -CH₂- |
| I-161 | 2-fluorophenyl | Q-2.16 | Rac | H | H | H | H | -CH₂- |
| I-162 | 2-fluorophenyl | Q-2.17 | Rac | H | H | H | H | -CH₂- |
| I-163 | 2-fluorophenyl | Q-2.6 | Rac | H | H | H | H | -CH₂- |
| I-164 | 2-chlorophenyl | Q-2.6 | Rac | H | H | H | H | -CH₂- |
| I-165 | 2-fluorophenyl | Q-2.18 | Rac | H | H | H | H | -CH₂- |
| I-166 | 2,4-dichlorophenyl | Q-2.1 | Rac | H | H | H | H | -CH₂- |
| I-167 | 2,5-difluorophenyl | Q-2.12 | Rac | H | H | H | H | -CH₂- |
| I-168 | 2,5-difluorophenyl | Q-2.15 | Rac | H | H | H | H | -CH₂- |
| I-169 | 2,4-difluorophenyl | Q-2.12 | Rac | H | H | H | H | -CH₂- |
| I-170 | 2-chlorophenyl | Q-2.1 | Rac | H | H | H | H | -CH₂- |
| I-171 | 2-fluorophenyl | Q-2.1 | Rac | H | H | H | H | -CH₂- |
| I-172 | 2-fluorophenyl | Q-2.20 | Rac | H | H | H | H | -CH₂- |
| I-173 | 2-fluorophenyl | Q-2.21 | Rac | H | H | H | H | -CH₂- |
| I-174 | 2-fluorophenyl | Q-2.25 | Rac | H | H | H | H | -CH₂- |
| I-175 | 2-fluorophenyl | Q-2.26 | Rac | H | H | H | H | -CH₂- |
| I-176 | 2-fluorophenyl | Q-2.27 | Rac | H | H | H | H | -CH₂- |
| I-177 | 2-fluorophenyl | Q-2.29 | Rac | H | H | H | H | -CH₂- |
| I-178 | 2-fluorophenyl | Q-2.28 | Rac | H | H | H | H | -CH₂- |
| I-179 | 2-fluorophenyl | Q-2.31 | Rac | H | H | H | H | -CH₂- |
| I-180 | 2-fluorophenyl | Q-2.32 | Rac | H | H | H | H | -CH₂- |
| I-181 | 2-fluorophenyl | Q-2.33 | Rac | H | H | H | H | -CH₂- |
| I-182 | 2-fluorophenyl | Q-2.34 | Rac | H | H | H | H | -CH₂- |
| I-183 | 2-fluorophenyl | Q-2.34 | Ent-1 | H | H | H | H | -CH₂- |
| I-184 | 2-fluorophenyl | Q-1.21 | Ent-2 | H | H | OH | H | -CH₂- |
| I-185 | 2-fluorophenyl | Q-2.21 | Ent-1 | H | H | H | H | -CH₂- |
| I-186 | 2,5-difluorophenyl | Q-2.1 | Rac | H | H | H | H | -CH₂- |
| I-187 | 2,5-difluorophenyl | Q-2.1 | Ent-1 | H | H | H | H | -CH₂- |
| I-188 | 2,5-difluorophenyl | Q-2.1 | Ent-2 | H | H | H | H | -CH₂- |
| I-189 | 2-chloro-6-fluorophenyl | Q-2.1 | Ent-1 | H | H | H | H | -CH₂- |
| I-190 | 2-chloro-6-fluorophenyl | Q-2.1 | Ent-2 | H | H | H | H | -CH₂- |
| I-191 | 2,4-difluorophenyl | Q-2.1 | Ent-1 | H | H | H | H | -CH₂- |
| I-192 | 2-fluorophenyl | Q-1.1 | Ent-1 | H | H | H | H | direct bond |
| I-193 | 2-fluorophenyl | Q-1.1 | Ent-2 | H | H | H | H | direct bond |
| I-194 | 2-chlorophenyl | Q-1.1 | Ent-2 | H | H | H | H | direct bond |
| I-195 | 2-chlorophenyl | Q-1.1 | Ent-1 | H | H | H | H | direct bond |
| I-196 | 2-fluorophenyl | Q-2.1 | Rac | H | H | H | H | direct bond |
| I-197 | 2-chlorophenyl | Q-2.1 | Rac | H | H | H | H | direct bond |
| I-198 | 2-chloro-6-fluorophenyl | Q-2.1 | Rac | H | H | H | H | direct bond |
| I-199 | 2-fluorophenyl | Q-2.5 | Rac | H | H | H | H | direct bond |
| I-200 | 2-chlorophenyl | Q-2.5 | Rac | H | H | H | H | direct bond |
| I-201 | 2-fluorophenyl | Q-1.1 | Rac | H | H | H | H | -CH₂-CH₂- |
| I-202 | 2-fluorophenyl | Q-1.1 | Ent-1 | H | H | H | H | -CH₂-CH₂- |
| I-203 | 2-fluorophenyl | Q-1.1 | Ent-2 | H | H | H | H | -CH₂-CH₂- |
| I-204 | 2-fluorophenyl | Q-2.10 | Rac | H | H | H | H | direct bond |
| I-205 | 2-fluorophenyl | Q-2.12 | Rac | H | H | H | H | direct bond |

Spectroscopic data of selected table examples:
The spectroscopic data listed hereinafter for selected table examples were evaluated via conventional ¹H-NMR interpretation or via NMR peak list methods.
a) Conventional ¹H-NMR interpretation
   I-010: ¹H-NMR (400.0 MHz, CDCl₃): δ_{H} = 7.51 (s, 1H), 7.28-7.24 (m, 3H), 7.04-6.87 (m, 5H), 5.40-5.20 (m, 2H), 4.50 (m, 1H), 3.94 (s, 3H), 3.60-3.50 (m, 1H), 3.40-3.30 (m, 1H), 3.20-3.10 (m, 1H), 2.90-2.80 (m, 1H), 1.90-1.40 (m, 4H)
   I-018: ¹H-NMR (400.6 MHz, CDCl₃): δ_{H} = 7.92 (s, 1H), 7.50 (m, 1H), 7.21-7.17 (m, 1H), 7.08-6.99 (m, 2H), 4.75-4.53 (m, 2H), 3.91 (s, 3H), 3.57 (m, 1H), 3.47 (s, 3H), 3.22 (m, 1H), 2.93 (m, 1H), 2.08-1.68 (m, 6H)
   I-019: ¹H-NMR (400.6 MHz, CDCl₃): δ_{H} = 7.99 (m, 1H), 7.64 (s, 1H), 7.53 (m, 1H), 7.25 (m, 1H), 7.14 (m, 1H), 5.49 (br s, 1H), 4.76 (d, J 12 Hz, 1H), 4.64 (d, J 12 Hz, 1H), 3.92 (s, 3H), 3.87-3.81 (m, 1H), 3.78-3.74 (m, 1H), 3.45 (s, 3H), 2.43-2.41 (m, 1H), 2.09-2.02 (m, 3H)
   I-124: ¹H-NMR (400.0 MHz, CDCl₃): δ_{H} = 7.57 (s, 1H), 7.16-7.10 (m, 1H), 6.88-6.84 (m, 2H), 4.85-4.66 (m, 2H), 3.91 (s, 3H), 3.60-3.50 (m, 1H), 3.37 (m, 3H), 3.30-3.20 (m, 1H), 3.00-2.90 (m, 1H), 2.80-2.70 (m, 1H), 1.95-1.70 (m, 4H).
   I-125: ¹H-NMR (400.0 MHz, CDCl₃): δ_{H} = 7.58 (s, 1H), 7.16-7.14 (m, 1H), 6.87-6.84 (m, 2H), 4.69-4.66 (m, 2H), 3.91 (s, 3H), 3.70-3.50 (m, 2H), 3.37 (m, 3H), 3.30-3.20 (m, 1H), 3.00-2.90 (m, 1H), 1.93-1.70 (m, 4H).
   I-126: ¹H-NMR (400.0 MHz, CDCl₃): δ_{H} = 7.58 (s, 1H), 7.17-7.14 (m, 1H), 6.87-6.84 (m, 2H), 4.82-4.66 (m, 2H), 3.91 (s, 3H), 3.63-3.56 (m, 2H), 3.37 (m, 3H), 3.30-3.20 (m, 1H), 3.00-2.90 (m, 1H), 1.93-1.73 (m, 4H).
   I-129: ¹H-NMR (400.6 MHz, CDCl₃): δ_{H} = 7.56 (s, 1H), 7.31 (m, 1H), 7.20-7.18 (m, 1H), 7.08-7.00 (m, 2H), 5.41 (d, J 16.0 Hz, 1H), 5.22 (d, J 12.0 Hz, 1H), 4.54 (br s, 1H), 3.94 (s, 3H), 3.70-3.60 (m, 1H), 3.50-3.40 (m, 1H), 3.30-3.23 (m, 1H), 3.00-2.90 (m, 1H), 1.93-1.63 (m, 10H)
   I-134: ¹H-NMR (400.6 MHz, CDCl₃): δ_{H} = 7.56 (s, 1H), 7.28-7.26 (m, 1H), 7.20-7.19 (m, 1H), 7.09-7.00 (m, 2H), 6.00-5.85 (m, 1H), 5.31-5.19 (m, 2H), 4.93-4.77 (m, 2H), 4.53 (br s, 1H), 4.06 (br s, 2H), 3.93 (s, 3H), 3.64-3.60 (m, 1H), 3.50-3.40 (m, 1H), 3.30-3.21 (m, 1H), 3.00-2.90 (m, 1H), 1.95-1.58 (m, 3H)
   I-149: ¹H-NMR (400.6 MHz, CDCl₃): δ_{H} = 7.99 (s, 1H), 7.63 (s, 1H), 7.26-7.29 (m, 1H), 7.20-7.18 (m, 2H), 7.07-7.03 (m, 2H), 4.87-4.84 (m, 1H), 4.73-4.67 (m, 2H), 4.60-4.50 (m, 1H), 3.70-3.60 (m, 1H), 3.87 (s, 3H), 3.30-3.20 (m, 1H), 3.00-2.90 (m, 1H), 2.75-2.70 (m, 1H), 1.94-1.73 (m, 3H), 1.51 (s, 3H), 1.50 (s, 3H)
   I-150: ¹H-NMR (400.6 MHz, CDCl₃): δ_{H} = 7.95 (s, 1H), 7.61 (s, 1H), 7.31-7.27 (m, 1H), 7.22-7.17 (m, 1H), 7.09-7.00 (m, 2H), 4.89-4.86 (m, 1H), 4.75-4.72 (m, 1H), 4.54 (br s, 1H), 4.07-4.04 (m, 2H), 3.76-3.63 (m, 1H), 3.48-3.44 (m, 1H), 3.39 (s, 3H), 3.26-3.22 (m, 1H), 2.99-2.94 (m, 1H), 1.96-1.73 (m, 3H), 1.38-1.26 (m, 1H), 0.62-0.57 (m, 2H), 0.44-0.41 (m, 2H).
   I-159: ¹H-NMR (400.6 MHz, CDCl₃): δ = 7.56 (s, 1H), 7.26-7.20 (m, 1H), 7.26-7.20 (m, 1H), 6.81-6.79 (m, 2H), 4.83-4.73 (m, 2H), 4.50-4.40 (m, 1H), 3.92 (s, 3H), 3.70-3.60 (m, 1H), 3.50-3.40 (m, 4H), 3.20-3.10 (s, 1H), 2.95-2.85 (m, 1H), 1.95-1.60 (m, 4H).
   I-167: ¹H-NMR (400.6 MHz, CDCl₃): δ= 7.61 (s, 1H), 7.01-6.87 (m, 3H), 4.86-4.73 (m, 2H), 4.55-4.45 (m, 1H), 4.23 (q, J 8.0 Hz, 2H), 3.66-3.50 (m, 2H), 3.40 (s, 3H), 3.25-3.15 (m, 1H), 2.95-2.85 (m, 1H), 1.97-1.93 (m, 4H), 1.47 (t, *J* 8.0 Hz, 3H).
   I-168: ¹H-NMR (400.6 MHz, CDCl₃): δ= 7.63 (s, 1H), 7.01-6.96 (m, 2H), 6.89-6.85 (m, 1H), 4.87-4.71 (m, 2H), 4.51 (br s, 1H), 4.08-4.05 (m, 2H), 3.70-3.66 (m, 2H), 3.39 (s, 3H), 3.25-3.22 (m, 1H), 2.94-2.88 (m, 1H), 1.98-1.67 (m, 4H), 1.38-1.31 (m, 1H), 0.62-0.57 (m, 2H), 0.44-0.40 (m, 2H).
   I-197: ¹H-NMR (400.0 MHz, CDCl₃):δ = 7.55-7.50 (m, 1H), 7.39-7.35 (m, 1H), 7.20-7.17 (m, 3H), 4.97-4.95 (d, 1H), 4.96-4.86 (br. m, 1H), 4.83-4.81 (d, 1H), 4.18-4.08 (m, 2H), 3.92 (s, 3H), 3.60-3.51 (br. m, 1H), 3.40 (s, 3H), 3.28-3.19 (br. m, 1H), 2.38-2.29 (m, 1H), 2.14-2.07 (m, 1H).
   I-200: ¹H-NMR (400.0 MHz, CDCl₃):
      δ = 7.55-7.49 (m, 1H), 7.38-7.35 (m, 1H), 7.20-7.16 (m, 3H), 5.00-4.96 (d, 1H), 4.92-4.84 (m, 2H), 4.14-4.08 (m, 2H), 3.93 (s, 3H), 3.57-3.50 (br. m, 1H), 3.30-3.24 (m, 2H), 3.25-3.19 (br. m, 1H), 2.37-2.29 (m, 1H), 2.12-2.05 (m, 1H), 1.93-1.84 (sept, 1H), 0.91-0.88 (d, 6H).
   I-204: ¹H-NMR (400.0 MHz, CDCl₃):
      δ = 7.54-7.46 (br. s, 1H), 7.26-7.19 (m, 2H), 7.09-7.03 (m, 2H), 5.96-5.89 (m, 1H), 5.32-5.28 (m, 1H), 5.22-5.19 (m, 1H), 5.02-4.99 (d, 1H), 4.90-4.87 (d, 1H), 4.86-4.79 (m, 1H), 4.11-4.04 (m, 4H), 3.94 (s, 3H), 3.42-3.34 (br. m, 1H), 3.20-3.12 (br. m, 1H), 2.39-2.29 (br. m, 1H), 2.10-2.01 (br. m, 1H).
   I-205: ¹H-NMR (400.0 MHz, CDCl₃):
      δ = 7.58-7.51 (br. s, 1H), 7.25-7.19 (m, 2H), 7.09-7.03 (m, 2H), 4.97-4.94 (d, 1H), 4.86-4.79 (m, 1H), 4.83-4.80 (d, 1H), 4.26-4.21 (q, 2H), 4.11-4.07 (m, 2H), 3.40 (s, 3H), 3.39-3.34 (br. m, 1H), 3.20-3.12 (br. m, 1H), 2.38-2.29 (br. m, 1H), 2.10-2.01 (br. m, 1H), 1.48-1.44 (t, 3H).
b) NMR peak list method
   ¹H-NMR data of selected examples are written in form of ¹H-NMR-peak lists. To each signal peak are listed the δ-value in ppm and the signal intensity in round brackets. Between the δ-value - signal intensity pairs are semicolons as delimiters. The peak list of an example has therefore the form:
      δ ₁ (intensity,); δ₂ (intensity₂);........; δᵢ (intensityᵢ);......; 8ₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown. For calibrating chemical shift for ¹H spectra, we use tetramethylsilane and/or the chemical shift of the solvent used, especially in the case of spectra measured in DMSO. Therefore, in NMR peak lists, tetramethylsilane peak can occur but not necessarily. The ¹H-NMR peak lists are similar to classical ¹H-NMR prints and contains therefore usually all peaks, which are listed at classical NMR-interpretation. Additionally, they can show like classical ¹H-NMR prints signals of solvents, stereoisomers of the target compounds, which are also object of the invention, and/or peaks of impurities. To show compound signals in the delta-range of solvents and/or water the usual peaks of solvents, for example peaks of DMSO in DMSO-D₆ and the peak of water are shown in our ¹H-NMR peak lists and have usually on average a high intensity. The peaks of stereoisomers of the target compounds and/or peaks of impurities have usually on average a lower intensity than the peaks of target compounds (for example with a purity >90%). Such stereoisomers and/or impurities can be typical for the specific preparation process. Therefore, their peaks can help to recognize the reproduction of our preparation process via "side-products-fingerprints". An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking done in the course of a classical ¹H-NMR interpretation. Further details of NMR-data description with peak lists can be found in the publication "Citation of NMR Peaklist Data within Patent Applications" of the Research Disclosure Database Number 564025.

| |
|---|
| I-001: ¹H-NMR(400.0 MHz, CDCl₃): |
| δ= 7.4997 (0.9); 7.2665 (6.3); 7.0024 (0.5); 6.9802 (0.6); 6.9688 (0.6); 6.9052 (0.5); 6.8960 (0.7); 6.8867 (0.8); 6.8762 (0.8); 6.8683 (0.5); 3.9129 (16.0); 3.4681 (4.0); 1.9393 (0.6); 1.9262 (0.6); 1.9094 (0.6); - 0.0002 (7.8) |
| I-002: ¹H-NMR(400.6 MHz, CDCl₃): |
| δ= 7.4967 (1.0); 7.3050 (0.6); 7.2986 (0.5); 7.2653 (16.8); 7.2255 (0.6); 7.2123 (0.7); 7.2059 (1.3); 7.2027 (1.2); 7.1922 (1.2); 7.1875 (1.4); 7.1824 (1.0); 7.1735 (0.8); 7.1692 (0.8); 7.0836 (1.2); 7.0653 (2.0); 7.0461 (1.2); 7.0152 (1.1); 7.0015 (0.6); 6.9942 (0.7); 4.7479 (0.7); 4.6925 (0.8); 4.6649 (0.6); 4.5320 (0.6); 4.1304 (0.5); 4.1126 (0.5); 3.9081 (16.0); 3.5735 (0.6); 3.4701 (4.7); 2.0449 (2.6); 1.9259 (0.7); 1.9079 (1.0); 1.8911 (1.1); 1.7543 (1.0); 1.6429 (2.4); 1.2771 (0.8); 1.2592 (1.9); 1.2561 (1.8); 1.2414 (0.8); 0.0080 (0.6); -0.0002 (25.0); -0.0085 (0.7) |
| I-003: ¹H-NMR(400.6 MHz, CDCl₃): |
| δ= 7.4972 (1.0); 7.3051 (0.6); 7.2658 (11.4); 7.2253 (0.6); 7.2209 (0.5); 7.2120 (0.7); 7.2058 (1.3); 7.1881 (1.4); 7.1825 (1.0); 7.1733 (0.8); 7.1692 (0.8); 7.0839 (1.3); 7.0655 (2.0); 7.0461 (1.3); 7.0155 (1.2); 7.0022 (0.7); 6.9940 (0.8); 5.2993 (4.0); 4.7499 (0.8); 4.6919 (0.9); 4.6633 (0.6); 4.5432 (0.7); 4.5302 (0.6); 3.9081 (16.0); 3.5754 (0.6); 3.4701 (5.1); 3.4128 (0.9); 3.3992 (0.7); 3.2167 (0.5); 1.9259 (0.8); 1.9082 (1.1); 1.8900 (1.2); 1.7560 (1.1); 1.6605 (1.6); -0.0002 (16.6); -0.0085 (0.5) |
| I-004: ¹H-NMR(400.0 MHz, CDCl₃): |
| δ= 7.4951 (1.8); 7.2644 (9.3); 7.1826 (0.6); 7.1523 (1.0); 7.1377 (1.0); 7.1321 (0.8); 7.1178 (0.8); 7.0981 (1.2); 7.0795 (0.6); 7.0704 (3.6); 6.9325 (0.5); 6.9293 (0.5); 6.9094 (0.9); 4.0943 (1.7); 4.0757 (0.6); 4.0387 (0.8); 3.9834 (5.5); 3.9132 (16.0); 3.8876 (13.8); 3.8049 (0.6); 3.7136 (0.6); 2.8133 (0.7); 2.8080 (0.9); 2.8006 (0.7); 2.7928 (0.8); 2.7881 (1.2); 2.7827 (0.7); 2.1279 (2.0); 1.9363 (0.6); 1.4300 (0.8); 1.4158 (0.8); 1.2559 (0.9); -0.0002 (12.2) |
| I-005: ¹H-NMR(400.6 MHz, CDCl₃): |
| δ= 7.4708 (1.0); 7.3159 (0.8); 7.2978 (0.5); 7.2703 (7.8); 7.2228 (0.8); 7.2183 (0.7); 7.2097 (0.9); 7.2044 (1.6); 7.1997 (1.5); 7.1896 (1.4); 7.1845 (1.9); 7.1795 (1.4); 7.1709 (1.1); 7.1663 (1.3); 7.0820 (0.8); 7.0675 (1.3); 7.0472 (1.0); 7.0166 (1.0); 6.9988 (0.7); 4.7386 (0.7); 4.6472 (0.9); 4.6183 (0.8); 4.5256 (0.6); 3.8930 (16.0); 3.5502 (0.6); 3.3990 (0.6); 3.3098 (2.7); 3.2939 (3.0); 3.2557 (0.6); 3.2215 (0.6); 2.9281 (0.5); 2.0544 (0.7); 2.0439 (0.9); 1.9182 (1.3); 1.9005 (1.6); 1.8870 (1.6); 1.8643 (1.2); 1.8308 (0.8); 1.8180 (0.7); 1.7471 (1.4); 1.2848 (0.8); 1.2763 (0.6); 1.2562 (3.3); 0.8887 (7.9); 0.8796 (7.4); 0.8737 (8.4); -0.0002 (10.1) |
| I-006: ¹H-NMR(400.6 MHz, CDCl₃): |
| δ= 7.5193 (0.6); 7.5105 (0.8); 7.2937 (0.6); 7.2810 (1.0); 7.2755 (2.1); 7.2674 (1.0); 7.2666 (1.0); 7.2610 (22.4); 7.2582 (3.4); 7.2574 (3.4); 7.2567 (3.4); 7.2559 (3.3); 7.2551 (3.3); 7.2544 (3.3); 7.2408 (1.3); 7.2357 (2.3); 7.2178 (0.5); 7.1985 (1.0); 7.1807 (1.0); 7.1653 (0.6); 7.0660 (1.2); 7.0471 (2.8); 7.0295 (2.6); 7.0130 (1.7); 6.9973 (1.0); 6.9948 (0.9); 6.9879 (0.9); 6.9759 (0.6); 6.9730 (0.6); 6.9444 (1.3); 6.9261 (2.1); 6.9078 (1.0); 5.3552 (0.6); 5.2819 (0.6); 3.9470 (1.6); 3.9283 (16.0); 3.9007 (0.8); 1.8808 (0.6); 1.8152 (0.6); 1.7590 (0.9); 1.7474 (1.3); 1.7363 (1.1); 1.7281 (1.2); 1.7165 (0.9); 1.7015 (0.5); 1.2558 (1.2); 0.0080 (0.9); -0.0002 (34.8); -0.0085 (1.0) |
| I-007: ¹H-NMR(400.6 MHz, CDCl₃): |
| δ= 7.4601 (0.8); 7.2657 (10.6); 6.8118 (0.9); 6.7839 (0.8); 4.7291 (0.5); 4.6415 (0.6); 3.8971 (16.0); 3.3079 (2.1); 3.2912 (2.2); 2.0703 (0.5); 2.0452 (0.7); 1.9316 (0.9); 1.9139 (1.1); 1.9008 (1.0); 1.8847 (0.9); 1.7196 (0.7); 1.2847 (0.7); 1.2562 (2.6); 0.8894 (5.4); 0.8738 (5.7); -0.0002 (14.4) |
| I-008: ¹H-NMR(400.6 MHz, CDCl₃): |
| δ= 7.5033 (0.8); 7.2772 (1.1); 7.2637 (4.3); 7.2586 (2.0); 7.2561 (2.0); 7.2373 (1.5); 7.0386 (0.9); 7.0196 (0.8); 6.9471 (0.8); 6.9288 (1.3); 6.9105 (0.6); 6.7857 (0.9); 6.7662 (0.9); 3.9283 (16.0); 1.7068 (0.5); 1.6919 (0.6); 1.2565 (1.3); -0.0002 (5.2) |
| I-009: ¹H-NMR(400.6 MHz, CDCl₃): |
| δ= 7.9435 (0.5); 7.4753 (0.6); 7.2713 (3.7); 6.8946 (0.6); 6.8853 (0.7); 6.8749 (0.7); 4.7516 (1.7); 3.9006 (16.0); 3.8942 (2.8); 3.4113 (1.3); 3.3945 (1.3); 3.3107 (1.6); 3.2938 (1.6); 1.9358 (0.6); 1.9311 (0.6); 1.9179 (0.7); 1.9017 (0.6); 1.2568 (1.6); 0.9438 (3.3); 0.9271 (3.3); 0.8903 (3.7); 0.8744 (3.8); -0.0002 (5.2) |
| 1-011: ¹H-NMR(400.6 MHz, CDCl₃): |
| δ= 7.9426 (0.5); 7.4330 (1.4); 7.3164 (1.0); 7.2990 (0.9); 7.2710 (16.9); 7.2210 (1.0); 7.2180 (1.0); 7.2005 (2.4); 7.1847 (2.6); 7.1664 (1.4); 7.0819 (1.5); 7.0638 (2.2); 7.0443 (1.7); 7.0131 (1.8); 6.9919 (1.2); 5.2659 (0.6); 5.2365 (0.5); 5.2188 (0.7); 5.2012 (0.7); 4.5093 (0.8); 4.1208 (0.6); 4.1033 (0.6); 4.0888 (0.9); 4.0711 (1.5); 4.0532 (1.4); 4.0369 (0.7); 3.9460 (0.9); 3.8774 (16.0); 3.5286 (0.8); 3.4774 (0.5); 3.4753 (0.6); 3.3744 (0.9); 3.2304 (0.8); 2.9361 (0.7); 2.9237 (0.8); 2.9117 (0.8); 2.4094 (0.6); 2.3928 (0.7); 2.3779 (0.7); 2.3622 (0.6); 2.3457 (0.6); 2.3055 (0.9); 2.2867 (0.7); 2.1064 (2.2); 2.0911 (2.7); 2.0771 (2.5); 2.0655 (2.3); 2.0611 (2.3); 2.0534 (1.6); 2.0489 (1.4); 2.0281 (1.0); 1.9728 (1.9); 1.9565 (1.7); 1.9195 (1.6); 1.9013 (1.9); 1.8873 (1.9); 1.8696 (1.7); 1.7673 (2.0); 1.7492 (2.1); 1.7381 (2.1); 1.3341 (0.5); 1.2847 (0.9); 1.2563 (4.4); 0.8799 (0.6); 0.0715 (0.9); 0.0014 (6.4); -0.0002 (12.1); -0.0029 (3.3) |
| I-013: ¹H-NMR(400.6 MHz, CDCl₃): |
| δ= 7.9997 (1.1); 7.4571 (0.5); 7.2672 (5.4); 7.2005 (0.8); 7.1967 (0.7); 7.1819 (0.9); 7.1765 (0.6); 7.0693 (0.5); 7.0447 (0.5); 7.0192 (0.6); 4.7353 (2.8); 4.6150 (0.5); 4.1816 (1.0); 4.1634 (3.2); 4.1451 (3.2); 4.1390 (1.4); 4.1268 (1.1); 4.1206 (1.1); 1.8796 (0.5); 1.7627 (1.0); 1.7517 (0.8); 1.7441 (0.8); 1.7333 (0.8); 1.5094 (1.3); 1.5005 (7.4); 1.4910 (2.8); 1.4822 (16.0); 1.4727 (1.5); 1.4639 (7.3); 1.3663 (15.5); 1.3008 (0.8); 1.2701 (8.0); 1.2566 (5.3); -0.0002 (8.2) |
| I-014: ¹H-NMR(300.1 MHz, d₆-DMSO): |
| δ= 8.0309 (0.6); 7.2182 (0.7); 7.2130 (0.8); 7.1886 (1.7); 7.1615 (1.5); 6.9932 (0.4); 6.9411 (0.7); 6.9173 (0.6); 6.8898 (1.0); 6.8681 (1.1); 4.5452 (0.5); 4.4596 (0.6); 4.3929 (0.6); 3.8333 (16.0); 3.7711 (1.8); 3.6459 (0.6); 3.4977 (1.3); 3.4776 (2.5); 3.4537 (1.7); 3.3241 (46.6); 3.3005 (6.2); 3.2218 (3.4); 3.1594 (0.4); 3.0421 (0.4); 2.8087 (0.5); 2.7279 (0.6); 2.7222 (0.6); 2.7047 (0.6); 2.6888 (0.6); 2.6634 (0.5); 2.6412 (0.4); 2.6193 (0.4); 2.5984 (0.3); 2.5071 (30.9); 2.5013 (39.9); 2.4955 (28.4); 2.0742 (0.6); 1.8043 (0.6); 1.7539 (1.2); 1.7327 (1.2); 1.7145 (1.0); 1.6810 (0.8); 1.6570 (0.4); 1.6228 (0.7); 1.5988 (0.8); - 0.0004 (0.8) |
| I-015: ¹H-NMR(300.1 MHz, d₆-DMSO): |
| δ= 8.4912 (0.7); 8.3766 (1.6); 8.3621 (1.7); 8.3332 (0.8); 8.0472 (3.4); 7.6733 (0.7); 7.6424 (1.3); 7.6129 (0.8); 7.3687 (1.2); 7.3542 (1.8); 7.3401 (1.5); 7.3264 (1.3); 7.3116 (0.7); 4.5620 (1.3); 4.5299 (1.5); 4.4230 (1.7); 4.3891 (1.1); 3.8282 (16.0); 3.5318 (2.0); 3.5103 (2.5); 3.4956 (2.4); 3.3414 (6.1); 3.2219 (14.9); 3.1487 (0.4); 2.9046 (0.6); 2.8665 (0.9); 2.8298 (0.7); 2.5039 (9.2); 2.0775 (0.4); 1.8754 (1.6); 1.8543 (2.3); 1.8333 (2.4); 1.8173 (2.1); 1.6867 (0.8); 1.5847 (0.4); 1.2336 (1.3); -0.0006 (0.4) |
| I-016: ¹H-NMR(400.2 MHz, d₆-DMSO): |
| δ= 8.0572 (2.0); 7.5686 (2.1); 7.4650 (0.6); 7.4610 (0.5); 7.3716 (4.4); 5.7613 (1.3); 4.5632 (1.0); 4.5360 (1.4); 4.4320 (2.3); 4.4047 (1.5); 3.8282 (14.5); 3.5566 (1.0); 3.5233 (3.3); 3.5049 (4.1); 3.4868 (2.1); 3.4789 (1.4); 3.4604 (0.6); 3.3379 (58.2); 3.3142 (3.8); 3.2209 (16.0); 2.8308 (1.0); 2.6810 (0.3); 2.6764 (0.4); 2.6718 (0.3); 2.5164 (8.0); 2.5119 (16.5); 2.5073 (22.2); 2.5027 (15.8); 2.4982 (7.1); 1.9598 (0.3); 1.9149 (1.0); 1.8119 (2.5); 1.7992 (2.7); 1.7622 (1.0); 1.6377 (1.5); 1.6212 (1.7); 1.6117 (1.7) |
| I-017: ¹H-NMR(400.6 MHz, CDCl₃): |
| δ= 7.9375 (1.9); 7.5260 (2.3); 7.5224 (2.3); 7.3531 (2.9); 7.2822 (0.7); 7.2638 (55.0); 7.2356 (0.5); 7.2268 (0.6); 7.1933 (3.8); 7.1713 (9.6); 7.1661 (6.9); 7.1522 (8.2); 7.1475 (6.4); 7.1338 (2.9); 7.1291 (2.4); 7.0001 (0.6); 5.2997 (2.7); 4.7464 (1.8); 4.7327 (6.2); 4.6970 (2.0); 4.6020 (1.8); 3.9015 (16.0); 3.8478 (2.7); 3.7657 (1.2); 3.7205 (0.5); 3.5743 (1.9); 3.5206 (12.7); 3.4693 (9.9); 3.3880 (2.0); 2.9930 (1.4); 2.0837 (1.7); 2.0451 (1.4); 1.8546 (3.2); 1.8034 (4.2); 1.7838 (3.7); 1.7734 (3.2); 1.7578 (1.7); 1.6400 (0.7); 1.2771 (0.6); 1.2591 (1.4); 1.2414 (0.6); 0.8794 (0.5); 0.0079 (2.5); -0.0002 (82.9); -0.0085 (2.3) |
| I-020: ¹H-NMR(600.3 MHz, CDCl₃): |
| δ= 7.3513 (0.4); 7.3452 (1.6); 7.3317 (15.3); 7.3290 (24.5); 7.3254 (8.2); 7.3181 (1.8); 7.3167 (1.8); 7.3149 (1.1); 7.3128 (0.7); 7.2665 (9.6); 7.2549 (7.9); 4.7136 (7.1); 4.6936 (9.1); 4.5654 (5.4); 4.5453 (4.2); 4.4371 (0.9); 4.4324 (0.9); 4.4225 (1.7); 4.4121 (1.0); 4.4076 (0.9); 3.8852 (29.1); 3.4480 (0.3); 3.4401 (50.0); 3.4252 (1.0); 3.4195 (1.0); 3.4119 (1.1); 3.4077 (1.7); 3.4034 (1.2); 3.3960 (1.0); 3.3904 (0.9); 2.5565 (1.0); 2.5452 (1.2); 2.5405 (1.7); 2.5291 (1.8); 2.5245 (1.2); 2.5128 (1.6); 2.5035 (0.9); 2.4985 (0.9); 2.4897 (1.5); 2.4808 (0.9); 2.4760 (0.9); 2.4674 (0.8); 2.3169 (0.4); 2.3095 (0.4); 2.3035 (0.8); 2.2956 (1.2); 2.2885 (0.8); 2.2818 (1.3); 2.2740 (1.0); 2.2680 (0.5); 2.2605 (0.5); 2.0712 (0.6); 2.0589 (1.0); 2.0474 (1.0); 2.0438 (2.0); 2.0369 (1.0); 2.0332 (0.9); 2.0221 (0.9); 2.0099 (0.5); 1.7215 (0.8); 1.7145 (0.9); 1.7080 (0.9); 1.7001 (1.4); 1.6921 (1.0); 1.6858 (0.9); 1.6786 (0.9); 1.6574 (0.5); 1.5728 (0.6); 1.5672 (0.5); 1.5593 (1.0); 1.5537 (1.0); 1.5521 (1.0); 1.5459 (0.7); 1.5441 (0.7); 1.5387 (1.4); 1.5309 (0.3); 1.5252 (0.8); 1.4797 (0.3); 1.4731 (0.6); 1.4675 (0.9); 1.4612 (1.0); 1.4553 (1.0); 1.4481 (0.9); 1.4419 (0.8); 1.4350 (0.6); 1.4292 (0.4); 1.2708 (0.3); 1.2589 (0.9); 1.2566 (0.6); 1.2470 (0.3); 0.0053 (0.4); -0.0001 (12.1); -0.0057 (0.4) |
| I-021: ¹H-NMR(400.6 MHz, CDCl₃): |
| δ= 7.4736 (1.9); 7.3585 (0.6); 7.3417 (1.1); 7.3219 (0.7); 7.2642 (14.6); 7.2176 (0.6); 7.2124 (1.0); 7.2077 (0.9); 7.1927 (1.2); 7.1877 (0.8); 7.1794 (0.8); 7.1747 (0.8); 7.1339 (1.1); 7.1153 (1.4); 7.0967 (0.7); 7.0369 (1.0); 7.0122 (1.3); 6.9912 (1.0); 4.7885 (0.9); 4.7579 (1.4); 4.6979 (0.8); 4.6527 (1.6); 4.6221 (1.2); 3.9113 (16.0); 3.8813 (4.9); 3.6977 (0.6); 3.6801 (0.9); 3.6622 (0.6); 3.5156 (0.6); 3.4633 (11.0); 3.4202 (3.9); 3.1983 (0.7); 3.1830 (0.6); 1.8333 (0.8); 1.8234 (0.8); 1.8134 (0.8); 1.7134 (0.7); 1.6951 (1.1); 1.6793 (1.4); 1.6648 (1.6); 1.6465 (1.4); 1.6291 (0.6); 1.3534 (3.4); 1.3357 (3.4); 0.0078 (0.7); - 0.0002 (22.0); -0.0086 (0.7) |
| I-022: ¹H-NMR(400.6 MHz, CDCl₃): |
| δ= 7.5184 (2.8); 7.2873 (3.4); 7.2600 (484.4); 6.9964 (2.9); 4.7100 (2.4); 4.1474 (2.8); 4.1345 (2.6); 3.5232 (4.7); 3.4122 (8.4); 2.9363 (1.6); 2.9165 (2.6); 2.8971 (2.6); 2.6234 (2.8); 2.6053 (3.7); 2.5867 (2.8); 1.7357 (1.6); 1.5459 (16.0); 0.1461 (1.9); 0.0272 (4.4); 0.0145 (3.1); 0.0080 (21.0); -0.0002 (701.4); -0.0085 (20.9); -0.0365 (2.2); -0.1494 (2.1) |
| I-023: ¹H-NMR(400.6 MHz, CDCl₃): |
| δ= 7.7935 (2.2); 7.3305 (0.5); 7.3121 (0.6); 7.2629 (14.7); 7.1861 (0.7); 7.1658 (1.2); 7.1462 (1.0); 7.0996 (0.8); 7.0726 (0.9); 7.0517 (0.6); 5.4135 (1.7); 5.3820 (1.6); 5.0419 (0.7); 5.0261 (0.7); 4.8238 (1.0); 4.7931 (1.6); 4.6898 (1.6); 4.6589 (0.9); 3.9233 (1.1); 3.8628 (7.4); 3.8264 (0.6); 3.6481 (0.8); 3.6360 (0.7); 3.6175 (0.5); 3.4738 (16.0); 1.8917 (1.4); 1.8782 (1.3); 1.8488 (0.6); 1.6524 (0.6); 0.0080 (0.6); - 0.0002 (21.2); -0.0085 (0.7) |
| I-024: ¹H-NMR(400.1 MHz, d₆-DMSO): |
| δ= 8.2334 (2.1); 7.3786 (2.8); 7.3616 (8.4); 7.3431 (8.8); 7.3214 (4.6); 7.3038 (5.4); 7.2959 (2.8); 7.2706 (7.0); 7.2539 (5.3); 7.1506 (2.1); 7.1337 (2.1); 7.0717 (0.5); 7.0496 (0.4); 5.3221 (16.0); 4.5821 (0.9); 4.5515 (1.4); 4.4587 (1.5); 4.4317 (1.1); 4.3688 (1.2); 4.2973 (0.4); 4.2790 (0.4); 3.4978 (5.0); 3.3217 (65.2); 3.2164 (10.1); 3.1408 (1.0); 2.7605 (0.7); 2.7327 (1.0); 2.7193 (0.7); 2.7112 (0.7); 2.6707 (1.1); 2.6662 (0.9); 2.6202 (0.4); 2.5947 (0.4); 2.5238 (3.0); 2.5191 (4.4); 2.5058 (98.9); 2.5014 (135.5); 2.4971 (101.0); 2.3328 (0.6); 2.3282 (0.8); 2.3238 (0.6); 1.8251 (1.9); 1.8080 (3.0); 1.7954 (3.1); 1.7788 (2.6); 1.5954 (1.8); 1.5856 (1.7); 0.0081 (0.6); 0.0000 (17.4) |
| I-025: ¹H-NMR(300.1 MHz, d₆-DMSO): |
| δ= 8.1415 (2.9); 8.0682 (2.4); 7.2819 (1.1); 7.2602 (1.2); 7.1430 (1.5); 4.5915 (0.5); 4.5573 (0.9); 4.5383 (0.8); 4.5156 (1.3); 4.4933 (1.8); 4.4709 (2.0); 4.4499 (1.1); 4.4313 (0.8); 4.3511 (0.8); 3.5142 (2.4); 3.3425 (0.6); 3.2356 (4.8); 2.7103 (0.6); 2.6768 (0.5); 2.5101 (8.8); 2.5043 (11.3); 2.4985 (8.2); 2.0782 (2.4); 1.8028 (1.6); 1.7875 (1.8); 1.5970 (0.9); 1.4364 (16.0); 1.4142 (16.0); -0.0002 (2.2) |
| I-026: ¹H-NMR(300.1 MHz, d₆-DMSO): |
| δ= 8.0872 (5.5); 7.3077 (2.7); 7.2841 (3.5); 7.2662 (3.3); 7.1597 (4.2); 7.1420 (4.3); 4.6027 (1.6); 4.5605 (2.6); 4.4740 (2.8); 4.4334 (1.9); 4.3661 (2.2); 4.1751 (0.4); 3.9672 (14.2); 3.9434 (14.6); 3.7326 (0.3); 3.7030 (0.3); 3.6216 (0.4); 3.5942 (0.3); 3.5169 (4.6); 3.4967 (8.4); 3.4007 (1.2); 3.3389 (161.8); 3.2865 (1.4); 3.2390 (16.0); 3.1453 (2.0); 3.0305 (0.4); 3.0184 (0.4); 2.9096 (0.3); 2.8333 (0.4); 2.7287 (3.3); 2.6999 (1.8); 2.6252 (1.5); 2.5143 (113.5); 2.5087 (201.1); 2.5029 (252.4); 2.4971 (175.4); 2.3725 (1.2); 2.2784 (1.4); 2.2727 (1.7); 2.0772 (3.8); 1.8129 (4.3); 1.7940 (5.0); 1.5983 (2.2); 1.2976 (1.1); 1.2825 (2.0); 1.2574 (2.9); 1.2411 (2.0); 1.2328 (2.1); 1.2166 (1.2); 1.1909 (0.4); 0.5595 (2.1); 0.5389 (7.8); 0.5173 (7.2); 0.5125 (7.0); 0.4989 (2.9); 0.4763 (0.8); 0.4490 (0.7); 0.4271 (0.6); 0.3961 (3.1); 0.3804 (9.1); 0.3667 (7.9); 0.3468 (2.2); 0.3026 (0.4); 0.2648 (0.3); 0.0106 (2.1); -0.0002 (44.1); -0.0112 (2.0) |
| I-027: ¹H-NMR(300.1 MHz, CDCl₃): |
| δ= 7.5206 (2.0); 7.2639 (14.7); 7.2376 (0.9); 7.2115 (1.6); 7.1880 (1.7); 7.1684 (1.0); 7.1630 (1.0); 7.0879 (1.7); 7.0632 (2.5); 7.0419 (2.3); 7.0112 (1.9); 6.9828 (1.0); 5.3010 (1.5); 4.7570 (0.6); 4.7163 (1.4); 4.6731 (2.1); 4.6331 (0.8); 4.4926 (0.6); 4.2310 (1.7); 4.2064 (5.0); 4.1820 (5.0); 4.1578 (1.6); 3.6254 (0.8); 3.6131 (0.8); 3.4594 (16.0); 3.2250 (0.3); 3.1856 (0.4); 2.8837 (0.4); 2.8576 (0.4); 2.2664 (1.4); 2.0092 (6.7); 1.9382 (0.9); 1.9134 (1.6); 1.8921 (2.0); 1.8784 (1.9); 1.7817 (0.9); 1.7529 (1.0); 1.7377 (0.9); 1.5334 (6.1); 1.5091 (12.2); 1.4847 (5.3); 1.2560 (0.8); 0.8956 (0.3); 0.0002 (11.2) |
| I-028: ¹H-NMR(300.1 MHz, d₆-DMSO): |
| δ= 8.1366 (0.5); 8.0146 (8.5); 7.3105 (2.4); 7.2843 (3.2); 7.2635 (3.1); 7.1595 (3.9); 7.1381 (4.2); 7.0350 (0.3); 5.2027 (3.7); 5.1883 (8.8); 5.1742 (3.8); 4.5862 (1.3); 4.5496 (2.2); 4.4625 (2.3); 4.4250 (1.6); 4.3622 (1.8); 4.2478 (12.0); 4.2336 (11.3); 3.8473 (11.5); 3.8364 (16.0); 3.8240 (6.6); 3.7913 (1.3); 3.4734 (6.4); 3.3354 (104.3); 3.2751 (1.8); 3.2314 (15.2); 3.1391 (1.6); 2.7779 (1.0); 2.7337 (2.0); 2.7284 (2.1); 2.7222 (1.8); 2.6328 (0.9); 2.6251 (0.9); 2.5140 (67.8); 2.5083 (120.9); 2.5024 (151.9); 2.4965 (105.7); 2.2725 (1.1); 2.0753 (6.0); 1.8148 (3.6); 1.7912 (4.2); 1.5984 (2.2); 1.5855 (2.0); 0.0106 (1.2); - 0.0002 (25.4); -0.0113 (1.3) |
| I-029: ¹H-NMR(300.1 MHz, d₆-DMSO): |
| δ= 8.1943 (1.8); 8.1358 (2.5); 7.2855 (1.7); 7.2677 (1.6); 7.1436 (1.9); 5.4841 (16.0); 4.6083 (0.6); 4.5699 (1.3); 4.5031 (1.4); 4.4642 (0.9); 4.3792 (0.9); 3.4736 (2.4); 3.3308 (18.1); 3.2606 (7.3); 3.1654 (0.9); 3.1247 (0.8); 2.7989 (0.5); 2.7609 (0.7); 2.7298 (0.8); 2.6261 (0.4); 2.5143 (11.5); 2.5085 (21.4); 2.5026 (27.3); 2.4968 (19.2); 2.0757 (0.4); 1.8525 (1.0); 1.8271 (1.5); 1.7873 (2.3); 1.6121 (1.0); -0.0002 (6.8) |
| I-030: ¹H-NMR(300.1 MHz, d₆-DMSO): |
| δ= 8.0299 (2.5); 7.2843 (1.6); 7.2618 (1.5); 7.2403 (0.9); 7.1553 (1.9); 7.1370 (2.0); 7.0889 (0.5); 4.6001 (0.6); 4.5620 (1.0); 4.4701 (1.0); 4.4307 (0.8); 4.3573 (1.0); 4.2653 (2.7); 4.2480 (5.2); 4.2301 (3.0); 3.7847 (2.6); 3.7674 (4.5); 3.7504 (2.4); 3.5209 (3.0); 3.5073 (5.6); 3.4997 (4.9); 3.4902 (6.8); 3.4035 (4.0); 3.3868 (4.1); 3.3727 (2.7); 3.3274 (37.9); 3.2321 (7.9); 3.1936 (16.0); 3.1296 (1.0); 2.7766 (0.5); 2.7336 (1.3); 2.7278 (1.4); 2.7218 (1.2); 2.6508 (0.3); 2.5130 (66.6); 2.5075 (120.2); 2.5017 (152.6); 2.4959 (109.1); 2.2718 (1.0); 1.7997 (2.2); 1.7803 (2.0); 1.6000 (1.0); -0.0003 (30.6); -0.0110 (1.8) |
| I-031: ¹H-NMR(400.2 MHz, d₆-DMSO): |
| δ= 8.0789 (2.0); 8.0551 (4.4); 7.9532 (1.7); 7.3132 (1.4); 7.2831 (1.7); 7.2658 (1.8); 7.1820 (1.2); 7.1604 (2.6); 7.1439 (2.6); 4.7612 (16.0); 4.5914 (1.1); 4.5627 (1.6); 4.4620 (1.6); 4.4330 (1.2); 4.3739 (1.2); 4.0550 (0.5); 4.0373 (1.5); 4.0195 (1.5); 4.0017 (0.5); 3.5014 (2.4); 3.4867 (2.7); 3.4705 (1.7); 3.3374 (78.4); 3.3140 (1.1); 3.2348 (9.5); 3.1885 (1.5); 3.1533 (1.1); 2.8905 (11.7); 2.7685 (0.8); 2.7311 (10.9); 2.7164 (1.0); 2.6712 (0.8); 2.6671 (0.8); 2.6353 (10.0); 2.6243 (9.8); 2.5910 (0.6); 2.5568 (0.4); 2.5111 (14.7); 2.5070 (28.3); 2.5026 (37.1); 2.4983 (26.9); 2.0767 (4.2); 1.9898 (6.5); 1.8118 (2.7); 1.8004 (2.8); 1.7824 (2.6); 1.7690 (1.7); 1.7596 (1.6); 1.7434 (1.1); 1.5971 (1.6); 1.5863 (1.5); 1.1921 (1.8); 1.1743 (3.4); 1.1565 (1.7); -0.0001 (3.9) |
| I-032: ¹H-NMR(300.1 MHz, d₆-DMSO): |
| δ= 8.0344 (0.9); 7.3089 (0.4); 7.2842 (0.6); 7.2634 (0.6); 7.2415 (0.4); 7.1554 (0.8); 7.1363 (0.8); 4.5589 (0.4); 4.4700 (0.4); 4.3635 (0.4); 4.2705 (1.3); 4.2530 (2.4); 4.2352 (1.4); 3.7123 (1.3); 3.6948 (2.2); 3.6771 (1.2); 3.5064 (0.8); 3.4858 (1.5); 3.4645 (0.9); 3.3502 (3.8); 3.2326 (16.0); 3.1702 (0.4); 3.1448 (0.4); 2.7298 (0.3); 2.5153 (5.2); 2.5095 (9.4); 2.5037 (12.0); 2.4978 (8.5); 1.8119 (0.8); 1.7953 (0.9); 1.7741 (0.8); 1.5975 (0.4); 1.5840 (0.4); -0.0002 (2.6) |
| I-033: ¹H-NMR(300.1 MHz, d₆-DMSO): |
| δ= 8.0253 (7.2); 7.3850 (0.3); 7.3073 (2.6); 7.2819 (3.2); 7.2617 (3.0); 7.1568 (4.2); 7.1409 (4.2); 4.7611 (9.5); 4.6016 (1.4); 4.5614 (2.3); 4.4589 (2.2); 4.4230 (1.7); 4.3677 (1.9); 4.0307 (0.3); 3.4793 (5.0); 3.2296 (16.0); 3.1503 (1.8); 2.7722 (1.2); 2.7353 (1.8); 2.7007 (1.3); 2.6214 (0.6); 2.5728 (0.5); 2.5082 (52.0); 2.5030 (60.7); 2.4976 (43.3); 2.2730 (0.4); 2.0774 (1.1); 1.8075 (3.9); 1.7901 (4.5); 1.5890 (2.3); 1.5759 (2.2); -0.0001 (18.0) |
| I-034: ¹H-NMR(400.2 MHz, d₆-DMSO): |
| δ= 7.7699 (6.4); 7.3313 (0.9); 7.3111 (1.9); 7.2902 (2.0); 7.2707 (1.8); 7.2546 (1.3); 7.1850 (1.4); 7.1655 (2.8); 7.1491 (2.7); 5.0862 (16.0); 4.6952 (0.5); 4.6632 (4.3); 4.6578 (4.3); 4.6236 (0.6); 4.4918 (0.7); 4.3858 (1.6); 3.8152 (0.3); 3.7738 (0.7); 3.6763 (15.5); 3.6068 (0.3); 3.5346 (2.7); 3.5205 (4.5); 3.5057 (2.8); 3.3373 (70.8); 3.3136 (0.5); 3.2360 (0.5); 3.1853 (16.6); 2.7879 (1.0); 2.7652 (1.2); 2.7562 (1.1); 2.7342 (0.9); 2.6716 (0.6); 2.6106 (0.5); 2.5069 (62.4); 2.5025 (80.8); 2.4981 (57.8); 2.3297 (0.5); 1.8171 (2.4); 1.8017 (2.2); 1.7882 (1.7); 1.7730 (1.3); 1.7526 (1.0); 1.7324 (1.0); 1.5976 (1.4); -0.0002 (5.2) |
| I-035: ¹H-NMR(400.2 MHz, d₆-DMSO): |
| δ= 8.3037 (9.6); 8.0050 (1.1); 7.9915 (2.2); 7.9776 (1.1); 7.5953 (1.1); 7.5916 (1.2); 7.5764 (2.2); 7.5720 (2.4); 7.5555 (1.2); 7.5525 (1.2); 7.2869 (0.5); 7.2828 (0.6); 7.2732 (0.7); 7.2690 (1.4); 7.2656 (1.4); 7.2540 (1.5); 7.2482 (1.8); 7.2443 (1.3); 7.2349 (1.2); 7.2307 (1.1); 7.1837 (2.2); 7.1704 (2.2); 7.1659 (1.7); 7.1627 (1.8); 7.1551 (4.1); 7.1352 (2.7); 6.5532 (1.7); 6.5129 (3.5); 6.4550 (1.3); 6.4384 (2.8); 6.4217 (1.2); 6.4151 (0.6); 6.3984 (1.4); 6.3816 (0.7); 5.4844 (0.5); 5.4298 (0.4); 5.3627 (16.0); 4.6551 (0.7); 4.5869 (15.6); 4.5335 (0.5); 3.7823 (0.4); 3.3372 (26.6); 3.3137 (0.4); 3.2761 (37.5); 3.2590 (4.5); 3.2417 (39.3); 3.2304 (2.8); 3.2283 (2.7); 2.5122 (8.7); 2.5078 (17.2); 2.5034 (22.7); 2.4990 (16.1); 2.4947 (7.5); 2.2971 (1.2); 2.2791 (3.1); 2.2614 (3.2); 2.2438 (1.3); 1.7033 (0.8); 1.6854 (2.5); 1.6674 (3.4); 1.6494 (2.3); 1.6315 (0.7); -0.0002 (4.8) |
| I-036: ¹H-NMR(300.1 MHz, d₆-DMSO): |
| δ= 8.6105 (1.9); 8.4755 (5.4); 7.3265 (1.3); 7.2874 (1.3); 7.2631 (1.6); 7.1852 (1.1); 7.1431 (2.2); 7.0831 (1.1); 5.7505 (0.9); 4.6071 (0.8); 4.5674 (2.0); 4.5201 (2.2); 4.4809 (1.1); 4.3606 (1.2); 4.2829 (0.3); 4.2352 (0.3); 3.5182 (2.4); 3.4987 (4.9); 3.4752 (2.7); 3.3053 (142.3); 3.2720 (10.8); 3.1899 (1.0); 3.1433 (1.0); 2.7862 (2.2); 2.7188 (1.0); 2.6635 (0.5); 2.5624 (0.5); 2.5066 (34.6); 2.5008 (44.7); 2.4951 (32.6); 2.0716 (1.6); 1.7833 (2.5); 1.6149 (1.3); 0.7132 (16.0); 0.6938 (8.7); -0.0001 (9.8) |
| I-039: ¹H-NMR(400.1 MHz, d₆-DMSO): |
| δ= 8.0628 (2.0); 7.2979 (1.7); 7.2793 (2.3); 7.2635 (2.3); 7.2472 (1.3); 7.1524 (2.8); 7.1390 (2.9); 6.0768 (0.7); 6.0624 (1.4); 6.0507 (1.2); 6.0365 (2.0); 6.0214 (1.9); 6.0079 (1.3); 5.9940 (1.6); 5.9801 (0.8); 5.7581 (16.0); 5.2373 (2.6); 5.2119 (2.5); 5.1576 (1.4); 5.1160 (1.2); 4.7602 (6.7); 4.7462 (6.2); 4.5982 (1.0); 4.5699 (1.4); 4.5246 (0.4); 4.4756 (1.5); 4.4477 (1.1); 4.3743 (1.3); 4.2866 (0.4); 3.5029 (3.2); 3.4886 (5.4); 3.4716 (2.9); 3.3304 (9.1); 3.2743 (0.9); 3.2347 (8.9); 3.1822 (1.3); 3.1471 (1.1); 2.7414 (1.0); 2.6789 (0.4); 2.6285 (0.3); 2.6219 (0.3); 2.5048 (7.8); 1.9893 (0.5); 1.9708 (0.5); 1.8094 (2.9); 1.7974 (3.1); 1.7819 (2.8); 1.6011 (1.6); 1.2349 (0.4); -0.0002 (1.8) |
| I-040: ¹H-NMR(300.1 MHz, d₆-DMSO): |
| δ= 8.4871 (1.3); 8.3759 (0.4); 7.3393 (0.6); 7.3139 (0.6); 7.2729 (0.8); 7.1973 (0.6); 7.1550 (1.2); 7.1369 (0.9); 7.0956 (0.6); 7.0804 (0.6); 7.0511 (0.4); 4.5860 (0.4); 4.5454 (1.0); 4.5051 (1.2); 4.4641 (0.6); 4.3589 (0.8); 3.4862 (1.4); 3.3436 (5.5); 3.2732 (4.8); 3.2255 (1.8); 3.1700 (0.6); 2.7699 (0.4); 2.7380 (0.6); 2.7267 (0.6); 2.6970 (0.4); 2.5060 (14.9); 2.0798 (0.8); 1.7860 (1.4); 1.5977 (16.0); 1.4237 (0.8); - 0.0001 (1.6) |
| 1-041: ¹H-NMR(400.1 MHz, d₆-DMSO): |
| δ= 8.2194 (0.3); 8.1733 (1.2); 7.3365 (0.6); 7.2681 (0.8); 7.1616 (1.1); 7.1495 (1.1); 5.7577 (2.9); 4.5644 (0.5); 4.5367 (0.8); 4.4475 (0.7); 4.4173 (0.5); 4.3562 (0.7); 4.1196 (1.0); 4.1021 (2.6); 4.0844 (2.6); 4.0671 (1.0); 3.5427 (0.8); 3.5222 (0.9); 3.5028 (1.0); 3.3239 (21.6); 3.2179 (4.0); 3.1844 (1.3); 2.7370 (0.4); 2.7116 (0.5); 2.6811 (0.5); 2.5021 (16.3); 1.9889 (0.5); 1.7809 (16.0); 1.6048 (0.8); 1.4111 (0.5); 1.1936 (0.3); 1.1755 (0.4); 1.1564 (0.4); 1.1183 (2.9); 1.1026 (2.1); -0.0002 (3.3) |
| I-042: ¹H-NMR(600.3 MHz, CDCl₃): |
| δ= 7.5251 (3.5); 7.4348 (0.5); 7.3444 (4.1); 7.2631 (40.0); 7.1934 (4.8); 7.1650 (8.4); 7.1524 (10.4); 7.1404 (4.6); 7.0864 (0.6); 7.0005 (0.5); 4.7522 (2.8); 4.6945 (2.8); 4.6064 (2.8); 4.4888 (1.0); 4.3795 (0.5); 3.9109 (13.2); 3.5739 (2.8); 3.4735 (13.5); 3.3827 (3.3); 2.9935 (2.3); 2.8380 (0.5); 2.7469 (0.5); 1.9294 (2.6); 1.8523 (4.1); 1.7966 (5.4); 1.7847 (5.1); 1.7773 (4.6); 1.5979 (17.4); 1.3334 (0.3); 1.2844 (0.6); 1.2555 (3.0); 0.8804 (0.6); 0.8696 (0.5); 0.8408 (0.5); 0.0699 (11.7); -0.0001 (50.0) |
| I-043: ¹H-NMR(600.3 MHz, CDCl₃): |
| δ= 7.5257 (2.8); 7.4349 (0.4); 7.3709 (2.8); 7.3436 (3.2); 7.2852 (0.9); 7.2811 (0.8); 7.2632 (40.1); 7.1937 (3.9); 7.1653 (7.2); 7.1526 (8.9); 7.1403 (3.7); 7.0866 (0.5); 7.0010 (0.4); 4.7517 (2.2); 4.6948 (2.3); 4.6058 (2.2); 4.4873 (0.8); 4.3766 (0.4); 3.9109 (10.6); 3.5744 (2.2); 3.4736 (10.8); 3.3825 (2.6); 2.9948 (1.8); 2.8397 (0.4); 2.7514 (0.4); 2.0451 (0.6); 1.9294 (2.0); 1.8526 (3.2); 1.7966 (4.4); 1.7845 (4.1); 1.7773 (3.6); 1.6025 (18.1); 1.3333 (0.4); 1.2845 (0.6); 1.2553 (2.4); 0.8919 (0.3); 0.8807 (0.6); 0.8688 (0.4); 0.8410 (0.4); 0.0699 (8.8); 0.0219 (0.3); -0.0001 (50.0) |
| I-044: ¹H-NMR(400.6 MHz, CDCl₃): |
| δ= 7.4639 (0.6); 7.4378 (2.0); 7.2627 (22.7); 7.2554 (0.8); 7.2361 (0.8); 7.2306 (0.6); 7.1407 (1.2); 7.1379 (1.2); 7.1220 (1.8); 7.1191 (1.9); 7.1031 (0.8); 7.1004 (0.8); 7.0129 (0.8); 6.0570 (0.9); 5.9289 (0.9); 5.9185 (1.8); 5.9081 (0.9); 5.7800 (0.9); 5.4018 (0.6); 5.3001 (12.0); 4.9001 (2.2); 4.8699 (3.2); 4.8164 (0.6); 4.7691 (0.8); 4.7382 (0.5); 3.9353 (0.9); 3.9059 (16.0); 3.8151 (1.4); 3.8059 (1.4); 3.8046 (1.4); 3.7814 (2.8); 3.7804 (2.9); 3.7711 (2.6); 3.7699 (2.7); 3.7469 (1.4); 3.7455 (1.5); 3.7364 (1.3); 3.7351 (1.4); 2.1715 (4.2); 1.9146 (0.5); 1.7060 (0.5); 1.6893 (0.6); 1.6715 (0.7); 1.6503 (0.6); 1.6204 (0.6); 0.0079 (0.8); -0.0002 (32.1); -0.0052 (0.5); -0.0085 (1.0) |
| I-045: ¹H-NMR(300.1 MHz, d₆-DMSO): |
| δ= 8.3171 (0.3); 8.1697 (11.5); 7.3225 (1.8); 7.2889 (2.2); 7.2651 (2.2); 7.1489 (2.9); 7.0527 (0.6); 5.2917 (1.3); 5.2681 (3.9); 5.2441 (4.0); 5.2196 (1.2); 4.6144 (0.4); 4.5905 (1.2); 4.5487 (2.0); 4.4655 (2.0); 4.4261 (1.5); 4.3567 (1.7); 4.3011 (1.0); 4.1580 (2.8); 4.1344 (7.6); 4.1109 (7.6); 4.0873 (2.7); 4.0511 (0.3); 3.8844 (0.4); 3.7650 (0.4); 3.7272 (0.4); 3.6989 (0.4); 3.6848 (0.4); 3.6362 (0.4); 3.5917 (0.5); 3.5589 (0.7); 3.5494 (0.8); 3.4983 (5.7); 3.4574 (1.7); 3.4436 (1.3); 3.4117 (1.3); 3.3339 (452.3); 3.3110 (6.0); 3.2682 (2.7); 3.2227 (10.7); 2.7541 (1.0); 2.7292 (1.6); 2.7022 (1.0); 2.6181 (0.8); 2.5870 (0.7); 2.5139 (41.4); 2.5082 (75.5); 2.5023 (95.1); 2.4965 (65.0); 2.2722 (0.5); 2.0753 (6.2); 1.9168 (0.5); 1.8135 (3.1); 1.7968 (3.6); 1.6997 (16.0); 1.6755 (15.8); 1.6230 (1.5); 1.6009 (2.1); 1.5886 (1.9); 1.5266 (0.3); 1.1826 (5.8); 1.1594 (11.1); 1.1365 (5.8); 1.1114 (0.7); 1.0556 (0.5); 0.0109 (1.2); -0.0001 (27.5); - 0.0110 (1.3) |
| I-046: ¹H-NMR(300.1 MHz, d₆-DMSO): |
| δ= 8.2088 (5.2); 7.3124 (1.4); 7.2892 (1.6); 7.2679 (1.4); 7.2485 (1.0); 7.1861 (0.9); 7.1437 (2.0); 5.7444 (9.7); 4.6317 (0.8); 4.5922 (1.5); 4.5219 (1.6); 4.4815 (0.9); 4.3791 (0.9); 3.4734 (2.5); 3.4566 (2.2); 3.3514 (6.0); 3.2559 (8.9); 3.1929 (1.0); 3.1434 (0.9); 3.0606 (16.0); 2.8035 (0.6); 2.7731 (0.8); 2.7297 (0.6); 2.5096 (6.5); 2.5038 (8.5); 2.4981 (6.1); 2.0751 (1.2); 1.8613 (1.1); 1.8360 (1.7); 1.8167 (2.1); 1.7959 (2.2); 1.6354 (0.7); 1.6113 (1.2); 1.5978 (1.2); -0.0001 (0.8) |
| I-047: ¹H-NMR(300.1 MHz, d₆-DMSO): |
| δ= 8.0453 (5.1); 7.3069 (2.2); 7.2834 (3.2); 7.2631 (3.1); 7.2424 (1.9); 7.1805 (2.0); 7.1553 (3.9); 7.1358 (4.2); 7.0300 (0.3); 4.7412 (2.8); 4.7227 (6.3); 4.7046 (3.1); 4.5959 (1.3); 4.5583 (2.2); 4.4706 (2.1); 4.4318 (1.5); 4.3600 (1.8); 4.2134 (11.7); 4.1950 (10.8); 3.8167 (0.5); 3.4972 (3.8); 3.4763 (7.0); 3.4540 (3.9); 3.3282 (12.5); 3.2813 (82.9); 3.2321 (16.0); 3.1358 (1.8); 3.0426 (0.6); 2.7781 (0.9); 2.7355 (1.3); 2.5081 (18.1); 2.5025 (22.9); 2.4967 (16.4); 2.0750 (0.5); 1.8162 (3.8); 1.7966 (4.5); 1.6000 (2.2); 1.5869 (2.0); -0.0001 (6.0) |
| I-048: ¹H-NMR(300.1 MHz, d₆-DMSO): |
| δ= 8.0739 (0.7); 7.3078 (0.4); 7.2816 (0.5); 7.2622 (0.5); 7.1350 (0.6); 4.5525 (0.3); 4.4695 (0.4); 4.3408 (0.7); 4.1397 (0.8); 4.1167 (1.4); 4.0924 (0.8); 3.5113 (0.6); 3.4903 (1.2); 3.3251 (4.5); 3.2402 (16.0); 2.5133 (5.7); 2.5076 (10.8); 2.5017 (14.0); 2.4958 (9.8); 2.0966 (0.4); 2.0748 (1.2); 2.0520 (0.9); 2.0305 (0.5); 1.7939 (0.7); 1.5959 (0.3); -0.0002 (5.7) |
| I-049: ¹H-NMR(400.2 MHz, d₆-DMSO): |
| δ= 8.1854 (1.7); 7.8258 (13.1); 7.3824 (1.0); 7.3641 (2.1); 7.3426 (1.9); 7.3378 (1.9); 7.3177 (2.0); 7.3035 (1.4); 7.2329 (1.5); 7.2121 (3.3); 7.1954 (3.0); 7.1788 (1.6); 7.1212 (0.8); 7.0963 (0.9); 5.5235 (16.0); 4.7679 (1.1); 4.7373 (3.5); 4.7145 (3.5); 4.6838 (1.2); 4.6170 (0.3); 4.5867 (0.6); 4.5474 (0.5); 4.4345 (1.5); 3.6258 (3.1); 3.6059 (5.6); 3.5860 (4.1); 3.5493 (3.6); 3.5370 (4.6); 3.3847 (7.0); 3.3095 (19.2); 3.2414 (1.2); 3.2341 (1.2); 3.2089 (1.4); 3.2019 (1.4); 2.8420 (1.1); 2.8190 (1.3); 2.8102 (1.3); 2.7876 (1.0); 2.6497 (0.8); 2.5613 (5.4); 2.5571 (10.6); 2.5527 (13.8); 2.5482 (9.8); 2.5439 (4.5); 2.1262 (1.6); 1.9103 (1.1); 1.8906 (1.4); 1.8528 (2.4); 1.8226 (1.3); 1.8048 (1.0); 1.7800 (1.2); 1.7659 (1.2); 1.7516 (0.8); 1.6773 (1.2); 1.6639 (1.4); 1.6529 (1.6); 1.6400 (1.4); 0.9036 (7.1); 0.8962 (1.7); 0.8834 (9.3); 0.8707 (1.7); 0.8633 (6.9); 0.0484 (3.7); 0.0459 (1.9); 0.0002 (47.9); -0.0333 (7.0) |
| I-050: ¹H-NMR(300.1 MHz, d₆-DMSO): |
| δ= 8.1681 (1.3); 7.3080 (0.7); 7.2829 (0.9); 7.2674 (0.9); 7.1588 (1.1); 7.1392 (1.1); 5.2485 (8.2); 4.5993 (0.4); 4.5585 (0.7); 4.4799 (0.7); 4.4469 (0.5); 4.3820 (0.5); 4.3675 (0.5); 3.4829 (2.0); 3.4166 (0.4); 3.3932 (0.6); 3.3853 (0.4); 3.3251 (142.4); 3.2412 (4.4); 3.1320 (0.5); 2.7330 (0.6); 2.7213 (0.6); 2.5674 (0.4); 2.5132 (19.1); 2.5074 (36.1); 2.5015 (46.6); 2.4956 (32.6); 2.3956 (0.4); 2.2718 (0.4); 2.1458 (16.0); 2.0744 (0.9); 1.7957 (1.3); 1.6019 (0.6); -0.0002 (14.2) |
| I-051: ¹H-NMR(300.1 MHz, d₆-DMSO): |
| δ= 8.5381 (1.9); 8.5248 (2.2); 8.4942 (16.0); 7.3556 (1.0); 7.3316 (1.9); 7.3066 (1.7); 7.2921 (1.6); 7.2675 (2.0); 7.2486 (1.6); 7.1933 (1.7); 7.1685 (2.9); 7.1477 (2.8); 7.0834 (1.7); 4.8427 (0.5); 4.6285 (1.4); 4.5884 (3.4); 4.5453 (3.5); 4.5053 (1.7); 4.4693 (0.9); 4.3635 (1.9); 4.2566 (0.6); 4.2456 (0.5); 3.5142 (5.5); 3.4485 (0.8); 3.4118 (0.8); 3.3418 (176.6); 3.2902 (16.8); 3.2535 (4.3); 3.2429 (4.4); 3.1905 (1.5); 3.1803 (1.5); 3.1473 (1.5); 2.8302 (11.5); 2.8152 (10.7); 2.7916 (2.0); 2.7550 (1.5); 2.7418 (1.5); 2.7232 (1.0); 2.7132 (1.3); 2.6587 (0.6); 2.5871 (0.6); 2.5157 (14.0); 2.5099 (26.0); 2.5040 (33.5); 2.4980 (23.2); 2.0768 (1.1); 1.8449 (1.5); 1.7875 (3.5); 1.7404 (1.4); 1.6986 (0.4); 1.6389 (1.3); 1.6123 (1.6); 1.5971 (1.5); -0.0001 (8.7); -0.0112 (0.4) |
| I-052: ¹H-NMR(300.2 MHz, d₆-DMSO): |
| δ= 11.8098 (1.3); 8.1677 (5.2); 7.2921 (1.1); 7.2750 (1.2); 7.2493 (0.6); 7.1872 (0.7); 7.1609 (1.4); 7.1414 (1.5); 5.8261 (7.8); 4.6070 (0.6); 4.5680 (1.1); 4.4871 (1.1); 4.4469 (0.7); 4.3813 (0.6); 3.4928 (0.7); 3.4676 (1.4); 3.4455 (1.4); 3.4248 (0.7); 3.3272 (10.1); 3.2596 (7.0); 3.1750 (0.5); 3.1353 (0.6); 2.8071 (0.4); 2.7761 (0.5); 2.7674 (0.5); 2.7345 (0.5); 2.5133 (7.6); 2.5078 (14.9); 2.5020 (19.5); 2.4962 (13.6); 2.0751 (1.3); 2.0037 (16.0); 1.8640 (0.5); 1.8400 (1.0); 1.8198 (1.3); 1.7973 (1.3); 1.7855 (1.2); 1.7628 (0.8); 1.6115 (0.7); 1.5964 (0.8); 0.0000 (5.2) |
| I-053: ¹H-NMR(400.6 MHz, CDCl₃): |
| δ= 7.4833 (0.7); 7.4792 (0.7); 7.4602 (1.8); 7.2612 (35.1); 7.2555 (0.6); 7.1444 (0.6); 7.1416 (0.6); 7.1255 (0.9); 7.1227 (0.9); 4.7648 (0.6); 4.7340 (0.8); 4.1488 (0.8); 4.1310 (2.3); 4.1132 (2.4); 4.0953 (0.8); 3.9031 (7.2); 3.4410 (16.0); 2.0455 (11.4); 1.6182 (0.6); 1.5975 (0.6); 1.2775 (3.3); 1.2597 (6.7); 1.2418 (3.2); 0.0080 (1.5); -0.0002 (54.5); -0.0061 (0.8); -0.0070 (0.7); -0.0085 (1.7) |
| I-055: ¹H-NMR(600.3 MHz, CDCl₃): |
| δ= 7.6018 (5.1); 7.3106 (0.6); 7.2984 (1.2); 7.2868 (0.7); 7.2614 (34.1); 7.2045 (1.0); 7.1928 (1.2); 7.1833 (0.8); 7.0895 (0.9); 7.0847 (0.7); 7.0772 (1.5); 7.0651 (0.8); 7.0471 (0.9); 7.0318 (1.3); 7.0171 (0.9); 7.0088 (0.5); 7.0068 (0.5); 6.9931 (0.3); 6.9910 (0.4); 6.9889 (0.3); 6.9754 (0.4); 5.6048 (1.1); 5.5866 (2.2); 5.5716 (0.9); 5.5688 (1.0); 5.5650 (0.6); 5.5515 (2.8); 5.5333 (1.2); 5.4186 (0.4); 4.8994 (1.2); 4.8790 (1.7); 4.7934 (1.7); 4.7730 (1.2); 4.7611 (0.3); 4.7369 (0.4); 4.5401 (0.8); 3.8375 (0.7); 3.6112 (5.0); 3.5972 (8.6); 3.5835 (5.1); 3.5257 (0.4); 3.4660 (0.8); 3.4599 (0.4); 3.4484 (0.8); 3.4309 (0.9); 3.4116 (9.8); 3.3911 (1.0); 3.3857 (0.8); 3.3749 (0.7); 3.3437 (0.4); 3.2550 (0.8); 3.2373 (0.9); 2.9793 (0.7); 2.9650 (0.8); 2.9575 (0.7); 2.9432 (0.6); 1.9773 (0.3); 1.9651 (0.4); 1.9518 (0.7); 1.9405 (1.0); 1.9293 (0.9); 1.9126 (0.5); 1.8546 (0.5); 1.8448 (0.8); 1.8363 (0.7); 1.8186 (0.4); 1.7748 (0.6); 1.7629 (0.9); 1.7512 (1.4); 1.7427 (1.5); 1.7337 (1.0); 1.5766 (1.2); 1.5345 (0.3); 1.5191 (1.1); 1.4972 (1.0); 1.4836 (0.7); 1.4638 (0.5); 1.3838 (0.7); 1.3712 (0.7); 1.3017 (0.5); 1.2895 (0.6); 1.2798 (0.6); 1.2766 (0.6); 1.2647 (1.3); 1.2566 (0.9); 0.9417 (0.4); 0.9317 (2.6); 0.9275 (2.0); 0.9183 (3.4); 0.9128 (2.8); 0.9039 (2.5); 0.9000 (1.8); 0.8936 (1.7); 0.8820 (3.9); 0.8701 (1.9); 0.0263 (1.1); 0.0201 (0.7); 0.0053 (2.0); -0.0001 (50.0); -0.0056 (2.8); -0.0162 (42.7) |
| I-056: ¹H-NMR(400.6 MHz, CDCl₃): |
| δ= 7.3796 (1.0); 7.3613 (1.8); 7.3434 (1.0); 7.2652 (7.2); 7.2626 (12.9); 7.2240 (0.5); 7.2074 (1.3); 7.1899 (1.7); 7.1760 (1.1); 7.0971 (1.4); 7.0788 (2.2); 7.0520 (1.5); 7.0287 (2.2); 7.0078 (1.4); 6.9388 (0.6); 6.9193 (0.6); 6.9043 (0.6); 4.5489 (2.2); 4.5192 (4.3); 4.4790 (0.7); 4.4456 (3.2); 4.4159 (1.9); 4.3865 (0.8); 3.8681 (3.9); 3.8369 (14.8); 3.7051 (0.7); 3.6877 (1.2); 3.6704 (0.7); 3.3961 (16.0); 3.3730 (4.2); 3.3547 (5.3); 3.2873 (1.1); 3.2786 (1.1); 3.2535 (1.3); 3.2469 (1.3); 2.9537 (1.2); 2.9326 (1.2); 2.9211 (1.1); 2.8993 (0.9); 1.9545 (1.4); 1.9399 (1.3); 1.9237 (0.9); 1.8938 (0.6); 1.8537 (1.2); 1.8380 (1.3); 1.8124 (1.0); 1.7938 (1.4); 1.7813 (2.0); 1.7664 (2.3); 1.7496 (2.2); 1.7380 (2.0); 1.7136 (0.8); 1.0216 (0.5); 0.0079 (0.9); 0.0024 (10.9); -0.0002 (19.3) |
| 1-057: ¹H-NMR(400.6 MHz, CDCl₃): |
| δ= 7.3784 (0.8); 7.3600 (1.6); 7.3414 (0.9); 7.2626 (25.2); 7.2042 (1.1); 7.1889 (1.4); 7.1752 (1.0); 7.0955 (1.3); 7.0770 (2.0); 7.0578 (1.0); 7.0509 (1.3); 7.0276 (1.9); 7.0066 (1.3); 6.9389 (0.5); 6.9195 (0.5); 6.9044 (0.5); 4.5489 (2.1); 4.5193 (4.2); 4.4784 (0.6); 4.4454 (3.2); 4.4156 (1.9); 4.3863 (0.7); 3.8671 (3.5); 3.8359 (16.0); 3.7044 (0.6); 3.6860 (1.0); 3.6681 (0.6); 3.3964 (15.9); 3.3723 (3.5); 3.3536 (4.8); 3.2865 (0.9); 3.2775 (0.9); 3.2533 (1.1); 3.2450 (1.0); 2.9527 (1.1); 2.9314 (1.1); 2.9201 (1.0); 2.8981 (0.9); 1.9777 (0.8); 1.9547 (1.2); 1.9393 (1.1); 1.9219 (0.8); 1.8667 (0.5); 1.8511 (1.0); 1.8362 (1.1); 1.8097 (0.8); 1.7922 (1.2); 1.7798 (1.6); 1.7645 (2.0); 1.7473 (1.9); 1.7364 (1.7); 1.7115 (0.7); 0.0079 (1.0); -0.0002 (36.1); -0.0085 (1.2) |
| 1-058: ¹H-NMR(400.6 MHz, CDCl₃): |
| δ= 7.3794 (0.9); 7.3602 (1.7); 7.3421 (1.0); 7.2630 (19.7); 7.2073 (1.2); 7.1890 (1.6); 7.1750 (1.0); 7.0959 (1.4); 7.0775 (2.1); 7.0579 (1.2); 7.0513 (1.4); 7.0280 (2.0); 7.0067 (1.3); 6.9393 (0.5); 6.9182 (0.5); 6.9040 (0.5); 4.5490 (2.2); 4.5194 (4.3); 4.4779 (0.6); 4.4455 (3.2); 4.4157 (1.9); 4.3865 (0.8); 3.8674 (3.7); 3.8361 (16.0); 3.7037 (0.6); 3.6867 (1.0); 3.6692 (0.6); 3.3965 (15.4); 3.3727 (3.8); 3.3539 (5.0); 3.3032 (1.1); 3.2870 (1.0); 3.2776 (1.0); 3.2535 (1.2); 3.2448 (1.1); 2.9535 (1.2); 2.9317 (1.2); 2.9198 (1.1); 2.8983 (1.0); 2.0963 (0.8); 1.9793 (0.9); 1.9545 (1.2); 1.9390 (1.2); 1.9222 (0.8); 1.8944 (0.5); 1.8516 (1.1); 1.8372 (1.2); 1.8102 (0.8); 1.7927 (1.2); 1.7801 (1.8); 1.7650 (2.2); 1.7481 (2.0); 1.7369 (1.8); 1.7118 (0.7); 1.5667 (0.6); 1.5224 (0.7); 0.0079 (1.0); -0.0002 (28.6); -0.0085 (0.9) |
| 1-061: ¹H-NMR(400.6 MHz, CDCl₃): |
| δ= 7.2615 (19.4); 3.8933 (3.1); 3.7232 (0.6); 3.6796 (0.6); 3.6357 (2.3); 3.6259 (2.1); 3.6126 (1.6); 3.5414 (2.0); 3.5341 (1.2); 3.5289 (1.5); 3.5182 (1.0); 3.3647 (16.0); 1.5984 (0.8); 0.0080 (0.8); -0.0002 (30.6); - 0.0085 (0.9) |
| I-063: ¹H-NMR(400.6 MHz, CDCl₃): |
| δ= 7.4954 (1.4); 7.2945 (0.7); 7.2609 (57.5); 7.2259 (0.6); 7.2109 (1.1); 7.1938 (1.2); 7.1782 (0.7); 7.0878 (1.1); 7.0689 (1.7); 7.0497 (1.1); 7.0194 (1.0); 6.9974 (1.0); 5.3000 (3.3); 4.7445 (0.6); 4.6964 (0.8); 4.1222 (0.7); 3.9145 (16.0); 3.8890 (0.6); 3.8845 (0.8); 2.8488 (1.2); 2.6328 (0.8); 1.8834 (1.0); 1.7541 (1.1); 1.6739 (1.0); 1.5412 (1.5); 1.3207 (0.7); 1.2551 (1.8); 1.2227 (1.1); 0.0937 (0.5); 0.0691 (1.5); 0.0079 (1.5); -0.0002 (62.9); -0.0085 (3.0) |
| 1-064: ¹H-NMR(400.6 MHz, CDCl₃): |
| δ= 7.5194 (0.6); 7.4663 (1.2); 7.3091 (1.0); 7.2889 (1.0); 7.2611 (97.4); 7.2233 (1.2); 7.2046 (2.0); 7.1862 (2.2); 7.1804 (1.6); 7.1716 (1.3); 7.1672 (1.4); 7.0682 (1.8); 7.0190 (1.4); 6.9974 (1.6); 5.3001 (11.5); 4.8328 (0.8); 4.7239 (1.0); 4.5179 (0.7); 4.0933 (1.0); 3.8889 (16.0); 3.8720 (2.2); 3.8516 (1.3); 3.7276 (1.6); 3.7104 (1.6); 3.5472 (3.4); 3.4096 (0.9); 3.2201 (0.7); 2.9163 (0.7); 2.0754 (0.7); 1.9130 (2.6); 1.9000 (2.7); 1.8362 (3.6); 1.7503 (2.2); 1.6195 (1.2); 1.5414 (2.5); 1.2552 (1.7); 1.2225 (1.2); 0.0080 (3.5); -0.0002 (142.8); -0.0085 (6.1) |
| I-065: ¹H-NMR(400.6 MHz, CDCl₃): |
| δ= 7.5194 (0.6); 7.4782 (1.1); 7.2609 (85.1); 7.2259 (0.8); 7.2070 (1.5); 7.1886 (1.6); 7.1837 (1.2); 7.1749 (1.0); 7.0680 (1.5); 7.0467 (1.2); 7.0192 (1.2); 6.9973 (1.2); 5.3001 (3.9); 4.7843 (0.6); 4.6793 (0.7); 4.5285 (0.6); 3.9007 (16.0); 3.8166 (1.4); 3.7997 (1.6); 3.7228 (0.9); 3.7054 (1.5); 3.6863 (1.4); 3.5346 (2.0); 3.4612 (1.2); 3.2102 (0.5); 2.9314 (0.6); 2.5812 (0.7); 2.0757 (0.6); 2.0520 (0.6); 1.9713 (1.6); 1.7513 (1.5); 1.5900 (0.8); 1.2542 (1.9); 1.2227 (0.8); 0.0079 (4.4); -0.0002 (126.4); -0.0085 (5.2); - 0.0317 (0.6) |
| I-067: ¹H-NMR(400.6 MHz, CDCl₃): |
| δ= 7.5204 (0.5); 7.4747 (1.3); 7.3077 (1.1); 7.2877 (1.1); 7.2619 (60.4); 7.2245 (1.2); 7.2054 (2.0); 7.1871 (2.1); 7.1812 (1.5); 7.1727 (1.3); 7.1681 (1.3); 7.0888 (1.6); 7.0693 (2.0); 7.0507 (1.5); 7.0156 (1.5); 6.9983 (1.3); 4.8241 (1.0); 4.7464 (1.2); 4.7186 (1.0); 4.5215 (0.8); 3.8934 (16.0); 3.7225 (2.9); 3.5797 (3.0); 3.4115 (1.1); 3.3900 (1.2); 3.3516 (6.4); 3.2250 (0.6); 2.9191 (0.7); 2.1125 (0.5); 2.0754 (2.2); 2.0518 (1.5); 1.9295 (1.9); 1.9108 (2.2); 1.8984 (2.1); 1.8796 (1.8); 1.8420 (1.2); 1.7535 (1.9); 1.2551 (2.4); 1.2226 (0.6); 0.8796 (0.6); 0.0692 (1.0); 0.0079 (4.0); -0.0002 (67.9); -0.0085 (1.4) |
| I-068: ¹H-NMR(400.6 MHz, CDCl₃): |
| δ= 7.4896 (1.1); 7.2670 (8.9); 6.8287 (0.6); 6.8081 (1.4); 6.7867 (1.0); 6.7574 (0.5); 4.7366 (0.6); 4.6855 (0.7); 3.9113 (16.0); 3.4678 (4.3); 2.0077 (4.2); 1.9395 (0.6); 1.9215 (0.8); 1.9090 (0.8); 1.8929 (0.8); 1.8363 (0.6); 1.7189 (0.6); 1.7061 (0.6); 1.6923 (0.6); 1.6702 (0.6); -0.0002 (9.3) |
| I-069: ¹H-NMR(400.6 MHz, CDCl₃): |
| δ= 7.4991 (1.1); 7.2616 (33.9); 6.9980 (0.7); 6.9808 (0.7); 6.9697 (0.6); 6.8971 (0.7); 6.8875 (0.8); 6.8772 (0.9); 6.8692 (0.6); 6.8650 (0.5); 4.7386 (0.5); 4.6831 (0.5); 3.9137 (16.0); 3.4693 (4.3); 1.9401 (0.6); 1.9275 (0.6); 1.9108 (0.6); 1.6241 (0.9); 0.0080 (1.2); -0.0002 (38.5); -0.0084 (1.5) |
| I-070: ¹H-NMR(400.6 MHz, CDCl₃): |
| δ= 7.5200 (0.5); 7.4790 (1.3); 7.3111 (0.9); 7.2878 (0.8); 7.2615 (76.5); 7.2246 (1.1); 7.2055 (1.9); 7.1881 (2.1); 7.1817 (1.5); 7.1724 (1.3); 7.1684 (1.3); 7.0836 (1.5); 7.0659 (2.2); 7.0457 (1.6); 7.0155 (1.6); 6.9979 (1.5); 5.3000 (6.0); 4.7606 (1.0); 4.7167 (1.1); 4.5221 (0.7); 3.9259 (0.5); 3.9008 (16.0); 3.6323 (2.4); 3.4113 (0.8); 3.2148 (0.7); 2.9293 (0.7); 2.0753 (0.6); 1.8900 (1.5); 1.7464 (1.9); 1.6800 (1.6); 1.2537 (4.6); 1.2319 (3.5); 0.0079 (1.5); -0.0002 (82.8); -0.0085 (4.3); -0.0289 (0.7) |
| I-071: ¹H-NMR(400.6 MHz, CDCl₃): |
| δ= 7.5003 (1.0); 7.2646 (10.5); 7.0010 (0.6); 6.9928 (0.5); 6.9808 (0.7); 6.9700 (0.6); 6.9057 (0.5); 6.8971 (0.7); 6.8873 (0.8); 6.8835 (0.5); 6.8769 (0.9); 6.8689 (0.6); 6.8650 (0.6); 4.6827 (0.6); 3.9137 (16.0); 3.4694 (4.3); 1.9395 (0.6); 1.9269 (0.6); 1.9102 (0.6); 1.2566 (0.5); -0.0002 (11.8) |
| I-072: ¹H-NMR(400.6 MHz, CDCl₃): |
| δ= 7.5006 (1.1); 7.2651 (8.6); 7.0017 (0.6); 6.9911 (0.5); 6.9806 (0.7); 6.9697 (0.7); 6.9593 (0.5); 6.8968 (0.7); 6.8872 (0.9); 6.8768 (0.9); 6.8689 (0.6); 6.8650 (0.6); 4.6842 (0.6); 3.9137 (16.0); 3.4694 (4.4); 1.9396 (0.7); 1.9267 (0.6); 1.9099 (0.7); 1.7239 (0.5); 1.7129 (0.5); -0.0002 (9.5) |
| I-073: ¹H-NMR(400.6 MHz, CDCl₃): |
| δ= 7.2608 (34.8); 7.1362 (0.7); 7.1176 (0.7); 3.8923 (2.1); 3.4402 (1.2); 1.5505 (16.0); 0.0079 (1.1); - 0.0002 (38.8); -0.0085 (1.7) |
| I-074: ¹H-NMR(400.6 MHz, CDCl₃): |
| δ= 7.8176 (1.0); 7.4691 (0.9); 7.3004 (0.7); 7.2917 (0.6); 7.2619 (68.0); 7.2251 (0.8); 7.2205 (0.8); 7.2118 (1.0); 7.2052 (1.6); 7.1869 (1.8); 7.1818 (1.4); 7.1730 (1.0); 7.1687 (1.1); 7.0841 (1.3); 7.0652 (2.0); 7.0461 (1.4); 7.0149 (1.5); 6.9982 (1.2); 6.5600 (0.6); 5.2999 (1.5); 4.6579 (4.1); 4.6495 (1.3); 4.6342 (0.9); 4.5203 (0.7); 4.2892 (1.1); 4.2714 (1.1); 4.1171 (0.8); 4.0998 (1.0); 4.0814 (0.8); 3.8989 (16.0); 3.8954 (9.2); 3.8844 (2.9); 3.8790 (1.7); 3.5632 (0.7); 3.4078 (0.6); 3.2148 (0.6); 2.9278 (0.6); 2.2226 (1.7); 2.2167 (1.6); 2.2056 (1.6); 2.0756 (1.0); 2.0698 (0.6); 2.0517 (1.1); 2.0429 (1.1); 2.0203 (1.4); 1.9243 (1.0); 1.9059 (1.3); 1.8896 (1.4); 1.7424 (1.8); 1.7142 (1.6); 1.6872 (1.8); 1.6624 (1.6); 1.6382 (0.8); 1.5409 (3.6); 1.5198 (0.9); 1.5009 (1.3); 1.4750 (1.2); 1.4545 (0.6); 1.4487 (0.5); 1.4322 (0.8); 1.4211 (0.9); 1.3557 (1.2); 1.3379 (2.4); 1.3231 (1.3); 1.3201 (1.4); 1.2809 (1.5); 1.2701 (1.5); 1.2539 (2.7); 1.2223 (5.4); 0.8793 (0.5); 0.0693 (0.5); 0.0079 (2.4); -0.0002 (91.0); -0.0085 (2.9) |
| I-075: ¹H-NMR(400.6 MHz, CDCl₃): |
| δ= 7.4770 (0.5); 7.2616 (43.5); 7.2260 (0.5); 7.2128 (0.6); 7.2066 (0.8); 7.1876 (0.9); 7.1826 (0.7); 7.1738 (0.5); 7.1696 (0.6); 7.0893 (0.7); 7.0679 (1.0); 7.0490 (0.7); 7.0148 (0.7); 6.9979 (0.7); 5.3001 (16.0); 3.8980 (9.2); 3.4571 (0.6); 1.9057 (0.7); 1.8847 (0.7); 1.7574 (0.8); 1.2542 (1.4); 1.2225 (0.8); 1.0436 (0.5); 1.0250 (0.6); 0.6728 (1.1); 0.4790 (1.5); 0.4635 (1.5); 0.0692 (1.5); 0.0080 (1.2); -0.0002 (48.0); - 0.0085 (1.5) |
| I-076: ¹H-NMR(400.6 MHz, CDCl₃): |
| δ= 7.5200 (0.5); 7.4969 (1.0); 7.3011 (0.9); 7.2911 (0.7); 7.2818 (0.8); 7.2614 (61.3); 7.2289 (1.0); 7.2235 (0.8); 7.2093 (1.5); 7.1915 (1.5); 7.1858 (1.1); 7.1767 (0.9); 7.1727 (1.0); 7.0984 (0.7); 7.0833 (1.3); 7.0691 (1.9); 7.0485 (1.5); 7.0211 (1.3); 6.9979 (1.2); 5.3000 (4.7); 4.9539 (0.7); 4.9170 (0.8); 4.8738 (0.7); 4.8423 (0.6); 4.5290 (0.5); 3.9815 (1.4); 3.9733 (1.5); 3.9594 (1.6); 3.9514 (1.5); 3.9392 (1.2); 3.9295 (0.9); 3.9129 (16.0); 3.8925 (1.2); 3.8843 (1.3); 3.8770 (0.7); 3.5482 (0.8); 3.4084 (1.0); 3.1985 (0.6); 2.9304 (0.6); 2.0761 (0.7); 2.0524 (0.5); 1.9102 (1.2); 1.8940 (1.3); 1.7504 (1.5); 1.7399 (1.6); 1.6555 (1.2); 1.5431 (1.6); 1.4322 (0.5); 1.4088 (0.5); 1.2542 (2.1); 1.0123 (1.7); 0.9944 (2.7); 0.9773 (1.2); 0.0692 (3.6); 0.0080 (1.1); -0.0002 (82.1); -0.0085 (3.5); -0.0315 (0.5) |
| I-077: ¹H-NMR(400.6 MHz, CDCl₃): |
| δ= 7.5046 (0.6); 7.2635 (13.6); 7.0406 (0.8); 7.0361 (0.8); 7.0215 (1.2); 7.0181 (1.3); 7.0110 (1.0); 7.0052 (1.6); 6.9952 (0.8); 6.9913 (0.7); 5.3003 (1.2); 3.9142 (16.0); 3.4632 (3.4); 1.9592 (0.5); 1.9411 (0.7); 1.9284 (0.6); 1.9116 (0.6); 0.0079 (0.6); -0.0002 (21.0); -0.0085 (0.6) |
| I-078: ¹H-NMR(400.6 MHz, CDCl₃): |
| δ= 7.5367 (2.0); 7.5198 (1.4); 7.2613 (87.0); 7.1632 (4.1); 7.1467 (7.3); 7.1296 (12.8); 7.1136 (7.3); 6.9976 (0.6); 5.2992 (16.0); 4.7316 (1.9); 4.6789 (2.1); 4.5298 (1.9); 3.9117 (7.3); 3.7922 (1.2); 3.5681 (2.5); 3.4642 (10.0); 2.5901 (5.1); 2.5664 (4.1); 2.5568 (5.0); 2.5330 (3.7); 2.4619 (6.3); 2.4469 (4.3); 2.4021 (1.2); 2.3392 (0.7); 2.3219 (1.0); 2.3073 (6.0); 2.1159 (1.9); 1.9556 (2.0); 1.8290 (3.4); 1.7727 (2.2); 1.6605 (0.7); 1.0199 (0.7); 1.0026 (1.2); 0.0694 (0.8); 0.0080 (3.7); -0.0002 (130.8); -0.0085 (4.0) |
| I-079: ¹H-NMR(400.6 MHz, CDCl₃): |
| δ= 7.4893 (1.0); 7.2647 (12.0); 6.8290 (0.6); 6.8080 (1.4); 6.7869 (1.0); 4.7366 (0.6); 4.6862 (0.6); 3.9114 (16.0); 3.4689 (4.1); 3.4236 (0.5); 1.9395 (0.6); 1.9217 (0.8); 1.9087 (0.7); 1.8920 (0.7); 1.7190 (0.5); 1.7067 (0.5); -0.0002 (18.0); -0.0085 (0.6) |
| I-080: ¹H-NMR(400.6 MHz, CDCl₃): |
| δ= 7.4889 (1.0); 7.2639 (12.8); 6.8291 (0.6); 6.8082 (1.3); 6.7874 (0.9); 4.7369 (0.5); 4.6859 (0.6); 3.9118 (16.0); 3.4688 (3.9); 3.4212 (0.5); 1.9396 (0.6); 1.9218 (0.7); 1.9085 (0.7); 1.8931 (0.7); 1.6054 (1.2); 0.0080 (0.5); -0.0002 (19.3); -0.0085 (0.6) |
| I-081: ¹H-NMR(400.6 MHz, CDCl₃): |
| δ= 7.5105 (1.0); 7.2617 (18.0); 7.1691 (0.7); 6.8713 (0.7); 6.8522 (1.1); 6.8334 (0.6); 5.3002 (1.3); 3.9098 (16.0); 3.4484 (1.8); 0.0079 (0.8); -0.0002 (27.7); -0.0085 (0.8) |
| I-082: ¹H-NMR(400.6 MHz, CDCl₃): |
| δ= 7.5100 (1.0); 7.2649 (6.2); 7.1680 (0.9); 7.1475 (0.5); 6.8706 (0.9); 6.8518 (1.4); 6.8327 (0.8); 4.6488 (0.6); 4.6209 (0.5); 3.9089 (16.0); 3.4514 (2.3); 1.8970 (0.6); -0.0002 (9.0) |
| I-083: ¹H-NMR(400.6 MHz, CDCl₃): |
| δ= 7.5118 (0.8); 7.2642 (8.5); 7.1681 (0.8); 6.8707 (0.8); 6.8519 (1.2); 6.8329 (0.7); 4.6477 (0.5); 3.9095 (16.0); 3.4514 (1.9); 1.8952 (0.5); -0.0002 (12.9) |
| I-084: ¹H-NMR(400.6 MHz, CDCl₃): |
| δ= 7.5348 (3.0); 7.5194 (3.2); 7.4415 (1.3); 7.2893 (0.6); 7.2609 (231.5); 7.2348 (0.9); 7.2303 (0.7); 7.1456 (9.2); 7.1285 (21.7); 7.1121 (12.5); 6.9972 (1.7); 4.7468 (2.7); 4.6957 (3.0); 4.5198 (2.7); 4.2400 (0.5); 3.9089 (12.2); 3.5700 (3.8); 3.4723 (16.0); 3.2764 (1.5); 3.2352 (3.3); 2.5893 (8.9); 2.5656 (7.6); 2.5560 (8.7); 2.5322 (7.1); 2.4659 (10.2); 2.3226 (0.9); 1.9512 (3.0); 1.8285 (5.2); 1.7658 (3.5); 1.6169 (4.0); 1.6009 (6.3); 0.1457 (1.0); 0.0284 (1.0); 0.0079 (8.4); -0.0002 (330.6); -0.0062 (4.3); -0.0085 (10.3); -0.0228 (0.6); -0.0263 (0.8); -0.0307 (0.7); -0.1492 (1.0) |
| I-085: ¹H-NMR(400.6 MHz, CDCl₃): |
| δ= 7.7475 (0.6); 7.5352 (3.4); 7.5212 (2.2); 7.2625 (75.7); 7.1599 (7.8); 7.1516 (9.2); 7.1455 (10.3); 7.1283 (21.9); 7.1116 (12.4); 7.0882 (2.0); 7.0617 (0.8); 6.9988 (0.6); 6.9288 (0.5); 4.7446 (2.8); 4.6927 (3.0); 4.5206 (2.5); 3.9115 (12.2); 3.7956 (1.7); 3.7628 (1.5); 3.5807 (3.8); 3.4708 (16.0); 3.2332 (0.8); 3.1814 (0.6); 2.8402 (0.6); 2.8205 (0.6); 2.7792 (0.7); 2.7623 (0.7); 2.7450 (0.7); 2.7282 (0.6); 2.5890 (9.0); 2.5652 (7.4); 2.5556 (8.8); 2.5319 (6.9); 2.4657 (10.7); 2.4606 (11.2); 2.4418 (4.8); 2.4014 (2.8); 2.3461 (7.3); 2.3234 (1.9); 2.3080 (4.2); 2.0805 (3.5); 2.0166 (2.6); 1.9522 (3.3); 1.8312 (5.2); 1.8236 (5.8); 1.7645 (3.2); 1.7408 (3.1); 1.2552 (0.9); 0.0080 (3.2); -0.0002 (107.6); -0.0085 (3.0) |
| I-086: ¹H-NMR(400.6 MHz, CDCl₃): |
| δ= 7.5000 (1.1); 7.2631 (23.8); 7.1466 (4.4); 7.1365 (5.2); 7.1173 (4.6); 7.0976 (1.4); 6.9677 (1.3); 6.9455 (2.4); 6.9280 (1.6); 4.7882 (0.9); 4.7069 (1.0); 4.6275 (1.3); 3.8915 (16.0); 3.7161 (0.8); 3.5830 (1.9); 3.4410 (8.2); 2.9616 (1.0); 2.3013 (0.5); 2.0141 (1.1); 1.9144 (1.0); 1.8962 (1.8); 1.8664 (2.8); 1.8583 (2.8); 1.8404 (2.2); 1.8226 (1.0); 1.7536 (2.1); 1.7456 (2.0); 1.7349 (2.5); 1.7265 (2.2); 1.7157 (1.6); 1.7078 (1.4); 1.6651 (1.5); 1.2554 (0.5); 0.0072 (1.0); -0.0002 (34.0); -0.0082 (1.2) |
| I-087: ¹H-NMR(400.6 MHz, CDCl₃): |
| δ= 7.5222 (1.0); 7.5039 (1.2); 7.2638 (53.9); 7.1477 (5.3); 7.1372 (6.2); 7.1323 (6.0); 7.1179 (5.7); 7.0981 (1.8); 6.9477 (2.8); 6.9290 (2.0); 4.7890 (1.1); 4.7078 (1.3); 4.6316 (1.6); 3.8930 (16.0); 3.7136 (1.0); 3.5827 (2.2); 3.4422 (9.5); 2.9701 (1.2); 2.0157 (1.4); 1.8597 (3.2); 1.7542 (2.4); 1.7465 (2.4); 1.7356 (2.9); 1.7170 (1.9); 1.6647 (1.2); 1.2541 (0.5); 0.0080 (2.4); -0.0002 (83.0); -0.0085 (2.6) |
| 1-088: ¹H-NMR(400.6 MHz, CDCl₃): |
| δ= 7.5016 (0.9); 7.2678 (3.9); 7.0595 (0.6); 7.0541 (0.5); 7.0342 (0.9); 7.0160 (1.6); 7.0034 (2.0); 6.9928 (1.0); 4.7377 (0.6); 4.6841 (0.6); 3.9134 (16.0); 3.4677 (4.6); 1.9551 (0.6); 1.9372 (0.8); 1.9199 (0.8); 1.9076 (0.8); 1.8610 (0.5); 1.7348 (0.6); 1.7223 (0.6); -0.0002 (5.3) |
| I-089: ¹H-NMR(400.6 MHz, CDCl₃): |
| δ= 7.5013 (0.6); 7.2638 (10.8); 7.0391 (0.7); 7.0346 (0.7); 7.0198 (1.2); 7.0166 (1.2); 7.0093 (1.0); 7.0037 (1.6); 6.9936 (0.8); 6.9898 (0.7); 3.9140 (16.0); 3.4674 (3.4); 1.9557 (0.5); 1.9376 (0.7); 1.9203 (0.6); 1.9085 (0.6); -0.0002 (17.3) |
| I-090: ¹H-NMR(400.0 MHz, CDCl₃): |
| δ= 7.4854 (1.9); 7.2621 (19.2); 7.2027 (2.6); 7.1879 (2.6); 7.0837 (2.4); 7.0648 (3.0); 7.0157 (2.2); 5.2997 (1.7); 4.8553 (1.4); 4.7790 (1.6); 4.6410 (2.3); 4.5237 (3.1); 3.9030 (16.0); 3.8642 (2.6); 3.7878 (2.5); 3.5524 (1.3); 3.4024 (1.4); 3.2132 (1.0); 2.9255 (1.2); 1.8943 (2.4); 1.7482 (2.7); 1.2537 (2.4); 1.0015 (0.9); 0.0689 (4.0); -0.0002 (25.6) |
| I-091: ¹H-NMR(400.6 MHz, CDCl₃): |
| δ= 7.5256 (0.7); 7.5192 (0.7); 7.4852 (0.8); 7.2984 (0.5); 7.2607 (91.1); 7.2240 (0.5); 7.2155 (0.6); 7.2090 (1.0); 7.1904 (1.2); 7.1854 (0.8); 7.1765 (0.7); 7.1721 (0.7); 7.0865 (0.7); 7.0681 (1.2); 7.0485 (0.9); 7.0163 (0.8); 6.9971 (1.0); 5.3001 (1.3); 4.7665 (1.4); 4.4466 (0.9); 4.4327 (2.1); 4.4198 (2.3); 4.4059 (1.0); 3.9046 (16.0); 3.8170 (1.9); 3.2446 (0.6); 1.8863 (0.8); 1.7596 (0.9); 1.5603 (2.5); 1.2539 (1.6); 0.0689 (0.6); 0.0080 (3.4); -0.0002 (131.3); -0.0085 (4.3) |
| I-092: ¹H-NMR(400.6 MHz, CDCl₃): |
| δ= 7.5194 (0.6); 7.4744 (0.8); 7.2975 (0.7); 7.2896 (0.8); 7.2857 (0.5); 7.2610 (91.4); 7.2249 (0.6); 7.2202 (0.6); 7.2115 (0.8); 7.2045 (1.4); 7.1881 (1.5); 7.1814 (1.1); 7.1726 (0.8); 7.1683 (1.0); 7.0836 (1.1); 7.0658 (1.8); 7.0467 (1.0); 7.0119 (1.1); 6.9973 (1.0); 4.7832 (0.6); 4.7252 (0.8); 4.5233 (0.5); 3.8983 (16.0); 3.5692 (0.6); 3.4108 (2.6); 3.3957 (2.6); 1.9323 (0.9); 1.9142 (1.2); 1.9006 (1.2); 1.7445 (1.2); 1.6307 (1.9); 1.2541 (0.6); 1.1108 (0.6); 0.5106 (2.0); 0.4940 (2.0); 0.2042 (2.4); 0.1456 (0.5); 0.0080 (3.4); -0.0002 (135.6); -0.0085 (4.2) |
| I-095: ¹H-NMR(400.6 MHz, CDCl₃): |
| δ= 7.4901 (0.8); 7.3004 (0.6); 7.2611 (51.9); 7.2297 (0.6); 7.2241 (0.5); 7.2161 (0.7); 7.2095 (1.0); 7.1909 (1.0); 7.1861 (0.8); 7.1774 (0.6); 7.1729 (0.7); 7.0861 (0.9); 7.0683 (1.4); 7.0481 (0.9); 7.0158 (0.8); 6.9975 (0.8); 5.3000 (6.3); 4.8358 (0.6); 4.8101 (0.5); 4.0743 (0.9); 4.0408 (1.6); 4.0065 (0.9); 3.9236 (1.1); 3.9115 (16.0); 3.8908 (0.6); 2.1718 (0.6); 1.8932 (0.9); 1.8533 (0.9); 1.8140 (0.6); 1.7699 (0.9); 1.2542 (1.2); 1.2226 (0.8); 1.0493 (0.6); 1.0311 (0.9); 0.0080 (1.8); -0.0002 (74.8); -0.0085 (2.3) |
| I-096: ¹H-NMR(400.0 MHz, CDCl₃): |
| δ= 7.5177 (4.0); 7.3773 (0.8); 7.3609 (0.8); 7.2657 (8.9); 7.1114 (2.4); 6.8718 (0.6); 6.8559 (0.6); 6.8482 (0.6); 6.8400 (0.8); 6.8192 (0.9); 6.7949 (0.7); 6.7891 (0.8); 6.7718 (0.6); 6.7661 (0.7); 4.0942 (1.2); 4.0388 (0.9); 3.9910 (12.7); 3.9285 (9.1); 3.9247 (16.0); 3.8916 (9.1); 3.2791 (1.5); 3.2627 (2.2); 3.2462 (1.2); 2.8948 (0.6); 2.8714 (0.6); 2.1242 (1.6); 1.7878 (0.6); 1.7718 (0.8); 1.7575 (0.7); 1.7420 (0.8); 1.7302 (0.5); 1.4276 (0.8); 1.4134 (0.8); 1.2556 (1.0); -0.0002 (11.9) |
| I-097: ¹H-NMR(400.0 MHz, CDCl₃): |
| δ= 7.5308 (4.4); 7.2677 (6.2); 7.1194 (2.6); 7.1100 (0.9); 6.9938 (1.0); 6.9825 (1.0); 6.9715 (0.6); 6.9601 (0.6); 6.9074 (0.7); 6.9012 (0.9); 6.8916 (1.5); 6.8876 (1.0); 6.8829 (0.9); 6.8765 (0.9); 6.8732 (0.8); 4.4956 (0.5); 3.9922 (13.8); 3.9340 (7.8); 3.9273 (16.0); 3.8946 (8.2); 3.3062 (0.6); 3.2999 (0.6); 3.2777 (0.8); 3.2610 (2.0); 3.2445 (1.0); 2.9347 (0.6); 2.9118 (0.6); 2.9016 (0.5); 2.8785 (0.5); 2.0792 (5.7); 2.0456 (1.0); 1.8354 (0.6); 1.8183 (0.8); 1.8021 (0.5); 1.7958 (0.6); 1.7869 (0.8); 1.7736 (1.2); 1.7638 (0.8); 1.7561 (0.8); 1.7436 (0.6); 1.3337 (0.7); 1.2848 (1.0); 1.2568 (1.8); -0.0002 (8.2) |
| I-098: ¹H-NMR(400.6 MHz, CDCl₃): |
| δ= 7.5203 (0.6); 7.4790 (1.0); 7.3336 (0.7); 7.3152 (0.9); 7.2899 (1.0); 7.2618 (81.4); 7.2248 (1.3); 7.2117 (1.4); 7.2052 (2.1); 7.1872 (2.2); 7.1816 (1.7); 7.1728 (1.3); 7.1684 (1.5); 7.1067 (0.8); 7.0878 (1.6); 7.0651 (2.2); 7.0455 (1.8); 7.0141 (1.9); 6.9981 (1.7); 5.9602 (0.6); 5.3000 (16.0); 5.2711 (0.9); 5.1848 (1.2); 5.1592 (1.1); 4.7701 (1.2); 4.7211 (1.0); 4.6910 (0.8); 4.5220 (0.7); 4.1166 (2.4); 3.9380 (2.2); 3.9022 (15.7); 3.8664 (0.8); 3.7631 (0.6); 3.7465 (1.1); 3.7299 (0.6); 3.5533 (0.9); 3.4073 (1.1); 3.3706 (1.0); 3.2115 (1.1); 3.1106 (0.6); 3.0857 (0.5); 2.9264 (0.8); 2.1718 (1.5); 1.9232 (1.4); 1.9052 (1.6); 1.8878 (1.6); 1.8697 (1.7); 1.8532 (2.2); 1.8366 (1.4); 1.7570 (2.1); 1.7399 (2.0); 1.7025 (1.5); 1.4637 (0.7); 1.4322 (1.1); 1.2547 (2.0); 1.0360 (1.8); 1.0178 (3.0); 0.9998 (1.4); 0.0080 (2.9); -0.0002 (119.1); - 0.0085 (3.9) |
| I-099: ¹H-NMR(400.6 MHz, CDCl₃): |
| δ= 7.4730 (0.6); 7.2987 (0.5); 7.2885 (0.5); 7.2614 (63.2); 7.2247 (0.6); 7.2114 (0.7); 7.2046 (1.2); 7.2016 (1.1); 7.1914 (1.1); 7.1864 (1.3); 7.1813 (1.0); 7.1726 (0.7); 7.1682 (0.9); 7.0840 (0.9); 7.0653 (1.4); 7.0450 (0.9); 7.0135 (0.9); 6.9977 (0.8); 4.6762 (0.7); 3.8983 (16.0); 3.5115 (2.1); 1.9042 (0.9); 1.8885 (1.0); 1.7437 (1.1); 1.6253 (3.8); 1.2539 (0.8); 0.9137 (2.2); 0.8950 (4.0); 0.8788 (2.4); 0.0080 (2.2); - 0.0002 (92.1); -0.0085 (2.8) |
| I-101: ¹H-NMR(400.6 MHz, CDCl₃): |
| δ= 7.5195 (0.8); 7.4968 (1.1); 7.3227 (0.8); 7.3042 (0.9); 7.2994 (1.1); 7.2900 (0.9); 7.2610 (86.5); 7.2290 (0.8); 7.2159 (1.0); 7.2102 (1.4); 7.1912 (1.4); 7.1770 (0.8); 7.1730 (0.8); 7.1107 (0.5); 7.0918 (1.4); 7.0695 (1.8); 7.0509 (1.2); 7.0420 (1.1); 7.0170 (1.2); 6.9973 (1.1); 4.9887 (0.7); 4.9525 (0.9); 4.9071 (0.8); 4.8799 (0.6); 4.5325 (0.5); 4.0912 (1.2); 4.0629 (2.0); 4.0346 (1.2); 3.9397 (0.7); 3.9233 (3.0); 3.9133 (16.0); 3.5453 (0.6); 3.4084 (0.8); 3.2029 (0.6); 2.9274 (0.7); 2.7491 (0.8); 1.9281 (0.9); 1.9099 (1.1); 1.8959 (1.1); 1.7666 (1.3); 1.6857 (0.6); 1.6596 (1.0); 1.6509 (1.5); 1.4322 (0.6); 1.2544 (1.2); 1.2226 (1.1); 1.0158 (1.4); 0.9979 (2.3); 0.9804 (0.9); 0.1458 (0.5); 0.0382 (0.6); 0.0080 (4.0); -0.0002 (133.1); -0.0085 (3.3) |
| I-102: ¹H-NMR(400.6 MHz, CDCl₃): |
| δ= 7.5204 (0.6); 7.4797 (1.4); 7.3105 (1.3); 7.2619 (80.6); 7.2330 (0.7); 7.2247 (1.4); 7.2198 (1.1); 7.2115 (1.4); 7.2057 (2.2); 7.2016 (2.1); 7.1866 (2.5); 7.1814 (1.9); 7.1726 (1.5); 7.1683 (1.6); 7.1102 (0.7); 7.0858 (1.6); 7.0684 (2.2); 7.0382 (1.7); 7.0167 (1.8); 6.9983 (1.6); 4.9094 (0.7); 4.8946 (0.8); 4.8803 (1.1); 4.8076 (1.2); 4.7790 (1.0); 4.5163 (0.8); 4.2142 (0.5); 4.1997 (0.5); 3.8974 (16.0); 3.8553 (2.7); 3.7992 (2.6); 3.5515 (0.9); 3.4151 (1.1); 3.2174 (0.8); 2.9189 (1.8); 1.9512 (0.9); 1.9327 (1.5); 1.9138 (1.9); 1.9016 (1.9); 1.8838 (1.7); 1.7410 (2.2); 1.6897 (0.8); 1.6619 (1.5); 1.6526 (1.6); 1.4322 (0.8); 1.3331 (0.6); 1.2543 (2.9); 1.2224 (0.9); 1.2103 (0.8); 1.0752 (2.0); 1.0191 (2.7); 1.0025 (2.9); 0.9845 (1.3); 0.9596 (0.7); 0.9059 (0.6); 0.8965 (0.5); 0.8791 (1.1); 0.8617 (0.5); 0.7128 (2.3); 0.6967 (2.5); 0.0079 (2.9); -0.0002 (120.1); -0.0085 (3.8) |
| I-103: ¹H-NMR(400.6 MHz, CDCl₃): |
| δ= 7.4723 (0.6); 7.2620 (24.5); 7.2282 (0.6); 7.2240 (0.5); 7.2156 (0.6); 7.2090 (1.0); 7.1908 (1.1); 7.1854 (0.8); 7.1767 (0.6); 7.1722 (0.7); 7.0875 (0.9); 7.0693 (1.2); 7.0508 (0.8); 7.0178 (0.8); 6.9983 (0.7); 5.2998 (8.9); 4.8707 (0.6); 4.0400 (0.7); 4.0236 (1.2); 4.0073 (1.5); 3.9910 (1.1); 3.9004 (16.0); 3.5361 (0.5); 3.4061 (0.5); 2.1716 (2.0); 1.8870 (0.7); 1.8687 (0.7); 1.7544 (1.1); 1.6770 (0.5); 1.3340 (3.1); 1.3180 (3.3); 1.2546 (0.9); 1.0260 (0.7); 1.0091 (1.1); 0.0080 (1.1); -0.0002 (35.9); -0.0085 (1.0) |
| I-104: ¹H-NMR(400.6 MHz, CDCl₃): |
| δ= 7.4551 (0.5); 7.2885 (0.6); 7.2610 (77.9); 7.2245 (0.5); 7.2113 (0.6); 7.2047 (1.0); 7.2014 (1.0); 7.1880 (1.1); 7.1811 (0.9); 7.1725 (0.6); 7.1680 (0.8); 7.0852 (0.8); 7.0656 (1.2); 7.0463 (0.7); 7.0143 (0.9); 6.9973 (0.8); 4.7008 (0.8); 3.8933 (16.0); 3.7675 (0.6); 3.7527 (0.8); 3.7382 (0.7); 1.9034 (0.8); 1.8860 (0.8); 1.7461 (0.9); 1.6067 (4.4); 1.2534 (0.9); 1.2165 (5.6); 1.2023 (5.8); 0.0691 (0.9); 0.0273 (0.5); 0.0080 (3.0); -0.0002 (114.5); -0.0085 (3.2) |
| I-105: ¹H-NMR(400.6 MHz, CDCl₃): |
| δ= 7.2629 (11.6); 7.2518 (0.6); 7.2477 (0.5); 7.2325 (1.2); 7.2185 (1.1); 7.2135 (1.0); 7.2087 (0.5); 7.1997 (0.6); 7.0932 (1.0); 7.0902 (1.3); 7.0718 (3.0); 7.0559 (0.7); 7.0526 (1.5); 7.0476 (1.2); 7.0266 (0.7); 7.0236 (0.5); 4.8827 (1.7); 4.8520 (3.6); 4.8012 (3.8); 4.7882 (0.5); 4.7705 (1.9); 4.0309 (0.9); 4.0152 (1.2); 4.0086 (1.1); 3.9927 (1.0); 3.9011 (16.0); 3.7067 (1.8); 3.6892 (5.6); 3.6717 (5.7); 3.6541 (1.9); 3.3426 (0.5); 3.3347 (0.5); 2.0451 (1.3); 1.6016 (0.7); 1.2977 (6.1); 1.2802 (12.6); 1.2627 (6.0); 1.2596 (1.0); -0.0002 (18.2); -0.0085 (0.5) |
| I-106: ¹H-NMR(400.6 MHz, CDCl₃): |
| δ= 7.4635 (0.6); 7.3857 (0.9); 7.3799 (0.5); 7.3730 (0.9); 7.3696 (0.8); 7.3625 (1.1); 7.2618 (20.6); 7.2109 (0.6); 7.1990 (2.5); 7.1968 (2.7); 7.1927 (1.9); 7.1863 (2.9); 7.1800 (1.7); 7.1755 (1.7); 7.1737 (1.5); 5.3000 (1.8); 4.8773 (1.0); 4.8590 (0.7); 4.8467 (2.1); 4.8002 (1.5); 4.7694 (0.7); 4.0529 (1.0); 4.0324 (1.6); 4.0147 (1.1); 3.8948 (16.0); 3.7049 (1.4); 3.6873 (4.4); 3.6698 (4.4); 3.6523 (1.4); 2.0453 (1.4); 1.5773 (1.7); 1.2966 (5.4); 1.2791 (11.2); 1.2616 (5.3); 0.0079 (0.9); -0.0002 (31.5); -0.0085 (0.9) |
| I-107: ¹H-NMR(400.6 MHz, CDCl₃): |
| δ= 7.2627 (12.1); 7.1998 (0.5); 7.1842 (2.8); 7.1794 (1.8); 7.1707 (1.3); 7.1649 (1.4); 7.1509 (1.4); 7.1308 (0.5); 7.0033 (0.8); 6.9974 (0.6); 6.9856 (0.7); 6.9799 (1.4); 6.9762 (0.8); 6.9605 (0.6); 6.9565 (0.6); 6.0897 (1.0); 5.9615 (1.0); 5.9509 (2.0); 5.9402 (1.0); 5.8121 (1.0); 4.9761 (0.9); 4.9454 (1.8); 4.8838 (1.4); 4.8529 (1.0); 4.2112 (0.9); 4.1955 (1.0); 4.1895 (0.6); 4.1735 (0.5); 4.1307 (0.6); 4.1129 (0.6); 4.0610 (0.5); 4.0523 (0.7); 4.0379 (0.7); 3.9057 (16.0); 3.8534 (1.3); 3.8430 (1.3); 3.8188 (2.7); 3.8082 (2.6); 3.7841 (1.3); 3.7736 (1.3); 2.0452 (2.6); 1.5935 (4.0); 1.2773 (0.8); 1.2595 (1.5); 1.2416 (0.7); - 0.0002 (14.7) |
| I-108: ¹H-NMR(400.6 MHz, CDCl₃): |
| δ= 7.2620 (15.2); 7.2524 (0.6); 7.2359 (1.1); 7.2333 (1.0); 7.2217 (1.0); 7.2175 (1.0); 7.2037 (0.6); 7.0953 (1.0); 7.0923 (1.2); 7.0740 (2.8); 7.0579 (0.7); 7.0546 (1.4); 7.0497 (1.2); 7.0287 (0.7); 7.0255 (0.5); 6.0981 (0.9); 5.9699 (0.9); 5.9593 (1.8); 5.9487 (0.9); 5.8205 (0.9); 4.9981 (1.5); 4.9677 (2.9); 4.9027 (2.0); 4.8723 (1.1); 4.0384 (0.8); 4.0228 (1.1); 4.0161 (1.0); 4.0003 (1.0); 3.9092 (16.0); 3.8682 (1.4); 3.8577 (1.4); 3.8335 (2.8); 3.8230 (2.7); 3.7989 (1.4); 3.7883 (1.4); 3.3402 (0.5); 3.3327 (0.5); 1.5842 (7.0); -0.0002 (17.1) |
| I-109: ¹H-NMR(400.6 MHz, CDCl₃): |
| δ= 7.4780 (0.6); 7.3866 (0.9); 7.3743 (1.0); 7.3634 (1.1); 7.2611 (35.2); 7.2382 (0.8); 7.2116 (0.7); 7.1990 (3.7); 7.1934 (2.2); 7.1883 (2.5); 7.1825 (2.1); 7.1763 (2.6); 6.0968 (1.0); 5.9687 (1.0); 5.9580 (2.0); 5.9474 (1.0); 5.8193 (1.0); 4.9912 (0.8); 4.9607 (1.6); 4.8968 (1.1); 4.8834 (0.6); 4.8633 (1.1); 4.8513 (0.7); 4.0612 (1.0); 4.0402 (1.6); 4.0231 (1.2); 3.9014 (15.0); 3.8655 (1.7); 3.8549 (1.7); 3.8308 (3.4); 3.8202 (3.3); 3.7962 (1.7); 3.7855 (1.6); 2.0453 (0.8); 1.5630 (16.0); 1.2595 (0.6); 0.0080 (1.0); -0.0002 (38.0); -0.0085 (1.2) |
| I-110: ¹H-NMR(400.6 MHz, CDCl₃): |
| δ= 7.2618 (19.5); 7.1988 (0.5); 7.1832 (2.5); 7.1802 (2.2); 7.1681 (1.1); 7.1623 (1.3); 7.1482 (1.3); 7.0030 (0.7); 6.9977 (0.6); 6.9849 (0.6); 6.9796 (1.3); 6.9760 (0.8); 6.9601 (0.6); 6.9562 (0.6); 5.3002 (2.5); 4.8659 (1.1); 4.8348 (2.3); 4.7899 (2.4); 4.7588 (1.0); 4.2071 (0.9); 4.1915 (0.9); 4.1855 (0.5); 4.0539 (0.5); 4.0453 (0.6); 4.0307 (0.7); 3.8990 (16.0); 3.6940 (1.4); 3.6765 (4.5); 3.6590 (4.7); 3.6414 (1.5); 2.0454 (1.3); 1.5719 (2.7); 1.2897 (5.5); 1.2774 (0.7); 1.2722 (11.3); 1.2596 (1.1); 1.2547 (5.4); 0.0080 (0.8); -0.0002 (30.5); -0.0085 (0.9) |
| 1-111: ¹H-NMR(400.6 MHz, CDCl₃): |
| δ= 7.2634 (5.7); 4.5945 (0.7); 4.5647 (1.1); 4.4568 (2.5); 4.4270 (1.7); 3.8241 (13.5); 3.4208 (16.0); - 0.0002 (8.1) |
| 1-112: ¹H-NMR(400.6 MHz, CDCl₃): |
| δ= 7.9403 (2.8); 7.2631 (5.0); 4.7372 (7.6); 3.9182 (10.6); 3.5119 (16.0); -0.0002 (6.8) |
| 1-113: ¹H-NMR(400.0 MHz, CDCl₃): |
| δ= 7.4732 (1.8); 7.3645 (2.0); 7.2613 (15.8); 7.1949 (3.8); 7.1888 (3.8); 5.2999 (2.1); 4.8447 (2.4); 4.8140 (2.9); 4.7612 (2.8); 4.7320 (1.3); 4.0585 (2.0); 4.0386 (3.5); 4.0201 (2.1); 3.8986 (14.9); 3.4987 (16.0); 3.1818 (0.5); 2.3088 (1.1); 2.0805 (1.0); 1.5644 (5.0); -0.0002 (20.6) |
| 1-114: ¹H-NMR(400.6 MHz, CDCl₃): |
| δ= 7.2614 (19.2); 7.1846 (1.9); 7.1814 (1.8); 7.1694 (0.8); 7.1636 (0.9); 7.1495 (1.0); 7.0044 (0.6); 6.9810 (1.0); 6.9775 (0.6); 5.3002 (2.8); 4.8389 (0.9); 4.8075 (1.4); 4.7541 (1.9); 4.7227 (0.9); 4.2176 (0.7); 4.2021 (0.7); 3.9037 (11.8); 3.4897 (16.0); 1.5583 (5.9); 0.0079 (0.8); -0.0002 (27.6); -0.0085 (0.9) |
| 1-115: ¹H-NMR(400.6 MHz, CDCl₃): |
| δ= 7.8378 (1.8); 7.8045 (4.7); 7.2625 (22.8); 7.1851 (0.8); 7.1043 (1.0); 7.0906 (1.1); 7.0850 (1.4); 7.0811 (2.0); 7.0745 (3.6); 7.0606 (0.6); 6.9806 (0.5); 6.9207 (0.7); 6.9163 (0.7); 6.9015 (0.6); 6.8976 (1.2); 6.8903 (0.7); 6.8741 (0.6); 5.3006 (8.3); 4.7340 (0.5); 4.3258 (0.8); 4.3227 (0.9); 4.3185 (1.1); 4.3078 (1.0); 4.3048 (2.3); 4.2994 (2.4); 4.2870 (2.3); 4.2816 (2.3); 4.2691 (0.8); 4.2638 (0.7); 4.2252 (0.7); 4.2087 (0.8); 4.2025 (1.4); 4.1865 (1.3); 4.1790 (1.0); 4.1636 (0.8); 3.9209 (6.2); 3.9061 (16.0); 3.1981 (0.5); 3.1945 (0.5); 3.1733 (0.5); 3.1696 (0.6); 3.1653 (0.7); 3.1616 (0.7); 3.1403 (0.6); 3.1368 (0.6); 2.9173 (0.6); 2.9120 (0.6); 2.9052 (0.6); 2.8998 (0.6); 2.8790 (0.5); 2.3644 (0.6); 2.1144 (0.5); 1.5932 (1.1); 1.3737 (1.8); 1.3637 (4.8); 1.3559 (3.7); 1.3459 (9.9); 1.3381 (1.9); 1.3281 (4.7); 0.8819 (0.6); 0.0080 (0.9); -0.0002 (33.1); -0.0085 (0.9) |
| 1-116: ¹H-NMR(400.6 MHz, CDCl₃): |
| δ= 7.4638 (0.6); 7.2608 (61.9); 7.1955 (0.6); 7.1798 (1.4); 7.1753 (3.3); 7.1628 (1.2); 7.1570 (1.4); 7.1432 (1.3); 6.9977 (1.0); 6.9931 (0.6); 6.9806 (0.8); 6.9749 (1.6); 6.9557 (0.8); 6.9517 (0.8); 5.4015 (0.9); 5.3839 (0.5); 5.3002 (4.0); 4.1831 (1.0); 4.1680 (1.0); 4.1617 (0.7); 4.1459 (0.9); 4.1310 (1.9); 4.1132 (1.8); 4.1101 (1.0); 4.0952 (0.7); 4.0336 (0.8); 4.0247 (0.7); 4.0111 (0.8); 3.8995 (0.8); 3.8947 (0.6); 3.8823 (16.0); 3.8799 (10.9); 3.8662 (0.9); 3.8604 (1.0); 3.8459 (0.5); 2.4329 (0.5); 2.4143 (0.7); 2.1089 (0.5); 2.0945 (0.7); 2.0801 (0.8); 2.0689 (1.0); 2.0641 (1.2); 2.0565 (1.2); 2.0455 (7.4); 2.0349 (1.1); 2.0163 (1.1); 2.0013 (0.7); 1.9979 (0.8); 1.9761 (0.7); 1.5552 (5.0); 1.2775 (1.9); 1.2597 (4.0); 1.2418 (1.8); 0.0080 (2.6); 0.0056 (0.8); -0.0002 (93.6); -0.0085 (2.8) |
| 1-117: ¹H-NMR(400.6 MHz, CDCl₃): |
| δ= 7.4405 (0.5); 7.2613 (33.6); 7.2477 (1.0); 7.2297 (1.7); 7.2159 (1.5); 7.1954 (0.7); 7.0902 (1.6); 7.0709 (2.9); 7.0527 (1.2); 7.0437 (1.8); 7.0226 (1.0); 5.4397 (0.9); 5.4226 (1.7); 5.4056 (0.9); 5.3000 (2.8); 4.7874 (0.7); 4.7763 (0.8); 4.7654 (0.7); 4.1543 (0.6); 4.1461 (0.8); 4.1348 (1.0); 4.1267 (1.0); 4.1158 (0.6); 4.0210 (0.8); 4.0109 (1.1); 4.0057 (1.2); 3.9908 (1.6); 3.9831 (1.3); 3.9733 (1.2); 3.9232 (0.5); 3.9141 (0.7); 3.9041 (1.1); 3.8945 (1.5); 3.8868 (16.0); 3.8826 (14.0); 3.8692 (1.0); 3.3284 (0.6); 2.4549 (0.6); 2.4368 (0.8); 2.4161 (0.6); 2.3878 (0.7); 2.3094 (0.6); 2.0916 (0.6); 2.0807 (0.9); 2.0736 (1.4); 2.0653 (1.5); 2.0557 (1.2); 2.0479 (1.4); 2.0372 (1.5); 2.0311 (1.4); 2.0153 (1.8); 2.0046 (1.6); 1.9926 (1.2); 1.9863 (1.4); 1.9778 (0.8); 1.9674 (0.6); 1.5688 (6.0); 0.0080 (1.5); -0.0002 (48.9); -0.0086 (1.3) |
| 1-118: ¹H-NMR(400.6 MHz, CDCl₃): |
| δ= 7.8359 (1.7); 7.8110 (1.8); 7.2635 (8.8); 7.1069 (0.5); 7.0987 (0.6); 7.0957 (0.6); 7.0357 (0.7); 7.0172 (0.8); 7.0146 (0.8); 5.3003 (3.7); 4.3325 (0.8); 4.3242 (0.5); 4.3212 (0.8); 4.3150 (1.3); 4.3065 (0.9); 4.3035 (0.8); 4.2975 (1.0); 4.2888 (0.8); 3.9148 (16.0); 1.3761 (1.6); 1.3665 (1.8); 1.3583 (3.3); 1.3487 (3.6); 1.3405 (1.7); 1.3309 (1.7); -0.0002 (12.8) |
| 1-119: ¹H-NMR(400.6 MHz, CDCl₃): |
| δ= 7.5609 (1.8); 7.2665 (4.0); 7.2535 (0.6); 7.2362 (0.6); 6.8112 (1.2); 6.7899 (1.2); 6.7672 (0.5); 4.8585 (0.6); 4.8277 (1.0); 4.7269 (1.0); 4.6961 (0.7); 4.4879 (0.6); 3.9208 (16.0); 3.6487 (0.6); 3.4691 (0.6); 3.4467 (0.5); 3.4003 (8.1); 3.1664 (0.6); 1.9639 (0.6); 1.9471 (0.9); 1.9337 (0.8); 1.9168 (0.9); 1.8991 (0.5); 1.8549 (0.5); 1.8409 (0.5); 1.7754 (0.5); 1.7583 (0.5); 1.7118 (0.6); 1.6831 (0.7); -0.0002 (5.8) |
| I-120: ¹H-NMR(400.6 MHz, CDCl₃): |
| δ= 7.5574 (1.7); 7.2848 (0.8); 7.2633 (14.8); 7.2612 (18.1); 7.2020 (0.8); 7.1857 (1.0); 7.0844 (0.6); 7.0663 (1.0); 7.0479 (1.0); 7.0246 (0.9); 7.0001 (0.6); 5.3021 (1.8); 5.3000 (2.2); 4.9157 (0.6); 4.8853 (0.9); 4.7737 (1.0); 4.7420 (0.7); 4.5301 (0.6); 3.9327 (16.0); 3.6363 (0.6); 3.6036 (0.9); 3.5860 (1.9); 3.5684 (2.0); 3.5511 (0.8); 3.4505 (0.5); 3.2171 (0.6); 2.9630 (0.5); 1.9492 (0.6); 1.9327 (0.8); 1.9158 (0.8); 1.8360 (0.6); 1.7421 (1.0); 1.5730 (6.8); 1.2372 (1.8); 1.2200 (3.3); 1.2024 (2.0); 0.0078 (1.2); 0.0019 (21.4); -0.0002 (26.4); -0.0061 (0.9); -0.0085 (0.8) |
| I-121: ¹H-NMR(400.6 MHz, CDCl₃): |
| δ= 7.5926 (3.9); 7.5190 (0.8); 7.2911 (0.9); 7.2661 (2.3); 7.2606 (151.2); 7.2515 (1.9); 7.2476 (1.1); 7.2357 (0.5); 7.2323 (0.6); 7.2222 (0.7); 7.2078 (1.4); 7.1895 (1.8); 7.0892 (1.2); 7.0702 (1.9); 7.0502 (1.8); 7.0264 (1.8); 7.0042 (1.2); 6.9969 (1.6); 5.0509 (0.6); 5.0168 (3.7); 5.0096 (3.8); 4.9774 (0.6); 4.5373 (1.7); 4.0488 (0.8); 4.0269 (2.4); 4.0179 (2.2); 4.0051 (2.5); 3.9960 (6.9); 3.9832 (1.2); 3.9742 (7.5); 3.9685 (2.2); 3.9522 (4.2); 3.9404 (16.0); 3.6399 (1.2); 3.4894 (1.2); 3.2228 (1.0); 3.1970 (1.2); 2.9858 (1.0); 2.9656 (1.1); 2.9311 (0.8); 1.9432 (1.4); 1.8979 (0.9); 1.8556 (1.2); 1.8418 (1.2); 1.8022 (1.0); 1.7855 (1.3); 1.7514 (1.7); 1.5781 (2.9); 0.1458 (0.6); 0.0112 (0.8); 0.0105 (0.9); 0.0080 (6.2); 0.0065 (1.4); 0.0056 (1.4); 0.0049 (1.5); 0.0040 (2.1); -0.0002 (230.4); -0.0085 (6.6); -0.0130 (0.6); - 0.0286 (0.5); -0.1493 (0.6) |
| I-122: ¹H-NMR(400.6 MHz, CDCl₃): |
| δ= 7.5926 (4.0); 7.5762 (1.3); 7.5702 (1.2); 7.5190 (0.8); 7.2883 (1.2); 7.2605 (131.8); 7.2051 (1.5); 7.1904 (1.8); 7.0887 (1.3); 7.0702 (2.0); 7.0500 (1.9); 7.0274 (1.9); 7.0048 (1.3); 6.9969 (1.5); 5.0485 (0.6); 5.0171 (3.8); 5.0096 (3.8); 4.9785 (0.6); 4.5373 (1.8); 4.0491 (0.6); 4.0269 (2.0); 4.0179 (1.9); 4.0051 (2.1); 3.9961 (6.0); 3.9831 (1.2); 3.9742 (6.6); 3.9685 (2.2); 3.9522 (4.0); 3.9406 (16.0); 3.6295 (1.2); 3.4898 (1.3); 3.3032 (7.8); 3.2227 (1.0); 3.1956 (1.3); 3.1713 (0.8); 3.1284 (2.0); 3.0920 (1.9); 3.0494 (0.6); 2.9864 (1.0); 2.9634 (1.1); 2.9309 (0.8); 2.0966 (5.8); 1.9437 (1.4); 1.8991 (0.9); 1.8569 (1.2); 1.7863 (1.3); 1.7566 (1.8); 1.5664 (11.5); 1.5227 (4.8); 0.1462 (0.6); 0.0080 (5.5); -0.0002 (192.1); - 0.0085 (5.8); -0.1493 (0.7) |
| I-123: ¹H-NMR(400.6 MHz, CDCl₃): |
| δ= 7.5535 (1.1); 7.2608 (55.6); 7.2028 (0.5); 7.1844 (0.6); 7.0655 (0.7); 7.0459 (0.7); 7.0235 (0.6); 6.9972 (0.6); 4.9143 (0.6); 4.7960 (0.6); 3.9496 (16.0); 3.3650 (0.9); 3.3559 (1.0); 3.3479 (1.1); 3.3385 (1.0); 1.9312 (0.6); 1.9176 (0.5); 1.7286 (0.7); 1.6441 (0.7); 1.6258 (0.7); 1.3163 (0.5); 1.2761 (0.6); 1.2538 (1.1); 0.9411 (0.8); 0.9229 (1.7); 0.9047 (0.8); 0.5459 (0.9); 0.5273 (1.0); 0.2070 (1.2); 0.1956 (1.2); 0.0690 (0.6); 0.0080 (2.0); -0.0002 (81.0); -0.0085 (2.6) |
| I-127: ¹H-NMR(400.6 MHz, CDCl₃): |
| δ= 7.5574 (9.0); 7.3067 (0.7); 7.2609 (49.5); 7.1951 (0.7); 7.1790 (0.8); 7.0849 (0.5); 7.0665 (0.8); 7.0461 (0.8); 7.0216 (0.8); 6.9973 (0.8); 5.4261 (0.6); 5.3939 (0.8); 5.2324 (0.9); 5.2007 (0.6); 4.5392 (0.5); 3.9430 (16.0); 3.5959 (0.5); 3.2287 (0.6); 1.9269 (0.8); 1.9112 (0.8); 1.8384 (5.0); 1.8263 (5.4); 1.7368 (1.4); 1.6286 (1.2); 0.0080 (2.1); -0.0002 (75.2); -0.0085 (2.0) |
| I-128: ¹H-NMR(400.6 MHz, CDCl₃): |
| δ= 7.5610 (3.4); 7.3068 (1.0); 7.2901 (0.8); 7.2609 (68.4); 7.1793 (1.2); 7.0847 (0.8); 7.0665 (1.2); 7.0469 (1.2); 7.0214 (1.3); 6.9973 (1.2); 5.4300 (0.7); 5.3988 (1.0); 5.2358 (1.1); 5.2037 (0.8); 4.5380 (0.8); 3.9422 (16.0); 3.8969 (2.5); 3.7573 (0.7); 3.5979 (0.8); 3.4849 (2.6); 3.4540 (0.8); 3.4350 (0.7); 3.4097 (0.8); 3.2595 (0.7); 3.2255 (0.8); 2.9841 (0.6); 2.9619 (0.7); 2.9307 (0.5); 2.4370 (1.2); 1.9258 (1.2); 1.8381 (7.6); 1.8258 (7.8); 1.7356 (2.1); 1.5923 (2.1); 1.5510 (0.7); 0.0691 (0.5); 0.0079 (2.9); -0.0002 (101.5); -0.0085 (3.2) |
| I-130: ¹H-NMR(400.6 MHz, CDCl₃): |
| δ= 7.5647 (2.0); 7.5194 (0.7); 7.3233 (0.5); 7.2953 (0.6); 7.2754 (0.9); 7.2698 (1.0); 7.2667 (0.8); 7.2659 (0.8); 7.2610 (118.0); 7.2250 (1.0); 7.2061 (1.3); 7.1868 (1.3); 7.1044 (0.8); 7.0854 (1.6); 7.0668 (1.6); 7.0484 (1.5); 7.0297 (1.6); 7.0116 (1.2); 6.9973 (1.4); 4.8997 (0.6); 4.8622 (0.8); 4.8429 (0.9); 4.8082 (0.8); 4.5333 (0.7); 4.2847 (1.6); 4.2758 (1.3); 4.0977 (0.8); 3.9255 (16.0); 3.9069 (1.3); 3.8813 (1.0); 3.8654 (1.2); 3.8363 (1.8); 3.8138 (2.0); 3.7945 (2.9); 3.7832 (2.4); 3.7695 (1.6); 3.7582 (1.4); 3.6494 (0.9); 3.4911 (0.6); 3.4415 (0.8); 3.4008 (0.8); 3.2286 (0.6); 3.2002 (0.7); 2.9836 (0.6); 2.9622 (0.7); 2.9317 (0.6); 2.0750 (0.7); 2.0203 (2.0); 2.0028 (1.9); 1.9386 (1.8); 1.9210 (1.8); 1.8905 (1.4); 1.8445 (1.3); 1.7395 (1.7); 1.6618 (1.0); 1.3326 (0.5); 1.2542 (4.4); 1.2321 (1.4); 0.9984 (0.8); 0.9819 (1.0); 0.8797 (0.8); 0.1456 (0.6); 0.0692 (0.6); 0.0079 (4.5); 0.0054 (0.6); 0.0046 (0.6); -0.0002 (183.6); -0.0085 (5.6); -0.0124 (0.9); -0.0140 (0.6); -0.0285 (0.5); -0.1493 (0.6) |
| I-131: ¹H-NMR(400.6 MHz, CDCl₃): |
| δ= 7.5739 (3.2); 7.2838 (1.0); 7.2610 (74.8); 7.2281 (1.0); 7.2086 (1.6); 7.1922 (1.7); 7.0891 (1.2); 7.0709 (1.9); 7.0509 (1.8); 7.0276 (1.7); 7.0065 (1.2); 6.9975 (1.1); 4.8727 (0.7); 4.8402 (2.4); 4.8165 (2.4); 4.7841 (0.9); 4.7611 (1.6); 4.7462 (4.8); 4.7418 (4.4); 4.7373 (4.8); 4.7192 (2.5); 4.7035 (1.9); 4.5639 (1.3); 4.5456 (5.2); 4.5405 (4.9); 4.5208 (2.2); 4.5123 (2.1); 4.5029 (2.1); 3.9654 (15.1); 3.6351 (1.2); 3.4814 (1.1); 3.2225 (0.8); 3.1913 (1.1); 2.9805 (0.8); 2.9606 (1.0); 2.9269 (0.7); 2.0754 (0.6); 1.9598 (1.1); 1.9421 (1.5); 1.9292 (1.4); 1.9145 (1.4); 1.8967 (1.1); 1.8508 (1.1); 1.7531 (1.7); 1.5596 (16.0); 0.0078 (2.9); -0.0002 (85.5); -0.0085 (3.4) |
| I-132: ¹H-NMR(400.6 MHz, CDCl₃): |
| δ= 7.5740 (3.3); 7.2614 (55.8); 7.2268 (0.8); 7.2088 (1.5); 7.1912 (1.8); 7.1780 (1.0); 7.0887 (1.2); 7.0705 (1.9); 7.0515 (1.8); 7.0271 (1.8); 7.0054 (1.2); 6.9978 (1.1); 4.8718 (0.8); 4.8403 (2.5); 4.8164 (2.6); 4.7847 (0.9); 4.7613 (1.7); 4.7464 (5.0); 4.7419 (4.6); 4.7373 (5.1); 4.7191 (2.6); 4.7029 (1.9); 4.5643 (1.3); 4.5457 (5.5); 4.5406 (5.3); 4.5210 (2.3); 4.5120 (2.1); 4.5022 (2.1); 3.9654 (16.0); 3.6347 (1.2); 3.4818 (1.1); 3.2249 (0.9); 3.1939 (1.1); 2.9806 (0.9); 2.9590 (1.0); 2.9266 (0.8); 1.9603 (1.1); 1.9428 (1.6); 1.9296 (1.4); 1.9136 (1.4); 1.8976 (1.1); 1.8640 (1.1); 1.8495 (1.1); 1.7545 (1.7); 1.5746 (6.3); 0.0079 (2.0); -0.0002 (59.8); -0.0084 (2.4) |
| I-133: ¹H-NMR(400.0 MHz, CDCl₃): |
| δ= 7.5738 (2.4); 7.2626 (16.7); 7.2270 (0.5); 7.2086 (1.1); 7.1914 (1.3); 7.1767 (0.7); 7.0887 (1.0); 7.0706 (1.4); 7.0503 (1.4); 7.0265 (1.3); 7.0041 (0.8); 4.8709 (0.6); 4.8393 (1.9); 4.8157 (1.8); 4.7843 (0.8); 4.7607 (1.5); 4.7459 (3.8); 4.7414 (3.3); 4.7366 (3.7); 4.7183 (1.8); 4.7033 (1.3); 4.5636 (1.1); 4.5453 (4.1); 4.5398 (3.9); 4.5295 (1.5); 4.5209 (1.6); 4.5108 (1.5); 4.5013 (1.4); 3.9648 (11.9); 3.6347 (0.8); 3.4812 (0.8); 3.2213 (0.6); 3.1926 (0.8); 2.9804 (0.6); 2.9595 (0.7); 2.0067 (16.0); 1.9597 (0.9); 1.9422 (1.2); 1.9292 (1.0); 1.9136 (1.0); 1.8969 (0.8); 1.8625 (0.8); 1.8345 (0.6); 1.7835 (0.9); 1.7550 (1.2); 1.6524 (0.7); 0.0078 (1.0); -0.0002 (22.9); -0.0085 (0.7) |
| I-135: ¹H-NMR(400.6 MHz, CDCl₃): |
| δ= 7.5698 (2.1); 7.2642 (4.3); 7.0364 (1.4); 7.0172 (1.6); 7.0044 (1.7); 4.8553 (0.7); 4.8244 (1.0); 4.7278 (1.1); 4.6968 (0.7); 4.5191 (0.7); 3.9236 (16.0); 3.6552 (0.7); 3.4838 (0.6); 3.4621 (0.6); 3.4030 (8.3); 3.2836 (0.5); 3.2529 (0.6); 3.0107 (0.5); 2.9885 (0.5); 1.9813 (0.7); 1.9646 (1.0); 1.9499 (0.9); 1.9347 (0.9); 1.9170 (0.6); 1.8683 (0.6); 1.8538 (0.6); 1.7857 (0.6); 1.7676 (0.7); 1.7314 (0.8); 1.0067 (0.5); - 0.0002 (6.4) |
| I-136: ¹H-NMR(400.0 MHz, CDCl₃): |
| δ= 7.5671 (3.0); 7.2615 (13.6); 7.0156 (2.8); 4.8535 (1.0); 4.8228 (1.6); 4.7253 (1.6); 4.6958 (1.1); 4.5130 (1.2); 3.9221 (16.0); 3.6480 (1.3); 3.4791 (1.0); 3.4004 (10.9); 3.2806 (0.9); 3.2495 (1.1); 2.9849 (0.9); 1.9615 (1.5); 1.9331 (1.4); 1.8569 (1.2); 1.7297 (1.3); -0.0002 (18.6) |
| I-137: ¹H-NMR(400.0 MHz, CDCl₃): |
| δ= 7.5653 (3.0); 7.2652 (3.5); 7.0339 (2.1); 7.0150 (2.7); 7.0033 (2.8); 4.8537 (1.0); 4.8232 (1.6); 4.7267 (1.6); 4.6947 (1.1); 4.5150 (1.2); 3.9219 (16.0); 3.6491 (1.2); 3.4808 (1.0); 3.4008 (11.1); 3.2797 (0.9); 3.2482 (1.1); 3.0062 (0.7); 2.9826 (0.9); 2.9538 (0.6); 1.9778 (0.9); 1.9621 (1.4); 1.9467 (1.4); 1.9321 (1.3); 1.9146 (0.8); 1.8503 (1.0); 1.7817 (1.0); 1.7643 (1.0); 1.7275 (1.2); -0.0002 (4.6) |
| I-138: ¹H-NMR(400.6 MHz, CDCl₃): |
| δ= 7.6064 (3.9); 7.2608 (21.7); 7.1570 (4.5); 7.1441 (4.9); 7.1312 (6.1); 4.8659 (1.7); 4.8352 (2.5); 4.7278 (2.6); 4.6970 (1.8); 4.5239 (2.0); 3.9202 (15.5); 3.6521 (2.1); 3.6124 (1.9); 3.4472 (2.0); 3.4002 (16.0); 2.6265 (2.8); 2.6025 (3.2); 2.5939 (2.7); 2.5693 (2.4); 2.4626 (11.5); 2.3433 (3.1); 2.0935 (1.0); 2.0352 (1.2); 1.9297 (1.8); 1.8610 (1.7); 1.8281 (2.4); 1.7936 (1.8); 1.7766 (1.9); 1.7531 (2.2); 1.6697 (0.6); 1.0230 (1.2); 1.0047 (1.9); 0.9872 (0.8); 0.0078 (1.6); -0.0002 (30.7) |
| I-139: ¹H-NMR(400.6 MHz, CDCl₃): |
| δ= 7.6059 (3.9); 7.2604 (46.2); 7.1552 (5.7); 7.1312 (6.4); 4.8679 (1.7); 4.8375 (2.6); 4.7289 (2.6); 4.6990 (1.9); 4.5219 (2.3); 3.9197 (15.4); 3.6542 (2.2); 3.6134 (2.1); 3.4476 (1.9); 3.4001 (16.0); 2.6259 (2.7); 2.6014 (3.2); 2.5934 (2.9); 2.5683 (2.4); 2.4625 (12.2); 2.3458 (1.6); 2.0870 (1.8); 2.0255 (2.2); 1.9306 (2.0); 1.8280 (2.8); 1.7446 (2.1); 1.2521 (0.6); -0.0002 (70.4) |
| I-140: ¹H-NMR(400.6 MHz, CDCl₃): |
| δ= 7.6077 (3.8); 7.2617 (5.4); 7.1312 (9.7); 6.9192 (0.6); 4.8406 (2.9); 4.7273 (2.9); 4.5226 (3.4); 3.9183 (14.6); 3.6539 (3.9); 3.3994 (16.0); 2.5974 (3.6); 2.4632 (11.8); 2.3350 (1.6); 2.0360 (2.2); 1.9277 (3.3); 1.8229 (4.4); 1.2539 (0.8); -0.0002 (8.1) |
| I-141: ¹H-NMR(400.2 MHz, d₆-DMSO): |
| δ= 7.7200 (16.0); 7.3230 (1.0); 7.3045 (2.2); 7.2849 (2.6); 7.2646 (2.3); 7.2518 (1.8); 7.1814 (1.5); 7.1595 (3.3); 7.1450 (3.3); 7.0221 (0.3); 4.7268 (1.0); 4.6966 (3.1); 4.6741 (3.2); 4.6434 (1.1); 4.5922 (0.3); 4.5627 (0.5); 4.5468 (0.4); 4.5413 (0.4); 4.5332 (0.4); 4.4503 (0.4); 4.4417 (0.4); 4.3823 (1.8); 4.3215 (0.6); 4.2971 (4.0); 4.2834 (7.9); 4.2698 (4.8); 4.2478 (0.7); 3.7811 (6.9); 3.7670 (13.8); 3.7527 (6.0); 3.5140 (5.1); 3.4975 (12.7); 3.4871 (10.3); 3.4817 (10.4); 3.4753 (7.9); 3.3881 (5.7); 3.3331 (28.1); 3.2502 (24.2); 3.2049 (17.9); 3.1541 (1.8); 2.7875 (1.1); 2.7647 (1.4); 2.7336 (1.0); 2.5979 (0.6); 2.5112 (12.3); 2.5070 (23.9); 2.5026 (31.0); 2.4981 (21.7); 2.4938 (9.9); 1.8031 (2.9); 1.7872 (2.4); 1.7546 (1.1); 1.7285 (1.2); 1.7132 (1.2); 1.6043 (1.4); 1.5928 (1.6); 1.5822 (1.4); 1.4125 (0.8); 1.3954 (0.7); 0.0078 (0.6); -0.0002 (11.9); -0.0085 (0.4) |
| I-142: ¹H-NMR(400.6 MHz, CDCl₃): |
| δ= 7.5562 (1.5); 7.2836 (0.7); 7.2611 (25.7); 7.2010 (0.7); 7.1848 (0.9); 7.0828 (0.6); 7.0656 (0.9); 7.0466 (0.9); 7.0233 (0.8); 6.9977 (0.6); 4.9157 (0.5); 4.8845 (0.8); 4.7734 (0.9); 4.7408 (0.6); 4.5330 (0.5); 3.9321 (16.0); 3.6360 (0.6); 3.6031 (0.8); 3.5845 (1.6); 3.5674 (1.7); 3.5504 (0.8); 3.2152 (0.5); 1.9306 (0.7); 1.9159 (0.7); 1.7341 (0.8); 1.5654 (5.0); 1.2361 (1.6); 1.2188 (2.9); 1.2018 (1.8); 0.0078 (1.0); - 0.0002 (27.7); -0.0084 (1.4) |
| I-143: ¹H-NMR(400.6 MHz, CDCl₃): |
| δ= 7.5560 (0.6); 7.2615 (16.2); 3.9322 (7.4); 3.5851 (0.7); 3.5673 (0.7); 2.6155 (16.0); 1.5708 (3.0); 1.2361 (0.6); 1.2189 (1.2); 1.2019 (0.7); 0.0079 (0.6); -0.0002 (18.1); -0.0085 (0.6) |
| I-144: ¹H-NMR(400.6 MHz, CDCl₃): |
| δ= 7.5617 (1.4); 7.2831 (0.7); 7.2618 (17.6); 7.2027 (0.8); 7.1858 (0.9); 7.0859 (0.6); 7.0670 (1.0); 7.0487 (1.0); 7.0257 (0.9); 7.0035 (0.6); 5.3085 (0.9); 5.2658 (0.8); 5.2146 (0.9); 5.1886 (0.9); 4.9342 (0.6); 4.9032 (0.9); 4.7980 (0.9); 4.7670 (0.6); 4.5312 (0.6); 4.0557 (1.9); 3.9338 (16.0); 3.6355 (0.6); 3.2116 (0.6); 1.9508 (0.5); 1.9342 (0.8); 1.9187 (0.8); 1.8418 (0.5); 1.8283 (0.5); 1.7338 (0.9); 1.5847 (2.0); 0.0079 (0.6); -0.0002 (19.5); -0.0085 (0.9) |
| I-145: ¹H-NMR(400.2 MHz, d₆-DMSO): |
| δ= 7.7070 (4.8); 7.3025 (1.2); 7.2829 (1.4); 7.2651 (1.3); 7.1800 (0.8); 7.1590 (1.7); 7.1442 (1.7); 4.7176 (0.5); 4.6865 (1.5); 4.6609 (1.6); 4.6307 (0.7); 4.3866 (1.2); 4.1651 (1.1); 4.1474 (3.2); 4.1295 (3.5); 4.1120 (1.5); 3.5258 (2.2); 3.5095 (4.4); 3.4927 (2.5); 3.3360 (12.0); 3.2567 (11.2); 3.2094 (1.0); 3.1898 (0.6); 3.1770 (0.8); 3.1640 (0.7); 3.1433 (0.8); 2.7882 (0.5); 2.7638 (0.7); 2.7352 (0.6); 2.5127 (3.3); 2.5083 (6.9); 2.5038 (9.4); 2.4993 (6.9); 2.4950 (3.5); 2.0780 (1.6); 1.8029 (1.6); 1.7288 (0.7); 1.7153 (0.7); 1.5912 (0.8); 1.4103 (0.4); 1.3945 (0.6); 1.3870 (0.5); 1.3658 (7.4); 1.3479 (16.0); 1.3298 (7.2); - 0.0002 (1.0) |
| I-148: ¹H-NMR(400.0 MHz, CDCl₃): |
| δ= 7.9716 (3.4); 7.6058 (4.3); 7.2641 (15.2); 7.2446 (0.7); 6.8351 (0.6); 6.8149 (1.4); 6.7930 (1.3); 6.7676 (0.6); 4.8701 (6.6); 4.8439 (1.2); 4.7425 (1.2); 4.7114 (0.8); 4.4984 (0.7); 4.0771 (3.1); 4.0631 (6.9); 4.0456 (6.6); 3.6712 (0.8); 3.4719 (0.7); 3.4475 (0.6); 3.3920 (11.9); 3.3780 (16.0); 3.2098 (0.6); 3.1747 (0.7); 2.9528 (0.5); 2.9313 (0.6); 2.1030 (1.1); 2.0458 (0.6); 1.9678 (0.8); 1.9505 (1.1); 1.9375 (1.0); 1.9200 (1.1); 1.9021 (0.6); 1.8544 (0.6); 1.8402 (0.6); 1.7719 (0.6); 1.7558 (0.6); 1.7107 (0.7); 1.6955 (0.6); 1.6807 (0.5); 1.3744 (0.6); 1.3643 (1.1); 1.3622 (1.1); 1.3565 (1.0); 1.3526 (0.8); 1.3444 (2.0); 1.3326 (1.3); 1.3244 (1.2); 1.3123 (0.7); 1.3066 (0.6); 1.2846 (0.6); 1.2572 (2.0); 0.6187 (1.1); 0.6077 (4.2); 0.6035 (4.3); 0.5918 (2.8); 0.5872 (4.1); 0.5834 (3.5); 0.5763 (0.9); 0.5722 (1.5); 0.4419 (1.9); 0.4301 (6.0); 0.4182 (4.5); 0.4152 (5.6); 0.4031 (1.5); 0.0079 (0.5); -0.0002 (19.6); -0.0085 (0.6) |
| I-151: ¹H-NMR(400.0 MHz, CDCl₃): |
| δ= 7.5866 (1.0); 7.2637 (17.4); 7.1548 (0.6); 7.1418 (0.7); 7.1222 (0.8); 7.1075 (0.8); 4.8557 (0.8); 4.8175 (0.5); 4.7881 (0.8); 4.6647 (0.5); 3.9337 (1.6); 3.8945 (5.6); 3.6525 (0.6); 3.6354 (0.6); 3.3770 (2.1); 3.3576 (16.0); 1.8576 (0.5); 1.2555 (0.5); -0.0002 (12.3) |
| I-152: ¹H-NMR(400.6 MHz, CDCl₃): |
| δ= 7.5882 (2.1); 7.2778 (4.7); 7.2635 (6.2); 7.1999 (1.2); 7.1864 (1.6); 7.1559 (2.4); 7.1398 (2.7); 7.1226 (3.0); 7.1063 (2.4); 6.9653 (1.6); 6.9520 (1.6); 4.7949 (2.5); 4.6598 (1.3); 4.6423 (1.3); 4.5385 (0.8); 3.9095 (8.1); 3.8949 (9.1); 3.6488 (2.2); 3.4518 (0.5); 3.3729 (15.3); 3.3582 (16.0); 3.0210 (1.1); 2.8647 (0.6); 2.0921 (0.6); 2.0000 (1.4); 1.8726 (2.4); 1.8061 (1.5); 1.7678 (2.6); 1.7520 (2.6); 1.6800 (1.3); 1.0419 (1.1); 1.0250 (2.8); 1.0081 (2.8); 0.9918 (1.3); 0.0139 (7.0); -0.0002 (9.4) |
| I-153: ¹H-NMR(400.0 MHz, CDCl₃): |
| δ= 7.6167 (0.6); 7.2658 (7.1); 4.7920 (0.6); 4.2104 (0.8); 4.1928 (0.9); 3.3544 (16.0); 1.4711 (3.7); 1.4530 (8.1); 1.4349 (3.7); -0.0002 (5.0) |
| I-154: ¹H-NMR(400.0 MHz, CDCl₃): |
| δ= 7.2627 (11.7); 4.8636 (0.8); 3.3688 (2.0); 3.3463 (16.0); 1.5127 (1.5); 1.4947 (8.4); 1.4781 (7.8); - 0.0002 (8.0) |
| I-155: ¹H-NMR(400.0 MHz, CDCl₃): |
| δ= 7.6275 (0.5); 7.2639 (9.4); 4.0354 (1.0); 4.0179 (1.1); 3.3499 (16.0); 1.3284 (0.6); 1.2552 (0.5); 0.5966 (1.0); 0.5928 (1.3); 0.5814 (0.6); 0.5761 (1.1); 0.5725 (1.2); 0.4153 (1.3); 0.4016 (1.2); -0.0002 (7.0) |
| I-158: ¹H-NMR(400.0 MHz, CDCl₃): |
| δ= 7.5615 (1.5); 7.2641 (7.7); 6.8115 (0.9); 6.7896 (0.9); 4.8509 (1.0); 4.8259 (0.7); 4.7261 (0.7); 4.6949 (0.5); 3.9348 (1.8); 3.9216 (16.0); 3.3998 (6.3); 3.3799 (0.6); 3.3746 (2.0); 2.1188 (1.2); 2.0915 (2.0); 1.9467 (0.7); 1.9340 (0.6); 1.9165 (0.7); 1.4304 (0.6); 1.4162 (0.6); 1.2568 (0.9); -0.0002 (10.3) |
| I-160: ¹H-NMR(400.6 MHz, CDCl₃): |
| δ= 7.5571 (1.1); 7.2633 (8.8); 7.1843 (0.5); 7.0664 (0.6); 7.0473 (0.6); 7.0237 (0.5); 4.8643 (0.6); 4.7630 (0.6); 3.9325 (16.0); 3.2743 (1.4); 3.2580 (1.6); 1.9143 (0.5); 1.8895 (0.6); 1.8727 (0.5); 1.8555 (0.5); 1.7372 (0.6); 1.2560 (1.5); 0.9054 (3.7); 0.8891 (3.9); -0.0002 (11.1) |
| I-161: ¹H-NMR(400.6 MHz, CDCl₃): |
| δ= 7.5513 (1.5); 7.2809 (0.6); 7.2650 (10.6); 7.2027 (0.6); 7.1845 (0.8); 7.0856 (0.5); 7.0672 (0.8); 7.0484 (0.8); 7.0242 (0.7); 7.0013 (0.5); 4.9739 (0.5); 4.9417 (0.8); 4.8450 (0.8); 4.8132 (0.6); 3.9485 (16.0); 3.6776 (1.6); 3.6656 (1.7); 3.6435 (0.7); 3.6327 (0.7); 3.6187 (0.6); 3.5943 (1.4); 3.5834 (1.9); 3.5294 (0.6); 3.5120 (1.6); 3.4946 (1.7); 3.4770 (0.9); 3.4535 (0.6); 3.2119 (0.5); 1.9499 (0.6); 1.9317 (0.7); 1.9168 (0.7); 1.7621 (0.6); 1.7465 (0.8); 1.7302 (0.8); 1.2846 (0.6); 1.2555 (1.9); 1.2143 (1.8); 1.1969 (3.5); 1.1795 (2.0); -0.0002 (15.2) |
| I-162: ¹H-NMR(400.6 MHz, CDCl₃): |
| δ= 7.5831 (0.8); 7.2991 (1.0); 7.2941 (0.5); 7.2807 (1.6); 7.2775 (1.7); 7.2606 (5.9); 7.0159 (1.6); 6.9945 (1.6); 6.9735 (0.9); 3.9657 (16.0); -0.0002 (7.3) |
| I-163: ¹H-NMR(400.6 MHz, CDCl₃): |
| δ= 7.5920 (4.2); 7.2912 (0.8); 7.2889 (0.8); 7.2707 (1.7); 7.2699 (1.7); 7.2691 (1.7); 7.2668 (1.6); 7.2627 (29.1); 7.2594 (1.4); 7.2577 (1.2); 7.2569 (1.1); 7.2560 (1.2); 7.2553 (1.2); 7.2545 (1.2); 7.2537 (1.2); 7.2529 (1.1); 7.2521 (1.1); 7.2230 (0.6); 7.2077 (1.3); 7.1900 (1.7); 7.1752 (1.0); 7.0895 (1.3); 7.0712 (2.0); 7.0508 (1.8); 7.0261 (1.8); 7.0040 (1.2); 5.0488 (0.6); 5.0168 (3.9); 5.0085 (4.0); 4.9765 (0.6); 4.5379 (1.6); 4.5283 (1.4); 4.5081 (0.6); 4.0492 (0.7); 4.0273 (2.2); 4.0183 (2.1); 4.0055 (2.4); 3.9965 (6.6); 3.9836 (1.2); 3.9746 (7.2); 3.9696 (2.1); 3.9527 (3.8); 3.9403 (16.0); 3.6888 (0.7); 3.6673 (0.8); 3.6504 (1.2); 3.6391 (1.2); 3.6284 (1.2); 3.6125 (0.7); 3.5066 (0.6); 3.4900 (1.2); 3.4655 (1.0); 3.2229 (1.0); 3.1971 (1.2); 3.1897 (1.2); 2.9845 (1.0); 2.9636 (1.1); 2.9520 (0.9); 2.9313 (0.8); 1.9596 (1.0); 1.9424 (1.3); 1.9313 (1.1); 1.9164 (1.1); 1.8976 (0.8); 1.8708 (0.8); 1.8559 (1.2); 1.8420 (1.2); 1.8032 (1.0); 1.7855 (1.3); 1.7674 (1.6); 1.7528 (1.6); 1.7163 (0.6); 1.6218 (0.9); 1.2562 (1.8); 0.0079 (1.1); - 0.0002 (38.2); -0.0027 (1.9); -0.0035 (1.4); -0.0051 (0.6); -0.0060 (0.5); -0.0068 (0.5); -0.0085 (1.2) |
| I-164: ¹H-NMR(400.6 MHz, CDCl₃): |
| δ= 7.5914 (3.4); 7.2883 (0.8); 7.2609 (82.8); 7.2216 (0.5); 7.2050 (1.1); 7.1899 (1.4); 7.1763 (0.8); 7.0898 (1.0); 7.0712 (1.6); 7.0510 (1.5); 7.0269 (1.5); 7.0036 (1.0); 6.9973 (1.0); 5.0165 (3.2); 5.0082 (3.2); 4.5375 (1.4); 4.0487 (0.6); 4.0268 (1.8); 4.0178 (1.7); 4.0050 (1.9); 3.9960 (5.3); 3.9832 (0.9); 3.9741 (5.7); 3.9690 (1.7); 3.9522 (3.2); 3.9404 (13.1); 3.6518 (0.9); 3.6389 (1.0); 3.6284 (1.0); 3.4901 (1.0); 3.4662 (0.8); 3.2238 (0.8); 3.1894 (1.0); 2.9843 (0.8); 2.9643 (0.9); 2.9520 (0.8); 2.9319 (0.7); 2.0076 (16.0); 1.9597 (0.8); 1.9431 (1.1); 1.9296 (0.9); 1.8978 (0.7); 1.8562 (1.0); 1.8411 (1.0); 1.8024 (0.8); 1.7863 (1.1); 1.7502 (1.4); 1.5607 (9.0); 0.0080 (3.4); 0.0056 (1.0); 0.0048 (1.1); 0.0039 (1.5); -0.0002 (106.4); -0.0067 (1.2); -0.0084 (2.9) |
| I-165: ¹H-NMR(400.6 MHz, CDCl₃): |
| δ= 7.9413 (1.3); 7.5696 (0.6); 7.2646 (2.9); 4.8394 (2.3); 4.2837 (0.5); 4.2656 (1.6); 4.2476 (1.6); 4.2296 (0.6); 1.5013 (1.2); 1.4972 (2.1); 1.4833 (2.7); 1.4792 (4.7); 1.4653 (1.2); 1.4612 (2.1); 1.2889 (16.0); 1.2760 (5.8); 1.2607 (1.2); 1.2573 (1.1); -0.0002 (4.4) |
| I-166: ¹H-NMR(400.2 MHz, d₆-DMSO): |
| δ= 8.0563 (2.0); 7.5657 (2.1); 7.3696 (4.3); 5.7584 (7.6); 5.2004 (1.4); 4.5632 (1.0); 4.5359 (1.4); 4.4321 (2.4); 4.4038 (1.5); 3.8275 (14.4); 3.5683 (0.6); 3.5562 (1.0); 3.5228 (3.4); 3.5043 (4.1); 3.4861 (2.1); 3.4781 (1.3); 3.4597 (0.6); 3.2204 (16.0); 2.8297 (1.0); 2.6811 (0.3); 2.6765 (0.4); 2.5297 (0.5); 2.5165 (7.4); 2.5120 (15.5); 2.5074 (21.0); 2.5028 (14.8); 2.4983 (6.6); 2.0779 (3.5); 1.8110 (2.5); 1.7975 (2.7); 1.6382 (1.5); 1.6205 (1.7); 1.6102 (1.7) |
| I-169: ¹H-NMR(400.0 MHz, CDCl₃): |
| δ= 7.5886 (3.2); 7.2638 (11.0); 7.2386 (0.5); 6.8126 (1.2); 6.7904 (1.1); 4.8599 (0.6); 4.8282 (0.9); 4.7250 (1.0); 4.6945 (0.7); 4.4924 (0.6); 4.2540 (1.0); 4.2388 (2.5); 4.2358 (2.9); 4.2206 (2.5); 4.2178 (2.9); 4.2002 (1.0); 3.6558 (0.7); 3.4639 (0.5); 3.4428 (0.5); 3.3961 (9.7); 3.1691 (0.6); 1.9651 (0.7); 1.9485 (1.0); 1.9351 (0.8); 1.9177 (0.9); 1.9000 (0.5); 1.8354 (0.5); 1.7702 (0.5); 1.7092 (0.6); 1.4888 (7.3); 1.4707 (16.0); 1.4526 (7.3); 1.2557 (1.7); -0.0002 (14.3) |
| I-170: ¹H-NMR(300.1 MHz, d₆-DMSO): |
| δ= 8.0779 (1.7); 8.0100 (0.3); 7.4227 (2.5); 7.3840 (2.3); 7.3480 (1.5); 7.3005 (3.1); 7.2849 (5.1); 7.2616 (3.7); 7.1331 (0.4); 4.5576 (1.7); 4.4717 (2.8); 4.3296 (0.7); 4.2808 (0.6); 4.2040 (0.4); 4.1835 (0.4); 4.1670 (0.3); 4.0818 (0.4); 4.0760 (0.4); 4.0588 (0.4); 4.0037 (0.5); 3.8442 (16.0); 3.6801 (0.7); 3.5518 (4.7); 3.5278 (5.7); 3.5045 (3.3); 3.4673 (2.1); 3.3707 (74.0); 3.2424 (14.2); 3.1900 (3.9); 3.1090 (0.5); 3.0830 (0.4); 3.0077 (0.4); 2.9546 (0.4); 2.8376 (1.4); 2.7498 (1.0); 2.7224 (0.8); 2.6979 (0.8); 2.6544 (0.9); 2.6385 (0.8); 2.5294 (25.7); 2.5235 (34.7); 2.5176 (26.8); 2.2962 (0.3); 2.0958 (4.5); 2.0723 (0.5); 1.9123 (1.5); 1.8306 (3.0); 1.8097 (3.2); 1.7947 (2.9); 1.7588 (1.9); 1.7340 (1.0); 1.6782 (1.8); 1.6538 (2.2); 1.6408 (1.8) |
| I-171: ¹H-NMR(400.2 MHz, d₆-DMSO): |
| δ= 7.7964 (6.3); 7.7527 (1.0); 7.3363 (1.2); 7.3175 (2.5); 7.2978 (2.2); 7.2911 (2.2); 7.2714 (2.3); 7.2570 (1.8); 7.1893 (1.9); 7.1691 (3.9); 7.1527 (3.7); 7.1359 (2.0); 7.0807 (1.0); 7.0625 (1.0); 7.0368 (0.7); 5.4450 (16.0); 5.3806 (0.6); 5.3720 (0.8); 5.3552 (0.4); 4.7203 (1.4); 4.6895 (4.3); 4.6651 (4.5); 4.6345 (1.6); 4.6174 (0.5); 4.6042 (0.4); 4.5345 (0.4); 4.5032 (0.8); 4.4678 (0.7); 4.4361 (0.4); 4.3852 (1.7); 4.3247 (0.6); 3.5140 (4.0); 3.4995 (6.2); 3.4857 (3.6); 3.4318 (0.7); 3.3459 (56.6); 3.2561 (86.0); 3.2187 (2.8); 3.1947 (2.3); 3.1860 (2.2); 3.1611 (2.0); 3.1524 (1.9); 3.0756 (0.6); 2.7904 (1.4); 2.7683 (1.6); 2.7581 (1.6); 2.7358 (1.3); 2.6727 (0.4); 2.6063 (1.0); 2.5933 (1.0); 2.5125 (15.0); 2.5082 (31.2); 2.5037 (42.5); 2.4992 (31.5); 2.4949 (15.9); 2.3307 (0.4); 2.0790 (1.6); 1.8881 (1.0); 1.8599 (1.0); 1.8413 (1.6); 1.8227 (2.6); 1.8070 (3.1); 1.7911 (2.8); 1.7749 (1.9); 1.7614 (1.5); 1.7366 (1.5); 1.7216 (1.6); 1.7083 (1.1); 1.6745 (0.6); 1.6131 (1.4); 1.6010 (1.8); 1.5888 (1.7); 0.9499 (0.4); 0.9326 (0.4); -0.0002 (3.4) |
| I-172: ¹H-NMR(400.2 MHz, d₆-DMSO): |
| δ= 8.0880 (0.4); 7.7301 (16.0); 7.3619 (0.4); 7.3262 (1.4); 7.3070 (2.9); 7.2869 (3.2); 7.2664 (2.9); 7.2530 (2.4); 7.2157 (0.8); 7.1835 (2.1); 7.1632 (4.2); 7.1473 (4.0); 7.0064 (0.4); 6.9910 (0.4); 5.2243 (3.6); 5.2132 (8.3); 5.2023 (4.0); 5.1879 (0.6); 5.1777 (0.5); 4.8791 (0.5); 4.7249 (1.2); 4.6941 (4.1); 4.6739 (4.2); 4.6433 (1.5); 4.5925 (0.6); 4.5766 (0.6); 4.5483 (0.8); 4.5198 (0.7); 4.4439 (1.2); 4.3789 (2.1); 4.2845 (12.4); 4.2734 (12.0); 4.2527 (1.4); 4.2414 (1.1); 3.8908 (2.6); 3.8745 (7.3); 3.8706 (6.6); 3.8661 (7.2); 3.8611 (7.2); 3.8563 (9.5); 3.8493 (6.8); 3.8370 (7.7); 3.8298 (9.7); 3.8251 (8.2); 3.8200 (8.6); 3.8157 (7.7); 3.8117 (8.1); 3.8067 (3.9); 3.7954 (3.2); 3.7869 (1.2); 3.7617 (1.0); 3.5103 (4.9); 3.4940 (8.1); 3.4779 (4.8); 3.4225 (0.8); 3.4026 (1.6); 3.3930 (1.6); 3.3376 (162.1); 3.3147 (2.2); 3.2484 (25.0); 3.2038 (1.8); 3.1920 (2.1); 3.1810 (2.1); 3.1485 (2.0); 2.7864 (1.4); 2.7636 (1.7); 2.7551 (1.7); 2.7326 (1.4); 2.6759 (0.9); 2.6716 (1.1); 2.6670 (0.9); 2.5955 (1.0); 2.5113 (44.8); 2.5070 (88.5); 2.5025 (117.2); 2.4981 (84.8); 2.4938 (41.5); 2.4445 (2.2); 2.3339 (0.6); 2.3294 (0.8); 2.3248 (0.7); 1.8354 (2.2); 1.8017 (3.6); 1.7870 (3.2); 1.7714 (2.3); 1.7321 (1.7); 1.7175 (1.7); 1.6042 (1.6); 1.5927 (2.0); 1.5814 (1.8); 1.4144 (1.1); 1.3976 (1.0); 0.0080 (0.4); -0.0002 (8.9); -0.0084 (0.4) |
| I-173: ¹H-NMR(400.2 MHz, d₆-DMSO): |
| δ= 8.1111 (0.4); 7.7207 (16.0); 7.3805 (0.4); 7.3610 (0.5); 7.3056 (4.3); 7.2851 (4.8); 7.2668 (4.4); 7.1812 (3.1); 7.1611 (6.1); 7.1469 (6.0); 7.0987 (1.6); 7.0006 (0.6); 4.7167 (2.1); 4.6867 (5.2); 4.6562 (5.1); 4.6259 (2.2); 4.5863 (0.6); 4.5567 (0.8); 4.5255 (0.9); 4.5106 (1.0); 4.4727 (0.8); 4.4423 (1.1); 4.3844 (3.4); 4.3402 (1.3); 4.3245 (1.5); 4.3004 (7.6); 4.2882 (13.1); 4.2745 (8.0); 4.2363 (0.8); 4.2219 (0.4); 3.7091 (11.4); 3.6952 (20.8); 3.6814 (9.8); 3.5209 (8.0); 3.5047 (13.6); 3.4884 (7.3); 3.4261 (0.5); 3.4007 (0.7); 3.3343 (38.4); 3.3108 (1.3); 3.2526 (37.5); 3.2268 (73.2); 3.2094 (3.5); 3.1939 (2.2); 3.1755 (2.6); 3.1461 (2.6); 3.0483 (0.4); 2.7875 (2.0); 2.7650 (2.5); 2.7346 (1.8); 2.6721 (0.6); 2.6029 (1.2); 2.5069 (33.0); 2.5030 (38.4); 2.0770 (6.3); 1.8052 (5.6); 1.7316 (2.4); 1.7190 (2.4); 1.5928 (3.0); 1.4146 (1.2); 1.3979 (1.2); -0.0001 (2.5) |
| I-174: ¹H-NMR(300.1 MHz, d₆-DMSO): |
| δ= 7.7720 (5.8); 7.3351 (1.0); 7.3121 (2.0); 7.2887 (2.3); 7.2685 (1.8); 7.1937 (1.4); 7.1500 (2.6); 7.1292 (1.8); 6.5689 (1.9); 5.0869 (13.0); 4.7067 (0.4); 4.6615 (4.6); 4.6168 (0.6); 4.4908 (0.7); 4.3832 (1.5); 3.6754 (14.3); 3.5405 (2.5); 3.5201 (4.3); 3.4416 (1.0); 3.3481 (288.1); 3.1840 (16.0); 2.7955 (0.8); 2.7675 (1.0); 2.7523 (1.0); 2.7289 (1.7); 2.7235 (1.6); 2.6095 (0.5); 2.5087 (142.3); 2.5032 (181.7); 2.4977 (134.9); 2.2732 (1.6); 2.2337 (0.4); 2.1991 (0.4); 1.8210 (2.2); 1.7968 (2.4); 1.7298 (1.1); 1.5966 (1.3); 1.2356 (0.4); 1.2136 (0.4); 1.1507 (0.3); -0.0002 (7.6) |
| I-177: ¹H-NMR(300.1 MHz, d₆-DMSO): |
| δ= 7.7692 (2.5); 7.3074 (1.2); 7.2889 (1.4); 7.2662 (1.5); 7.2451 (1.1); 7.1503 (2.0); 5.3587 (0.6); 5.3343 (1.7); 5.3107 (1.9); 5.2884 (0.6); 4.7613 (0.4); 4.7239 (0.8); 4.6986 (1.0); 4.6648 (1.1); 4.6517 (1.1); 4.6069 (0.8); 4.5544 (0.5); 4.5085 (0.4); 4.3881 (1.1); 4.1320 (1.0); 4.1084 (3.2); 4.0847 (3.5); 4.0609 (1.7); 3.5140 (2.8); 3.5022 (2.8); 3.4425 (0.6); 3.4190 (0.4); 3.3275 (372.1); 3.2070 (6.5); 3.1912 (16.0); 2.7769 (0.7); 2.7655 (0.8); 2.7332 (1.6); 2.7278 (2.0); 2.6177 (0.6); 2.5995 (0.6); 2.5132 (104.3); 2.5075 (195.0); 2.5017 (249.8); 2.4958 (174.5); 2.2780 (1.6); 2.2714 (2.0); 2.2658 (1.5); 2.0754 (0.8); 1.9751 (0.4); 1.9549 (0.3); 1.8314 (2.4); 1.7998 (1.8); 1.7244 (0.8); 1.6792 (5.3); 1.6660 (6.3); 1.6556 (5.7); 1.6425 (6.0); 1.2366 (0.3); 1.1640 (2.6); 1.1412 (5.4); 1.1177 (3.1); 1.0776 (0.8); 1.0549 (1.0); 1.0307 (0.6); 0.9940 (0.4); 0.0106 (4.1); -0.0001 (87.8); -0.0111 (4.2); -0.0630 (0.5); -0.1983 (0.4) |
| I-178: ¹H-NMR(300.1 MHz, d₆-DMSO): |
| δ= 7.9116 (2.7); 7.8638 (0.4); 7.3470 (0.5); 7.3213 (1.2); 7.2955 (1.3); 7.2677 (1.1); 7.2501 (0.8); 7.1929 (0.9); 7.1543 (1.8); 7.1309 (1.2); 7.0835 (0.5); 5.7318 (7.1); 4.7341 (3.6); 4.6915 (0.4); 4.5795 (0.6); 4.3906 (0.7); 3.4928 (2.2); 3.3714 (0.6); 3.3322 (1.3); 3.2755 (11.1); 3.2044 (0.8); 3.1951 (0.8); 3.1495 (0.9); 3.1027 (16.0); 2.8014 (0.6); 2.7718 (0.7); 2.7580 (0.6); 2.7293 (0.6); 2.6027 (0.4); 2.5086 (7.6); 2.5028 (9.6); 2.4969 (6.6); 2.0753 (0.3); 1.8569 (0.9); 1.8138 (1.4); 1.7928 (1.4); 1.7549 (0.8); 1.7303 (0.6); 1.6418 (0.6); 1.6113 (0.8); 1.5950 (0.7); -0.0001 (3.2) |
| I-179: ¹H-NMR(300.1 MHz, d₆-DMSO): |
| δ= 15.7837 (0.5); 8.0751 (0.4); 7.7303 (16.0); 7.3733 (0.5); 7.3080 (2.8); 7.2842 (3.5); 7.1485 (3.9); 4.9204 (0.4); 4.7478 (3.7); 4.7295 (9.7); 4.7117 (4.6); 4.6841 (3.8); 4.6657 (4.0); 4.5477 (0.8); 4.3836 (2.1); 4.3733 (2.1); 4.3037 (0.7); 4.2447 (12.0); 4.2267 (11.3); 3.6369 (0.4); 3.5196 (4.3); 3.4971 (7.0); 3.4750 (4.2); 3.4363 (1.2); 3.3909 (0.8); 3.3224 (274.9); 3.2774 (97.6); 3.2519 (36.1); 3.1818 (2.2); 3.1478 (1.9); 3.0868 (0.5); 3.0699 (0.4); 3.0553 (0.4); 3.0368 (0.5); 2.7921 (1.2); 2.7595 (1.5); 2.7212 (1.7); 2.6114 (0.8); 2.5127 (45.9); 2.5071 (89.3); 2.5012 (117.3); 2.4954 (83.8); 2.2712 (1.0); 2.0743 (1.3); 1.8010 (3.4); 1.7170 (1.7); 1.5974 (2.0); 1.2349 (0.4); 1.1484 (0.6); -0.0002 (33.6) |
| I-180: ¹H-NMR(300.1 MHz, d₆-DMSO): |
| δ= 7.7202 (1.7); 7.2794 (0.6); 7.1439 (0.7); 4.6705 (0.6); 4.6506 (0.6); 4.4238 (0.6); 4.4055 (1.2); 4.3878 (0.9); 4.1511 (0.7); 4.1262 (1.2); 4.1026 (0.8); 3.5268 (0.8); 3.5063 (1.5); 3.4851 (0.9); 3.3242 (6.7); 3.2557 (6.2); 3.2408 (16.0); 3.2084 (0.8); 3.1867 (0.7); 3.1468 (0.5); 2.7286 (0.3); 2.5132 (8.0); 2.5075 (14.8); 2.5016 (18.9); 2.4958 (13.1); 2.0799 (0.6); 2.0748 (0.5); 2.0601 (1.0); 2.0341 (1.0); 2.0131 (0.5); 1.8011 (0.7); 1.5988 (0.4); 0.0105 (0.3); -0.0002 (6.7) |
| I-181: ¹H-NMR(300.1 MHz, d₆-DMSO): |
| δ= 7.7501 (1.5); 7.3108 (0.7); 7.2845 (0.8); 7.2659 (0.7); 7.1877 (0.5); 7.1510 (1.0); 5.2689 (5.7); 4.7084 (1.3); 4.3734 (0.5); 3.5267 (1.0); 3.5053 (1.9); 3.4833 (1.1); 3.3230 (19.8); 3.2584 (6.4); 3.1917 (0.6); 3.1455 (0.5); 2.7955 (0.3); 2.7651 (0.4); 2.7274 (0.5); 2.5129 (18.2); 2.5072 (35.3); 2.5013 (46.4); 2.4954 (33.2); 2.2707 (0.4); 2.1801 (16.0); 2.0744 (0.9); 1.8085 (0.8); 1.7851 (0.8); 1.6013 (0.5); 0.0107 (0.6); - 0.0003 (16.3) |
| I-182: ¹H-NMR(600.3 MHz, CDCl₃): |
| δ= 7.7204 (1.2); 7.3272 (0.7); 7.3127 (0.5); 7.2999 (0.4); 7.2970 (0.4); 7.2870 (0.3); 7.2840 (0.4); 7.2756 (27.8); 7.2299 (0.7); 7.2270 (0.6); 7.2211 (0.8); 7.2176 (1.4); 7.2148 (1.3); 7.2045 (1.5); 7.2012 (1.0); 7.1951 (0.8); 7.1924 (0.9); 7.1184 (0.3); 7.1166 (0.3); 7.1060 (0.5); 7.1042 (0.6); 7.0989 (0.9); 7.0972 (0.9); 7.0938 (0.8); 7.0919 (0.8); 7.0866 (1.3); 7.0847 (1.4); 7.0743 (0.9); 7.0724 (0.9); 7.0684 (0.8); 7.0512 (0.8); 7.0377 (1.1); 7.0233 (0.9); 7.0092 (0.4); 5.4467 (6.0); 4.8134 (0.6); 4.7935 (0.8); 4.7201 (0.8); 4.6998 (0.6); 4.5599 (0.6); 3.6350 (5.2); 3.6306 (1.0); 3.6212 (5.3); 3.6117 (1.0); 3.6073 (5.4); 3.5818 (0.7); 3.4782 (4.0); 3.4570 (1.0); 3.4531 (0.9); 3.4483 (1.3); 3.4428 (1.1); 3.4382 (1.4); 3.4265 (1.1); 3.4163 (1.0); 3.4133 (0.8); 3.3999 (0.8); 3.3865 (0.4); 3.3836 (0.3); 3.2649 (0.5); 3.2447 (0.6); 2.9685 (0.5); 2.9470 (0.6); 2.9373 (0.5); 2.8413 (0.3); 2.0883 (4.6); 2.0645 (2.6); 1.9358 (0.6); 1.9275 (0.7); 1.9167 (0.8); 1.9097 (0.8); 1.8989 (0.7); 1.8880 (0.7); 1.8843 (0.7); 1.8799 (0.7); 1.8714 (1.0); 1.8681 (0.9); 1.8620 (0.8); 1.8583 (0.9); 1.8490 (0.7); 1.8371 (0.4); 1.8179 (0.5); 1.8056 (0.7); 1.7971 (0.7); 1.7846 (1.0); 1.7805 (0.9); 1.7725 (1.0); 1.7684 (1.0); 1.7568 (0.9); 1.6036 (5.2); 1.3272 (0.4); 1.3154 (0.6); 1.3066 (0.6); 1.3028 (0.7); 1.2931 (0.7); 1.2900 (0.6); 1.2781 (1.5); 1.2605 (0.5); 0.9480 (2.7); 0.9340 (3.6); 0.9204 (2.8); 0.9069 (1.9); 0.8953 (4.8); 0.8834 (2.3); 0.0186 (1.1); 0.0131 (39.9); 0.0091 (2.6); 0.0074 (3.3); -0.0001 (50.0); -0.0126 (2.2); -0.1006 (0.3) |
| I-183: ¹H-NMR(400.2 MHz, d₆-DMSO): |
| δ= 8.2973 (3.0); 7.3572 (1.4); 7.3297 (1.9); 7.3120 (2.0); 7.2260 (1.0); 7.2026 (2.4); 7.1834 (2.5); 5.4486 (16.0); 4.6511 (1.1); 4.6215 (1.8); 4.5360 (1.9); 4.5062 (1.2); 4.4178 (1.2); 4.3526 (0.4); 4.3325 (0.4); 3.6177 (4.9); 3.5975 (7.2); 3.5774 (5.2); 3.5431 (3.2); 3.5268 (5.7); 3.5099 (3.4); 3.4245 (3.2); 3.2853 (9.3); 3.2205 (1.4); 3.1841 (1.1); 2.8343 (0.7); 2.8120 (1.0); 2.7815 (0.8); 2.6765 (0.4); 2.5587 (5.0); 2.5544 (10.3); 2.5500 (13.8); 2.5455 (10.1); 2.5412 (5.0); 1.9039 (0.8); 1.8868 (1.4); 1.8669 (1.9); 1.8526 (2.3); 1.8362 (2.6); 1.8204 (2.1); 1.6729 (0.9); 1.6609 (1.2); 1.6513 (1.6); 1.6404 (1.5); 0.9092 (3.6); 0.8885 (5.7); 0.8690 (3.7); 0.0462 (3.3); 0.0360 (1.2); -0.0002 (38.0) |
| I-186: ¹H-NMR(400.6 MHz, CDCl₃): |
| δ= 7.5838 (2.6); 7.2623 (8.5); 6.9991 (1.0); 6.9878 (1.1); 6.8957 (0.7); 6.8858 (0.8); 6.8758 (0.9); 6.8544 (0.5); 4.8530 (0.6); 4.8225 (1.0); 4.7260 (1.0); 4.6943 (0.7); 4.5077 (0.7); 3.9240 (16.0); 3.6499 (0.7); 3.5065 (0.6); 3.4849 (0.6); 3.4023 (9.5); 3.2394 (0.5); 3.2105 (0.6); 2.9153 (0.5); 1.9765 (0.7); 1.9600 (1.0); 1.9459 (0.9); 1.9295 (0.9); 1.9121 (0.6); 1.8670 (0.6); 1.8533 (0.6); 1.7881 (0.7); 1.7733 (0.7); 1.7425 (0.7); 1.7283 (0.8); 1.6987 (0.6); 1.0132 (0.7); 0.0078 (0.7); -0.0002 (12.4) |
| I-187: ¹H-NMR(400.0 MHz, CDCl₃): |
| δ= 7.5781 (3.7); 7.2677 (2.2); 6.9850 (1.8); 6.8737 (1.6); 4.8532 (1.0); 4.8223 (1.5); 4.7254 (1.6); 4.6949 (1.1); 4.4992 (1.2); 3.9215 (16.0); 3.6530 (1.3); 3.5033 (1.0); 3.4866 (0.9); 3.4001 (12.5); 3.2386 (0.9); 3.2080 (1.1); 2.9334 (0.8); 2.9082 (1.0); 2.8802 (0.7); 1.9728 (0.9); 1.9558 (1.4); 1.9408 (1.4); 1.9272 (1.3); 1.9089 (0.9); 1.8513 (1.0); 1.7832 (1.0); 1.7679 (1.0); 1.7245 (1.1); -0.0002 (3.0) |
| I-188: ¹H-NMR(400.0 MHz, CDCl₃): |
| δ= 7.5804 (3.4); 7.2630 (6.0); 6.9863 (1.8); 6.8755 (1.5); 4.8519 (1.0); 4.8230 (1.5); 4.7249 (1.6); 4.6947 (1.1); 4.5031 (1.2); 3.9222 (16.0); 3.6523 (1.2); 3.5019 (1.0); 3.4003 (12.5); 3.2383 (0.9); 3.2078 (1.0); 2.9341 (0.8); 2.9076 (0.9); 1.9710 (0.8); 1.9564 (1.3); 1.9411 (1.4); 1.9271 (1.3); 1.9098 (0.8); 1.8504 (1.0); 1.7843 (1.0); 1.7240 (1.1); -0.0002 (8.1) |
| I-189: ¹H-NMR(400.6 MHz, CDCl₃): |
| δ= 7.5909 (0.8); 7.2624 (21.2); 7.1559 (0.7); 7.1433 (0.7); 7.1238 (0.8); 7.1087 (0.8); 6.9548 (0.5); 4.8140 (0.6); 4.7840 (0.8); 4.6618 (0.5); 3.8955 (5.3); 3.6669 (0.6); 3.6493 (0.7); 3.3587 (16.0); 3.3034 (0.8); 2.6237 (1.5); 2.0965 (0.6); 1.8616 (0.6); 1.7677 (0.6); 1.7515 (0.6); 1.7372 (0.6); 1.5668 (0.5); 0.0080 (0.7); -0.0002 (31.8); -0.0067 (0.5); -0.0084 (1.1) |
| I-190: ¹H-NMR(400.6 MHz, CDCl₃): |
| δ= 7.5869 (1.0); 7.2612 (38.4); 7.1544 (0.7); 7.1426 (0.6); 7.1228 (0.8); 7.1081 (0.8); 4.8157 (0.6); 4.7873 (0.8); 4.6624 (0.5); 3.9077 (0.7); 3.8943 (5.5); 3.6513 (0.6); 3.3581 (16.0); 2.0358 (0.5); 2.0255 (1.2); 2.0079 (0.9); 1.8618 (0.6); 0.0080 (1.6); -0.0002 (55.1); -0.0068 (0.7); -0.0085 (1.6) |
| I-191: ¹H-NMR(400.6 MHz, CDCl₃): |
| δ= 7.5596 (1.4); 7.2642 (8.0); 6.8107 (0.9); 6.7892 (0.9); 4.8266 (0.7); 4.7257 (0.7); 3.9208 (16.0); 3.3999 (6.4); 1.9466 (0.7); 1.9334 (0.6); 1.9163 (0.7); -0.0002 (12.2) |
| I-192: ¹H-NMR(400.6 MHz, CDCl₃): |
| δ= 7.2643 (5.9); 7.2532 (0.8); 7.2346 (1.4); 7.2198 (1.3); 7.2012 (0.6); 7.0942 (1.2); 7.0915 (1.2); 7.0733 (2.8); 7.0538 (1.5); 7.0489 (1.3); 7.0277 (0.8); 4.8512 (1.4); 4.8202 (2.9); 4.8032 (0.6); 4.7923 (0.7); 4.7815 (0.7); 4.7655 (2.9); 4.7344 (1.4); 4.0379 (1.0); 4.0218 (1.4); 4.0160 (1.4); 3.9997 (1.1); 3.9055 (12.8); 3.5013 (16.0); 3.3848 (0.6); 3.3765 (0.6); 3.3504 (0.8); 3.3431 (0.7); 1.6236 (1.4); 0.0079 (0.6); - 0.0002 (8.7) |
| I-193: ¹H-NMR(400.6 MHz, CDCl₃): |
| δ= 7.4641 (0.5); 7.2636 (6.0); 7.2535 (1.0); 7.2350 (1.8); 7.2202 (1.6); 7.2014 (0.7); 7.0935 (1.5); 7.0738 (3.4); 7.0539 (1.8); 7.0492 (1.6); 7.0275 (0.9); 4.8526 (1.5); 4.8216 (3.2); 4.8037 (0.8); 4.7926 (0.8); 4.7815 (0.8); 4.7667 (3.3); 4.7356 (1.5); 4.0383 (1.3); 4.0220 (1.7); 4.0163 (1.8); 4.0004 (1.3); 3.9060 (15.3); 3.5031 (16.0); 3.3839 (0.7); 3.3773 (0.8); 3.3506 (0.9); 3.3428 (0.9); 2.3216 (0.6); 2.0533 (0.5); 2.0372 (0.6); 2.0229 (0.5); 1.6142 (2.1); 0.0078 (0.5); 0.0013 (7.9); -0.0002 (8.7) |
| I-194: ¹H-NMR(400.6 MHz, CDCl₃): |
| δ= 7.4770 (0.9); 7.3869 (1.3); 7.3743 (1.4); 7.3640 (1.5); 7.2632 (11.0); 7.2623 (11.0); 7.2482 (1.1); 7.2122 (0.7); 7.1994 (3.6); 7.1946 (2.4); 7.1877 (3.0); 7.1814 (1.9); 7.1762 (2.3); 4.8858 (0.6); 4.8766 (0.9); 4.8650 (1.1); 4.8500 (1.5); 4.8185 (2.0); 4.7662 (1.9); 4.7354 (0.9); 4.0607 (1.5); 4.0420 (2.3); 4.0227 (1.5); 3.8996 (16.0); 3.5018 (15.2); 2.3385 (0.5); 2.3120 (0.7); 2.2910 (0.5); 2.0845 (0.6); 1.5843 (1.3); 0.0077 (0.9); 0.0008 (15.4); -0.0002 (15.9) |
| I-195: ¹H-NMR(400.6 MHz, CDCl₃): |
| δ= 7.4745 (0.8); 7.3859 (1.2); 7.3802 (0.8); 7.3733 (1.2); 7.3629 (1.3); 7.2631 (7.9); 7.2475 (1.0); 7.2114 (0.8); 7.1979 (3.3); 7.1932 (2.0); 7.1867 (2.8); 7.1804 (1.7); 7.1747 (1.9); 4.8847 (0.6); 4.8757 (0.8); 4.8642 (1.0); 4.8471 (1.4); 4.8311 (0.5); 4.8162 (2.0); 4.7643 (1.8); 4.7332 (0.9); 4.0598 (1.4); 4.0419 (2.0); 4.0218 (1.4); 3.8986 (14.9); 3.4996 (16.0); 2.3092 (0.6); 2.0825 (0.5); 1.6020 (0.7); -0.0002 (11.1) |

The present invention furthermore provides the use of one or more compounds of the general formula (I) and/or salts thereof, as defined above, preferably in one of the embodiments identified as preferred or particularly preferred, in particular one or more compounds of the formulae (I-001) to (I-205) and/or salts thereof, in each case as defined above, as herbicide and/or plant growth regulator, preferably in crops of useful plants and/or ornamental plants.

The present invention furthermore provides a method for controlling harmful plants and/or for regulating the growth of plants, characterized in that an effective amount
- of one or more compounds of the general formula (I) and/or salts thereof, as defined above, preferably in one of the embodiments identified as preferred or particularly preferred, in particular one or more compounds of the formulae (I-001) to (I-205) and/or salts thereof, in each case as defined above, or
- of a composition according to the invention, as defined below, is applied to the (harmful) plants, seeds of (harmful) plants, the soil in which or on which the (harmful) plants grow or the area under cultivation.

The present invention also provides a method for controlling unwanted plants, preferably in crops of useful plants, characterized in that an effective amount
- of one or more compounds of the general formula (I) and/or salts thereof, as defined above, preferably in one of the embodiments identified as preferred or particularly preferred, in particular one or more compounds of the formulae (I-001) to (I-205) and/or salts thereof, in each case as defined above, or
- of a composition according to the invention, as defined below,
   is applied to unwanted plants (for example harmful plants such as mono- or dicotyledonous weeds or unwanted crop plants), the seed of the unwanted plants (i.e. plant seeds, for example grains, seeds or vegetative propagation organs such as tubers or shoot parts with buds), the soil in which or on which the unwanted plants grow (for example the soil of crop land or non-crop land) or the area under cultivation (i.e. the area on which the unwanted plants will grow).

The present invention furthermore also provides methods for regulating the growth of plants, preferably of useful plants, characterized in that an effective amount
- of one or more compounds of the general formula (I) and/or salts thereof, as defined above, preferably in one of the embodiments identified as preferred or particularly preferred, in particular one or more compounds of the formulae (I-001) to (I-205) and/or salts thereof, in each case as defined above, or
- of a composition according to the invention, as defined below, is applied to the plant, the seed of the plant (i.e. plant seed, for example grains, seeds or vegetative propagation organs such as tubers or shoot parts with buds), the soil in which or on which the plants grow (for example the soil of crop land or non-crop land) or the area under cultivation (i.e. the area on which the plants will grow).

In this context, the compounds according to the invention or the compositions according to the invention can be applied for example by pre-sowing (if appropriate also by incorporation into the soil), pre-emergence and/or post-emergence processes. Specific examples of some representatives of the monocotyledonous and dicotyledonous weed flora which can be controlled by the compounds according to the invention are as follows, though there is no intention to restrict the enumeration to particular species.

In a method according to the invention for controlling harmful plants or for regulating the growth of plants, one or more compounds of the general formula (I) and/or salts thereof are preferably employed for controlling harmful plants or for regulating growth in crops of useful plants or ornamental plants, where in a preferred embodiment the useful plants or ornamental plants are transgenic plants.

The compounds of the general formula (I) according to the invention and/or their salts are suitable for controlling the following genera of monocotyledonous and dicotyledonous harmful plants:
Monocotyledonous harmful plants of the genera: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Dicotyledonous harmful plants of the genera: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

When the compounds according to the invention are applied to the soil surface before germination of the harmful plants (weed grasses and/or broad-leaved weeds) (pre-emergence method), either the seedlings of the weed grasses or broad-leaved weeds are prevented completely from emerging or they grow until they have reached the cotyledon stage, but then stop growing and eventually, after three to four weeks have elapsed, die completely.

If the active compounds are applied post-emergence to the green parts of the plants, growth stops after the treatment, and the harmful plants remain at the growth stage at the time of application, or they die completely after a certain time, so that in this manner competition by the weeds, which is harmful to the crop plants, is eliminated very early and in a sustained manner.

Although the compounds according to the invention display an outstanding herbicidal activity against monocotyledonous and dicotyledonous weeds, crop plants of economically important crops, for example dicotyledonous crops of the genera Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, or monocotyledonous crops of the genera Allium, Ananas, Asparagus, Avena, Hordeum, Oryza, Panicum, Saccharum, Secale, Sorghum, triticale, triticum, Zea, are damaged only to an insignificant extent, or not at all, depending on the structure of the respective compound according to the invention and its application rate. For these reasons, the present compounds are very suitable for selective control of unwanted plant growth in plant crops such as agriculturally useful plants or ornamental plants.

In addition, the compounds of the invention (depending on their particular structure and the application rate deployed) have outstanding growth-regulating properties in crop plants. They intervene in the plants' own metabolism with regulatory effect and can thus be used for the controlled influencing of plant constituents and to facilitate harvesting, for example by triggering desiccation and stunted growth. Furthermore, they are also suitable for the general control and inhibition of unwanted vegetative growth without killing the plants in the process. Inhibition of vegetative growth plays a major role for many mono- and dicotyledonous crops since, for example, this can reduce or completely prevent lodging.

By virtue of their herbicidal and plant growth regulatory properties, the active compounds can also be used to control harmful plants in crops of genetically modified plants or plants modified by conventional mutagenesis. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material.

It is preferred with a view to transgenic crops to use the compounds according to the invention and/or their salts in economically important transgenic crops of useful plants and ornamentals, for example of cereals such as wheat, barley, rye, oats, millet, rice and corn or else crops of sugar beet, cotton, soybean, oilseed rape, potato, tomato, peas and other vegetables.

It is preferred to employ the compounds according to the invention as herbicides in crops of useful plants which are resistant, or have been made resistant by recombinant means, to the phytotoxic effects of the herbicides.

By virtue of their herbicidal and plant growth regulatory properties, the active compounds can also be used to control harmful plants in crops of genetically modified plants which are known or are yet to be developed. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material. Further special properties may be tolerance or resistance to abiotic stressors, for example heat, cold, drought, salinity and ultraviolet radiation.

Preference is given to the use of the compounds of the general formula (I) according to the invention or salts thereof in economically important transgenic crops of useful plants and ornamental plants, for example of cereals such as wheat, barley, rye, oats, triticale, millet, rice, cassava and corn, or else crops of sugar beet, cotton, soybean, oilseed rape, potatoes, tomatoes, peas and other vegetables.

The compounds of the general formula (I) can preferably be used as herbicides in crops of useful plants which are resistant, or have been made resistant by recombinant means, to the phytotoxic effects of the herbicides.

Conventional ways of producing novel plants which have modified properties in comparison to existing plants consist, for example, in traditional cultivation methods and the generation of mutants. Alternatively, novel plants with altered properties can be generated with the aid of recombinant methods.

A large number of molecular-biological techniques by means of which novel transgenic plants with modified properties can be generated are known to the person skilled in the art. For such recombinant manipulations, nucleic acid molecules which allow mutagenesis or sequence alteration by recombination of DNA sequences can be introduced into plasmids. With the aid of standard methods, it is possible, for example, to undertake base exchanges, remove parts of sequences or add natural or synthetic sequences. To connect the DNA fragments to each other, adapters or linkers may be added to the fragments.

For example, the generation of plant cells with a reduced activity of a gene product can be achieved by expressing at least one corresponding antisense RNA, a sense RNA for achieving a co-suppression effect, or by expressing at least one suitably constructed ribozyme which specifically cleaves transcripts of the abovementioned gene product.

To this end, it is firstly possible to use DNA molecules which encompass the entire coding sequence of a gene product inclusive of any flanking sequences which may be present, and also DNA molecules which only encompass portions of the coding sequence, in which case it is necessary for these portions to be long enough to have an antisense effect in the cells. It is also possible to use DNA sequences which have a high degree of homology to the coding sequences of a gene product but are not completely identical to them.

When expressing nucleic acid molecules in plants, the protein synthesized may be localized in any desired compartment of the plant cell. However, to achieve localization in a particular compartment, it is possible, for example, to join the coding region to DNA sequences which ensure localization in a particular compartment. Such sequences are known to those skilled in the art (see, for example, Braun et al., EMBO J. 11 (1992), 3219-3227). The nucleic acid molecules can also be expressed in the organelles of the plant cells.

The transgenic plant cells can be regenerated by known techniques to give rise to entire plants. In principle, the transgenic plants may be plants of any desired plant species, i.e., not only monocotyledonous but also dicotyledonous plants.

Thus, transgenic plants can be obtained whose properties are altered by overexpression, suppression or inhibition of homologous (= natural) genes or gene sequences or expression of heterologous (= foreign) genes or gene sequences.

It is preferred to employ the compounds (I) according to the invention in transgenic crops which are resistant to growth regulators such as, for example, dicamba, or to herbicides which inhibit essential plant enzymes, for example acetolactate synthases (ALS), EPSP synthases, glutamine synthases (GS) or hydroxyphenylpyruvate dioxygenases (HPPD), or to herbicides from the group of the sulfonylureas, glyphosate, glufosinate or benzoylisoxazoles and analogous active compounds.

When the active compounds of the invention are employed in transgenic crops, not only do the effects toward harmful plants observed in other crops occur, but frequently also effects which are specific to application in the particular transgenic crop, for example an altered or specifically widened spectrum of weeds which can be controlled, altered application rates which can be used for the application, preferably good combinability with the herbicides to which the transgenic crop is resistant, and influencing of growth and yield of the transgenic crop plants.

The invention therefore also relates to the use of the compounds of the general formula (I) according to the invention and/or their salts as herbicides for controlling harmful plants in crops of useful plants or ornamentals, optionally in transgenic crop plants.

Preference is given to the use in cereals, here preferably corn, wheat, barley, rye, oats, millet or rice, by the pre- or post-emergence method.

Preference is also given to the use in soybeans by the pre- or post-emergence method.

The use according to the invention for the control of harmful plants or for growth regulation of plants also includes the case in which the active compound of the general formula (I) or its salt is not formed from a precursor substance ("prodrug") until after application on the plant, in the plant or in the soil. The invention also provides for the use of one or more compounds of the general formula (I) or salts thereof or of a composition according to the invention (as defined below) (in a method) for controlling harmful plants or for regulating the growth of plants which comprises applying an effective amount of one or more compounds of the general formula (I) or salts thereof onto the plants (harmful plants, if appropriate together with the useful plants), plant seeds, the soil in which or on which the plants grow or the area under cultivation.

The invention also provides an herbicidal and/or plant growth-regulating composition, characterized in that the composition comprises
(a) one or more compounds of the general formula (I) and/or salts thereof, as defined above, preferably in one of the embodiments identified as preferred or particularly preferred, in particular one or more compounds of the formulae (I-001) to (I-205) and/or salts thereof, in each case as defined above,
   and
(b) one or more further substances selected from groups (i) and/or (ii):
   (i) one or more further agrochemically active substances, preferably selected from the group consisting of insecticides, acaricides, nematicides, further herbicides (i.e. those not corresponding to the general formula (I) defined above), fungicides, safeners, fertilizers and/or further growth regulators,
   (ii) one or more formulation auxiliaries customary in crop protection.

Here, the further agrochemical active substances of component (i) of a composition according to the invention are preferably selected from the group of substances mentioned in "The Pesticide Manual", 16th edition, The British Crop Protection Council and the Royal Soc. of Chemistry, 2012.

A herbicidal or plant growth-regulating composition according to the invention comprises preferably one, two, three or more formulation auxiliaries (ii) customary in crop protection selected from the group consisting of surfactants, emulsifiers, dispersants, film-formers, thickeners, inorganic salts, dusting agents, carriers solid at 25 °C and 1013 mbar, preferably adsorbent granulated inert materials, wetting agents, antioxidants, stabilizers, buffer substances, antifoam agents, water, organic solvents, preferably organic solvents miscible with water in any ratio at 25 °C and 1013 mbar.

The compounds of general formula (I) according to the invention can be used in the form of wettable powders, emulsifiable concentrates, sprayable solutions, dusting products or granules in the customary formulations. The invention therefore also provides herbicidal and plant growth-regulating compositions which comprise compounds of the general formula (I) and/or salts thereof.

The compounds of the general formula (I) and/or salts thereof can be formulated in various ways according to which biological and/or physicochemical parameters are required. Possible formulations include, for example: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), dispersions based on oil or water, oil-miscible solutions, capsule suspensions (CS), dusting products (DP), dressings, granules for scattering and soil application, granules (GR) in the form of microgranules, spray granules, absorption and adsorption granules, water-dispersible granules (WG), water-soluble granules (SG), ULV formulations, microcapsules and waxes.

These individual formulation types and the formulation assistants, such as inert materials, surfactants, solvents and further additives, are known to the person skilled in the art and are described, for example, in: Watkins, "Handbook of Insecticide Dust Diluents and Carriers", 2nd Ed., Darland Books, Caldwell N.J.; H.v. Olphen, "Introduction to Clay Colloid Chemistry", 2nd ed., J. Wiley & Sons, N.Y.; C. Marsden, "Solvents Guide", 2nd ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte" [Interface-active Ethylene Oxide Adducts], Wiss. Verlagsgesellschaft, Stuttgart 1976; Winnacker-Küchler, "Chemische Technologie" [Chemical Technology], volume 7, C. Hanser Verlag Munich, 4th Ed. 1986.

Wettable powders are preparations which can be dispersed uniformly in water and, in addition to the active compound, apart from a diluent or inert substance, also comprise surfactants of the ionic and/or nonionic type (wetting agents, dispersants), for example polyoxyethylated alkylphenols, polyoxyethylated fatty alcohols, polyoxyethylated fatty amines, fatty alcohol polyglycol ether sulfates, alkanesulfonates, alkylbenzenesulfonates, sodium lignosulfonate, sodium 2,2'-dinaphthylmethane-6,6'-disulfonate, sodium dibutylnaphthalenesulfonate or else sodium oleoylmethyltaurate. To produce the wettable powders, the herbicidally active compounds are finely ground, for example in customary apparatuses such as hammer mills, blower mills and air-jet mills, and simultaneously or subsequently mixed with the formulation auxiliaries.

Emulsifiable concentrates are produced by dissolving the active compound in an organic solvent, for example butanol, cyclohexanone, dimethylformamide, xylene, or else relatively high-boiling aromatics or hydrocarbons or mixtures of the organic solvents, with addition of one or more ionic and/or nonionic surfactants (emulsifiers). Examples of emulsifiers which may be used are: calcium alkylarylsulfonates such as calcium dodecylbenzenesulfonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters, or polyoxyethylene sorbitan esters, for example polyoxyethylene sorbitan fatty acid esters.

Dusting products are obtained by grinding the active compound with finely distributed solids, for example talc, natural clays, such as kaolin, bentonite and pyrophyllite, or diatomaceous earth. Suspension concentrates may be water- or oil-based. They may be prepared, for example, by wet-grinding by means of commercial bead mills and optional addition of surfactants as have, for example, already been listed above for the other formulation types.

Emulsions, for example oil-in-water emulsions (EW), can be produced, for example, by means of stirrers, colloid mills and/or static mixers using aqueous organic solvents and optionally surfactants as already listed above, for example, for the other formulation types.

Granules can be produced either by spraying the active compound onto adsorptive granular inert material or by applying active compound concentrates to the surface of carriers, such as sand, kaolinites or granular inert material, by means of adhesives, for example polyvinyl alcohol, sodium polyacrylate or else mineral oils. Suitable active compounds can also be granulated in the manner customary for the production of fertilizer granules - if desired as a mixture with fertilizers.

Water-dispersible granules are produced generally by the customary processes such as spray-drying, fluidized-bed granulation, pan granulation, mixing with high-speed mixers and extrusion without solid inert material.

For the production of pan, fluidized-bed, extruder and spray granules, see e.g. processes in "Spray Drying Handbook" 3rd Ed. 1979, G. Goodwin Ltd., London, J.E. Browning, "Agglomeration", Chemical and Engineering 1967, pages 147 ff; "Perry's Chemical Engineer's Handbook", 5th Ed., McGraw Hill, New York 1973, p. 8-57.

For further details regarding the formulation of crop protection compositions, see, for example, G.C. Klingman, "Weed Control as a Science", John Wiley and Sons, Inc., New York, 1961, pages 81-96 and J.D. Freyer, S.A. Evans, "Weed Control Handbook", 5th Ed., Blackwell Scientific Publications, Oxford, 1968, pages 101-103.

The agrochemical preparations, preferably herbicidal or plant growth-regulating compositions, of the present invention preferably comprise a total amount of from 0.1 to 99% by weight, preferably 0.5 to 95% by weight, particularly preferably 1 to 90% by weight, especially preferably 2 to 80% by weight, of active compounds of the general formula (I) and their salts.

In wettable powders, the active compound concentration is, for example, about 10 to 90% by weight, the remainder to 100% by weight consisting of customary formulation constituents. In emulsifiable concentrates, the active compound concentration may be about 1% to 90% and preferably 5% to 80% by weight. Formulations in the form of dusts comprise 1% to 30% by weight of active compound, preferably usually 5% to 20% by weight of active compound; sprayable solutions contain about 0.05% to 80% by weight, preferably 2% to 50% by weight of active compound. In the case of water-dispersible granules, the active compound content depends partially on whether the active compound is in liquid or solid form and on which granulation auxiliaries, fillers, etc., are used. In the water-dispersible granules, the content of active compound is, for example, between 1% and 95% by weight, preferably between 10% and 80% by weight.

In addition, the active compound formulations mentioned optionally comprise the respective customary stickers, wetters, dispersants, emulsifiers, penetrants, preservatives, antifreeze agents and solvents, fillers, carriers and dyes, defoamers, evaporation inhibitors and agents which influence the pH and the viscosity. Examples of formulation auxiliaries are described inter alia in "Chemistry and Technology of Agrochemical Formulations", ed. D.A. Knowles, Kluwer Academic Publishers (1998).

The compounds of the general formula (I) or salts thereof can be used as such or in the form of their preparations (formulations) in a combination with other pesticidally active substances, for example insecticides, acaricides, nematicides, herbicides, fungicides, safeners, fertilizers and/or growth regulators, for example in the form of a finished formulation or of a tank mix. The combination formulations can be prepared on the basis of the abovementioned formulations, while taking account of the physical properties and stabilities of the active compounds to be combined.

Active compounds which can be employed in combination with the compounds of general formula (I) according to the invention in mixture formulations or in a tank mix are, for example, known active compounds based on inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoendesaturase, photosystem I, photosystem II, protoporphyrinogen oxidase, as described, for example, in Weed Research 26 (1986) 441-445 or "The Pesticide Manual", 16th edition, The British Crop Protection Council and the Royal Soc. of Chemistry, 2012 and literature cited therein.

Of particular interest is the selective control of harmful plants in crops of useful plants and ornamentals. Although the compounds of the general formula (I) according to the invention have already demonstrated very good to adequate selectivity in a large number of crops, in principle, in some crops and in particular also in the case of mixtures with other, less selective herbicides, phytotoxicities on the crop plants may occur. In this connection, combinations of compounds of general formula (I) according to the invention are of particular interest which comprise the compounds of general formula (I) or their combinations with other herbicides or pesticides and safeners. The safeners, which are used in an antidotically effective amount, reduce the phytotoxic side effects of the herbicides/pesticides employed, for example in economically important crops, such as cereals (wheat, barley, rye, corn, rice, millet), sugarbeet, sugarcane, oilseed rape, cotton and soybeans, preferably cereals.

The weight ratios of herbicide (mixture) to safener depend generally on the herbicide application rate and the efficacy of the safener in question and may vary within wide limits, for example in the range from 200:1 to 1:200, preferably 100:1 to 1:100, in particular 20:1 to 1:20. Analogously to the compounds (I) or mixtures thereof, the safeners can be formulated with further herbicides/pesticides and be provided and employed as a finished formulation or tank mix with the herbicides.

For application, the herbicide or herbicide/safener formulations present in commercial form are, if appropriate, diluted in a customary manner, for example in the case of wettable powders, emulsifiable concentrates, dispersions and water-dispersible granules with water. Dust-type preparations, granules for soil application or granules for scattering and sprayable solutions are not normally diluted further with other inert substances prior to application.

The application rate of the compounds of the general formula (I) and/or their salts is affected to a certain extent by external conditions such as temperature, humidity, etc. Here, the application rate may vary within wide limits. For the application as a herbicide for controlling harmful plants, the total amount of compounds of the general formula (I) and/or their salts is preferably in the range from 0.001 to 10.0 kg/ha, with preference in the range from 0.005 to 5 kg/ha, more preferably in the range from 0.01 to 1.5 kg/ha, in particular preferably in the range from 0.05 to 1 kg/ha. This applies both to the pre-emergence and the post-emergence application.

When the compounds of the general formula (I) and/or their salts are used as plant growth regulator, for example as culm stabilizer for crop plants like those mentioned above, preferably cereal plants, such as wheat, barley, rye, triticale, millet, rice or corn, the total application rate is preferably in the range of from 0.001 to 2 kg/ha, preferably in the range of from 0.005 to 1 kg/ha, in particular in the range of from 10 to 500 g/ha, very particularly in the range from 20 to 250 g/ha. This applies both to the pre-emergence and the post-emergence application.

The application as culm stabilizer may take place at various stages of the growth of the plants. Preferred is, for example, the application after the tilling phase, at the beginning of the longitudinal growth.

As an alternative, application as plant growth regulator is also possible by treating the seed, which includes various techniques for dressing and coating seed. Here, the application rate depends on the particular techniques and can be determined in preliminary tests.

Active compounds which can be employed in combination with the compounds of the general formula (I) according to the invention in compositions according to the invention (for example in mixed formulations or in the tank mix) are, for example, known active compounds which are based on the inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoene desaturase, photosystem I, photosystem II, protoporphyrinogen oxidase, as are described in, for example, Weed Research 26 (1986) 441-445 or "The Pesticide Manual", 16th edition, The British Crop Protection Council and the Royal Soc. of Chemistry, 2012 and the literature cited therein. Known herbicides or plant growth regulators which can be combined with the compounds according to the invention are, for example, the following active compounds, where the compounds are designated either with the "common name" in accordance with the International Organization for Standardization (ISO) or with the chemical name or with the code number. They always encompass all of the application forms such as, for example, acids, salts, esters and also all isomeric forms such as stereoisomers and optical isomers, even if not explicitly mentioned. Examples of such herbicidal mixing partners are:
acetochlor, acifluorfen, acifluorfen-methyl, acifluorfen-sodium, aclonifen, alachlor, allidochlor, alloxydim, alloxydim-sodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlor-potassium, aminocyclopyrachlor-methyl, aminopyralid, aminopyralid-dimethylammonium, aminopyralid-tripromine, amitrole, ammoniumsulfamate, anilofos, asulam, asulam-potassium, asulam sodium, atrazine, azafenidin, azimsulfuron, beflubutamid, (S)-(-)-beflubutamid, beflubutamid-M, benazolin, benazolin-ethyl, benazolin-dimethylammonium, benazolin-potassium, benfluralin, benfuresate, bensulfuron, bensulfuron-methyl, bensulide, bentazone, bentazone-sodium, benzobicyclon, benzofenap, bicyclopyrone, bifenox, bilanafos, bilanafos-sodium, bipyrazone, bispyribac, bispyribac-sodium, bixlozone, bromacil, bromacil-lithium, bromacil-sodium, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, cambendichlor, carbetamide, carfentrazone, carfentrazone-ethyl, chloramben, chloramben-ammonium, chloramben-diolamine, chlroamben-methyl, chloramben-methylammonium, chloramben-sodium, chlorbromuron, chlorfenac, chlorfenac-ammonium, chlorfenac-sodium, chlorfenprop, chlorfenprop-methyl, chlorflurenol, chlorflurenol-methyl, chloridazon, chlorimuron, chlorimuron-ethyl, chlorophthalim, chlorotoluron, chlorsulfuron, chlorthal, chlorthal-dimethyl, chlorthal-monomethyl, cinidon, cinidon-ethyl, cinmethylin, exo-(+)-cinmethylin, i.e. (1R,2S,4S)-4-isopropyl-1-methyl-2-[(2-methylbenzyl)oxy]-7-oxabicyclo[2.2.1]heptane, exo-(-)-cinmethylin, i.e. (1R,2S,4S)-4-isopropyl-1-methyl-2-[(2-methylbenzyl)oxy]-7-oxabicyclo[2.2.1]heptane, cinosulfuron, clacyfos, clethodim, clodinafop, clodinafop-ethyl, clodinafop-propargyl, clomazone, clomeprop, clopyralid, clopyralid-methyl, clopyralid-olamine, clopyralid-potassium, clopyralid-tripomine, cloransulam, cloransulam-methyl, cumyluron, cyanamide, cyanazine, cycloate, cyclopyranil, cyclopyrimorate, cyclosulfamuron, cycloxydim, cyhalofop, cyhalofop-butyl, cyprazine, 2,4-D (including thea mmonium, butotyl, -butyl, choline, diethylammonium, -dimethylammonium, -diolamine, -doboxyl, -dodecylammonium, etexyl, ethyl, 2-ethylhexyl, heptylammonium, isobutyl, isooctyl, isopropyl, isopropylammonium, lithium, meptyl, methyl, potassium, tetradecylammonium, triethylammonium, triisopropanolammonium, tripromine and trolamine salt thereof), 2,4-DB, 2,4-DB-butyl, -dimethylammonium, isooctyl, - potassium und -sodium, daimuron (dymron), dalapon, dalapon-calcium, dalapon-magnesium, dalapon-sodium, dazomet, dazomet-sodium, n-decanol, 7-deoxy-D-sedoheptulose, desmedipham, detosyl-pyrazolate (DTP), dicamba and its salts, e. g. dicamba-biproamine, dicamba-N,N-Bis(3-aminopropyl)methylamine, dicamba-butotyl, dicamba-choline, dicamba-diglycolamine, dicamba-dimethylammonium, dicamba-diethanolamine ammonium, dicamba-diethylammonium, dicamba-isopropylammonium, dicamba-methyl, dicamba-monoethanolamine, dicamba-olamine, dicamba-potassium, dicamba-sodium, dicamba-triethanolamine, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-butotyl, dichlorprop-dimethylammonium, dichhlorprop-etexyl, dichlorprop-ethylammonium, dichlorprop-isoctyl, dichlorprop-methyl, dichlorprop-potassium, dichlorprop-sodium, dichlorprop-P, dichlorprop-P-dimethylammonium, dichlorprop-P-etexyl, dichlorprop-P-potassium, dichlorprop-sodium, diclofop, diclofop-methyl, diclofop-P, diclofop-P-methyl, diclosulam, difenzoquat, difenzoquat-metilsulfate, diflufenican, diflufenzopyr, diflufenzopyr-sodium, dimefuron, dimepiperate, dimesulfazet, dimethachlor, dimethametryn, dimethenamid, dimethenamid-P, dimetrasulfuron, dinitramine, dinoterb, dinoterb-acetate, diphenamid, diquat, diquat-dibromid, diquat-dichloride, dithiopyr, diuron, DNOC, DNOC-ammonium, DNOC-potassium, DNOC-sodium, endothal, endothal-diammonium, endothal-dipotassium, endothal-disodium, Epyrifenacil (S-3100), EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dione, fenoxaprop, fenoxaprop-P, fenoxaprop-ethyl, fenoxaprop-P-ethyl, fenoxasulfone, fenpyrazone, fenquinotrione, fentrazamide, flamprop, flamprop-isoproyl, flamprop-methyl, flamprop-M-isopropyl, flamprop-M-methyl, flazasulfuron, florasulam, florpyrauxifen, florpyrauxifen-benzyl, fluazifop, fluazifop-butyl, fluazifop-methyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium und -methyl, fluoroglycofen, fluoroglycofen-ethyl, flupropanate, flupropanate-sdium, flupyrsulfuron, flupyrsulfuron-methyl, flupyrsulfuron-methyl-sodium, fluridone, flurochloridone, fluroxypyr, fluroxypyr-butometyl, fluroxypyr-meptyl, flurtamone, fluthiacet, fluthiacet-methyl, fomesafen, fomesafen-sodium, foramsulfuron, foramsulfuron sodium salt, fosamine, fosamine-ammonium, glufosinate, glufosinate-ammonium, glufosinate-sodium, L-glufosinate-ammonium, L-glufosiante-sodium, glufosinate-P-sodium, glufosinate-P-ammonium, glyphosate, glyphosate-ammonium, -isopropylammonium, -diammonium, -dimethylammonium, -potassium, -sodium, sesquisodium and -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, haloxifop-sodium, hexazinone, HNPC-A8169, i.e. prop-2-yn-1-yl (2S)-2-{3-[(5-tert-butylpyridin-2-yl)oxy]phenoxy}propanoate, HW-02, i.e. 1-(Dimethoxyphosphoryl)-ethyl-(2,4-dichlorphenoxy)acetat, hydantocidin, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazaquin.methyl, imazethapyr, imazethapyr-immonium, imazosulfuron, indanofan, indaziflam, iodosulfuron, iodosulfuron-methyl, iodosulfuron-methyl-sodium, ioxynil, ioxynil-lithium, -octanoate, - potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl)sulfonyl)-5,5-dimethyl-4,5-dihydro-1,2-oxazol, ketospiradox, ketospiradox-potassium, lactofen, lancotrione, lenacil, linuron, MCPA, MCPA-butotyl, -butyl, -dimethylammonium, -diolamine, -2-ethylhexyl, -ethyl, - isobutyl, isoctyl, -isopropyl, -isopropylammonium, -methyl, olamine, -potassium, -sodium and - trolamine, MCPB, MCPB-methyl, -ethyl und -sodium, mecoprop, mecoprop-butotyl, mecoprop-demethylammonium, mecoprop-diolamine, mecoprop-etexyl, mecoprop-ethadyl, mecoprop-isoctyl, mecoprop-methyl, mecoprop-potassium, mecoprop-sodium, and mecoprop-trolamine, mecoprop-P, mecoprop-P-butotyl, -dimethylammonium, -2-ethylhexyl and -potassium, mefenacet, mefluidide, mefluidide-diolamine, mefluidide-potassium, mesosulfuron, mesosulfuron-methyl, mesosulfuron sodium salt, mesotrione, methabenzthiazuron, metam, metamifop, metamitron, metazachlor, metazo-sulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinate, monolinuron, monosulfuron, monosulfuron-methyl, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, NC-656, i.e. 3-[(isopropylsulfonyl)methyl]-N-(5-methyl-1,3,4-oxadiazol-2-yl)-5-(trifluoromethyl)[1,2,4]triazolo[4,3-a]pyridine-8-carboxamide, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefone, oxyfluorfen, paraquat, paraquat-dichloride, paraquat-dimethylsulfate, pebulate, pendimethalin, penoxsulam, pentachlorphenol, pentoxazone, pethoxamid, petroleum oils, phenmedipham, phenmedipham-ethyl, picloram, picloram-dimethylammonium, picloram-etexyl, picloram-isoctyl, picloram-methyl, picloram-olamine, picloram-potassium, picloram-triethylammonium, picloram-tripromine, picloram-trolamine, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinclorac-dimethylammonium, quinclorac-methyl, quinmerac, quinoclamine, quizalofop, quizalofop-ethyl, quizalofop-P, quizalofop-P-ethyl, quizalofop-P-tefuryl, QYM201, i.e. 1-{2-chloro-3-[(3-cyclopropyl-5-hydroxy-1-methyl-1H-pyrazol-4-yl)carbonyl]-6-(trifluoromethyl)phenyl}piperidin-2-one, rimsulfuron, saflufenacil, sethoxydim, siduron, simazine, simetryn, SL-261, sulcotrione, sulfentrazone, sulfometuron, sulfometuron-methyl, sulfosulfuron, , SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (trichloro acetic acid) and its salts, e.g. TCA-ammonium, TCA-calcium, TCA-ethyl, TCA-magnesium, TCA-sodium, tebuthiuron, tefuryltrione, tembotrione, tepraloxydim, terbacil, terbucarb, terbumeton, terbuthylazine, terbutryn, tetflupyrolimet, thaxtomin, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, triclopyr-butotyl, triclopyr-choline, triclopyr-ethyl, triclopyr-triethylammonium, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, 3-(2-chloro-4-fluoro-5-(3-methyl-2,6-dioxo-4-trifluoromethyl-3,6-dihydropyrimidin-1 (2H)-yl)phenyl)-5-methyl-4,5-dihydroisoxazole-5-carboxylic acid ethyl ester, ethyl-[(3-{2-chlor-4-fluor-5-[3-methyl-2,6-dioxo-4-(trifluormethyl)-3,6-dihydropyrimidin-1(2H)-yl]phenoxy }pyridin-2-yl)oxy] acetate, 3-chloro-2-[3-(difluoromethyl)isoxazolyl-5-yl]phenyl-5-chloropyrimidin-2-yl ether, 2-(3,4-dimethoxyphenyl)-4-[(2-hydroxy-6-oxocyclohex-1-en-1-yl)carbonyl]-6-methylpyridazine-3(2*H*)-one, 2-({2-[(2-methoxyethoxy)methyl]-6-methylpyridin-3-yl)carbonyl)cyclohexane-1,3-dione, (5-hydroxy-1-methyl-1H-pyrazol-4-yl)(3,3,4-trimethyl-1,1-dioxido-2,3-dihydro-1-benzothiophen-5-yl)methanone, 1-methyl-4-[(3,3,4-trimethyl-1,1-dioxido-2,3-dihydro-1-benzothiophen-5-yl)carbonyl]-1H-pyrazol-5-yl propane-1-sulfonate, 4-{2-chloro-3-[(3,5-dimethyl-1H-pyrazol-1-yl)methyl] -4-(methylsulfonyl)benzoyl-4-(methylsulfonyl)benzoyl}-1-methyl-1H-pyrazol-5-yl-1,3-dimethyl-1H-pyrazole-4-carboxylate; cyanomethyl 4-amino-3-chloro-5-fluoro-6-(7-fluoro-1H-indol-6-yl)pyridine-2-carboxylate, prop-2-yn-1-yl 4-amino-3-chloro-5-fluoro-6-(7-fluoro-1H-indol-6-yl)pyridine-2-carboxylate, methyl 4-amino-3-chloro-5-fluoro-6-(7-fluoro-1H-indol-6-yl)pyridine-2-carboxylate, 4-amino-3-chloro-5-fluoro-6-(7-fluoro-1H-indol-6-yl)pyridine-2-carboxylic acid, benzyl 4-amino-3-chloro-5-fluoro-6-(7-fluoro-1H-indol-6-yl)pyridine-2-carboxylate, ethyl 4-amino-3-chloro-5-fluoro-6-(7-fluoro-1H-indol-6-yl)pyridine-2-carboxylate, methyl 4-amino-3-chloro-5-fluoro-6-(7-fluoro-1-isobutyryl-1H-indol-6-yl)pyridine-2-carboxylate, methyl 6-(1-acetyl-7-fluoro-1H-indol-6-yl)-4-amino-3-chloro-5-fluoropyridine-2-carboxylate, methyl 4-amino-3-chloro-6-[1-(2,2-dimethylpropanoyl)-7-fluoro-1H-indol-6-yl]-5-fluoropyridine-2-carboxylate, methyl 4-amino-3-chloro-5-fluoro-6-[7-fluoro-1-(methoxyacetyl)-1H-indol-6-yl]pyridine-2-carboxylate, potassium 4-amino-3-chloro-5-fluoro-6-(7-fluoro-1H-indol-6-yl)pyridine-2-carboxylate, sodium 4-amino-3-chloro-5-fluoro-6-(7-fluoro-1H-indol-6-yl)pyridine-2-carboxylate, butyl 4-amino-3-chloro-5-fluoro-6-(7-fluoro-1H-indol-6-yl)pyridine-2-carboxylate, 4-hydroxy-1-methyl-3-[4-(trifluoromethyl)pyridin-2-yl]imidazolidin-2-one, 3-(5-tert-butyl-1,2-oxazol-3-yl)-4-hydroxy-1-methylimidazolidin-2-one, 3-[5-chloro-4-(trifluormethyl)pyridin-2-yl]-4-hydroxy-1-methylimidazolidin-2-one, 4-hydroxy-1-methoxy-5-methyl-3-[4-(trifluormethyl)pyridin-2-yl]imidazolidin-2-one, 6-[(2-hydroxy-6-oxocyclohex-1-en-1-yl)carbonyl]-1,5-dimethyl-3-(2-methylphenyl)quinazolin-2,4(1H,3H)-dione, 3-(2,6-dimethylphenyl)-6-[(2-hydroxy-6-oxocyclohex-1-en-1-yl)carbonyl]-1-methylquinazolin-2,4(1H,3H)-dione, 2-[2-chloro-4-(methylsulfonyl)-3-(morpholin-4-ylmethyl)benzoyl]-3-hydroxycyclohex-2-en-1-one, 1-(2-carboxyethyl)-4-(pyrimidin-2-yl)pyridazin-1-ium salt (with anions such as chloride, acetate or trifluoroacetate), 1-(2-carboxyethyl)-4-(pyridazin-3-yl)pyridazin-1-ium salt (with anions such as chloride, acetate or trifluoroacetate), 4-(pyrimidin-2-yl)-1-(2-sulfoethyl)pyridazin-1-ium salt (with anions such as chloride, acetate or trifluoroacetate), 4-(pyridazin-3-yl)-1-(2-sulfoethyl)pyridazin-1-ium salt (with anions such as chloride, acetate or trifluoroacetate), 1-(2-Carboxyethyl)-4-(1,3-thiazol-2-yl)pyridazin-1-ium salt (with anions such as chloride, acetate or trifluoroacetate), 1-(2-Carboxyethyl)-4-(1,3-thiazol-2-yl)pyridazin-1-ium salt (with anions such as chloride, acetate or trifluoroacetate), methyl (2R)-2-{ [(E)-({2-chloro-4-fluoro-5-[3-methyl-2,6-dioxo-4-(trifluoromethyl)-3,6-dihydropyrimidin-1(2H)-yl]phenyl}methylidene)amino]oxy}propanoate, (E)-2-(trifluoromethyl)benzaldehyde O-{2,6-bis[(4,6-dimethoxypyrimidin-2-yl)oxy]benzoyl}oxime, 2-fluoro-N-(5-methyl-1,3,4-oxadiazol-2-yl)-3-[(R)-propylsulfinyl]-4-(trifluoromethyl)benzamide, (2R)-2-[(4-amino-3,5-dichloro-6-fluoro-2-pyridyl)oxy]propanoic acid.

Examples of plant growth regulators as possible mixing partners are: Abscisic acid and related analogs [e.g. (2Z,4E)-5-[6-Ethynyl-1-hydroxy-2,6-dimethyl-4-oxocyclohex-2-en-1-yl]-3-methylpenta-2,4-dienoic acid, methyl-(2Z,4E)-5-[6-ethynyl-1-hydroxy-2,6-dimethyl-4-oxocyclohex-2-en-1-yl]-3-methylpenta-2,4-dienoate, (2Z,4E)-3-ethyl-5-(1-hydroxy-2,6,6-trimethyl-4-oxocyclohex-2-en-1-yl)penta-2,4-dienoic acid, (2E,4E)-5-(1-hydroxy-2,6,6-trimethyl-4-oxocyclohex-2-en-1-yl)-3-(trifluoromethyl)penta-2,4-dienoic acid, methyl (2E,4E)-5-(1-hydroxy-2,6,6-trimethyl-4-oxocyclohex-2-en-1-yl)-3-(trifluoromethyl)penta-2,4-dienoate, (2Z,4E)-5-(2-hydroxy-1,3-dimethyl-5-oxobicyclo[4.1.0]hept-3-en-2-yl)-3-methylpenta-2,4-dienoic acid], acibenzolar, acibenzolar-S-methyl, S-adenosylhomocysteine, allantoin, 2-Aminoethoxyvinylglycine (AVG), aminooxyacetic acid and related esters [e.g. (Isopropylidene)-aminooxyacetic acid-2-(methoxy)-2-oxoethylester, (Isopropylidene)-aminooxyacetic acid-2-(hexyloxy)-2-oxoethylester, (Cyclohexylidene)-aminooxyacetic acid-2-(isopropyloxy)-2-oxoethylester], 1-aminocycloprop-1-yl carboxylic acid and derivatives thereof, e.g. disclosed in DE3335514, EP30287, DE2906507 or US5123951, 5-aminolevulinic acid, ancymidol, 6-benzylaminopurine, bikinin, brassinolide, brassinolide-ethyl, L-canaline, catechin and catechines (e.g. (2S,3R)-2-(3,4-Dihydroxyphenyl)-3,4-dihydro-2H-chromen-3,5,7-triol), chitooligosaccharides (CO; COs differ from LCOs in that they lack the pendant fatty acid chain that is characteristic of LCOs. COs, sometimes referred to as N-acetylchitooligosaccharides, are also composed of GlcNAc residues but have side chain decorations that make them different from chitin molecules [(C₈H₁₃NO₅)ₙ, CAS No. 1398-61-4] and chitosan molecules [(C₅H₁₁NO₄)ₙ, CAS No. 9012-76-4]), chitinous compounds, chlormequat chloride, cloprop, cyclanilide, 3-(Cycloprop-1-enyl)propionic acid, 1-[2-(4-cyano-3,5-dicyclopropylphenyl)acetamido]cyclohexanecarboxylic acid, 1-[2-(4-cyano-3-cyclopropylphenyl)acetamido]cyclohexanecarboxylic acid, daminozide, dazomet, dazomet-sodium, n-decanol, dikegulac, dikegulac-sodium, endothal, endothal-dipotassium, -disodium, and mono(N,N-dimethylalkylammonium), ethephon, flumetralin, flurenol, flurenol-butyl, flurenol-methyl, flurprimidol, forchlorfenuron, gibberellic acid, inabenfide, indol-3-acetic acid (IAA), 4-indol-3-ylbutyric acid, isoprothiolane, probenazole, jasmonic acid, Jasmonic acid or derivatives thereof (e.g. jasmonic acid methyl ester, jasmonic acid ethyl ester), lipo-chitooligosaccharides (LCO, sometimes referred to as symbiotic nodulation (Nod) signals (or Nod factors) or as Myc factors, consist of an oligosaccharide backbone of β*-*1,4-linked *N*-acetyl-D-glucosamine ("GlcNAc") residues with an N-linked fatty acyl chain condensed at the non-reducing end. As understood in the art, LCOs differ in the number of GlcNAc residues in the backbone, in the length and degree of saturation of the fatty acyl chain and in the substitutions of reducing and non-reducing sugar residues), linoleic acid or derivatives thereof, linolenic acid or derivatives thereof, maleic hydrazide, mepiquat chloride, mepiquat pentaborate, 1-methylcyclopropene, 3-methylcyclopropene, 1-ethylcyclopropene, 1-n-propylcyclopropene, 1-cyclopropenylmethanol, methoxyvinylglycin (MVG), 3'-methyl abscisic acid, 1-(4-methylphenyl)-N-(2-oxo-1-propyl-1,2,3,4-tetrahydroquinolin-6-yl)methanesulfonamide and related substituted tetrahydroquinolin-6-yl)methanesulfonamides, (3E,3aR,8bS)-3-({ [(2R)-4-Methyl-5-oxo-2,5-dihydrofuran-2-yl]oxy)methylen)-3,3a,4,8b-tetrahydro-2H-indeno[1,2-b]furan-2-one and related lactones as outlined in EP2248421, 2-(1-naphthyl)acetamide, 1-naphthylacetic acid, 2-naphthyloxyacetic acid, nitrophenolate-mixture, 4-Oxo-4[(2-phenylethyl)amino]butyric acid, paclobutrazol, 4-phenylbutyric acid and its related salts (e.g. sodium-4-phenylbutanoate, potassium-4-phenylbutanoate), phenylalanine, N-phenylphthalamic acid, prohexadione, prohexadione-calcium, putrescine, prohydrojasmon, rhizobitoxin, salicylic acid, salicylic acid methyl ester, sarcosine, sodium cycloprop-1-en-1-yl acetate, sodium cycloprop-2-en-1-yl acetate, sodium-3-(cycloprop-2-en-1-yl)propanoate, sodium-3-(cycloprop-1-en-1-yl) propanoate, sidefungin, spermidine, spermine, strigolactone, tecnazene, thidiazuron, triacontanol, trinexapac, trinexapac-ethyl, tryptophan, tsitodef, uniconazole, uniconazole-P, 2-fluoro-N-(3-methoxyphenyl)-9H-purin-6-amine.

Suitable combination partners for the compounds of the general formula (I) according to the invention also include, for example, the following safeners:
S1) Compounds from the group of heterocyclic carboxylic acid derivatives: 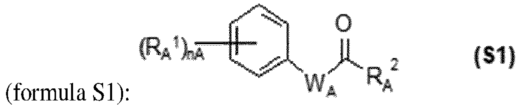 wherein symbols and indices are defined as follows:
   n_{A} is an integer value in the range of 0 to 5, preferably 0 to 3;
   R_{A}¹ is halogen, (C₁-C₄)-alkyl, (C₁-C₄)-alkoxy, nitro or (C₁-C₄)-haloalkyl; 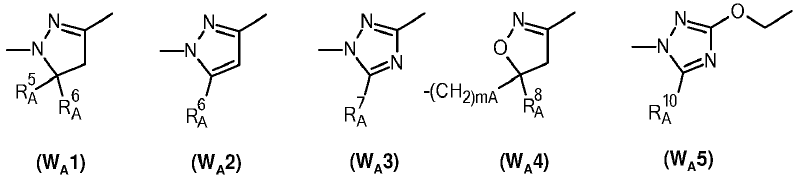
   W_{A} is an unsubstituted or substituted divalent heterocyclic moiety selected from the group of partially unsaturated or aromatic five-membered heterocycles carrying 1 to 3 hetero ring atoms selected from the group of nitrogen (N) und oxygen (O), and carrying at least one N-atom and not more than one O-atom in the ring, preferably a five-membered heterocyclic moiety selected from the group (W_{A}¹) to (W_{A}⁴), m_{A} is 0 or 1;

   - R_{A}²: is OR_{A}³, SR_{A}³ or NR_{A}³R_{A}⁴ or a saturated or unsaturated 3- to 7-membered heterocycle containing at least one N-atom and up to 3 heteroatoms, preferably combined with other heteroatoms from the group of O (oxygen) and S (sulfur), and which is linked to the carbonyl group in (S1) via a nitrogen atom, and which ist unsubstituted or substituted by moieties selected from the group of (C₁-C₄)-alkyl, (C₁C₄)-alkoxy or possibly substituted phenyl, preferably OR_{A}³, NHR_{A}⁴ or N(CH₃)₂, particularly OR_{A}³
   - R_{A}³: is hydrogen or an unsubstituted or substituted aliphatic hydrocarbon moiety, preferably containing 1 to 18 C-atoms;
   - R_{A}⁴: is hydrogen, (C₁-C₆)-alkyl, (C₁-C₆)-alkoxy or substituted or unsubstituted phenyl;
   - R_{A}⁵: is hydrogen, (C₁-C₈)-alkyl, (C₁-C₈)-haloalkyl, (C₁-C₄)-alkoxy-(C₁-C₈)-alkyl, cyano or COOR_{A}⁹, wherein R_{A}⁹ is hydrogen, (C₁-C₈)-alkyl, (C₁-C₈)-haloalkyl, (C₁-C₄)-alkoxy-(C₁C₄)-alkyl, (C₁-C₆)-hydroxyalkyl, (C₃-C₁₂)-cycloalkyl oder tris-(C₁-C₄)-alkylsilyl;
   - R_{A}⁶, R_{A}⁷, R_{A}⁸: are independently hydrogen, (C₁-C₈)-alkyl, (C₁-C₈)-haloalkyl, (C₃-C₁₂)-cycloalkyl or substituted or unsubstituted phenyl;
   - R_{A}¹⁰: is H, (C₃-C₁₂)-cycloalkyl, substituted or unsubstituted phenyl or substituted or unsubstituted heteroaryl;
   preferably
S1^{a}) Compounds of the dichlorophenylpyrazoline-3-carboxylic acid type (S1^{a}), preferably compounds such as
   1-(2,4-dichlorophenyl)-5-(ethoxycarbonyl)-5-methyl-2-pyrazoline-3-carboxylic acid, ethyl 1-(2,4-dichlorophenyl)-5-(ethoxycarbonyl)-5-methyl-2-pyrazoline-3-carboxylate (S1-1) ("mefenpyr-diethyl"), and related compounds as described in WO-A-91/07874;
S1^{b}) Derivatives of dichlorophenylpyrazolecarboxylic acid (S1^{b}), preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S1-3), ethyl 1-(2,4-dichlorophenyl)-5-(1,1-dimethylethyl)pyrazole-3-carboxylate (S1-4) and related compounds as described in EP-A-333131 131 and EP-A-269806;
S1^{c}) Derivatives of 1,5-diphenylpyrazole-3-carboxylic acid (S1^{c}), preferably compounds such as ethyl 1-(2,4-dichlorophenyl)-5-phenylpyrazole-3-carboxylate (S1-5), methyl 1-(2-chlorophenyl)-5-phenylpyrazole-3-carboxylate (S1-6) and related compounds as described, for example, in EP-A-268554;
S1^{d}) Compounds of the triazolecarboxylic acid type (S1^{d}), preferably compounds such as fenchlorazole (ethyl ester), i.e. ethyl 1-(2,4-dichlorophenyl)-5-trichloromethyl-1H-1,2,4-triazole-3-carboxylate (S1-7), and related compounds, as described in EP-A-174562 and EP-A-346620;
S1^{e}) Compounds of the 5-benzyl- or 5-phenyl-2-isoxazoline-3-carboxylic acid or of the 5,5-diphenyl-2-isoxazoline-3-carboxylic acid type (S1^{e}), preferably compounds such as ethyl 5-(2,4-dichlorobenzyl)-2-isoxazoline-3-carboxylate (S1-8) or ethyl 5-phenyl-2-isoxazoline-3-carboxylate (S1-9) and related compounds as described in WO-A-91/08202, or 5,5-diphenyl-2-isoxazolinecarboxylic acid (S1-10) or ethyl 5,5-diphenyl-2-isoxazoline-3-carboxylate (S1-11) ("isoxadifen-ethyl") or n-propyl 5,5-diphenyl-2-isoxazoline-3-carboxylate (S1-12) or ethyl 5-(4-fluorophenyl)-5-phenyl-2-isoxazoline-3-carboxylate (S1-13), as described in patent application WO-A-95/07897.
S1^{f}) compounds of the triazolyloxy acetic acid type (S1^{f}), preferably compounds such as methyl-{ [1,5-bis(4-chloro-2-fluorophenyl)-1H-1,2,4-triazol-3-yl]oxy}acetate (S1-14) or {[1,5-bis(4-chloro-2-fluorophenyl)-1H-1,2,4-triazol-3-yl]oxy}acetic acid (S1-15) or methyl-{[5-(4-chloro-2-fluorophenyl)-1-(2,4-difluorophenyl)-1H-1,2,4-triazol-3-yl]oxy}acetate (S1-16) or {[5-(4-chloro-2-fluorophenyl)-1-(2,4-difluorophenyl)-1H-1,2,4-triazol-3-yl]oxy}acetic acid (S1-17) or methyl-{[1-(4-chloro-2-fluorophenyl)-5-(2,4-difluorophenyl)-1H-1,2,4-triazol-3-yl]oxy}acetate (S1-18) or {[1-(4-chloro-2-fluorophenyl)-5-(2,4-difluorophenyl)-1H-1,2,4-triazol-3-yl]oxy}acetic acid (S1-19), as described in patent application WO2021/105101.
S2) Compounds from the group of the 8-quinolinoxy derivatives (S2):
   wobei die Symbole und Indizes folgende Bedeutungen haben:
   - R_{B}¹: is halogen, (C₁-C₄)-alkyl, (C₁-C₄)-alkoxy, nitro or (C₁-C₄)-haloalkyl;
   - n_{B}: an integer from 0 to 5, preferably 0 to 3;
   - R_{B}²: is OR_{B}³, SR_{B}³ or NR_{B}³R_{B}⁴ or a saturated or unsaturated 3- to 7-membered heterocycle containing at least one nitrogen atom and up to 3 heteroatoms, preferably from the group consisting of O (oxygen) and S (sulfur), which is connected to the carbonyl group in (S2) via a nitrogen atom and which is unsubstituted or substituted by (C₁-C₄)-alkyl, (C₁-C₄)-alkoxy or potentially substituted phenyl, preferably OR_{B}³, NHR_{B}⁴ oder N(CH₃)₂, in particular OR_{B}³;
   - R_{B}³: is hydrogen or an unsubstituted or substituted aliphatic hydrocarbon moiety preferably consisting of 1 to 18 carbon atoms;
   - R_{B}⁴: is hydrogen, (C₁-C₆)-alkyl, (C₁-C₆)-alkoxy or substituted or unsubstituted phenyl;
   - T_{B}: is a (C₁ or C₂)-alkandiyl chain, which is unsubstituted or substituted by one or two (C₁-C₄)-Alkyl moieties or by a [(C₁-C₃)-alkoxy]carbonyl moiety;
   preferably:
S2^{a}) Compounds of the 8-quinolinoxyacetic acid type (S2^{a}), preferably 1-methylhexyl (5-chloro-8-quinolinoxy)acetate ("cloquintocet-mexyl") (S2-1), 1,3-dimethylbut-1-yl (5-chloro-8-quinolinoxy)-acetate (S2-2), 4-allyloxybutyl (5-chloro-8-quinolinoxy)acetate (S2-3), 1-allyloxyprop-2-yl (5-chloro-8-quinolinoxy)acetate (S2-4), ethyl (5-chloro-8-quinolinoxy)acetate (S2-5), methyl 5-chloro-8-quinolinoxyacetate (S2-6), allyl (5-chloro-8-quinolinoxy)acetate (S2-7), 2-(2-propylideneiminoxy)-1-ethyl (5-chloro-8-quinolinoxy)acetate (S2-8), 2-oxoprop-1-yl (5-chloro-8-quinolinoxy)acetate (S2-9) and related compounds, as described in EP-A-86750, EP-A-94349 and EP-A-191736 or EP-A-0 492 366, and also (5-chloro-8-quinolinoxy)acetic acid (S2-10), hydrates and salts thereof, for example the lithium, sodium, potassium, calcium, magnesium, aluminum, iron, ammonium, quaternary ammonium, sulfonium or phosphonium salts thereof, as described in WO-A-2002/34048;
S2^{b}) Compounds of the (5-chloro-8-quinolinoxy)malonic acid type (S2^{b}), preferably compounds such as diethyl (5-chloro-8-quinolinoxy)malonate, diallyl (5-chloro-8-quinolinoxy)malonate, methyl ethyl (5-chloro-8-quinolinoxy)malonate and related compounds, as described in EP-A-0 582 198.
S3) Active compounds of the dichloroacetamide type (S3), which are frequently used as pre-emergence safeners (soil-acting safeners), for example
   "dichlormid" (N,N-diallyl-2,2-dichloroacetamide) (S3-1),
   "R-29148" (3-dichloroacetyl-2,2,5-trimethyl-1,3-oxazolidine) from Stauffer (S3-2),
   "R-28725" (3-dichloroacetyl-2,2-dimethyl-1,3-oxazolidine) from Stauffer (S3-3),
   "benoxacor" (4-dichloroacetyl-3,4-dihydro-3-methyl-2H-1,4-benzoxazine) (S3-4),
   "PPG-1292" (N-allyl-N-[(1,3-dioxolan-2-yl)methyl]dichloroacetamide) from PPG Industries (S3-5),
   "DKA-24" (N-allyl-N-[(allylaminocarbonyl)methyl]dichloroacetamide) from Sagro-Chem (S3-6),
   "AD-67" or "MON 4660" (3-dichloroacetyl-1-oxa-3-azaspiro[4.5]decane) from Nitrokemia or Monsanto (S3-7),
   "TI-35" (1-dichloroacetylazepane) from TRI-Chemical RT (S3-8),
   "Diclonon" (Dicyclonon) or "BAS145138" or "LAB145138" (S3-9)
   ((RS)-1-dichloroacetyl-3,3,8a-trimethylperhydropyrrolo[1,2-a]pyrimidin-6-one) from BASF, "furilazole" or "MON 13900" ((RS)-3-dichloroacetyl-5-(2-furyl)-2,2-dimethyloxazolidine) (S3-10), and the (R) isomer thereof (S3-11).
S4) Compounds from the class of the acylsulfonamides (S4):
S4^{a}) N-Acylsulfonamides of the formula (S4^{a}) and salts thereof, as described in WO-A-97/45016, 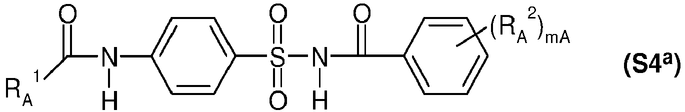 in which
   - R_{A}¹: is (C₁-C₆)-alkyl, (C₃-C₆)-cycloalkyl, where the 2 latter radicals are substituted by v_{A} substituents from the group of halogen, (C₁-C₄)-alkoxy, (C₁-C₆)-haloalkoxy and (C₁-C₄)-alkylthio and, in the case of cyclic radicals, also by (C₁-C₄)-alkyl and (C₁-C₄)-haloalkyl;
   - R_{A}²: is halogen, (C₁-C₄)-alkyl, (C₁-C₄)-alkoxy, CF₃;
   - m_{A}: is 1 or 2;
   - v_{A}: is 0, 1, 2 or 3;
   - S4^{b}): Compounds of the 4-(benzoylsulfamoyl)benzamide type of the formula (S4^{b}) and salts thereof, as described in WO-A-99/16744, 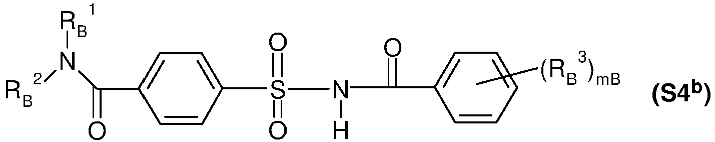
   in which
   R_{B}¹, R_{B}² are independently hydrogen, (C₁-C₆)-alkyl, (C₃-C₆)-cycloalkyl, (C₃-C₆)-alkenyl, (C₃-C₆)-alkynyl,
   R_{B}³ is halogen, (C₁-C₄)-alkyl, (C₁-C₄)-haloalkyl or (C₁-C₄)-alkoxy and
   m_{B} is 1 or 2,
   for example those in which
   R_{B}¹ = cyclopropyl, R_{B}² = hydrogen and (R_{B}³) = 2-OMe ("cyprosulfamide", S4-1),
   R_{B}¹ = cyclopropyl, R_{B}² = hydrogen and (R_{B}³) = 5-Cl-2-OMe (S4-2),
   R_{B}¹ = ethyl, R_{B}² = hydrogen and (R_{B}³) = 2-OMe (S4-3),
   R_{B}¹ = isopropyl, R_{B}² = hydrogen and (R_{B}³) = 5-Cl-2-OMe (S4-4) and
   R_{B}¹ = isopropyl, R_{B}² = hydrogen and (R_{B}³) = 2-OMe (S4-5);
S4^{c}) Compounds from the class of the benzoylsulfamoylphenylureas of the formula (S4^{c}), as described in EP-A-365484, 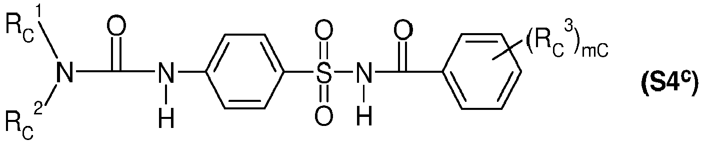 in which
   - R_{C}¹, R_{C}²: are independently hydrogen, (C₁-C₈)-alkyl, (C₃-C₈)-cycloalkyl, (C₃-C₆)-alkenyl, (C₃-C₆)-alkynyl,
   - R_{C}³: is halogen, (C₁-C₄)-alkyl, (C₁-C₄)-alkoxy, CF₃ and
   - m_{C}: is 1 or 2;
   for example
   1-[4-(N-2-methoxybenzoylsulfamoyl)phenyl]-3-methylurea,
   1-[4-(N-2-methoxybenzoylsulfamoyl)phenyl]-3,3-dimethylurea,
   1-[4-(N-4,5-dimethylbenzoylsulfamoyl)phenyl]-3-methylurea;
S4') Compounds of the N-phenylsulfonylterephthalamide type of the formula (S4^{d}) and salts thereof, which are known, for example, from CN 101838227, 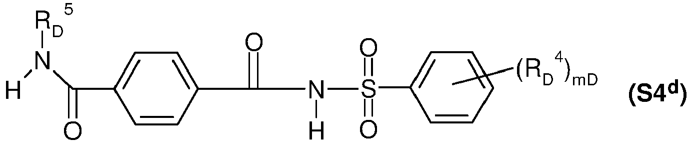 in which
   - R_{D}⁴: is halogen, (C₁-C₄)-alkyl, (C₁-C₄)-alkoxy, CF₃;
   - m_{D}: is 1 or 2;
   - R_{D}⁵: is hydrogen, (C₁-C₆)-alkyl, (C₃-C₆)-cycloalkyl, (C₂-C₆)-alkenyl, (C₂-C₆)-alkynyl or (C₅-C₆)-cycloalkenyl.
S5) Active compounds from the class of the hydroxyaromatics and the aromatic-aliphatic carboxylic acid derivatives (S5), for example ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicyclic acid, 2-hydroxycinnamic acid, 2,4-dichlorocinnamic acid, as described in WO-A-2004/084631, WO-A-2005/015994, WO-A-2005/016001.
S6) Active compounds from the class of the 1,2-dihydroquinoxalin-2-ones (S6), for example 1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-1,2-dihydroquinoxalin-2-one hydrochloride, 1-(2-methyl-sulfonylaminoethyl)-3-(2-thienyl)-1,2-dihydroquinoxalin-2-one, as described in WO-A-2005/112630.
S7) Compounds from the class of the diphenylmethoxyacetic acid derivatives (S7), e.g. methyl diphenylmethoxyacetate (CAS Reg. No. 41858-19-9) (S7-1), ethyl diphenylmethoxyacetate or diphenylmethoxyacetic acid, as described in WO-A-98/38856.
S8) Compounds of the formula (S8), as described in WO-A-98/27049, 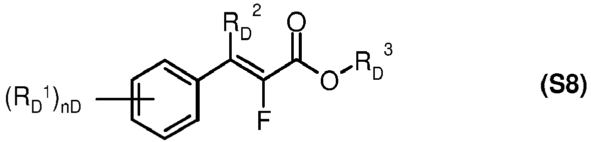 in which the symbols and indices are defined as follows:
   - R_{D}¹: is halogen, (C₁-C₄)-alkyl, (C₁-C₄)-haloalkyl, (C₁-C₄)-alkoxy, (C₁-C₄)-haloalkoxy,
   - R_{D}²: is hydrogen or (C₁-C₄)-alkyl,
   - R_{D}³: is hydrogen, (C₁-C₈)-alkyl, (C₂-C₄)-alkenyl, (C₂-C₄)-alkynyl or aryl, where each of the aforementioned carbon-containing radicals is unsubstituted or substituted by one or more, preferably up to three identical or different radicals from the group consisting of halogen and alkoxy; or salts thereof,
   - n_{D}: is an integer from 0 to 2.
S9) active compounds from the class of the 3-(5-tetrazolylcarbonyl)-2-quinolones (S9), for example 1,2-dihydro-4-hydroxy-1-ethyl-3-(5-tetrazolylcarbonyl)-2-quinolone (CAS Reg. No. 219479-18-2), 1,2-dihydro-4-hydroxy-1-methyl-3-(5-tetrazolylcarbonyl)-2-quinolone (CAS Reg. No. 95855-00-8), as described in WO-A-199/000020;
S10) Compounds of the formula (S10^{a}) or (S10^{b})
   as described in WO-A-2007/023719 and WO-A-2007/023764 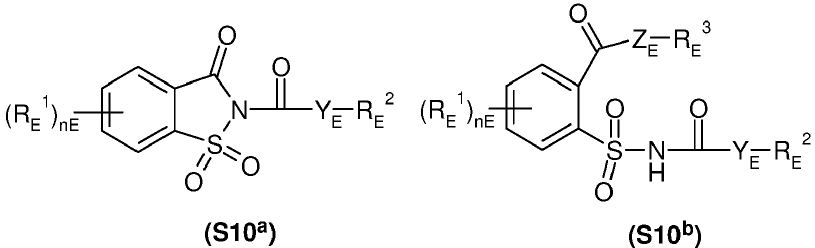 in which
   - R_{E}¹: is halogen, (C₁-C₄)-alkyl, methoxy, nitro, cyano, CF₃, OCF₃
   - Y_{E}, Z_{E}: are independently O or S,
   - n_{E}: is an integer from 0 to 4,
   - R_{E}²: is (C₁-C₁₆)-alkyl, (C₂-C₆)-alkenyl, (C₃-C₆)-cycloalkyl, aryl; benzyl, halobenzyl,
   - R_{E}³: is hydrogen or (C₁-C₆)-alkyl.
S11) Active compounds of the oxyimino compound type (S11), which are known as seed-dressing agents, for example
   "oxabetrinil" ((Z)-1,3-dioxolan-2-ylmethoxyimino(phenyl)acetonitrile) (S11-1), which is known as a seed-dressing safener for millet/sorghum against metolachlor damage,
   "fluxofenim" (1-(4-chlorophenyl)-2,2,2-trifluoro-1-ethanone O-(1,3-dioxolan-2-ylmethyl)oxime) (S11-2), which is known as a seed-dressing safener for millet/sorghum against metolachlor damage, and "cyometrinil" or "CGA-43089" ((Z)-cyanomethoxyimino(phenyl)acetonitrile) (S11-3), which is known as a seed-dressing safener for millet/sorghum against metolachlor damage.
S12) active compounds from the class of the isothiochromanones (S12), for example methyl [(3-oxo-1H-2-benzothiopyran-4(3H)-ylidene)methoxy]acetate (CAS Reg. No. 205121-04-6) (S12-1) and related compounds from WO-A-1998/13361.
S13) One or more compounds from group (S13):
   "naphthalic anhydride" (1,8-naphthalenedicarboxylic anhydride) (S13-1), which is known as a seed-dressing safener for corn against thiocarbamate herbicide damage,
   "fenclorim" (4,6-dichloro-2-phenylpyrimidine) (S13-2), which is known as a safener for pretilachlor in sown rice,
   "flurazole" (benzyl 2-chloro-4-trifluoromethyl-1,3-thiazole-5-carboxylate) (S13-3), which is known as a seed-dressing safener for millet/sorghum against alachlor and metolachlor damage,
   "CL 304415" (CAS Reg. No. 31541-57-8)
   (4-carboxy-3,4-dihydro-2H-1-benzopyran-4-acetic acid) (S13-4) from American Cyanamid, which is known as a safener for corn against damage by imidazolinones,
   "MG 191" (CAS Reg. No. 96420-72-3) (2-dichloromethyl-2-methyl-1,3-dioxolane) (S13-5) from Nitrokemia, which is known as a safener for corn,
   "MG 838" (CAS Reg. No. 133993-74-5)
   (2-propenyl 1-oxa-4-azaspiro[4.5]decane-4-carbodithioate) (S13-6) from Nitrokemia "disulfoton" (O,O-diethyl S-2-ethylthioethyl phosphorodithioate) (S13-7),
   "dietholate" (O,O-diethyl O-phenyl phosphorothioate) (S13-8),
   "mephenate" (4-chlorophenyl methylcarbamate) (S13-9).
S14) active compounds which, in addition to herbicidal action against weeds, also have safener action on crop plants such as rice, for example
   "dimepiperate" or "MY-93" (S-1-methyl 1-phenylethylpiperidine-1-carbothioate), which is known as a safener for rice against damage by the herbicide molinate,
   "daimuron" or "SK 23" (1-(1-methyl-1-phenylethyl)-3-p-tolylurea), which is known as safener for rice against imazosulfuron herbicide damage,
   "cumyluron" = "JC-940" (3-(2-chlorophenylmethyl)-1-(1-methyl-1-phenylethyl)urea, see JP-A-60087254), which is known as safener for rice against damage by some herbicides,
   "methoxyphenone" or "NK 049" (3,3'-dimethyl-4-methoxybenzophenone), which is known as a safener for rice against damage by some herbicides,
   "CSB" (1-bromo-4-(chloromethylsulfonyl)benzene) from Kumiai, (CAS Reg. No. 54091-06-4), which is known as a safener against damage by some herbicides in rice.
S15) Compounds of the formula (S15) or tautomers thereof 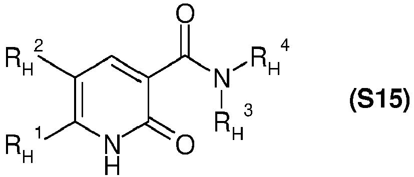 as described in WO-A-2008/131861 and WO-A-2008/131860 in which
   - R_{H}¹: is a (C₁-C₆)-haloalkyl radical and
   - R_{H}²: is hydrogen or halogen and
   - R_{H}³, R_{H}⁴: are each independently hydrogen, (C₁-C₁₆)-alkyl, (C₂-C₁₆)-alkenyl or (C₂-C₁₆)-alkynyl, where each of the 3 latter radicals is unsubstituted or substituted by one or more radicals from the group of halogen, hydroxyl, cyano, (C₁-C₄)-alkoxy, (C₁-C₄)-haloalkoxy, (C₁-C₄)-alkylthio, (C₁-C₄)-alkylamino, di[(C₁-C₄)-alkyl]amino, [(C₁-C₄)-alkoxy]carbonyl, [(C₁-C₄)-haloalkoxy]carbonyl, (C₃-C₆)-cycloalkyl which is unsubstituted or substituted, phenyl which is unsubstituted or substituted, and heterocyclyl which is unsubstituted or substituted, or (C₃-C₆)-cycloalkyl, (C₄-C₆)-cycloalkenyl, (C₃-C₆)-cycloalkyl fused on one side of the ring to a 4 to 6-membered saturated or unsaturated carbocyclic ring, or (C₄-C₆)-cycloalkenyl fused on one side of the ring to a 4 to 6-membered saturated or unsaturated carbocyclic ring, where each of the 4 latter radicals is unsubstituted or substituted by one or more radicals from the group of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di[(C₁-C₄)-alkyl]amino, [(C₁-C₄)alkoxy]carbonyl, [(C₁-C₄)haloalkoxy]carbonyl, (C₃-C₆)-cycloalkyl which is unsubstituted or substituted, phenyl which is unsubstituted or substituted, and heterocyclyl which is unsubstituted or substituted,
   or
   - R_{H}³: is (C₁-C₄)-alkoxy, (C₂-C₄)-alkenyloxy, (C₂-C₆)-alkynyloxy or (C₂-C₄)-haloalkoxy and
   - R_{H}⁴: is hydrogen or (C₁-C₄)-alkyl or
   - R_{H}³ and R_{H}⁴: together with the directly bonded nitrogen atom are a four- to eight-membered heterocyclic ring which, as well as the nitrogen atom, may also contain further ring heteroatoms, preferably up to two further ring heteroatoms from the group of N, O and S, and which is unsubstituted or substituted by one or more radicals from the group of halogen, cyano, nitro, (C₁-C₄)-alkyl, (C₁-C₄)-haloalkyl, (C₁-C₄)-alkoxy, (C₁-C₄)-haloalkoxy and (C₁-C₄)-alkylthio.
S16) Active compounds which are used primarily as herbicides but also have safener action on crop plants, for example
   (2,4-dichlorophenoxy)acetic acid (2,4-D),
   (4-chlorophenoxy)acetic acid,
   (R,S)-2-(4-chloro-o-tolyloxy)propionic acid (mecoprop),
   4-(2,4-dichlorophenoxy)butyric acid (2,4-DB),
   (4-chloro-o-tolyloxy)acetic acid (MCPA),
   4-(4-chloro-o-tolyloxy)butyric acid,
   4-(4-chlorophenoxy)butyric acid,
   3,6-dichloro-2-methoxybenzoic acid (dicamba),
   1-(ethoxycarbonyl)ethyl 3,6-dichloro-2-methoxybenzoate (lactidichlor-ethyl).

Preferred safeners in combination with the compounds of the general formula (I) according to the invention and/or salts thereof, in particular with the compounds of the formulae (I-001) to (I-205) and and/or salts thereof, are: cloquintocet-mexyl, cyprosulfamide, fenchlorazole-ethyl, isoxadifen-ethyl, mefenpyr-diethyl, fenclorim, cumyluron, S1-14, S1-15, S1-16, S1-17, S1-18, S1-19, S4-1 and S4-5, and particularly preferred safeners are: cloquintocet-mexyl, cyprosulfamide, isoxadifen-ethyl and mefenpyr-diethyl.

### Biological examples:

The following abbreviations are used in the examples and tables below:

### Tested harmful plants:

- ABUTH:: Abutilon theophrasti
- AGSTE:: Agrostis tenuis
- ALOMY:: Alopecurus myosuroides
- AMARE: Amaranthus retroflexus
- AVEFA:: Avena fatua
- DIGSA:: Digitaria sanguinalis
- ECHCG:: Echinochloa crus-galli
- KCHSC:: Kochia scoparia
- LOLRI:: Lolium rigidum
- MATIN:: Matricaria inodora
- PHBPU:: Pharbitis purpurea
- POAAN:: Poa annua
- POLCO:: Polygonum convolvulus
- SETVI:: Setaria viridis
- STEME:: Stellaria media
- VERPE:: Veronica persica
- VIOTR:: Viola tricolor

### Tested crop plants:

- BRSNW:: Brassica napus
- GLXMA:: Glycine max
- ORYSA:: Oryza sativa
- TRZAS:: Triticum aestivum
- ZEAMX:: Zea mays

### A. Herbicidal pre-emergence action

Seeds of mono- and dicotyledonous weed plants were sown in plastic pots (double sowings with one species of mono- and one species of dicotyledonous weed plants per pot), in sandy loam, and covered with soil. The compounds according to the invention, formulated in the form of wettable powders (WP) or as emulsifiable concentrates (EC), were applied to the surface of the covering soil as aqueous suspension or emulsion, with the addition of 0.5% of an additive, at an application rate of 6001 of water per hectare (converted). Following treatment, the pots were placed in a greenhouse and kept under optimum growth conditions for the test plants. The visual grading of the damage to the test plants was carried out after ca. 3 weeks in comparison to untreated controls
(herbicidal effect in percent (%): 100% effect = plants have died off, 0% effect = as control plants).

Tables A1 to A12, below, show the effects of selected compounds of the general formula (I) according to table 1 on various harmful plants and an application rate corresponding to 1280 g/ha or below obtained by the experimental procedure mentioned above.

**Table A1: pre-emergence activity at 1280g/ha against ABUTH in %**

| Example number | application rate [g/ha] | ABUTH |
|---|---|---|
| I-170 | 1280 | 90 |
| I-189 | 1280 | 100 |
| I-122 | 1280 | 100 |
| I-071 | 1280 | 90 |
| I-072 | 1280 | 100 |

**Table A2a: pre-emergence activity at 1280g/ha against ECHCG in %**

| Example number | application rate [g/ha] | ECHCG |
|---|---|---|
| | | |
| I-001 | 1280 | 100 |
| I-002 | 1280 | 100 |
| I-097 | 1280 | 100 |
| I-168 | 1280 | 100 |
| I-096 | 1280 | 100 |
| I-158 | 1280 | 100 |
| I-147 | 1280 | 100 |
| I-148 | 1280 | 100 |
| I-003 | 1280 | 100 |
| I-151 | 1280 | 100 |
| I-150 | 1280 | 100 |
| I-157 | 1280 | 100 |
| I-005 | 1280 | 100 |
| I-006 | 1280 | 90 |
| I-007 | 1280 | 100 |
| I-008 | 1280 | 90 |
| I-009 | 1280 | 100 |
| I-163 | 1280 | 90 |
| I-011 | 1280 | 100 |
| I-014 | 1280 | 100 |
| I-015 | 1280 | 100 |
| I-166 | 1280 | 90 |
| I-170 | 1280 | 90 |
| I-016 | 1280 | 100 |
| I-018 | 1280 | 100 |
| I-019 | 1280 | 90 |
| I-021 | 1280 | 100 |
| I-022 | 1280 | 100 |
| I-023 | 1280 | 100 |
| I-025 | 1280 | 90 |
| I-026 | 1280 | 90 |
| I-027 | 1280 | 90 |
| I-028 | 1280 | 90 |
| I-029 | 1280 | 90 |
| I-030 | 1280 | 90 |
| I-032 | 1280 | 90 |
| I-171 | 1280 | 100 |
| I-036 | 1280 | 100 |
| I-039 | 1280 | 90 |
| I-040 | 1280 | 100 |
| I-042 | 1280 | 100 |
| I-044 | 1280 | 100 |
| I-047 | 1280 | 100 |
| I-048 | 1280 | 90 |
| I-178 | 1280 | 90 |
| I-179 | 1280 | 90 |
| I-180 | 1280 | 90 |
| I-049 | 1280 | 90 |
| I-051 | 1280 | 90 |
| I-053 | 1280 | 90 |
| I-054 | 1280 | 100 |
| I-055 | 1280 | 90 |
| I-124 | 1280 | 100 |
| I-119 | 1280 | 90 |
| I-126 | 1280 | 90 |
| I-137 | 1280 | 100 |
| I-139 | 1280 | 100 |
| I-188 | 1280 | 100 |
| I-190 | 1280 | 100 |
| I-058 | 1280 | 100 |
| I-122 | 1280 | 100 |
| I-108 | 1280 | 100 |
| I-109 | 1280 | 100 |
| I-071 | 1280 | 100 |
| I-105 | 1280 | 100 |
| I-110 | 1280 | 100 |
| I-106 | 1280 | 100 |
| I-107 | 1280 | 100 |
| I-111 | 1280 | 90 |
| I-077 | 1280 | 100 |
| I-078 | 1280 | 100 |
| I-113 | 1280 | 90 |
| I-114 | 1280 | 90 |
| I-112 | 1280 | 100 |
| I-079 | 1280 | 90 |
| I-081 | 1280 | 100 |
| I-082 | 1280 | 100 |
| I-084 | 1280 | 90 |
| I-087 | 1280 | 100 |
| I-088 | 1280 | 100 |
| I-142 | 1280 | 100 |
| I-144 | 1280 | 100 |
| I-116 | 1280 | 100 |
| I-117 | 1280 | 100 |

**Table A2b: pre-emergence activity at 320g/ha against ECHCG in %**

| Example number | application rate [g/ha] | ECHCG |
|---|---|---|
| I-001 | 320 | 100 |
| I-002 | 320 | 100 |
| I-096 | 320 | 100 |
| I-003 | 320 | 100 |
| I-157 | 320 | 100 |
| I-014 | 320 | 90 |
| I-015 | 320 | 90 |
| I-170 | 320 | 90 |
| I-016 | 320 | 90 |
| I-018 | 320 | 90 |
| I-022 | 320 | 90 |
| I-023 | 320 | 90 |
| I-027 | 320 | 90 |
| I-032 | 320 | 90 |
| I-171 | 320 | 90 |
| I-036 | 320 | 90 |
| I-040 | 320 | 90 |
| I-042 | 320 | 100 |
| I-044 | 320 | 90 |
| I-179 | 320 | 90 |
| I-051 | 320 | 90 |
| I-053 | 320 | 90 |
| I-054 | 320 | 90 |
| I-124 | 320 | 90 |
| I-126 | 320 | 90 |
| I-137 | 320 | 90 |
| I-188 | 320 | 90 |
| I-190 | 320 | 90 |
| I-058 | 320 | 90 |
| I-122 | 320 | 100 |
| I-108 | 320 | 100 |
| I-109 | 320 | 100 |
| I-071 | 320 | 100 |
| I-105 | 320 | 90 |
| I-110 | 320 | 90 |
| I-106 | 320 | 90 |
| I-107 | 320 | 90 |
| I-077 | 320 | 90 |
| I-078 | 320 | 90 |
| I-079 | 320 | 90 |
| I-081 | 320 | 100 |
| I-082 | 320 | 90 |
| I-084 | 320 | 90 |
| I-087 | 320 | 100 |
| I-088 | 320 | 90 |
| I-142 | 320 | 90 |
| I-144 | 320 | 90 |

**Table A2c: pre-emergence activity at 80g/ha against ECHCG in %**

| Example number | application rate [g/ha] | ECHCG |
|---|---|---|
| I-002 | 80 | 90 |
| I-003 | 80 | 100 |
| I-157 | 80 | 100 |
| I-170 | 80 | 90 |
| I-018 | 80 | 90 |
| I-042 | 80 | 90 |
| I-051 | 80 | 90 |
| I-126 | 80 | 90 |
| I-137 | 80 | 90 |
| I-122 | 80 | 90 |
| I-071 | 80 | 90 |
| I-106 | 80 | 90 |
| I-077 | 80 | 90 |
| I-079 | 80 | 90 |
| I-081 | 80 | 90 |
| I-082 | 80 | 90 |
| I-084 | 80 | 90 |
| I-087 | 80 | 90 |
| I-088 | 80 | 90 |
| I-142 | 80 | 90 |

**Table A3a: pre-emergence activity at 1280g/ha against LOLRI in %**

| Example number | application rate [g/ha] | LOLRI |
|---|---|---|
| | | |
| I-001 | 1280 | 100 |
| I-002 | 1280 | 100 |
| I-167 | 1280 | 90 |
| I-158 | 1280 | 90 |
| I-147 | 1280 | 90 |
| I-003 | 1280 | 100 |
| I-151 | 1280 | 90 |
| I-157 | 1280 | 90 |
| I-006 | 1280 | 90 |
| I-011 | 1280 | 90 |
| I-014 | 1280 | 100 |
| I-015 | 1280 | 100 |
| I-166 | 1280 | 100 |
| I-170 | 1280 | 90 |
| I-018 | 1280 | 100 |
| I-021 | 1280 | 100 |
| I-022 | 1280 | 100 |
| I-023 | 1280 | 100 |
| I-025 | 1280 | 90 |
| I-027 | 1280 | 90 |
| I-032 | 1280 | 100 |
| I-171 | 1280 | 100 |
| I-036 | 1280 | 90 |
| I-039 | 1280 | 90 |
| I-040 | 1280 | 100 |
| I-042 | 1280 | 100 |
| I-044 | 1280 | 90 |
| I-047 | 1280 | 90 |
| I-048 | 1280 | 90 |
| I-179 | 1280 | 90 |
| I-180 | 1280 | 90 |
| I-051 | 1280 | 100 |
| I-053 | 1280 | 90 |
| I-054 | 1280 | 90 |
| I-124 | 1280 | 100 |
| I-119 | 1280 | 100 |
| I-126 | 1280 | 100 |
| I-137 | 1280 | 100 |
| I-139 | 1280 | 90 |
| I-188 | 1280 | 100 |
| I-190 | 1280 | 90 |
| I-058 | 1280 | 90 |
| I-122 | 1280 | 100 |
| I-108 | 1280 | 90 |
| I-071 | 1280 | 100 |
| I-105 | 1280 | 90 |
| I-110 | 1280 | 100 |
| I-106 | 1280 | 90 |
| I-107 | 1280 | 90 |
| I-077 | 1280 | 100 |
| I-078 | 1280 | 100 |
| I-079 | 1280 | 100 |
| I-081 | 1280 | 100 |
| I-082 | 1280 | 100 |
| I-084 | 1280 | 100 |
| I-087 | 1280 | 100 |
| I-088 | 1280 | 100 |
| I-142 | 1280 | 100 |

**Table A3b: pre-emergence activity at 320g/ha against LOLRI in %**

| Example number | application rate [g/ha] | LOLRI |
|---|---|---|
| I-002 | 320 | 90 |
| I-003 | 320 | 100 |
| I-157 | 320 | 90 |
| I-014 | 320 | 90 |
| I-170 | 320 | 90 |
| I-018 | 320 | 100 |
| I-021 | 320 | 100 |
| I-022 | 320 | 100 |
| I-023 | 320 | 100 |
| I-171 | 320 | 90 |
| I-036 | 320 | 90 |
| I-039 | 320 | 90 |
| I-040 | 320 | 90 |
| I-042 | 320 | 90 |
| I-044 | 320 | 90 |
| I-179 | 320 | 90 |
| I-051 | 320 | 100 |
| I-053 | 320 | 90 |
| I-124 | 320 | 90 |
| I-126 | 320 | 90 |
| I-137 | 320 | 90 |
| I-122 | 320 | 100 |
| I-071 | 320 | 100 |
| I-110 | 320 | 90 |
| I-077 | 320 | 90 |
| I-078 | 320 | 100 |
| I-079 | 320 | 100 |
| I-081 | 320 | 100 |
| I-082 | 320 | 100 |
| I-084 | 320 | 90 |
| I-087 | 320 | 100 |
| I-088 | 320 | 100 |
| I-142 | 320 | 90 |

**Table A3c: pre-emergence activity at 80g/ha against LOLRI in %**

| Example number | application rate [g/ha] | LOLRI |
|---|---|---|
| I-002 | 80 | 90 |
| I-003 | 80 | 100 |
| I-018 | 80 | 90 |
| I-042 | 80 | 90 |
| I-051 | 80 | 100 |
| I-071 | 80 | 90 |
| I-079 | 80 | 90 |
| I-081 | 80 | 100 |
| I-082 | 80 | 90 |
| I-087 | 80 | 100 |
| I-088 | 80 | 90 |

**Table A4a: pre-emergence activity at 1280g/ha against MATIN in %**

| Example number | application rate [g/ha] | MATIN |
|---|---|---|
| I-001 | 1280 | 90 |
| I-002 | 1280 | 90 |
| I-158 | 1280 | 90 |
| I-003 | 1280 | 90 |
| I-157 | 1280 | 90 |
| I-006 | 1280 | 90 |
| I-008 | 1280 | 90 |
| I-170 | 1280 | 90 |
| I-018 | 1280 | 90 |
| I-021 | 1280 | 90 |
| I-022 | 1280 | 90 |
| I-023 | 1280 | 90 |
| I-024 | 1280 | 90 |
| I-025 | 1280 | 90 |
| I-029 | 1280 | 90 |
| I-030 | 1280 | 90 |
| I-171 | 1280 | 100 |
| I-036 | 1280 | 90 |
| I-039 | 1280 | 90 |
| I-040 | 1280 | 90 |
| I-042 | 1280 | 90 |
| I-044 | 1280 | 90 |
| I-051 | 1280 | 90 |
| I-053 | 1280 | 90 |
| I-054 | 1280 | 90 |
| I-182 | 1280 | 90 |
| I-124 | 1280 | 100 |
| I-126 | 1280 | 90 |
| I-137 | 1280 | 90 |
| I-188 | 1280 | 90 |
| 1-058 | 1280 | 90 |
| I-122 | 1280 | 100 |
| I-108 | 1280 | 100 |
| I-109 | 1280 | 100 |
| I-071 | 1280 | 90 |
| I-110 | 1280 | 90 |
| I-107 | 1280 | 100 |
| I-077 | 1280 | 90 |
| I-078 | 1280 | 90 |
| I-113 | 1280 | 90 |
| I-079 | 1280 | 90 |
| I-081 | 1280 | 90 |
| I-082 | 1280 | 90 |
| I-083 | 1280 | 90 |
| I-084 | 1280 | 90 |
| I-087 | 1280 | 90 |
| I-088 | 1280 | 90 |
| I-142 | 1280 | 90 |

**Table A4b: pre-emergence activity at 320g/ha against MATIN in %**

| Example number | application rate [g/ha] | MATIN |
|---|---|---|
| I-002 | 320 | 90 |
| I-003 | 320 | 90 |
| I-018 | 320 | 90 |
| I-023 | 320 | 90 |
| I-024 | 320 | 90 |
| I-042 | 320 | 90 |
| I-054 | 320 | 90 |
| I-137 | 320 | 90 |
| I-071 | 320 | 90 |
| I-077 | 320 | 90 |
| I-078 | 320 | 90 |
| I-079 | 320 | 90 |
| I-081 | 320 | 90 |
| I-082 | 320 | 90 |
| I-084 | 320 | 90 |
| I-087 | 320 | 90 |
| I-088 | 320 | 90 |

**Table A4c: pre-emergence activity at 80g/ha against MATIN in %**

| Example number | application rate [g/ha] | MATIN |
|---|---|---|
| I-018 | 80 | 90 |
| I-024 | 80 | 90 |
| I-079 | 80 | 90 |
| I-082 | 80 | 90 |
| I-084 | 80 | 90 |
| I-088 | 80 | 90 |

**Table A5a: pre-emergence activity at 1280g/ha and against SETVI in %**

| Example number | application rate [g/ha] | SETVI |
|---|---|---|
| I-001 | 1280 | 100 |
| I-002 | 1280 | 90 |
| I-158 | 1280 | 90 |
| I-147 | 1280 | 100 |
| I-003 | 1280 | 100 |
| I-151 | 1280 | 90 |
| I-150 | 1280 | 100 |
| I-157 | 1280 | 90 |
| I-008 | 1280 | 90 |
| I-009 | 1280 | 100 |
| I-014 | 1280 | 90 |
| I-015 | 1280 | 90 |
| I-166 | 1280 | 90 |
| I-170 | 1280 | 90 |
| I-016 | 1280 | 90 |
| I-018 | 1280 | 100 |
| I-019 | 1280 | 100 |
| I-021 | 1280 | 100 |
| I-022 | 1280 | 90 |
| I-023 | 1280 | 90 |
| I-024 | 1280 | 90 |
| I-025 | 1280 | 90 |
| I-026 | 1280 | 90 |
| I-027 | 1280 | 100 |
| I-028 | 1280 | 90 |
| I-029 | 1280 | 100 |
| I-030 | 1280 | 90 |
| I-032 | 1280 | 90 |
| I-171 | 1280 | 100 |
| I-036 | 1280 | 90 |
| I-039 | 1280 | 90 |
| I-040 | 1280 | 90 |
| I-042 | 1280 | 90 |
| I-044 | 1280 | 90 |
| I-179 | 1280 | 90 |
| I-180 | 1280 | 90 |
| I-051 | 1280 | 90 |
| I-053 | 1280 | 90 |
| I-054 | 1280 | 90 |
| I-124 | 1280 | 90 |
| I-119 | 1280 | 100 |
| I-126 | 1280 | 100 |
| I-137 | 1280 | 100 |
| I-139 | 1280 | 100 |
| I-188 | 1280 | 100 |
| I-190 | 1280 | 100 |
| I-058 | 1280 | 100 |
| I-122 | 1280 | 100 |
| I-128 | 1280 | 90 |
| I-132 | 1280 | 100 |
| I-108 | 1280 | 100 |
| I-109 | 1280 | 90 |
| I-071 | 1280 | 90 |
| I-105 | 1280 | 100 |
| I-110 | 1280 | 100 |
| I-106 | 1280 | 100 |
| I-107 | 1280 | 100 |
| I-077 | 1280 | 90 |
| I-078 | 1280 | 100 |
| I-079 | 1280 | 90 |
| I-081 | 1280 | 100 |
| I-082 | 1280 | 100 |
| I-084 | 1280 | 100 |
| I-087 | 1280 | 100 |
| I-088 | 1280 | 100 |
| I-142 | 1280 | 100 |
| I-144 | 1280 | 90 |
| I-116 | 1280 | 90 |

**Table A5b: pre-emergence activity at 320g/ha and against SETVI in %**

| Example number | application rate [g/ha] | SETVI |
|---|---|---|
| I-001 | 320 | 90 |
| I-002 | 320 | 90 |
| I-003 | 320 | 100 |
| I-157 | 320 | 90 |
| I-170 | 320 | 90 |
| I-018 | 320 | 100 |
| I-021 | 320 | 100 |
| I-022 | 320 | 90 |
| I-023 | 320 | 90 |
| I-024 | 320 | 90 |
| I-027 | 320 | 100 |
| I-032 | 320 | 90 |
| I-171 | 320 | 90 |
| I-039 | 320 | 90 |
| I-040 | 320 | 90 |
| I-042 | 320 | 90 |
| I-051 | 320 | 90 |
| I-053 | 320 | 90 |
| I-054 | 320 | 90 |
| I-124 | 320 | 90 |
| I-119 | 320 | 90 |
| I-126 | 320 | 90 |
| I-188 | 320 | 100 |
| I-058 | 320 | 90 |
| I-122 | 320 | 90 |
| I-071 | 320 | 90 |
| I-106 | 320 | 100 |
| I-077 | 320 | 90 |
| I-078 | 320 | 90 |
| I-079 | 320 | 90 |
| I-081 | 320 | 100 |
| I-082 | 320 | 100 |
| I-084 | 320 | 90 |
| I-087 | 320 | 100 |
| I-088 | 320 | 100 |
| I-142 | 320 | 90 |

**Table A5c: pre-emergence activity at 80g/ha and against SETVI in %**

| Example number | application rate [g/ha] | SETVI |
|---|---|---|
| I-003 | 80 | 100 |
| I-024 | 80 | 90 |
| I-042 | 80 | 90 |
| I-126 | 80 | 90 |
| I-188 | 80 | 90 |
| I-122 | 80 | 90 |
| I-071 | 80 | 90 |
| I-106 | 80 | 90 |
| I-079 | 80 | 90 |
| I-081 | 80 | 90 |
| I-082 | 80 | 90 |
| I-084 | 80 | 90 |
| I-087 | 80 | 100 |
| I-088 | 80 | 90 |

**Table A6a: pre-emergence activity at 1280g/ha against STEME in %**

| Example number | application rate [g/ha] | STEME |
|---|---|---|
| I-001 | 1280 | 90 |
| I-002 | 1280 | 90 |
| I-096 | 1280 | 100 |
| I-158 | 1280 | 90 |
| I-147 | 1280 | 90 |
| I-148 | 1280 | 90 |
| I-003 | 1280 | 90 |
| I-151 | 1280 | 90 |
| I-153 | 1280 | 100 |
| I-150 | 1280 | 90 |
| I-157 | 1280 | 100 |
| I-006 | 1280 | 90 |
| I-008 | 1280 | 90 |
| I-010 | 1280 | 90 |
| I-163 | 1280 | 90 |
| I-011 | 1280 | 90 |
| I-166 | 1280 | 90 |
| I-018 | 1280 | 90 |
| I-023 | 1280 | 90 |
| I-024 | 1280 | 100 |
| I-027 | 1280 | 90 |
| I-171 | 1280 | 90 |
| I-036 | 1280 | 90 |
| I-039 | 1280 | 90 |
| I-040 | 1280 | 90 |
| I-042 | 1280 | 90 |
| I-044 | 1280 | 90 |
| I-051 | 1280 | 90 |
| I-053 | 1280 | 90 |
| I-054 | 1280 | 90 |
| I-124 | 1280 | 90 |
| I-126 | 1280 | 90 |
| I-188 | 1280 | 90 |
| I-190 | 1280 | 90 |
| I-058 | 1280 | 90 |
| I-122 | 1280 | 100 |
| I-108 | 1280 | 90 |
| I-071 | 1280 | 90 |
| I-110 | 1280 | 90 |
| I-106 | 1280 | 90 |
| I-107 | 1280 | 90 |
| I-077 | 1280 | 90 |
| I-078 | 1280 | 90 |
| I-079 | 1280 | 90 |
| I-081 | 1280 | 90 |
| I-082 | 1280 | 90 |
| I-084 | 1280 | 90 |
| I-087 | 1280 | 90 |
| I-088 | 1280 | 90 |
| I-142 | 1280 | 90 |
| I-144 | 1280 | 90 |

**Table A6b: pre-emergence activity at 320g/ha against STEME in %**

| Example number | application rate [g/ha] | STEME |
|---|---|---|
| I-001 | 320 | 90 |
| I-003 | 320 | 90 |
| I-157 | 320 | 90 |
| I-006 | 320 | 90 |
| I-018 | 320 | 90 |
| I-023 | 320 | 90 |
| I-024 | 320 | 90 |
| I-036 | 320 | 90 |
| I-040 | 320 | 90 |
| I-042 | 320 | 90 |
| I-051 | 320 | 90 |
| I-053 | 320 | 90 |
| I-054 | 320 | 90 |
| I-124 | 320 | 90 |
| I-126 | 320 | 90 |
| I-122 | 320 | 90 |
| I-071 | 320 | 90 |
| I-077 | 320 | 90 |
| I-078 | 320 | 90 |
| I-079 | 320 | 90 |
| I-082 | 320 | 90 |
| I-084 | 320 | 90 |
| I-087 | 320 | 90 |
| I-088 | 320 | 90 |
| I-142 | 320 | 90 |

**Table A6c: pre-emergence activity at 80g/ha against STEME in %**

| Example number | application rate [g/ha] | STEME |
|---|---|---|
| I-003 | 80 | 90 |
| I-077 | 80 | 90 |
| I-078 | 80 | 90 |
| I-079 | 80 | 90 |
| I-082 | 80 | 90 |
| I-088 | 80 | 90 |

**Table A7a: pre-emergence activity at 1280g/ha and against POAAN in %**

| Example number | application rate [g/ha] | POAAN |
|---|---|---|
| I-001 | 1280 | 100 |
| I-002 | 1280 | 100 |
| I-097 | 1280 | 90 |
| I-167 | 1280 | 100 |
| I-146 | 1280 | 100 |
| I-168 | 1280 | 100 |
| I-096 | 1280 | 100 |
| I-158 | 1280 | 100 |
| I-147 | 1280 | 100 |
| I-148 | 1280 | 100 |
| I-149 | 1280 | 100 |
| I-003 | 1280 | 100 |
| I-151 | 1280 | 100 |
| I-153 | 1280 | 100 |
| I-150 | 1280 | 100 |
| I-157 | 1280 | 100 |
| I-005 | 1280 | 100 |
| I-006 | 1280 | 100 |
| I-007 | 1280 | 100 |
| I-008 | 1280 | 100 |
| I-009 | 1280 | 100 |
| I-010 | 1280 | 100 |
| I-163 | 1280 | 100 |
| I-011 | 1280 | 100 |
| I-014 | 1280 | 100 |
| I-015 | 1280 | 100 |
| I-166 | 1280 | 100 |
| I-170 | 1280 | 100 |
| I-016 | 1280 | 100 |
| I-018 | 1280 | 100 |
| I-021 | 1280 | 100 |
| I-022 | 1280 | 100 |
| I-023 | 1280 | 100 |
| I-024 | 1280 | 100 |
| I-025 | 1280 | 100 |
| I-026 | 1280 | 100 |
| I-028 | 1280 | 100 |
| I-029 | 1280 | 90 |
| I-030 | 1280 | 100 |
| I-032 | 1280 | 100 |
| I-171 | 1280 | 100 |
| I-036 | 1280 | 100 |
| I-038 | 1280 | 90 |
| I-039 | 1280 | 100 |
| I-040 | 1280 | 100 |
| I-042 | 1280 | 100 |
| I-044 | 1280 | 100 |
| I-047 | 1280 | 100 |
| I-048 | 1280 | 100 |
| I-178 | 1280 | 90 |
| I-179 | 1280 | 100 |
| I-180 | 1280 | 90 |
| I-049 | 1280 | 100 |
| I-051 | 1280 | 100 |
| I-053 | 1280 | 100 |
| I-054 | 1280 | 100 |
| I-182 | 1280 | 90 |
| I-124 | 1280 | 100 |
| I-119 | 1280 | 100 |
| I-126 | 1280 | 100 |
| I-137 | 1280 | 100 |
| I-139 | 1280 | 100 |
| I-188 | 1280 | 100 |
| I-190 | 1280 | 100 |
| I-058 | 1280 | 100 |
| I-122 | 1280 | 100 |
| I-128 | 1280 | 90 |
| I-132 | 1280 | 100 |
| I-108 | 1280 | 100 |
| I-109 | 1280 | 100 |
| I-071 | 1280 | 100 |
| I-105 | 1280 | 100 |
| I-110 | 1280 | 100 |
| I-106 | 1280 | 100 |
| I-107 | 1280 | 100 |
| I-111 | 1280 | 90 |
| I-077 | 1280 | 100 |
| I-078 | 1280 | 100 |
| I-113 | 1280 | 100 |
| I-114 | 1280 | 100 |
| I-112 | 1280 | 100 |
| I-079 | 1280 | 100 |
| I-081 | 1280 | 100 |
| I-082 | 1280 | 100 |
| I-083 | 1280 | 100 |
| I-084 | 1280 | 100 |
| I-086 | 1280 | 100 |
| I-087 | 1280 | 100 |
| I-088 | 1280 | 100 |
| I-142 | 1280 | 100 |
| I-144 | 1280 | 100 |
| I-116 | 1280 | 100 |

**Table A7b: pre-emergence activity at 320g/ha and against POAAN in %**

| Example number | application rate [g/ha] | POAAN |
|---|---|---|
| I-001 | 320 | 100 |
| I-002 | 320 | 100 |
| I-167 | 320 | 100 |
| I-146 | 320 | 100 |
| I-168 | 320 | 100 |
| I-096 | 320 | 100 |
| I-158 | 320 | 100 |
| I-148 | 320 | 100 |
| I-149 | 320 | 100 |
| I-003 | 320 | 100 |
| I-150 | 320 | 100 |
| I-157 | 320 | 100 |
| I-006 | 320 | 100 |
| I-008 | 320 | 100 |
| I-010 | 320 | 90 |
| I-014 | 320 | 100 |
| I-015 | 320 | 100 |
| I-166 | 320 | 90 |
| I-170 | 320 | 100 |
| I-016 | 320 | 100 |
| I-018 | 320 | 100 |
| I-021 | 320 | 100 |
| I-022 | 320 | 100 |
| I-023 | 320 | 100 |
| I-024 | 320 | 90 |
| I-025 | 320 | 100 |
| I-030 | 320 | 90 |
| I-032 | 320 | 100 |
| I-171 | 320 | 100 |
| I-036 | 320 | 90 |
| I-039 | 320 | 100 |
| I-040 | 320 | 100 |
| I-042 | 320 | 100 |
| I-044 | 320 | 100 |
| I-048 | 320 | 90 |
| I-049 | 320 | 90 |
| I-051 | 320 | 100 |
| I-053 | 320 | 90 |
| I-054 | 320 | 90 |
| I-124 | 320 | 100 |
| I-126 | 320 | 90 |
| I-137 | 320 | 100 |
| I-139 | 320 | 100 |
| I-188 | 320 | 100 |
| I-190 | 320 | 100 |
| I-122 | 320 | 100 |
| I-108 | 320 | 100 |
| I-109 | 320 | 100 |
| I-071 | 320 | 100 |
| I-110 | 320 | 90 |
| I-106 | 320 | 100 |
| I-107 | 320 | 100 |
| I-111 | 320 | 90 |
| I-077 | 320 | 100 |
| I-078 | 320 | 100 |
| I-113 | 320 | 100 |
| I-079 | 320 | 100 |
| I-081 | 320 | 100 |
| I-082 | 320 | 100 |
| I-083 | 320 | 100 |
| I-084 | 320 | 100 |
| I-086 | 320 | 100 |
| I-087 | 320 | 100 |
| I-088 | 320 | 100 |
| I-142 | 320 | 100 |
| I-144 | 320 | 100 |

**Table A7c: pre-emergence activity at 80g/ha and against POAAN in %**

| Example number | application rate [g/ha] | POAAN |
|---|---|---|
| I-001 | 80 | 100 |
| I-002 | 80 | 100 |
| I-167 | 80 | 100 |
| I-146 | 80 | 100 |
| I-168 | 80 | 100 |
| I-003 | 80 | 100 |
| I-157 | 80 | 100 |
| I-006 | 80 | 100 |
| I-008 | 80 | 100 |
| I-015 | 80 | 100 |
| I-166 | 80 | 90 |
| I-170 | 80 | 100 |
| I-018 | 80 | 100 |
| I-022 | 80 | 90 |
| I-023 | 80 | 100 |
| I-024 | 80 | 90 |
| I-030 | 80 | 90 |
| I-171 | 80 | 90 |
| I-036 | 80 | 90 |
| I-039 | 80 | 90 |
| I-042 | 80 | 100 |
| I-044 | 80 | 90 |
| I-051 | 80 | 90 |
| I-054 | 80 | 90 |
| I-124 | 80 | 90 |
| I-126 | 80 | 90 |
| I-137 | 80 | 100 |
| I-139 | 80 | 100 |
| I-188 | 80 | 90 |
| I-190 | 80 | 90 |
| I-122 | 80 | 90 |
| I-108 | 80 | 90 |
| I-071 | 80 | 100 |
| I-077 | 80 | 100 |
| I-079 | 80 | 100 |
| I-081 | 80 | 100 |
| I-082 | 80 | 100 |
| I-084 | 80 | 100 |
| I-087 | 80 | 100 |
| I-088 | 80 | 90 |
| I-142 | 80 | 90 |
| I-144 | 80 | 100 |

**Table A8a: pre-emergence activity at 1280g/ha against AMARE in %**

| Example number | application rate [g/ha] | AMARE |
|---|---|---|
| I-003 | 1280 | 90 |
| I-157 | 1280 | 90 |
| I-009 | 1280 | 100 |
| I-166 | 1280 | 90 |
| I-023 | 1280 | 90 |
| I-024 | 1280 | 90 |
| I-042 | 1280 | 90 |
| I-139 | 1280 | 90 |
| I-058 | 1280 | 100 |
| I-122 | 1280 | 100 |
| I-071 | 1280 | 90 |
| I-077 | 1280 | 90 |
| I-079 | 1280 | 90 |
| I-081 | 1280 | 90 |
| I-082 | 1280 | 90 |
| I-084 | 1280 | 90 |
| I-087 | 1280 | 90 |
| I-142 | 1280 | 90 |

**Table A8b: pre-emergence activity at 320g/ha against AMARE in %**

| Example number | application rate [g/ha] | AMARE |
|---|---|---|
| I-003 | 320 | 90 |
| I-157 | 320 | 90 |
| I-024 | 320 | 90 |
| I-071 | 320 | 90 |
| I-079 | 320 | 90 |
| I-082 | 320 | 90 |
| I-084 | 320 | 90 |
| I-087 | 320 | 90 |

**Table A9a: pre-emergence activity at 1280g/ha against ALOMY in %**

| Example number | application rate [g/ha] | ALOMY |
|---|---|---|
| I-158 | 1280 | 100 |
| I-147 | 1280 | 100 |
| I-149 | 1280 | 100 |
| I-003 | 1280 | 100 |
| I-157 | 1280 | 100 |
| I-006 | 1280 | 100 |
| I-008 | 1280 | 100 |
| I-010 | 1280 | 100 |
| I-163 | 1280 | 100 |
| I-014 | 1280 | 90 |
| I-015 | 1280 | 90 |
| I-170 | 1280 | 90 |
| I-018 | 1280 | 100 |
| I-021 | 1280 | 90 |
| I-022 | 1280 | 90 |
| I-023 | 1280 | 90 |
| I-027 | 1280 | 90 |
| I-036 | 1280 | 90 |
| I-039 | 1280 | 90 |
| I-040 | 1280 | 90 |
| I-042 | 1280 | 90 |
| I-044 | 1280 | 90 |
| I-051 | 1280 | 90 |
| I-126 | 1280 | 90 |
| I-188 | 1280 | 90 |
| I-071 | 1280 | 90 |
| I-077 | 1280 | 90 |
| I-078 | 1280 | 90 |
| I-079 | 1280 | 90 |
| I-081 | 1280 | 100 |
| I-082 | 1280 | 90 |
| I-084 | 1280 | 90 |
| I-087 | 1280 | 90 |
| I-088 | 1280 | 100 |
| I-142 | 1280 | 90 |

**Table A9b: pre-emergence activity at 320g/ha against ALOMY in %**

| Example number | application rate [g/ha] | ALOMY |
|---|---|---|
| I-158 | 320 | 100 |
| I-003 | 320 | 100 |
| I-157 | 320 | 100 |
| I-006 | 320 | 90 |
| I-008 | 320 | 100 |
| I-018 | 320 | 90 |
| I-022 | 320 | 90 |
| I-042 | 320 | 90 |
| I-051 | 320 | 90 |
| I-126 | 320 | 90 |
| I-071 | 320 | 90 |
| I-077 | 320 | 90 |
| I-079 | 320 | 90 |
| I-081 | 320 | 90 |
| I-082 | 320 | 90 |
| I-088 | 320 | 90 |

**Table A10: pre-emergence activity at 1280g/ha against KCHSC in %**

| Example number | application rate [g/ha] | KCHSC |
|---|---|---|
| I-024 | 1280 | 90 |

**Table A11: pre-emergence activity at 1280g/ha against VERPE in %**

| Example number | application rate [g/ha] | VERPE |
|---|---|---|
| I-004 | 1280 | 100 |
| I-157 | 1280 | 90 |
| I-006 | 1280 | 100 |
| I-163 | 1280 | 100 |
| I-071 | 1280 | 90 |
| I-077 | 1280 | 90 |
| I-079 | 1280 | 90 |
| I-082 | 1280 | 90 |

**Table A12a: pre-emergence activity at 1280g/ha against DIGSA in %**

| Example number | application rate [g/ha] | DIGSA |
|---|---|---|
| I-167 | 1280 | 90 |
| I-096 | 1280 | 100 |
| I-158 | 1280 | 100 |
| I-147 | 1280 | 100 |
| I-003 | 1280 | 100 |
| I-151 | 1280 | 100 |
| I-153 | 1280 | 100 |
| I-155 | 1280 | 100 |
| I-157 | 1280 | 100 |
| I-005 | 1280 | 100 |
| I-006 | 1280 | 100 |
| I-008 | 1280 | 100 |
| I-009 | 1280 | 90 |
| I-010 | 1280 | 90 |
| I-163 | 1280 | 90 |
| I-011 | 1280 | 100 |
| I-018 | 1280 | 100 |
| I-019 | 1280 | 100 |
| I-021 | 1280 | 100 |
| I-022 | 1280 | 90 |
| I-023 | 1280 | 90 |
| I-024 | 1280 | 90 |
| I-025 | 1280 | 100 |
| I-026 | 1280 | 100 |
| I-027 | 1280 | 90 |
| I-028 | 1280 | 90 |
| I-029 | 1280 | 100 |
| I-030 | 1280 | 90 |
| I-032 | 1280 | 100 |
| I-171 | 1280 | 100 |
| I-036 | 1280 | 100 |
| I-038 | 1280 | 90 |
| I-039 | 1280 | 90 |
| I-040 | 1280 | 100 |
| I-042 | 1280 | 100 |
| I-044 | 1280 | 100 |
| I-047 | 1280 | 90 |
| I-048 | 1280 | 90 |
| I-178 | 1280 | 90 |
| I-179 | 1280 | 90 |
| I-180 | 1280 | 90 |
| I-049 | 1280 | 90 |
| I-051 | 1280 | 90 |
| I-053 | 1280 | 90 |
| I-054 | 1280 | 100 |
| I-124 | 1280 | 90 |
| I-119 | 1280 | 100 |
| I-126 | 1280 | 100 |
| I-137 | 1280 | 100 |
| I-139 | 1280 | 100 |
| I-188 | 1280 | 100 |
| I-190 | 1280 | 100 |
| I-058 | 1280 | 100 |
| I-122 | 1280 | 100 |
| I-108 | 1280 | 100 |
| I-109 | 1280 | 100 |
| I-071 | 1280 | 100 |
| I-105 | 1280 | 100 |
| I-110 | 1280 | 90 |
| I-106 | 1280 | 100 |
| I-107 | 1280 | 100 |
| I-077 | 1280 | 100 |
| I-078 | 1280 | 90 |
| I-114 | 1280 | 90 |
| I-079 | 1280 | 90 |
| I-081 | 1280 | 100 |
| I-082 | 1280 | 100 |
| I-084 | 1280 | 90 |
| I-087 | 1280 | 100 |
| I-088 | 1280 | 100 |
| I-142 | 1280 | 100 |
| I-144 | 1280 | 90 |
| I-116 | 1280 | 90 |
| I-117 | 1280 | 90 |

**Table A12b: pre-emergence activity at 320g/ha against DIGSA in %**

| Example number | application rate [g/ha] | DIGSA |
|---|---|---|
| I-003 | 320 | 100 |
| I-157 | 320 | 100 |
| I-006 | 320 | 90 |
| I-008 | 320 | 90 |
| 1-018 | 320 | 100 |
| I-021 | 320 | 90 |
| I-022 | 320 | 90 |
| I-023 | 320 | 90 |
| I-024 | 320 | 90 |
| I-025 | 320 | 90 |
| I-027 | 320 | 90 |
| I-171 | 320 | 90 |
| I-036 | 320 | 90 |
| I-039 | 320 | 90 |
| I-040 | 320 | 90 |
| I-042 | 320 | 90 |
| I-044 | 320 | 90 |
| I-051 | 320 | 90 |
| I-053 | 320 | 90 |
| I-054 | 320 | 90 |
| I-119 | 320 | 90 |
| I-126 | 320 | 100 |
| I-137 | 320 | 100 |
| I-139 | 320 | 90 |
| I-188 | 320 | 90 |
| I-190 | 320 | 100 |
| I-058 | 320 | 90 |
| I-122 | 320 | 90 |
| I-109 | 320 | 90 |
| I-071 | 320 | 100 |
| I-105 | 320 | 100 |
| I-110 | 320 | 90 |
| I-106 | 320 | 90 |
| I-107 | 320 | 90 |
| I-077 | 320 | 100 |
| I-078 | 320 | 90 |
| I-079 | 320 | 90 |
| I-081 | 320 | 100 |
| I-082 | 320 | 100 |
| I-084 | 320 | 90 |
| I-087 | 320 | 100 |
| I-088 | 320 | 100 |
| I-142 | 320 | 90 |

**Table A12c: pre-emergence activity at 80g/ha against DIGSA in %**

| Example number | application rate [g/ha] | DIGSA |
|---|---|---|
| | | |
| I-003 | 80 | 100 |
| I-157 | 80 | 100 |
| 1-018 | 80 | 90 |
| I-042 | 80 | 90 |
| I-126 | 80 | 90 |
| I-137 | 80 | 100 |
| I-122 | 80 | 90 |
| I-071 | 80 | 90 |
| I-079 | 80 | 90 |
| I-081 | 80 | 90 |
| I-082 | 80 | 100 |
| I-084 | 80 | 90 |
| I-087 | 80 | 90 |
| I-088 | 80 | 100 |

As the results show (Tables A), various compounds of the general formula (I) according the invention have very good herbicidal pre-emergence efficacy against a broad spectrum of mono- and dicotyledonous weeds such as Abutilon theophrasti, Alopecurus myosuroides, Amaranthus retroflexus, Kochia scoparia, Digitaria sanguinalis, Echinochloa crus-galli, Lolium rigidum, Matricaria inodora, Poa annua, Setaria viridis, Stellaria media and Veronica persica at an application rate of 1280 g or below of active ingredient per hectare.

### B. Herbicidal post-emergence action

Seeds of mono- and dicotyledonous weed plants were placed in plastic pots in sandy loam soil (doubly sown with in each case one species of mono- or dicotyledonous weed plants per pot), covered with soil and cultivated in a greenhouse under controlled growth conditions. 2 to 3 weeks after sowing, the test plants were treated at the one-leaf stage. The compounds of the invention, formulated in the form of wettable powders (WP) or as emulsion concentrates (EC), were applied onto the green parts of the plants as aqueous suspension or emulsion with addition of 0.5% additive at a water application rate of 600 liters per hectare (converted). After the test plants had been kept in the greenhouse under optimum growth conditions for about 3 weeks, the activity of the preparations was rated visually in comparison to untreated controls. For example, 100% activity = the plants have died, 0% activity = like control plants.

Tables B1 to B12, below, show the effects of selected compounds of the general formula (I) according to table 1 on various harmful plants and an application rate corresponding to 1280 g/ha or below obtained by the experimental procedure mentioned above.

**Table B1a: post-emergence activity at 1280g/ha against ABUTH in %**

| Example number | application rate [g/ha] | ABUTH |
|---|---|---|
| I-003 | 1280 | 90 |
| I-005 | 1280 | 90 |
| I-006 | 1280 | 90 |
| I-010 | 1280 | 90 |
| I-077 | 1280 | 90 |
| I-078 | 1280 | 90 |
| I-079 | 1280 | 90 |
| I-082 | 1280 | 90 |
| I-084 | 1280 | 90 |

**Table B1b: post-emergence activity at 320g/ha against ABUTH in %**

| Example number | application rate [g/ha] | ABUTH |
|---|---|---|
| I-003 | 320 | 90 |
| I-006 | 320 | 90 |
| I-082 | 320 | 90 |

**Table B2a: post-emergence activity at 1280g/ha against ECHCG in %**

| Example number | application rate [g/ha] | ECHCG |
|---|---|---|
| I-002 | 1280 | 90 |
| I-158 | 1280 | 90 |
| I-003 | 1280 | 90 |
| I-006 | 1280 | 100 |
| I-007 | 1280 | 90 |
| I-010 | 1280 | 90 |
| I-166 | 1280 | 90 |
| I-170 | 1280 | 90 |
| I-016 | 1280 | 90 |
| I-018 | 1280 | 90 |
| I-021 | 1280 | 90 |
| I-023 | 1280 | 90 |
| I-035 | 1280 | 90 |
| I-039 | 1280 | 90 |
| I-040 | 1280 | 90 |
| I-042 | 1280 | 90 |
| I-051 | 1280 | 90 |
| I-053 | 1280 | 90 |
| I-071 | 1280 | 90 |
| I-077 | 1280 | 90 |
| I-078 | 1280 | 90 |
| I-079 | 1280 | 90 |
| I-082 | 1280 | 90 |
| I-084 | 1280 | 90 |

**Table B2b: post-emergence activity at 320g/ha against ECHCG in %**

| Example number | application rate [g/ha] | ECHCG |
|---|---|---|
| | | |
| I-003 | 320 | 90 |
| I-170 | 320 | 90 |
| I-042 | 320 | 90 |
| I-079 | 320 | 90 |
| I-082 | 320 | 90 |

**Table B3: post-emergence activity at 1280g/ha against LOLRI in %**

| Example number | application rate [g/ha] | LOLRI |
|---|---|---|
| I-170 | 1280 | 90 |
| I-018 | 1280 | 90 |
| I-023 | 1280 | 90 |
| I-124 | 1280 | 90 |
| I-079 | 1280 | 90 |

**Table B4a: post-emergence activity at 1280g/ha against MATIN in %**

| Example number | application rate [g/ha] | MATIN |
|---|---|---|
| I-009 | 1280 | 90 |
| I-166 | 1280 | 90 |
| I-170 | 1280 | 90 |
| I-018 | 1280 | 90 |
| I-021 | 1280 | 90 |
| I-042 | 1280 | 90 |
| I-077 | 1280 | 90 |
| I-079 | 1280 | 90 |
| I-081 | 1280 | 90 |
| I-082 | 1280 | 90 |
| I-084 | 1280 | 90 |
| I-088 | 1280 | 90 |

**Table B4b: post-emergence activity at 320g/ha against MATIN in %**

| Example number | application rate [g/ha] | MATIN |
|---|---|---|
| I-018 | 320 | 90 |
| I-077 | 320 | 90 |
| I-082 | 320 | 90 |

**Table B5: post-emergence activity at 1280g/ha against SETVI in %**

| Example number | application rate [g/ha] | SETVI |
|---|---|---|
| I-170 | 1280 | 90 |
| I-021 | 1280 | 90 |
| I-042 | 1280 | 90 |
| I-053 | 1280 | 90 |
| I-054 | 1280 | 90 |
| I-124 | 1280 | 90 |
| I-122 | 1280 | 90 |
| I-079 | 1280 | 90 |
| I-082 | 1280 | 90 |
| I-088 | 1280 | 90 |

**Table B6: post-emergence activity at 1280g/ha against STEME in %**

| Example number | application rate [g/ha] | STEME |
|---|---|---|
| I-148 | 1280 | 100 |
| I-157 | 1280 | 100 |
| I-018 | 1280 | 90 |
| I-043 | 1280 | 90 |
| I-051 | 1280 | 90 |
| I-122 | 1280 | 90 |
| I-105 | 1280 | 90 |
| I-107 | 1280 | 90 |
| I-114 | 1280 | 90 |
| I-079 | 1280 | 90 |
| I-082 | 1280 | 90 |
| I-084 | 1280 | 90 |
| I-087 | 1280 | 90 |

**Table B7a: post-emergence activity at 1280g/ha against POAAN in %**

| Example number | application rate [g/ha] | POAAN |
|---|---|---|
| I-002 | 1280 | 90 |
| I-096 | 1280 | 100 |
| I-158 | 1280 | 100 |
| I-003 | 1280 | 90 |
| I-004 | 1280 | 100 |
| I-153 | 1280 | 100 |
| I-154 | 1280 | 100 |
| I-155 | 1280 | 100 |
| I-157 | 1280 | 100 |
| I-006 | 1280 | 90 |
| I-008 | 1280 | 90 |
| I-163 | 1280 | 100 |
| I-015 | 1280 | 90 |
| I-166 | 1280 | 90 |
| I-170 | 1280 | 90 |
| I-016 | 1280 | 90 |
| I-018 | 1280 | 90 |
| I-021 | 1280 | 90 |
| I-022 | 1280 | 90 |
| I-023 | 1280 | 90 |
| I-042 | 1280 | 90 |
| I-051 | 1280 | 90 |
| I-054 | 1280 | 90 |
| I-124 | 1280 | 90 |
| I-119 | 1280 | 90 |
| I-126 | 1280 | 90 |
| I-137 | 1280 | 90 |
| I-188 | 1280 | 90 |
| I-190 | 1280 | 90 |
| I-058 | 1280 | 90 |
| I-122 | 1280 | 90 |
| I-108 | 1280 | 90 |
| I-071 | 1280 | 90 |
| I-105 | 1280 | 90 |
| I-077 | 1280 | 90 |
| I-078 | 1280 | 90 |
| I-079 | 1280 | 90 |
| I-081 | 1280 | 100 |
| I-082 | 1280 | 100 |
| I-084 | 1280 | 100 |
| I-088 | 1280 | 90 |
| I-142 | 1280 | 90 |

**Table B7b: post-emergence activity at 320g/ha against POAAN in %**

| Example number | application rate [g/ha] | POAAN |
|---|---|---|
| I-096 | 320 | 100 |
| I-003 | 320 | 90 |
| I-004 | 320 | 100 |
| I-154 | 320 | 100 |
| I-155 | 320 | 100 |
| I-157 | 320 | 100 |
| I-015 | 320 | 90 |
| I-170 | 320 | 90 |
| I-018 | 320 | 90 |
| I-126 | 320 | 90 |
| I-071 | 320 | 90 |
| I-077 | 320 | 90 |
| I-079 | 320 | 90 |
| I-081 | 320 | 90 |
| I-082 | 320 | 90 |
| I-084 | 320 | 100 |

**Table B7c: post-emergence activity at 80g/ha against POAAN in %**

| Example number | application rate [g/ha] | POAAN |
|---|---|---|
| I-003 | 80 | 90 |
| I-004 | 80 | 100 |
| I-126 | 80 | 90 |
| I-079 | 80 | 90 |
| I-081 | 80 | 90 |
| I-082 | 80 | 90 |

**Table B8: post-emergence activity at 1280g/ha against AMARE in %**

| Example number | application rate [g/ha] | AMARE |
|---|---|---|
| I-002 | 1280 | 90 |
| I-154 | 1280 | 100 |
| I-006 | 1280 | 90 |
| I-009 | 1280 | 90 |
| 1-018 | 1280 | 90 |
| I-042 | 1280 | 90 |
| I-048 | 1280 | 90 |
| I-122 | 1280 | 90 |
| I-105 | 1280 | 90 |
| I-110 | 1280 | 100 |
| I-106 | 1280 | 90 |
| I-107 | 1280 | 90 |
| I-113 | 1280 | 90 |
| I-112 | 1280 | 90 |
| I-079 | 1280 | 90 |
| I-086 | 1280 | 90 |
| I-087 | 1280 | 90 |
| I-144 | 1280 | 90 |

**Table B9a: post-emergence activity at 1280g/ha against ALOMY in %**

| Example number | application rate [g/ha] | ALOMY |
|---|---|---|
| I-003 | 1280 | 100 |
| I-154 | 1280 | 100 |
| I-005 | 1280 | 90 |
| I-006 | 1280 | 90 |
| I-008 | 1280 | 90 |
| I-010 | 1280 | 90 |
| I-170 | 1280 | 90 |

**Table B9b: post-emergence activity at 320g/ha against ALOMY in %**

| Example number | application rate [g/ha] | ALOMY |
|---|---|---|
| I-003 | 320 | 100 |
| I-154 | 320 | 100 |
| I-006 | 320 | 90 |

**Table B9c: post-emergence activity at 80g/ha against ALOMY in %**

| Example number | application rate [g/ha] | ALOMY |
|---|---|---|
| I-003 | 80 | 100 |

**Table B10a: post-emergence activity at 1280g/ha against KCHSC in %**

| Example number | application rate [g/ha] | KCHSC |
|---|---|---|
| I-005 | 1280 | 90 |
| I-009 | 1280 | 100 |
| I-163 | 1280 | 90 |
| I-171 | 1280 | 90 |
| I-122 | 1280 | 90 |
| I-132 | 1280 | 90 |

**Table BlOb: post-emergence activity at 320g/ha against KCHSC in %**

| Example number | application rate [g/ha] | KCHSC |
|---|---|---|
| I-009 | 320 | 100 |

**Table B11a: post-emergence activity at 1280g/ha against VERPE in %**

| Example number | application rate [g/ha] | VERPE |
|---|---|---|
| I-003 | 1280 | 90 |
| I-010 | 1280 | 90 |
| I-014 | 1280 | 90 |
| I-015 | 1280 | 90 |
| I-166 | 1280 | 90 |
| I-170 | 1280 | 90 |
| I-018 | 1280 | 90 |
| I-024 | 1280 | 90 |
| I-036 | 1280 | 90 |
| I-042 | 1280 | 90 |
| I-107 | 1280 | 90 |
| I-077 | 1280 | 90 |
| I-078 | 1280 | 90 |
| I-079 | 1280 | 90 |
| I-082 | 1280 | 90 |
| I-084 | 1280 | 90 |

**Table B11b: post-emergence activity at 320g/ha against VERPE in %**

| Example number | application rate [g/ha] | VERPE |
|---|---|---|
| I-003 | 320 | 90 |
| I-042 | 320 | 90 |
| I-079 | 320 | 90 |
| I-082 | 320 | 90 |
| I-084 | 320 | 90 |

**Table B11c: post-emergence activity at 80g/ha against VERPE in %**

| Example number | application rate [g/ha] | VERPE |
|---|---|---|
| I-003 | 80 | 90 |

**Table B12a: post-emergence activity at 1280g/ha against DIGSA in %**

| Example number | application rate [g/ha] | DIGSA |
|---|---|---|
| I-003 | 1280 | 100 |
| I-006 | 1280 | 90 |
| I-009 | 1280 | 100 |
| I-023 | 1280 | 90 |
| I-039 | 1280 | 90 |
| I-041 | 1280 | 90 |
| I-077 | 1280 | 90 |
| I-079 | 1280 | 90 |
| I-082 | 1280 | 90 |
| I-084 | 1280 | 90 |
| I-088 | 1280 | 90 |

**Table B12b: post-emergence activity at 320g/ha against DIGSA in %**

| Example number | application rate [g/ha] | DIGSA |
|---|---|---|
| I-003 | 320 | 100 |

**Table B12c: post-emergence activity at 80g/ha against DIGSA in %**

| Example number | application rate [g/ha] | DIGSA |
|---|---|---|
| I-003 | 80 | 100 |

As the results show (Tables B), various compounds of the general formula (I) according to the invention, in post-emergence applications, have very good herbicidal activity against harmful plants plants such as Abutilon theophrasti, Alopecurus myosuroides, Amaranthus retroflexus, Kochia scoparia, Digitaria sanguinalis, Echinochloa crus-galli, Lolium rigidum, Matricaria inodora, Poa annua, Setaria viridis, Stellaria media and Veronica persica at an application rate of 1280 g or below of active substance per hectare.

### C. Herbicidal pre-emergence action

Seeds of mono- and dicotyledonous weed plants and crop plants were sown, in plastic or organic planting pots, in sandy loam and covered with soil. The compounds according to the invention, formulated in the form of wettable powders (WP) or as emulsifiable concentrates (EC), were applied to the surface of the covering soil as aqueous suspension or emulsion, with the addition of 0.5% of an additive, at an application rate of 6001 of water/ha (converted).

Following treatment, the pots were placed in a greenhouse and kept under optimum growth conditions for the test plants. The visual grading of the damage to the test plants was carried out after ca. 3 weeks in comparison to untreated controls (herbicidal effect in percent (%): 100% effect = plants have died off, 0% effect = as control plants).

Tables C1 to C12 below show the effects of selected compounds of the general formula (I) according to table 1 on various harmful plants and an application rate corresponding to 320 g/ha or below obtained by the experimental procedure mentioned above.

**Table C1: pre-emergence activity at 320g/ha against KCHSC in %**

| Example number | application rate [g/ha] | KCHSC |
|---|---|---|
| I-066 | 320 | 100 |

**Table C2a: pre-emergence activity at 320g/ha against ALOMY in %**

| Example number | application rate [g/ha] | ALOMY |
|---|---|---|
| I-002 | 320 | 90 |
| I-003 | 320 | 100 |
| I-157 | 320 | 90 |
| I-006 | 320 | 90 |
| I-008 | 320 | 100 |
| I-170 | 320 | 90 |
| I-023 | 320 | 100 |
| I-040 | 320 | 80 |
| I-042 | 320 | 90 |
| I-044 | 320 | 90 |
| I-051 | 320 | 90 |
| I-053 | 320 | 90 |

**Table C2b: pre-emergence activity at 80g/ha against ALOMY in %**

| Example number | application rate [g/ha] | ALOMY |
|---|---|---|
| I-003 | 80 | 90 |
| I-170 | 80 | 90 |
| I-051 | 80 | 80 |
| I-053 | 80 | 80 |

**Table C3: pre-emergence activity at 320g/ha against AMARE in %**

| Example number | application rate [g/ha] | AMARE |
|---|---|---|
| I-003 | 320 | 90 |
| I-053 | 320 | 90 |

**Table C4: pre-emergence activity at 320g/ha against AVEFA in %**

| Example number | application rate [g/ha] | AVEFA |
|---|---|---|
| I-002 | 320 | 80 |
| I-003 | 320 | 90 |
| I-042 | 320 | 80 |

**Table C5a: pre-emergence activity at 320g/ha against DIGSA in %**

| Example number | application rate [g/ha] | DIGSA |
|---|---|---|
| I-001 | 320 | 100 |
| I-002 | 320 | 90 |
| I-157 | 320 | 90 |
| I-006 | 320 | 100 |
| I-008 | 320 | 90 |
| I-022 | 320 | 90 |
| I-023 | 320 | 100 |
| I-040 | 320 | 90 |
| I-042 | 320 | 90 |
| I-044 | 320 | 90 |
| I-051 | 320 | 100 |
| I-053 | 320 | 90 |

**Table C5b: pre-emergence activity at 80g/ha against DIGSA in %**

| Example number | application rate [g/ha] | DIGSA |
|---|---|---|
| I-001 | 80 | 90 |
| I-002 | 80 | 90 |
| I-023 | 80 | 90 |
| I-040 | 80 | 80 |
| I-042 | 80 | 90 |
| I-051 | 80 | 90 |
| I-053 | 80 | 80 |

**Table C6a: pre-emergence activity at 320g/ha against ECHCG in %**

| Example number | application rate [g/ha] | ECHCG |
|---|---|---|
| I-001 | 320 | 90 |
| I-002 | 320 | 90 |
| I-003 | 320 | 90 |
| I-157 | 320 | 80 |
| I-008 | 320 | 90 |
| I-170 | 320 | 90 |
| I-022 | 320 | 90 |
| I-023 | 320 | 90 |
| I-040 | 320 | 90 |
| I-042 | 320 | 90 |
| I-044 | 320 | 90 |
| I-051 | 320 | 100 |
| I-053 | 320 | 100 |

**Table C6b: pre-emergence activity at 80g/ha against ECHCG in %**

| Example number | application rate [g/ha] | ECHCG |
|---|---|---|
| I-003 | 80 | 90 |
| I-170 | 80 | 90 |
| I-040 | 80 | 80 |
| I-042 | 80 | 80 |
| I-051 | 80 | 100 |
| I-053 | 80 | 90 |

**Table C7a: pre-emergence activity at 320g/ha against LOLRI in %**

| Example number | application rate [g/ha] | LOLRI |
|---|---|---|
| I-001 | 320 | 80 |
| I-002 | 320 | 90 |
| I-003 | 320 | 90 |
| I-157 | 320 | 90 |
| I-170 | 320 | 90 |
| I-022 | 320 | 100 |
| I-023 | 320 | 100 |
| I-040 | 320 | 100 |
| I-042 | 320 | 90 |
| I-051 | 320 | 90 |
| I-053 | 320 | 90 |

**Table C7b: pre-emergence activity at 320g/ha against LOLRI in %**

| Example number | application rate [g/ha] | LOLRI |
|---|---|---|
| I-003 | 80 | 90 |
| I-042 | 80 | 90 |
| I-051 | 80 | 80 |

**Table C8: pre-emergence activity at 320g/ha against MATIN in %**

| Example number | application rate [g/ha] | MATIN |
|---|---|---|
| I-002 | 320 | 80 |
| I-003 | 320 | 80 |
| I-170 | 320 | 80 |
| I-023 | 320 | 80 |
| I-040 | 320 | 80 |
| I-042 | 320 | 80 |
| I-044 | 320 | 90 |
| I-053 | 320 | 80 |

**Table C9: pre-emergence activity at 320g/ha against POLCO in %**

| Example number | application rate [g/ha] | POLCO |
|---|---|---|
| I-023 | 320 | 80 |

**Table C10: pre-emergence activity at 320g/ha against SETVI in %**

| Example number | application rate [g/ha] | SETVI |
|---|---|---|
| I-002 | 320 | 80 |
| I-003 | 320 | 80 |
| I-008 | 320 | 90 |
| I-023 | 320 | 90 |
| I-040 | 320 | 80 |
| I-042 | 320 | 80 |
| I-051 | 320 | 80 |
| I-053 | 320 | 90 |

**Table C11: pre-emergence activity at 320g/ha against VERPE in %**

| Example number | application rate [g/ha] | VERPE |
|---|---|---|
| I-003 | 320 | 80 |
| I-157 | 320 | 80 |
| I-170 | 320 | 80 |
| I-053 | 320 | 80 |

**Table C12a: pre-emergence activity at 320g/ha against VIOTR in %**

| Example number | application rate [g/ha] | VIOTR |
|---|---|---|
| I-001 | 320 | 80 |
| I-002 | 320 | 80 |
| I-003 | 320 | 90 |
| I-157 | 320 | 90 |
| I-008 | 320 | 90 |
| I-022 | 320 | 80 |
| I-023 | 320 | 80 |
| I-044 | 320 | 90 |
| I-051 | 320 | 80 |
| I-066 | 320 | 80 |

**Table C12b: pre-emergence activity at 80g/ha against VIOTR in %**

| Example number | application rate [g/ha] | VIOTR |
|---|---|---|
| I-003 | 80 | 80 |
| I-023 | 80 | 80 |
| I-051 | 80 | 80 |

As the results show (Tables C), various compounds of the general formula (I) according the invention have very good herbicidal pre-emergence efficacy against a broad spectrum of mono- and dicotyledonous weeds such as Avena fatua, Alopecurus myosuroides, Amaranthus retroflexus, Kochia scoparia, Digitaria sanguinalis, Echinochloa crus-galli, Lolium rigidum, Matricaria inodora, Polygonum convolvulus, Setaria viridis, Veronica persica, and Viola triclolor at an application rate of 1280 g or below of active ingredient per hectare.

### D. Pre-emergence effects on crop plants

Tables D1 to D5 below show the effects of various compounds of the general formula (I) according to Table 1 on various crop plants and an application rate corresponding to 320 g/ha or below obtained by the experimental procedure mentioned above in Biological Examples, Section C.

**Table D1a: pre-emergence activity at 320g/ha against ZEAMX in %**

| Example number | application rate [g/ha] | ZEAMX |
|---|---|---|
| I-008 | 320 | 20 |
| I-020 | 320 | 0 |
| I-066 | 320 | 0 |
| I-091 | 320 | 0 |

**Table D1b: pre-emergence activity at 80g/ha against ZEAMX in %**

| Example number | application rate [g/ha] | ZEAMX |
|---|---|---|
| I-006 | 80 | 0 |
| I-008 | 80 | 10 |
| I-020 | 80 | 0 |
| I-022 | 80 | 0 |
| I-023 | 80 | 0 |
| I-040 | 80 | 20 |
| I-044 | 80 | 0 |
| I-066 | 80 | 0 |
| I-091 | 80 | 0 |

**Table D2a: pre-emergence activity at 320g/ha against TRZAS in %**

| Example number | application rate [g/ha] | TRZAS |
|---|---|---|
| I-006 | 320 | 20 |
| I-020 | 320 | 0 |
| I-066 | 320 | 0 |
| I-091 | 320 | 0 |

**Table D2b: pre-emergence activity at 80g/ha against TRZAS in %**

| Example number | application rate [g/ha] | TRZAS |
|---|---|---|
| I-006 | 80 | 0 |
| I-008 | 80 | 10 |
| I-020 | 80 | 0 |
| I-022 | 80 | 0 |
| I-066 | 80 | 0 |
| I-091 | 80 | 0 |

**Table D3a: pre-emergence activity at 320g/ha against ORYZA in %**

| Example number | application rate [g/ha] | ORYZA |
|---|---|---|
| I-006 | 320 | 0 |
| I-008 | 320 | 0 |
| I-020 | 320 | 0 |

**Table D3b: pre-emergence activity at 80g/ha against ORYZA in %**

| Example number | application rate [g/ha] | ORYZA |
|---|---|---|
| I-001 | 80 | 20 |
| I-006 | 80 | 0 |
| I-008 | 80 | 0 |
| I-020 | 80 | 0 |

**Table D4a: pre-emergence activity at 320g/ha against GLXMA in %**

| Example number | application rate [g/ha] | GLXMA |
|---|---|---|
| I-001 | 320 | 20 |
| I-157 | 320 | 0 |
| I-170 | 320 | 0 |
| I-020 | 320 | 0 |
| I-022 | 320 | 0 |
| I-023 | 320 | 0 |
| I-053 | 320 | 10 |
| I-066 | 320 | 0 |
| I-091 | 320 | 0 |

**Table D4b: pre-emergence activity at 80g/ha against GLXMA in %**

| Example number | application rate [g/ha] | GLXMA |
|---|---|---|
| I-001 | 80 | 0 |
| I-008 | 80 | 20 |
| I-020 | 80 | 0 |
| I-022 | 80 | 0 |
| I-023 | 80 | 0 |
| I-040 | 80 | 0 |
| I-042 | 80 | 0 |
| I-044 | 80 | 0 |
| I-051 | 80 | 0 |
| I-053 | 80 | 0 |
| I-066 | 80 | 0 |
| I-091 | 80 | 0 |

**Table D5a: pre-emergence activity at 320g/ha against BRSNW in %**

| Example number | application rate [g/ha] | BRSNW |
|---|---|---|
| I-002 | 320 | 0 |
| I-003 | 320 | 0 |
| I-157 | 320 | 0 |
| I-006 | 320 | 10 |
| I-008 | 320 | 10 |
| I-170 | 320 | 0 |
| I-020 | 320 | 0 |
| I-022 | 320 | 0 |
| I-023 | 320 | 0 |
| I-040 | 320 | 0 |
| I-042 | 320 | 10 |
| I-051 | 320 | 0 |
| I-066 | 320 | 0 |
| I-091 | 320 | 0 |

**Table D5b: pre-emergence activity at 80g/ha against BRSNW in %**

| Example number | application rate [g/ha] | BRSNW |
|---|---|---|
| I-001 | 80 | 0 |
| I-002 | 80 | 0 |
| I-003 | 80 | 0 |
| I-157 | 80 | 0 |
| I-006 | 80 | 0 |
| I-008 | 80 | 0 |
| I-170 | 80 | 0 |
| I-020 | 80 | 0 |
| I-022 | 80 | 0 |
| I-023 | 80 | 0 |
| I-040 | 80 | 0 |
| I-042 | 80 | 10 |
| I-044 | 80 | 0 |
| I-051 | 80 | 0 |
| I-053 | 80 | 10 |
| I-066 | 80 | 0 |
| I-091 | 80 | 0 |

As the results show (Tables D), compounds according to the invention show very low or no damaging effects in crop plants such as Triticum aestivum, Zea Mays, Oryza sativa, Glycine max, and Brassica napus.

### E. Herbicidal post-emergence action

Seeds of mono- and dicotyledonous weed plants and crop plants were sown, in plastic or organic planting pots, in sandy loam, covered with soil and grown in a greenhouse under controlled growth conditions. 2 to 3 weeks after sowing, the test plants were sprayed in the single-leaf stage. The compounds according to the invention, formulated in form of wettable powders (WP) or as emulsifiable concentrates (EC), were sprayed onto the green plant parts as aqueous suspension or emulsion, with the addition of 0.5% of an additive, at an application rate of 6001 of water/ha (converted). The test plants were placed in the greenhouse for ca. 3 weeks under optimum growth conditions, and then the effect of the preparations was assessed visually in comparison with untreated controls (herbicidal effect in percent (%): 100% effect = plants have died off, 0% effect = as control plants).

Tables E1 to E7 below show the effects of selected compounds of the general formula (I) according to table 1 on various harmful plants and an application rate corresponding to 320 g/ha or below obtained by the experimental procedure mentioned above.

**Table E1: post-emergence activity at 320g/ha against ALOMY in %**

| Example number | application rate [g/ha] | ALOMY |
|---|---|---|
| I-003 | 320 | 80 |
| I-157 | 320 | 80 |
| I-006 | 320 | 90 |
| I-170 | 320 | 80 |
| I-051 | 320 | 80 |

**Table E2: post-emergence activity at 320g/ha against ECHCG in %**

| Example number | application rate [g/ha] | ECHCG |
|---|---|---|
| I-002 | 320 | 80 |
| I-003 | 320 | 80 |
| I-170 | 320 | 80 |
| I-042 | 320 | 80 |

**Table E3: post-emergence activity at 320g/ha against LOLRI in %**

| Example number | application rate [g/ha] | LOLRI |
|---|---|---|
| I-003 | 320 | 80 |
| I-170 | 320 | 80 |
| I-042 | 320 | 90 |

**Table E4: post-emergence activity at 320g/ha against MATIN in %**

| Example number | application rate [g/ha] | MATIN |
|---|---|---|
| I-170 | 320 | 80 |

**Table E5: post-emergence activity at 320g/ha against SETVI in %**

| Example number | application rate [g/ha] | SETVI |
|---|---|---|
| I-003 | 320 | 80 |
| I-053 | 320 | 80 |

**Table E6: post-emergence activity at 320g/ha against VERPE in %**

| Example number | application rate [g/ha] | VERPE |
|---|---|---|
| I-003 | 320 | 80 |

**Table E7: post-emergence activity at 320g/ha against VIOTR in %**

| Example number | application rate [g/ha] | VIOTR |
|---|---|---|
| I-042 | 320 | 90 |

As the results show (Tables E), compounds according to the invention have good herbicidal post-emergence effectiveness against a broad spectrum of mono- and dicotyledonous weeds such as Alopecurus myosuroides, Echinochloa crus-galli, Lolium rigidum, Matricaria inodora, Setaria viridis, Veronica persica, and Viola tricolor at an application rate of 320 g and less of active ingredient per hectare.

### F. Post-emergence effects on crop plants

Tables F1 to F5 below show the effects of selected compounds of the general formula (I) according to table 1 on various crop plants and an application rate corresponding to 320 g/ha or below obtained by the experimental procedure mentioned above.

**Table F1a: post-emergence activity at 320g/ha against ZEAMX in %**

| Example number | application rate [g/ha] | ZEAMX |
|---|---|---|
| I-002 | 320 | 0 |
| I-157 | 320 | 0 |
| I-006 | 320 | 20 |
| I-008 | 320 | 0 |
| I-020 | 320 | 0 |
| I-022 | 320 | 0 |
| I-023 | 320 | 20 |
| I-040 | 320 | 0 |
| I-042 | 320 | 10 |
| I-044 | 320 | 0 |
| I-066 | 320 | 0 |
| I-091 | 320 | 0 |

**Table F1b: post-emergence activity at 80g/ha against ZEAMX in %**

| Example number | application rate [g/ha] | ZEAMX |
|---|---|---|
| I-001 | 80 | 0 |
| I-002 | 80 | 0 |
| I-157 | 80 | 0 |
| I-006 | 80 | 10 |
| I-008 | 80 | 0 |
| I-170 | 80 | 0 |
| I-020 | 80 | 0 |
| I-022 | 80 | 0 |
| I-023 | 80 | 0 |
| I-040 | 80 | 0 |
| I-042 | 80 | 0 |
| I-044 | 80 | 0 |
| I-051 | 80 | 20 |
| I-053 | 80 | 0 |
| I-066 | 80 | 0 |
| I-091 | 80 | 0 |

**Table F2a: post-emergence activity at 320g/ha against TRZAS in %**

| Example number | application rate [g/ha] | TRZAS |
|---|---|---|
| I-157 | 320 | 20 |
| I-006 | 320 | 20 |
| I-008 | 320 | 20 |
| I-022 | 320 | 0 |
| I-053 | 320 | 20 |
| I-066 | 320 | 0 |
| I-091 | 320 | 0 |

**Table F2b: post-emergence activity at 80g/ha against TRZAS in %**

| Example number | application rate [g/ha] | TRZAS |
|---|---|---|
| I-001 | 80 | 10 |
| I-157 | 80 | 0 |
| I-006 | 80 | 20 |
| I-008 | 80 | 20 |
| I-170 | 80 | 20 |
| I-022 | 80 | 0 |
| I-044 | 80 | 0 |
| I-053 | 80 | 10 |
| I-066 | 80 | 0 |
| I-091 | 80 | 0 |

**Table F3a: post-emergence activity at 320g/ha against ORYZA in %**

| Example number | application rate [g/ha] | ORYZA |
|---|---|---|
| I-006 | 320 | 0 |
| I-008 | 320 | 0 |
| I-020 | 320 | 0 |

**Table F3b: post-emergence activity at 80g/ha against ORYZA in %**

| Example number | application rate [g/ha] | ORYZA |
|---|---|---|
| I-001 | 80 | 0 |
| I-003 | 80 | 0 |
| I-157 | 80 | 10 |
| I-006 | 80 | 0 |
| I-008 | 80 | 0 |
| I-170 | 80 | 0 |
| I-020 | 80 | 0 |

**Table F4: post-emergence activity at 320g/ha and 80 g/ha against GLXMA in %**

| Example number | application rate [g/ha] | GLXMA |
|---|---|---|
| I-001 | 320 | 20 |
| I-002 | 80 | 20 |
| I-003 | 320 | 10 |
| I-157 | 320 | 0 |
| I-006 | 80 | 0 |
| I-008 | 320 | 0 |
| I-170 | 80 | 10 |
| I-020 | 320 | 0 |
| I-022 | 320 | 0 |
| I-040 | 80 | 0 |
| I-042 | 320 | 20 |
| I-044 | 320 | 0 |
| I-051 | 320 | 10 |
| I-053 | 320 | 20 |
| I-066 | 320 | 0 |
| I-091 | 320 | 0 |

**Table F5: post-emergence activity at 320 g/ha and 80 g/ha against BRSNW in %**

| Example number | application rate [g/ha] | BRSNW |
|---|---|---|
| I-001 | 80 | 10 |
| I-002 | 80 | 10 |
| I-003 | 320 | 0 |
| I-157 | 320 | 0 |
| I-006 | 320 | 10 |
| I-008 | 320 | 10 |
| I-170 | 320 | 0 |
| I-020 | 320 | 0 |
| I-022 | 320 | 0 |
| I-023 | 320 | 0 |
| I-040 | 320 | 0 |
| I-042 | 320 | 0 |
| I-044 | 80 | 0 |
| I-051 | 320 | 20 |
| I-053 | 320 | 20 |
| I-066 | 320 | 0 |
| I-091 | 320 | 0 |

As the results show (Tables F), compounds according to the invention show only low or no damaging effects in crop plants such as Triticum aestivum, Zea Mays, Oryza sativa, Glycine max, and Brassica napus.

## Claims

1. A substituted pyrazole carboxamide of the general formula (I), or salts thereof 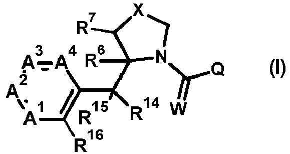 in which
Q represents a group Q-1 to Q-2 as outlined below 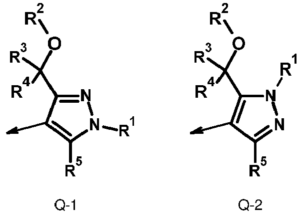
A¹, A², A³ and A⁴ are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
R¹⁷ in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
R¹⁷ in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
X represents a direct bond or a group CR⁸R⁹, -CR⁸R⁹CH₂-, -CH₂CR⁸R⁹-,
R¹ represents (C₁-C₈)-alkyl, (C₁-C₈)-haloalkyl, cyano-(C₁-C₈)-alkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, R¹²O-(C₁-C₈)-alkyl, R¹²O(R¹²O)CH-(C₁-C₈)-alkyl, tris[(C₁-C₈)-alkyl]silyl-O-(C₁-C₈)-alkyl, R¹⁰R¹¹-(C₁-C₈)-alkyl, C(=O)R¹², C(=O)OR¹², C(=O)NR¹⁰R¹¹, R¹²O(O=)C-(C₁-C₈)-alkyl, R¹²(O=)C-(C₁-C₈)-alkyl, R¹⁰R¹¹N(O=)C-(C₁-C₈)-alkyl, S(O)ₚR¹³, R¹³S(O)ₚ-(C₁-C₈)-alkyl, (C₃-C₈)-cycloalkyl-(C₁-C₈)-alkyl, (C₃-C₈)-halocycloalkyl-(C₁-C₈)-alkyl, (C₄-C₈)-cycloalkenyl-(C₁-C₈)-alkyl, aryl, heteroaryl, heterocyclyl, aryl-(C₁-C₈)-alkyl, heteroaryl-(C₁-C₈)-alkyl, (C₃-C₈)-heterocyclyl-(C₁-C₈)-alkyl, 1,3-dioxolan-2-yl-(C₁-C₈)-alkyl, 4,5-dimethyl-1,3-dioxolan-2-yl-(C₁-C₈)-alkyl, 1,3-dioxan-2-yl-(C₁-C₈)-alkyl,
R² represents (C₁-C₈)-alkyl, (C₁-C₈)-haloalkyl, cyano-(C₁-C₈)-alkyl, (C₂-C₈)-alkenyl, (C₂-C₈)-alkynyl, aryl, heteroaryl, heterocyclyl, aryl-(C₁-C₈)-alkyl, heteroaryl-(C₁-C₈)-alkyl, (C₃-C₈)-heterocyclyl-(C₁-C₈)-alkyl, (C₃-C₈)-cycloalkyl, (C₃-C₈)-cycloalkyl-(C₁-C₈)-alkyl, (C₃-C₈)-halocycloalkyl, (C₃-C₈)-halocycloalkyl-(C₁-C₈)-alkyl, (C₁-C₈)-alkoxy-(C₁-C₈)-alkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-haloalkoxy-(C₁-C₈)-alkyl, R¹³S(O)ₚ-(C₁-C₈)-alkyl, R¹⁰R¹¹N-(C₁-C₈)-alkyl, R¹²O(O=)C-(C₁-C₈)-alkyl, R¹²(O=)C-(C₁-C₈)-alkyl, R¹⁰R¹¹(O=)C-(C₁-C₈)-alkyl, tris[(C₁-C₈)-alkyl]silyl-O-(C₁-C₈)-alkyl, tris[(C₁-C₈)-alkyl]silyl-(C₁-C₈)-alkyl, tris[(C₁-C₈)-alkyl]silyl, bis-[(C₁-C₈)-alkyl](aryl)silyl, [(C₁-C₈)-alkyl]-bis-(aryl)silyl, bis-(C₁-C₈)-alkyl-C=N-, (C₁-C₈)-alkyl(aryl)-C=N-,
R³ and R⁴ independently of one another represent hydrogen, (C₁-C₈)-alkyl, (C₁-C₈)-haloalkyl, (C₃-C₈)-cycloalkyl, (C₂-C₈)-alkenyl, R¹²O-(C₁-C₈)-alkyl, (C₁-C₈)-alkoxy,
R⁵ represents hydrogen, halogen, (C₁-C₈)-alkyl, (C₁-C₈)-haloalkyl, (C₃-C₈)-cycloalkyl, (C₃-C₈)-halocycloalkyl,
R⁶ and R⁷ independently of one another represent hydrogen, (C₁-C₈)-alkyl, (C₁-C₈)-haloalkyl, (C₃-C₈)-cycloalkyl, (C₂-C₈)-alkenyl,
R⁸ and R⁹ independently of one another represent hydrogen, halogen, (C₁-C₈)-alkyl, (C₁-C₈)-haloalkyl, (C₃-C₈)-cycloalkyl, (C₂-C₈)-alkenyl, R¹²O-(C₁-C₈)-alkyl, OR¹², NR¹⁰R¹¹, SR¹³,
R³ and R⁴ together with the carbon atom to which they are bonded form a fully saturated or partly saturated 3- to 10-membered monocyclic or bicyclic ring optionally interrupted by heteroatoms and optionally having further substitution,
R³ and R⁴ together with the carbon atom to which they are bonded form a carbonyl group (C=O),
R² and R³ together with the atoms to which they are bonded form a fully saturated or partly saturated 4- to 10-membered monocyclic or bicyclic ring optionally interrupted by heteroatoms and optionally having further substitution,
R¹ and R⁵ together with the atoms to which they are bonded form a fully saturated or partly saturated 4- to 10-membered monocyclic or bicyclic ring optionally interrupted by heteroatoms and optionally having further substitution when Q represents a group Q-1,
R¹ and R² together with the atoms to which they are bonded form a fully saturated or partly saturated 4- to 10-membered monocyclic or bicyclic ring optionally interrupted by heteroatoms and optionally having further substitution when Q represents a group Q-2,
p is 0, 1, 2
R¹⁰ and R¹¹ independently of one another represent hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-haloalkoxy-(C₁-C₈)-alkyl, (C₁-C₈)-alkylthio-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, COR¹², SO₂R¹³, heterocyclyl, (C₁-C₈)-alkoxycarbonyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkyl-aminocarbonyl-(C₁-C₈)-alkyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl, aryl-(C₁-C₈)-alkoxycarbonyl, heteroaryl-(C₁-C₈)-alkoxycarbonyl, (C₂-C₈)-alkenyloxycarbonyl, (C₂-C₈)-alkynyloxycarbonyl, heterocyclyl-(C₁-C₈)-alkyl, or
R¹⁰ and R¹¹ together with the carbon atom to which they are bonded form a fully saturated or partly saturated 3- to 10-membered monocyclic or bicyclic ring optionally interrupted by heteroatoms and optionally having further substitution,
R¹² represents hydrogen, (C₁-C₈)-alkyl, (C₂-C₈)-alkenyl, (C₂-C₈)-alkinyl, (C₁-C₈)-cyanoalkyl, (C₁-C₁₀)-haloalkyl, (C₂-C₈)-haloalkenyl, (C₃-C₈)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (Ci-Cs)-alkoxy-(Ci-Cs)-alkyl, (Ci-Cs)-haloalkoxy-(Ci-Cs)-alkyl, (C₁-C₈)-alkoxy-(C₁-C₈)-haloalkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkoxy-(C₁-C₈)-alkyl, aryl, aryl-(C₁-C₈)-alkyl, aryl-(C₁-C₈)-alkoxy-(C₁-C₈)-alkyl, aryloxy-(C₁-C₈)-alkyl, heteroaryl-(C₁-C₈)-alkoxy-(C₁-C₈)-alkyl, heteroaryloxy-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkyl-aminocarbonyl-(C₁-C₈)-alkyl, bis-[(C₁-C₈)-alkyl]amino-(C₂-C₆)-alkyl, (C₁-C₈)-alkyl-amino-(C₂-C₆)-alkyl, aryl-(C₁-C₈)-alkylamino-(C₂-C₆)-alkyl, R¹³(O)ₚS-(C₁-C₈)-alkyl, hydroxycarbonyl-(C₁-C₈)-alkyl, heterocyclyl, heterocyclyl-(C₁-C₈)-alkyl, heterocyclyl-(C₁-C₈)-alkoxy-(C₁-C₈)-alkyl, heterocyclyloxy-(C₁-C₈)-alkyl, tris-[(C₁-C₈)-alkyl]silyl-(C₁-C₈)-alkyl, bis-[(C₁-C₈)-alkyl](aryl)silyl(C₁-C₈)-alkyl, [(C₁-C₈)-alkyl]-bis-(aryl)silyl-(C₁-C₈)-alkyl, (C₁-C₈)-alkylcarbonyloxy-(C₁-C₈)-alkyl, (C₃-C₈)-cycloalkylcarbonyloxy-(C₁-C₈)-alkyl, arylcarbonyloxy-(C₁-C₈)-alkyl, heteroarylcarbonyloxy-(C₁-C₈)-alkyl, Heterocyclylcarbonyloxy-(C₁-C₈)-alkyl, (C₁-C₈)-alkoxycarbonyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl,
R¹³ represents hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, heterocyclyl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]amino, (C₁-C₈)-alkyl-amino, aryl-(C₁-C₈)-amino, aryl-(C₁-C₆)-alkyl-amino, aryl-[(C₁-C₈)-alkyl]amino; heteroaryl-(C₁-C₈)-amino, heteroaryl-(C₁-C₆)-alkyl-amino, heteroaryl-[(C₁-C₈)-alkyl]amino; Hetercyclyl-(C₁-C₈)-amino, heterocyclyl-(C₁-C₆)-alkyl-amino, heterocyclyl-[(C₁-C₈)-alkyl]amino; (C₃-C₈)-cycloalkyl-amino, (C₃-C₈)-cycloalkyl-[(C₁-C₈)-alkyl]amino, (C₁-C₈)-alkylcarbonylamino, arylcarbonylamino, hetarylcarbonylamino, N-azetidinyl, N-pyrrolidinyl, N-piperidinyl, N-morpholinyl,
R¹⁴ and R¹⁵ independently of one another represent hydrogen, hydroxy, (C₁-C₈)-alkyl, (C₁-C₈)-haloalkyl, (C₃-C₈)-cycloalkyl, (C₂-C₈)-alkenyl, (C₁-C₈)-alkoxy, or
R¹⁴ and R¹⁵ together with the carbon atom to which they are bonded form a fully saturated or partly saturated 3- to 10-membered monocyclic or bicyclic ring optionally interrupted by heteroatoms and optionally having further substitution, or
R¹⁴ and R¹⁵ together with the carbon atom to which they are bonded form an ethylene group (C=CHz) or a carbonyl group (C=O),
R¹⁶ represents halogen, nitro, cyano, thiocyanato, (C₁-C₈)-alkyl, (C₁-C₈)-haloalkyl, cyano-(C₁-C₈)-alkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, OR¹², R¹²O-(C₁-C₈)-alkyl, R¹⁰R¹¹N-(C₁-C₈)-alkyl, NR¹⁰R¹¹, C(=O)R¹², C(=O)OR¹², C(=O)NR¹⁰R¹¹, R¹²O(O)C-(C₁-C₈)-alkyl, R¹²(O=)C-(C₁-C₈)-Alkyl, R¹⁰R¹¹N(O)C-(C₁-C₈)-alkyl, S(O)ₚR¹³, R¹³S(O)ₚ₋(C₁-C₈)-alkyl, (C₃-C₈)-cycloalkyl-(C₁-C₈)-alkyl, aryl, heteroaryl, heterocyclyl, aryl-(C₁-C₈)-alkyl, heteroaryl-(C₁-C₈)-alkyl, (C₃-C₈)-heterocyclyl-(C₁-C₈)-alkyl,
R¹⁷ represents hydrogen, halogen, nitro, cyano, thiocyanato, (C₁-C₈)-alkyl, (C₁-C₈)-haloalkyl, cyano-(C₁-C₈)-alkyl, (C₂-C₈)-alkenyl, (C₂-C₈)-haloalkenyl, (C₂-C₈)-alkynyl, (C₂-C₈)-haloalkynyl, (C₃-C₈)-cycloalkyl, (C₃-C₈)-halocycloalkyl, (C₄-C₈)-cycloalkenyl, (C₄-C₈)-halocycloalkenyl, OR¹², R¹²O-(C₁-C₈)-alkyl, R¹⁰R¹¹N-(C₁-C₈)-alkyl, NR¹⁰R¹¹, C(=O)R¹², C(=O)OR¹², C(=O)NR¹⁰R¹¹, R¹²O(O)C-(C₁-C₈)-alkyl, R¹²(O=)C-(C₁-C₈)-Alkyl, R¹⁰R¹¹N(O)C-(C₁-C₈)-alkyl, S(O)ₚR¹³, R¹³S(O)ₚ-(C₁-C₈)-alkyl, (C₃-C₈)-cycloalkyl-(C₁-C₈)-alkyl, (C₃-C₈)-halocycloalkyl-(C₁-C₈)-alkyl, (C₄-C₈)-cycloalkenyl-(C₁-C₈)-alkyl, (C₄-C₈)-halocycloalkenyl-(C₁-C₈)-alkyl, aryl, heterocyclyl, heteroaryl, aryl-(C₁-C₈)-alkyl, heteroaryl-(C₁-C₈)-alkyl, heterocyclyl-(C₁-C₈)-alkyl, tris[(C₁-C₈)alkyl]silyl, bis-[(C₁-C₈)-alkyl](aryl)silyl, (C₁-C₈)-alkyl-[bis-(aryl)]silyl, tris-[(C₁-C₈)-alkyl]silyl-(C₁-C₈)-alkyl, bis-[(C₁-C₈)-alkyl](aryl)silyl(C₁-C₈)-alkyl or (C₁-C₈)-alkyl-[bis-(aryl)] silyl-(C₁-C₈)-alkyl
and
W represents oxygen (O), sulfur (S).

2. The compound of the general formula (I) as claimed in claim 1 and/or the salt thereof, **characterized in that**
Q represents a group Q-1 to Q-2 as outlined below 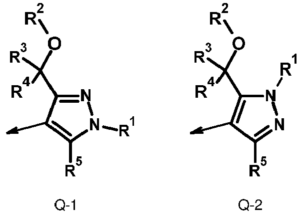
A¹, A², A³ and A⁴ are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
R¹⁷ in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
R¹⁷ in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
X represents a direct bond or a group CR⁸R⁹, -CR⁸R⁹CH₂-, -CH₂CR⁸R⁹-,
R¹ represents (C₁-C₇)-alkyl, (C₁-C₇)-haloalkyl, cyano-(C₁-C₇)-alkyl, (C₂-C₇)-alkenyl, (C₂-C₇)-haloalkenyl, (C₂-C₇)-alkynyl, (C₂-C₇)-haloalkynyl, (C₃-C₇)-cycloalkyl, (C₃-C₇)-halocycloalkyl, R¹²O-(C₁-C₇)-alkyl, R¹²O(R¹²O)CH-(C₁-C₇)-alkyl, tris[(C₁-C₇)-alkyl]silyl-O-(C₁-C₇)-alkyl, R¹⁰R¹¹N-(C₁-C₇)-alkyl, C(=O)R¹², C(=O)OR¹², C(=O)NR¹⁰R¹¹, R¹²O(O=)C-(C₁-C₇)-alkyl, R¹²(O=)C-(C₁-C₇)-alkyl, R¹⁰R¹¹N(O=)C-(C₁-C₇)-alkyl, S(O)ₚR¹³, R¹³S(O)ₚ-(C₁-C₇)-alkyl, (C₃-C₇)-cycloalkyl-(C₁-C₇)-alkyl, (C₃-C₇)-halocycloalkyl-(C₁-C₇)-alkyl, (C₄-C₇)-cycloalkenyl-(C₁-C₇)-alkyl, aryl, heteroaryl, heterocyclyl, aryl-(C₁-C₇)-alkyl, heteroaryl-(C₁-C₇)-alkyl, (C₃-C₇)-heterocyclyl-(C₁-C₇)-alkyl, 1,3-dioxolan-2-yl-(C₁-C₇)-alkyl, 4,5-dimethyl-1,3-dioxolan-2-yl-(C₁-C₇)-alkyl, 1,3-dioxan-2-yl-(C₁-C₇)-alkyl,
R² represents (C₁-C₇)-alkyl, (C₁-C₇)-haloalkyl, cyano-(C₁-C₇)-alkyl, (C₂-C₇)-alkenyl, (C₂-C₇)- (C₂-C₇)-alkynyl, aryl, heteroaryl, heterocyclyl, aryl-(C₁-C₇)-alkyl, heteroaryl-(C₁-C₇)-alkyl, (C₃-C₇)-heterocyclyl-(C₁-C₇)-alkyl, (C₃-C₇)-cycloalkyl, (C₃-C₇)-cycloalkyl-(C₁-C₇)-alkyl, (C₃-C₇)-halocycloalkyl, (C₃-C₇)-halocycloalkyl-(C₁-C₇)-alkyl, (C₁-C₇)-alkoxy-(C₁-C₇)-alkyl, (C₁-C₇)-alkoxy-(C₁-C₇)-alkoxy-(C₁-C₇)-alkyl, (C₁-C₇)-alkoxy-(C₁₋C₇)-alkoxy-(C₁-C₇)-alkoxy-(C₁-C₇)-alkyl, (C₁-C₇)-haloalkoxy-(C₁-C₇)-alkyl, R¹³S(O)ₚ-(C₁-C₇)-alkyl, R¹⁰R¹¹N-(C₁-C₇)-alkyl, R¹²O(O=)C-(C₁-C₇)-alkyl, R¹²(O=)C-(C₁-C₇)-alkyl, R¹⁰R¹¹N(O=)C-(C₁-C₇)-alkyl, tris[(C₁-C₇)-alkyl]silyl-O-(C₁-C₇)-alkyl, tris[(C₁-C₇)-alkyl]silyl-(C₁-C₇)-alkyl, tris[(C₁-C₇)-alkyl]silyl, bis-[(C₁-C₇)-alkyl](aryl)silyl, [(C₁-C₇)-alkyl]-bis-(aryl)silyl, bis-(C₁-C₇)-alkyl-C=N-, (C₁-C₇)-alkyl(aryl)-C=N-,
R³ and R⁴ independently of one another represent hydrogen, (C₁-C₇)-alkyl, (C₁-C₇)-haloalkyl, (C₃-C₇)-cycloalkyl, (C₂-C₇)-alkenyl, R¹²O-(C₁-C₇)-alkyl, (C₁-C₇)-alkoxy,
R⁵ represents hydrogen, halogen, (C₁-C₇)-alkyl, (C₁-C₇)-haloalkyl, (C₃-C₇)-cycloalkyl, (C₃-C₇)-halocycloalkyl,
R⁶ and R⁷ independently of one another represent hydrogen, (C₁-C₇)-alkyl, (C₁-C₇)-haloalkyl, (C₃-C₇)-cycloalkyl, (C₂-C₇)-alkenyl,
R⁸ and R⁹ independently of one another represent hydrogen, halogen, (C₁-C₇)-alkyl, (C₁-C₇)-haloalkyl, (C₃-C₇)-cycloalkyl, (C₂-C₇)-alkenyl, R¹²O-(C₁-C₇)-alkyl, OR¹², NR¹⁰R¹¹, SR¹³,
R³ and R⁴ together with the carbon atom to which they are bonded form a fully saturated or partly saturated 3- to 10-membered monocyclic or bicyclic ring optionally interrupted by heteroatoms and optionally having further substitution, or
R³ and R⁴ together with the carbon atom to which they are bonded form a carbonyl group (C=O),
R² and R³ together with the atoms to which they are bonded form a fully saturated or partly saturated 4- to 10-membered monocyclic or bicyclic ring optionally interrupted by heteroatoms and optionally having further substitution,
R¹ and R⁵ together with the atoms to which they are bonded form a fully saturated or partly saturated 4- to 10-membered monocyclic or bicyclic ring optionally interrupted by heteroatoms and optionally having further substitution when Q represents a group Q-1,
R¹ and R² together with the atoms to which they are bonded form a fully saturated or partly saturated 4- to 10-membered monocyclic or bicyclic ring optionally interrupted by heteroatoms and optionally having further substitution when Q represents a group Q-2,
p is 0, 1, 2
R¹⁰ and R¹¹ independently of one another represent hydrogen, (C₁-C₇)-alkyl, (C₂-C₇)-alkenyl, (C₂-C₇)-alkynyl, (C₁-C₇)-cyanoalkyl, (C₁-C₁₀)-haloalkyl, (C₂-C₇)-haloalkenyl, (C₃-C₇)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-haloalkoxy-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, (C₃-C₇)-cycloalkyl-(C₁-C₇)-alkyl, (C₄-C₁₀)-cycloalkenyl-(C₁-C₇)-alkyl, COR¹², SO₂R¹³, heterocyclyl, (C₁-C₇)-alkoxycarbonyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkyl-aminocarbonyl-(C₁-C₇)-alkyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl, aryl-(C₁-C₇)-alkoxycarbonyl, heteroaryl-(C₁-C₇)-alkoxycarbonyl, (C₂-C₇)-alkenyloxycarbonyl, (C₂-C₇)-alkynyloxycarbonyl, heterocyclyl-(C₁-C₇)-alkyl, or
R¹⁰ and R¹¹ together with the carbon atom to which they are bonded form a fully saturated or partly saturated 3- to 10-membered monocyclic or bicyclic ring optionally interrupted by heteroatoms and optionally having further substitution,
R¹² represents hydrogen, (C₁-C₇)-alkyl, (C₂-C₇)-alkenyl, (C₂-C₇)-alkinyl, (C₁-C₇)-cyanoalkyl, (C₁-C₁₀)-haloalkyl, (C₂-C₇)-haloalkenyl, (C₃-C₇)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-haloalkoxy-(C₁-C₇)-alkyl, (C₁-C₇)-alkoxy-(C₁-C₇)-haloalkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkoxy-(C₁-C₇)-alkyl, aryl, aryl-(C₁-C₇)-alkyl, aryl-(C₁-C₇)-alkoxy-(C₁-C₇)-alkyl, aryloxy-(C₁-C₇)-alkyl, heteroaryl-(C₁-C₇)-alkoxy-(C₁-C₇)-alkyl, heteroaryloxy-(C₁-C₇)-alkyl, heteroaryl, heteroaryl-(C₁-C₇)-alkyl, (C₃-C₇)-cycloalkyl-(C₁-C₇)-alkyl, (C₄-C₁₀)-cycloalkenyl-(C₁-C₇)-alkyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkyl-aminocarbonyl-(C₁-C₇)-alkyl, bis-[(C₁-C₇)-alkyl]amino-(C₂-C₆)-alkyl, (C₁-C₇)-alkyl-amino-(C₂-C₆)-alkyl, aryl-(C₁-C₇)-alkylamino-(C₂-C₆)-alkyl, R¹³(O)ₚS-(C₁-C₇)-alkyl, hydroxycarbonyl-(C₁-C₇)-alkyl, heterocyclyl, heterocyclyl-(C₁-C₇)-alkyl, heterocyclyl-(C₁-C₇)-alkoxy-(C₁-C₇)-alkyl, heterocyclyloxy-(C₁-C₇)-alkyl, tris-[(C₁-C₇)-alkyl]silyl-(C₁-C₇)-alkyl, bis-[(C₁-C₇)-alkyl](aryl)silyl(C₁-C₇)-alkyl, [(C₁-C₇)-alkyl]-bis-(aryl)silyl-(C₁-C₇)-alkyl, (C₁-C₇)-alkylcarbonyloxy-(C₁-C₇)-alkyl, (C₃-C₇)-cycloalkylcarbonyloxy-(C₁-C₇)-alkyl, arylcarbonyloxy-(C₁-C₇)-alkyl, heteroarylcarbonyloxy-(C₁-C₇)-alkyl, Heterocyclylcarbonyloxy-(C₁-C₇)-alkyl, (C₁-C₇)-alkoxycarbonyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl,
R¹³ represents hydrogen, (C₁-C₇)-alkyl, (C₂-C₇)-alkenyl, (C₂-C₇)-alkynyl, (C₁-C₇)-cyanoalkyl, (C₁-C₁₀)-haloalkyl, (C₂-C₇)-haloalkenyl, (C₃-C₇)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, heterocyclyl-(C₁-C₇)-alkyl, (C₃-C₇)-cycloalkyl-(C₁-C₇)-alkyl, (C₄-C₁₀)-cycloalkenyl-(C₁-C₇)-alkyl, bis-[(C₁-C₇)-alkyl]amino, (C₁-C₇)-alkyl-amino, aryl-(C₁-C₇)-amino, aryl-(C₁-C₆)-alkyl-amino, aryl-[(C₁-C₇)-alkyl]amino; heteroaryl-(C₁-C₇)-amino, heteroaryl-(C₁-C₆)-alkyl-amino, heteroaryl-[(C₁-C₇)-alkyl]amino; Hetercyclyl-(C₁-C₇)-amino, heterocyclyl-(C₁-C₆)-alkyl-amino, heterocyclyl-[(C₁-C₇)-alkyl]amino; (C₃-C₇)-cycloalkyl-amino, (C₃-C₇)-cycloalkyl-[(C₁-C₇)-alkyl]amino, (C₁-C₇)-alkylcarbonylamino, arylcarbonylamino, hetarylcarbonylamino, N-azetidinyl, N-pyrrolidinyl, N-piperidinyl, N-morpholinyl,
R¹⁴ and R¹⁵ independently of one another represent hydrogen, hydroxy, (C₁-C₇)-alkyl, (C₁-C₇)-haloalkyl, (C₃-C₇)-cycloalkyl, (C₂-C₇)-alkenyl, (C₁-C₇)-alkoxy, or
R¹⁴ and R¹⁵ together with the carbon atom to which they are bonded form a fully saturated or partly saturated 3- to 10-membered monocyclic or bicyclic ring optionally interrupted by heteroatoms and optionally having further substitution, or
R¹⁴ and R¹⁵ together with the carbon atom to which they are bonded form an ethylene group (C=CHz) or a carbonyl group (C=O),
R¹⁶ represents halogen, nitro, cyano, thiocyanato, (C₁-C₇)-alkyl, (C₁-C₇)-haloalkyl, cyano-(C₁-C₇)-alkyl, (C₂-C₇)-alkenyl, (C₂-C₇)-haloalkenyl, (C₂-C₇)-alkynyl, (C₂-C₇)-haloalkynyl, (C₃-C₇)-cycloalkyl, (C₃-C₇)-halocycloalkyl, OR¹², R¹²O-(C₁-C₇)-alkyl, R¹⁰R¹¹N-(C₁-C₇)-alkyl, NR¹⁰R¹¹, C(=O)R¹², C(=O)OR¹², C(=O)NR¹⁰R¹¹, R¹²O(O)C-(C₁-C₇)-alkyl, R¹²(O=)C-(C₁-C₇)-Alkyl, R¹⁰R¹¹N(O)C-(C₁-C₇)-alkyl, S(O)ₚR¹³, R¹³S(O)ₚ-(C₁-C₇)-alkyl, (C₃-C₇)-cycloalkyl-(C₁-C₇)-alkyl, aryl, heteroaryl, heterocyclyl, aryl-(C₁-C₇)-alkyl, heteroaryl-(C₁-C₇)-alkyl, (C₃-C₇)-heterocyclyl-(C₁-C₇)-alkyl,
R¹⁷ represents hydrogen, halogen, nitro, cyano, thiocyanato, (C₁-C₇)-alkyl, (C₁-C₇)-haloalkyl, cyano-(C₁-C₇)-alkyl, (C₂-C₇)-alkenyl, (C₂-C₇)-haloalkenyl, (C₂-C₇)-alkynyl, (C₂-C₇)-haloalkynyl, (C₃-C₇)-cycloalkyl, (C₃-C₇)-halocycloalkyl, (C₄-C₇)-cycloalkenyl, (C₄-C₇)-halocycloalkenyl, OR¹², R¹²O-(C₁-C₇)-alkyl, R¹⁰R¹¹N-(C₁-C₇)-alkyl, NR¹⁰R¹¹, C(=O)R¹², C(=O)OR¹², C(=O)NR¹⁰R¹¹, R¹²O(O)C-(C₁-C₇)-alkyl, R¹²(O=)C-(C₁-C₇)-Alkyl, R¹⁰R¹¹N(O)C-(C₁-C₇)-alkyl, S(O)ₚR¹³, R¹³S(O)ₚ-(C₁-C₇)-alkyl, (C₃-C₇)-cycloalkyl-(C₁-C₇)-alkyl, (C₃-C₇)-halocycloalkyl-(C₁-C₇)-alkyl, (C₄-C₇)-cycloalkenyl-(C₁-C₇)-alkyl, (C₄-C₇)-halocycloalkenyl-(C₁-C₇)-alkyl, aryl, heterocyclyl, heteroaryl, aryl-(C₁-C₇)-alkyl, heteroaryl-(C₁-C₇)-alkyl, heterocyclyl-(C₁-C₇)-alkyl, tris[(C₁-C₇)alkyl]silyl, bis-[(C₁-C₇)-alkyl](aryl)silyl, (C₁-C₇)-alkyl-[bis-(aryl)]silyl, tris-[(C₁-C₇)-alkyl]silyl-(C₁-C₇)-alkyl, bis-[(C₁-C₇)-alkyl](aryl)silyl(C₁-C₇)-alkyl or (C₁-C₇)-alkyl-[bis-(aryl)]silyl-(C₁-C₇)-alkyl
and
W represents oxygen.

3. The compound of the general formula (I) as claimed in claim 1 and/or the salt thereof, **characterized in that**
Q represents a group Q-1 to Q-2 as outlined below 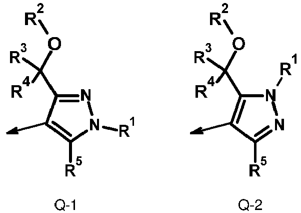
A¹, A², A³ and A⁴ are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
R¹⁷ in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
R¹⁷ in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
X represents a direct bond or a group CR⁸R⁹, -CR⁸R⁹CH₂-, -CH₂CR⁸R⁹-,
R¹ represents (C₁-C₆)-alkyl, (C₁-C₆)-haloalkyl, cyano-(C₁-C₆)-alkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, R¹²O-(C₁-C₆)-alkyl, R¹²O(R¹²O)CH-(C₁-C₆)-alkyl, tris[(C₁-C₆)-alkyl]silyl-O-(C₁-C₆)-alkyl, R¹⁰R¹¹N-(C₁-C₆)-alkyl, C(=O)R¹², C(=O)OR¹², C(=O)NR¹⁰R¹¹, R¹²O(O=)C-(C₁-C₆)-alkyl, R¹²(O=)C-(C₁-C₆)-alkyl, R¹⁰R¹¹N(O=)C-(C₁-C₆)-alkyl, S(O)ₚR¹³, R¹³S(O)ₚ-(C₁-C₆)-alkyl, (C₃-C₆)-cycloalkyl-(C₁-C₆)-alkyl, (C₃-C₆)-halocycloalkyl-(C₁-C₆)-alkyl, (C₄-C₆)-cycloalkenyl-(C₁-C₆)-alkyl, aryl, heteroaryl, heterocyclyl, aryl-(C₁-C₆)-alkyl, heteroaryl-(C₁-C₆)-alkyl, (C₃-C₆)-heterocyclyl-(C₁-C₆)-alkyl, 1,3-dioxolan-2-yl-(C₁-C₆)-alkyl, 4,5-dimethyl-1,3-dioxolan-2-yl-(C₁-C₆)-alkyl, 1,3-dioxan-2-yl-(C₁-C₆)-alkyl,
R² represents (C₁-C₆)-alkyl, (C₁-C₆)-haloalkyl, cyano-(C₁-C₆)-alkyl, (C₂-C₆)-alkenyl, (C₂-C₆)- (C₂-C₆)-alkynyl, aryl, heteroaryl, heterocyclyl, aryl-(C₁-C₆)-alkyl, heteroaryl-(C₁-C₆)-alkyl, (C₃-C₆)-heterocyclyl-(C₁-C₆)-alkyl, (C₃-C₆)-cycloalkyl, (C₃-C₆)-cycloalkyl-(C₁-C₆)-alkyl, (C₃-C₆)-halocycloalkyl, (C₃-C₆)-halocycloalkyl-(C₁-C₆)-alkyl, (C₁-C₆)-alkoxy-(C₁-C₆)-alkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-haloalkoxy-(C₁-C₆)-alkyl, R¹³S(O)ₚ-(C₁-C₆)-alkyl, R¹⁰R¹¹N-(C₁-C₆)-alkyl, R¹²O(O=)C-(C₁-C₆)-alkyl, R¹²(O=)C-(C₁-C₆)-alkyl, R¹⁰R¹¹N(O=)C-(C₁-C₆)-alkyl, tris[(C₁-C₆)-alkyl]silyl-O-(C₁-C₆)-alkyl, tris[(C₁-C₆)-alkyl]silyl-(C₁-C₆)-alkyl, tris[(C₁-C₆)-alkyl]silyl, bis-[(C₁-C₆)-alkyl](aryl)silyl, [(C₁-C₆)-alkyl]-bis-(aryl)silyl, bis-(C₁-C₆)-alkyl-C=N-, (C₁-C₆)-alkyl(aryl)-C=N-,
R³ and R⁴ independently of one another represent hydrogen, (C₁-C₆)-alkyl, (C₁-C₆)-haloalkyl, (C₃-C₆)-cycloalkyl, (C₂-C₆)-alkenyl, R¹²O-(C₁-C₆)-alkyl, (C₁-C₆)-alkoxy,
R⁵ represents hydrogen, halogen, (C₁-C₆)-alkyl, (C₁-C₆)-haloalkyl, (C₃-C₆)-cycloalkyl, (C₃-C₆)-halocycloalkyl,
R⁶ and R⁷ independently of one another represent hydrogen, (C₁-C₆)-alkyl, (C₁-C₆)-haloalkyl, (C₃-C₆)-cycloalkyl, (C₂-C₆)-alkenyl,
R⁸ and R⁹ independently of one another represent hydrogen, halogen, (C₁-C₆)-alkyl, (C₁-C₆)-haloalkyl, (C₃-C₆)-cycloalkyl, (C₂-C₆)-alkenyl, R¹²O-(C₁-C₆)-alkyl, OR¹², NR¹⁰R¹¹, SR¹³,
R³ and R⁴ together with the carbon atom to which they are bonded form a fully saturated or partly saturated 3- to 10-membered monocyclic or bicyclic ring optionally interrupted by heteroatoms and optionally having further substitution, or
R³ and R⁴ together with the carbon atom to which they are bonded form a carbonyl group (C=O),
R² and R³ together with the atoms to which they are bonded form a fully saturated or partly saturated 4- to 10-membered monocyclic or bicyclic ring optionally interrupted by heteroatoms and optionally having further substitution,
R¹ and R⁵ together with the atoms to which they are bonded form a fully saturated or partly saturated 4- to 10-membered monocyclic or bicyclic ring optionally interrupted by heteroatoms and optionally having further substitution when Q represents a group Q-1,
R¹ and R² together with the atoms to which they are bonded form a fully saturated or partly saturated 4- to 10-membered monocyclic or bicyclic ring optionally interrupted by heteroatoms and optionally having further substitution when Q represents a group Q-2,
p is 0, 1, 2
R¹⁰ and R¹¹ independently of one another represent hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₃-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-haloalkoxy-(C₁-C₆)-alkyl, (C₁-C₆)-alkylthio-(C₁-C₆)-alkyl, (C₁-C₆)-haloalkylthio-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, COR¹², SO₂R¹³, heterocyclyl, (C₁-C₆)-alkoxycarbonyl, bis-[(C₁-C₆)-alkyl]aminocarbonyl-(C₁-C₆)-alkyl, (C₁-C₆)-alkyl-aminocarbonyl-(C₁-C₆)-alkyl, aryl-(C₁-C₆)-alkyl-aminocarbonyl-(C₁-C₆)-alkyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl, aryl-(C₁-C₆)-alkoxycarbonyl, heteroaryl-(C₁-C₆)-alkoxycarbonyl, (C₂-C₆)-alkenyloxycarbonyl, (C₂-C₆)-alkynyloxycarbonyl, heterocyclyl-(C₁-C₆)-alkyl, or
R¹⁰ and R¹¹ together with the carbon atom to which they are bonded form a fully saturated or partly saturated 3- to 10-membered monocyclic or bicyclic ring optionally interrupted by heteroatoms and optionally having further substitution,
R¹² represents hydrogen, (C₁-C₆)-alkyl, (C₂-C₆)-alkenyl, (C₂-C₆)-alkinyl, (C₁-C₆)-cyanoalkyl, (C₁-C₁₀)-haloalkyl, (C₂-C₆)-haloalkenyl, (C₃-C₆)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-haloalkoxy-(C₁-C₆)-alkyl, (C₁-C₆)-alkoxy-(C₁-C₆)-haloalkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkoxy-(C₁-C₆)-alkyl, aryl, aryl-(C₁-C₆)-alkyl, aryl-(C₁-C₆)-alkoxy-(C₁-C₆)-alkyl, aryloxy-(C₁-C₆)-alkyl, heteroaryl-(C₁-C₆)-alkoxy-(C₁-C₆)-alkyl, heteroaryloxy-(C₁-C₆)-alkyl, heteroaryl, heteroaryl-(C₁-C₆)-alkyl, (C₃-C₆)-cycloalkyl-(C₁-C₆)-alkyl, (C₄-C₁₀)-cycloalkenyl-(C₁-C₆)-alkyl, bis-[(C₁-C₆)-alkyl]aminocarbonyl-(C₁-C₆)-alkyl, (C₁-C₆)-alkyl-aminocarbonyl-(C₁-C₆)-alkyl, aryl-(C₁-C₆)-alkyl-aminocarbonyl-(C₁-C₆)-alkyl, bis-[(C₁-C₆)-alkyl]amino-(C₂-C₆)-alkyl, (C₁-C₆)-alkyl-amino-(C₂-C₆)-alkyl, aryl-(C₁-C₆)-alkylamino-(C₂-C₆)-alkyl, R¹³(O)ₚS-(C₁-C₆)-alkyl, hydroxycarbonyl-(C₁-C₆)-alkyl, heterocyclyl, heterocyclyl-(C₁-C₆)-alkyl, heterocyclyl-(C₁-C₆)-alkoxy-(C₁-C₆)-alkyl, heterocyclyloxy-(C₁-C₆)-alkyl, tris-[(C₁-C₆)-alkyl]silyl-(C₁-C₆)-alkyl, bis-[(C₁-C₆)-alkyl](aryl)silyl(C₁-C₆)-alkyl, [(C₁-C₆)-alkyl]-bis-(aryl)silyl-(C₁-C₆)-alkyl, (C₁-C₆)-alkylcarbonyloxy-(C₁-C₆)-alkyl, (C₃-C₆)-cycloalkylcarbonyloxy-(C₁-C₆)-alkyl, arylcarbonyloxy-(C₁-C₆)-alkyl, heteroarylcarbonyloxy-(C₁-C₆)-alkyl, Heterocyclylcarbonyloxy-(C₁-C₆)-alkyl, (C₁-C₆)-alkoxycarbonyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl,
R¹³ represents hydrogen, (C₁-C₆)-alkyl, (C₂-C₆)-alkenyl, (C₂-C₆)-alkynyl, (C₁-C₆)-cyanoalkyl, (C₁-C₁₀)-haloalkyl, (C₂-C₆)-haloalkenyl, (C₃-C₆)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₆)-alkoxy-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, heterocyclyl-(C₁-C₆)-alkyl, (C₃-C₆)-cycloalkyl-(C₁-C₆)-alkyl, (C₄-C₁₀)-cycloalkenyl-(C₁-C₆)-alkyl, bis-[(C₁-C₆)-alkyl]amino, (C₁-C₆)-alkyl-amino, aryl-(C₁-C₆)-amino, aryl-(C₁-C₆)-alkyl-amino, aryl-[(C₁-C₆)-alkyl]amino; heteroaryl-(C₁-C₆)-amino, heteroaryl-(C₁-C₆)-alkyl-amino, heteroaryl-[(C₁-C₆)-alkyl]amino; Hetercyclyl-(C₁-C₆)-amino, heterocyclyl-(C₁-C₆)-alkyl-amino, heterocyclyl-[(C₁-C₆)-alkyl]amino; (C₃-C₆)-cycloalkyl-amino, (C₃-C₆)-cycloalkyl-[(C₁-C₆)-alkyl]amino, (C₁-C₆)-alkylcarbonylamino, arylcarbonylamino, hetarylcarbonylamino, N-azetidinyl, N-pyrrolidinyl, N-piperidinyl, N-morpholinyl,
R¹⁴ and R¹⁵ independently of one another represent hydrogen, hydroxy, (C₁-C₆)-alkyl, (C₁-C₆)-haloalkyl, (C₃-C₆)-cycloalkyl, (C₂-C₆)-alkenyl, (C₁-C₆)-alkoxy, or
R¹⁴ and R¹⁵ together with the carbon atom to which they are bonded form a fully saturated or partly saturated 3- to 10-membered monocyclic or bicyclic ring optionally interrupted by heteroatoms and optionally having further substitution, or
R¹⁴ and R¹⁵ together with the carbon atom to which they are bonded form an ethylene group (C=CHz) or a carbonyl group (C=O),
R¹⁶ represents halogen, nitro, cyano, thiocyanato, (C₁-C₆)-alkyl, (C₁-C₆)-haloalkyl, cyano-(C₁-C₆)-alkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, OR¹², R¹²O-(C₁-C₆)-alkyl, R¹⁰R¹¹N-(C₁-C₆)-alkyl, NR¹⁰R¹¹, C(=O)R¹², C(=O)OR¹², C(=O)NR¹⁰Rn, R¹²O(O)C-(C₁-C₆)-alkyl, R¹²(O=)C-(C₁-C₆)-Alkyl, R¹⁰R¹¹N(O)C-(C₁-C₆)-alkyl, S(O)ₚR¹³, R¹³S(O)ₚ-(C₁-C₆)-alkyl, (C₃-C₆)-cycloalkyl-(C₁-C₆)-alkyl, aryl, heteroaryl, heterocyclyl, aryl-(C₁-C₆)-alkyl, heteroaryl-(C₁-C₆)-alkyl, (C₃-C₆)-heterocyclyl-(C₁-C₆)-alkyl,
R¹⁷ represents hydrogen, halogen, nitro, cyano, thiocyanato, (C₁-C₆)-alkyl, (C₁-C₆)-haloalkyl, cyano-(C₁-C₆)-alkyl, (C₂-C₆)-alkenyl, (C₂-C₆)-haloalkenyl, (C₂-C₆)-alkynyl, (C₂-C₆)-haloalkynyl, (C₃-C₆)-cycloalkyl, (C₃-C₆)-halocycloalkyl, (C₄-C₆)-cycloalkenyl, (C₄-C₆)-halocycloalkenyl, OR¹², R¹²O-(C₁-C₆)-alkyl, R¹⁰R¹¹N-(C₁-C₆)-alkyl, NR¹⁰R¹¹, C(=O)R¹², C(=O)OR¹², C(=O)NR¹⁰R¹¹, R¹²O(O)C-(C₁-C₆)-alkyl, R¹²(O=)C-(C₁-C₆)-Alkyl, R¹⁰R¹¹N(O)C-(C₁-C₆)-alkyl, S(O)ₚR¹³, R¹³S(O)ₚ-(C₁-C₆)-alkyl, (C₃-C₆)-cycloalkyl-(C₁-C₆)-alkyl, (C₃-C₆)-halocycloalkyl-(C₁-C₆)-alkyl, (C₄-C₆)-cycloalkenyl-(C₁-C₆)-alkyl, (C₄-C₆)-halocycloalkenyl-(C₁-C₆)-alkyl, aryl, heterocyclyl, heteroaryl, aryl-(C₁-C₆)-alkyl, heteroaryl-(C₁-C₆)-alkyl, heterocyclyl-(C₁-C₆)-alkyl, tris[(C₁-C₆)alkyl]silyl, bis-[(C₁-C₆)-alkyl](aryl)silyl, (C₁-C₆)-alkyl-[bis-(aryl)]silyl, tris-[(C₁-C₆)-alkyl]silyl-(C₁-C₆)-alkyl, bis-[(C₁-C₆)-alkyl](aryl)silyl(C₁-C₆)-alkyl or (C₁-C₆)-alkyl-[bis-(aryl)]silyl-(C₁-C₆)-alkyl
and
W represents oxygen (O).

4. The compound of the general formula (I) as claimed in claim 1 and/or the salt thereof, **characterized in that**
Q represents a group Q-1 to Q-2 as outlined below 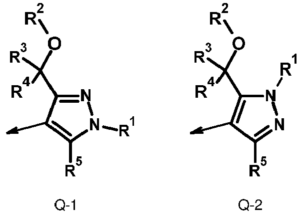
A¹, A², A³ and A⁴ are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
R¹⁷ in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
R¹⁷ in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
X represents a direct bond or a group CR⁸R⁹, -CR⁸R⁹CH₂-, -CH₂CR⁸R⁹-,
R¹ represents methyl, ethyl, prop-1-yl, 1-methylethyl, but-1-yl, 1-methylprop-1-yl, 2-methylprop-1-yl, 1,1-dimethylethyl, pent-1-yl, 1-methylbut-1-yl, 2-methylbut-1-yl, 3-methylbut-1-yl, 1,1-dimethylprop-1-yl, 1,2-dimethylprop-1-yl, 2,2-dimethylprop-1-yl, 1-ethylprop-1-yl, hex-1-yl, prop-2-en-1-yl, but-2-en-1-yl, but-3-en-1-yl, pent-2-en-1-yl, pent-3-en-1-yl, pent-4-en-1-yl, 1-methylprop-2-en-1-yl, 2-methylprop-2-en-1-yl, 1-methylbut-2-en-1-yl, 2-methylbut-2-en-1-yl, 3-methylbut-2-en-1-yl, 1-methylbut-3-en-1-yl, 2-methylbut-3-en-1-yl, 3-methylbut-3-en-1-yl, 1,1-dimethylprop-2-en-1-yl, 1,2-dimethylprop-2-en-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methylbuty-2-n-1-yl, 1-methylbut-3-yn-1-yl, 2-methylbut-3-yn-1-yl, cyclobutyl, cyclopentyl, cyclohexyl, cyclopropylmethyl, cyclobutylmethyl, cyclopentylmethyl, cyclohexylmethyl, 2-cyclopropyleth-1-yl, 1-cyclopropyleth-1-yl, cyanomethyl, 2-cyanoeth-1-yl, 1-cyanoeth-1-yl, 3-cyano-prop-1-yl, 2-cyano-prop-1-yl, prop-2-yn-1-yl, but-2-yn-1-yl, but-3-yn-1-yl, 1-methylprop-2-yn-1-yl, pent-2-yn-1-yl, pent-3-yn-1-yl, pent-4-yn-1-yl, 1-methylbut-2-yn-1-yl, 1-methylbut-3-yn-1-yl, 1,1-dimethylprop-2-yn-1-yl, 1-ethylprop-2-yn-1-yl, hex-2-yn-1-yl, hex-3-yn-1-yl, hex-4-yn-1-yl, hex-5-yn-1-yl, 1,3-dioxolan-2-ylmethyl, 4,5-dimethyl-1,3-dioxolan-2-ylmethyl, 1,3-dioxan-2-ylmethyl, trifluoromethyl, pentafluoroethyl, 1,1,2,2-tetrafluoroethyl, heptafluoropropyl, nona-fluorobutyl, 1-fluoroeth-1-yl, 2-fluoroeth-1-yl, difluoromethyl, 2,2-difluoroeth-1-yl, 2,2,2-trifluoroeth-1-yl, 3,3,3-trifluoroprop-1-yl, methoxymethyl, ethoxymethyl, 2-ethoxyeth-1-yl, 1-ethoxyeth-1-yl, 2-methoxyeth-1-yl, 1-methoxyeth-1-yl, 3-methoxy-prop-1-yl, 3-ethoxyprop-1-yl, 4-methoxybut-1-yl, 3-methoxyprop-2-yl, 2,2-di-methoxyeth-1-yl, 3,3-dimethoxyprop-1-yl, trifluoromethoxymethyl, benzyl, 4-fluoro-benzyl, 3-fluorobenzyl, 2-fluorobenzyl, 4-chlorobenzyl, 3-chlorobenzyl, 2-chloro-benzyl, phenyl, 4-fluorophenyl, 3-fluorophenyl, 2-fluorophenyl, 4-chlorophenyl, 3-chlorophenyl, 2-chlorophenyl, methylsulfanylmethyl, ethylsulfanylmethyl, 2-(methyl-sulfanyl)eth-1-yl, 2-(ethylsulfanyl)eth-1-yl, trifluoromethylsulfanylmethyl, pyridin-2-yl, pyridin-3-yl, pyridin-4-yl, pyridin-2-ylmethyl, pyridin-3-ylmethyl, pyridin-4-ylmethyl, 2-(2-methoxyethoxy)eth-1-yl, 2-(2-ethoxyethoxy)eth-1-yl, methylamino-carbonylmethyl, aminocarbonylmethyl, ethylaminocarbonylmethyl, dimethylamino-carbonylmethyl, diethylaminocarbonylmethyl, methyl(ethyl)aminocarbonylmethyl, hydroxycarbonylmethyl, methoxycarbonylmethyl, ethoxycarbonylmethyl, 1-methyleth-1-yloxycarbonylmethyl, 1,1-dimethyleth-1-yloxycarbonylmethyl, 2-hydroxycarbonyleth-1-yl, 2-methoxycarbonyleth-1-yl, 2-ethoxycarbonyleth-1-yl, 2-(1-methyleth-1-yloxycarbonyl)eth-1-yl, 2-(1,1-dimethyleth-1-yloxycarbonyl)eth-1-yl, 2-(benzyloxycarbonyl)eth-1-yl, 1-hydroxycarbonyleth-1-yl, 1-methoxycarbonyleth-1-yl, 1-ethoxycarbonyleth-1-yl, 1-(1-methyleth-1-yloxycarbonyl)eth-1-yl, 1-(1,1-dimethyleth-1-yloxycarbonyl)eth-1-yl, 1-(benzyloxycarbonyl)eth-1-yl, 1-hydroxy-carbonyl-1-methyleth-1-yl, 1-methoxycarbonyl-1-methyleth-1-yl, 1-ethoxycarbonyl-1-methyleth-1-yl, 1-(1-methyleth-1-yloxycarbonyl)-1-methyleth-1-yl, 1-(1,1-dimethyleth-1-yloxycarbonyl)1-methyleth-1-yl, 1-(benzyloxycarbonyl)1-methyleth-1-yl, methylaminocarbonyl, dimethylaminocarbonyl, diethylaminocarbonyl, methyl-(ethyl)aminocarbonyl, 1-methyleth-1-ylaminocarbonyl, cyclopropylaminocarbonyl, cyclobutylaminocarbonyl, cyclopentylaminocarbonyl, cyclohexylaminocarbonyl, phenylaminocarbonyl, benzylaminocarbonyl, methoxycarbonyl, ethoxycarbonyl, 1-methyleth-1-yloxycarbonyl, 1,1-dimethyleth-1-yloxycarbonyl, benzyloxycarbonyl, 2-(methylamino)eth-1-yl, 2-(dimethylamino)eth-1-yl, 2-(ethylamino)eth-1-yl, 2-(diethylamino)eth-1-yl, 2-(methylsulfonylamino)eth-1-yl, 2-(ethylsulfonylamino)eth-1-yl, 2-(benzylsulfonylamino)eth-1-yl, 2-(phenylsulfonylamino)eth-1-yl, 2-(methylcarbonylaminosulfonylamino)eth-1-yl, 2-(ethylcarbonylamino-sulfonylamino)eth-1-yl, methylsulfonylmethyl, 2-(methylsulfonyl)eth-1-yl, ethylsulfonylmethyl, 2-(ethylsulfonyl)eth-1-yl, (2-trismethylsilyleth-1-yloxy)methyl, (2-trismethylsilyleth-1-yloxy)eth-1-yl, methylcarbonylaminosulfonylmethyl, ethylcarbonylaminosulfonylmethyl, 2-(methylcarbonylaminosulfonyl)eth-1-yl, tetrahydrofuran-2-ylmethyl, tetrahydrofuran-3-ylmethyl, oxetan-3-ylmethyl, tetrahydropyran-2-ylmethyl, tetrahydropyran-3-ylmethyl, tetrahydropyran-4-ylmethyl, oxetan-3-yl, tetrahydrofuran-3-yl,
R² represents methyl, ethyl, prop-1-yl, 1-methylethyl, but-1-yl, 1-methylprop-1-yl, 2-methylprop-1-yl, 1,1-dimethylethyl, pent-1-yl, 1-methylbut-1-yl, 2-methylbut-1-yl, 3-methylbut-1-yl, 1,1-dimethylprop-1-yl, 1,2-dimethylprop-1-yl, 2,2-dimethylprop-1-yl, 1-ethylprop-1-yl, hex-1-yl, prop-2-en-1-yl, but-2-en-1-yl, but-3-en-1-yl, pent-2-en-1-yl, pent-3-en-1-yl, pent-4-en-1-yl, 1-methylprop-2-en-1-yl, 2-methylprop-2-en-1-yl, 1-methylbut-2-en-1-yl, 2-methylbut-2-en-1-yl, 3-methylbut-2-en-1-yl, 1-methylbut-3-en-1-yl, 2-methylbut-3-en-1-yl, 3-methylbut-3-en-1-yl, 1,1-dimethylprop-2-en-1-yl, 1,2-dimethylprop-2-en-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methylbuty-2-n-1-yl, 1-methylbut-3-yn-1-yl, 2-methylbut-3-yn-1-yl, cyclobutyl, cyclopentyl, cyclohexyl, cyclopropylmethyl, cyclobutylmethyl, cyclopentylmethyl, cyclohexylmethyl, 3,3-difluorocyclobutyl, 3,3-difluorocyclobutylmethyl, (1-fluorocycloprop-1-yl)methyl), (1-chlorocycloprop-1-yl)methyl), (1-cyanocycloprop-1-yl)methyl), (1-methylcyclo-prop-1-yl)methyl), 2-cyclopropyleth-1-yl, 1-cyclopropyleth-1-yl, cyanomethyl, 2-cyanoeth-1-yl, 1-cyanoeth-1-yl, 3-cyano-prop-1-yl, 2-cyano-prop-1-yl, prop-2-yn-1-yl, but-2-yn-1-yl, but-3-yn-1-yl, 1-methylprop-2-yn-1-yl, pent-2-yn-1-yl, pent-3-yn-1-yl, pent-4-yn-1-yl, 1-methylbut-2-yn-1-yl, 1-methylbut-3-yn-1-yl, 1,1-dimethylprop-2-yn-1-yl, 1-ethylprop-2-yn-1-yl, hex-2-yn-1-yl, hex-3-yn-1-yl, hex-4-yn-1-yl, hex-5-yn-1-yl, benzyl, 4-fluorobenzyl, 3-fluorobenzyl, 2-fluorobenzyl, 4-chlorobenzyl, 3-chlorobenzyl, 2-chlorobenzyl, phenyl, 4-fluorophenyl, 3-fluorophenyl, 2-fluoro-phenyl, 4-chlorophenyl, 3-chlorophenyl, 2-chlorophenyl, 4-methylphenyl, trifluoromethyl, pentafluoroethyl, 1,1,2,2-tetrafluoroethyl, heptafluoropropyl, nonafluorobutyl, 1-fluoroeth-1-yl, 2-fluoroeth-1-yl, difluoromethyl, 2,2-difluoroeth-1-yl, 2,2,2-trifluoroeth-1-yl, 3,3,3-trifluoroprop-1-yl, 2,2-difluoroprop-1-yl, 3,3,3,2,2-pentafluoroprop-1-yl, 3,3,2,2-tetrafluoroprop-1-yl, 2-chloro-2,2-difluoroeth-1-yl, 2,2,2-trifluoro-1-methyleth-1-yl, 2-ethoxyeth-1-yl, 1-ethoxyeth-1-yl, 2-methoxyeth-1-yl, 1-methoxyeth-1-yl, 3-methoxyprop-1-yl, 3-ethoxyprop-1-yl, 4-methoxybut-1-yl, 1-(methoxymethyl)eth-1-yl, 2-(2-methoxyethoxy)eth-1-yl, 2-(2-ethoxyethoxy)eth-1-yl, 2-(2-methoxyethoxyethoxy)eth-1-yl, 2-(2-ethoxyethoxyethoxy)eth-1-yl, 2-(2-methoxyethoxyethoxyethoxy)eth-1-yl, 2-(2-ethoxyethoxyethoxyethoxy)eth-1-yl, tetrahydrofuran-2-ylmethyl, tetrahydrofuran-3-ylmethyl, oxetan-3-ylmethyl, tetrahydropyran-2-ylmethyl, tetrahydropyran-3-ylmethyl, tetrahydropyran-4-ylmethyl, oxetan-3-yl, tetrahydrofuran-3-yl, dimethylimino, diethylamino,
R³ and R⁴ independently of one another represent hydrogen, methyl, ethyl, prop-1-yl, 1-methyleth-1-yl, 1,1-dimethyleth-1-yl, or R³ and R⁴ together with the carbon atom to which they are bonded form a carbonyl group (C=O),
R⁵ represents hydrogen, fluorine, chlorine, bromine, iodine, methyl, ethyl, 1-methyleth-1-yl, 1,1-dimethyleth-1-yl, prop-1-yl, difluoromethyl, trifluoromethyl, cyclopropyl, cyclobutyl, cyclopentyl, cyclohexyl,
R⁶ and R⁷ independently of one another represent hydrogen, methyl, ethyl, 1-methyleth-1-yl, 1,1-dimethyleth-1-yl, prop-1-yl, difluoromethyl, trifluoromethyl, cyclopropyl, cyclobutyl, cyclopentyl, cyclohexyl,
R⁸ and R⁹ independently of one another represent hydrogen, fluoro, chloro, bromo, methyl, ethyl, prop-1-yl, 1-methyleth-1-yl, 1,1-dimethyleth-1-yl, trifluoromethyl, cyclopropyl, cyclobutyl, cyclopentyl, methoxymethyl, methoxy, hydroxy, dimethylamino, methylsulfanyl,
R² and R³ together with the atoms to which they are bonded form a fully saturated or partly saturated 4- to 10-membered monocyclic or bicyclic ring optionally interrupted by heteroatoms and optionally having further substitution,
R¹ and R⁵ together with the atoms to which they are bonded form a fully saturated or partly saturated 4- to 10-membered monocyclic or bicyclic ring optionally interrupted by heteroatoms and optionally having further substitution when Q represents a group Q-1,
R¹ and R² together with the atoms to which they are bonded form a fully saturated or partly saturated 4- to 10-membered monocyclic or bicyclic ring optionally interrupted by heteroatoms and optionally having further substitution when Q represents a group Q-2,
R¹⁴ and R¹⁵ independently of one another represent hydrogen, hydroxy, methyl, ethyl, prop-1-yl, 1-methyl-eth-1-yl, trifluoromethyl, difluoromethyl, 2,2-difluoroeth-1-yl, cyclo-propyl, cyclobutyl, cyclopentyl, cyclohexyl, prop-2-en-1-yl, methoxy,
R¹⁴ and R¹⁵ together with the carbon atom to which they are bonded form a fully saturated or partly saturated 3- to 10-membered monocyclic or bicyclic ring optionally interrupted by heteroatoms and optionally having further substitution,
R¹⁴ and R¹⁵ together with the carbon atom to which they are bonded form an ethylene group (C=CHz) or a carbonyl group (C=O),
R¹⁶ represents fluoro, chloro, bromo, iodo, nitro, cyano, thiocyanato, methyl, ethyl, 1-methyleth-1-yl, 1,1-dimethyleth-1-yl, prop-1-yl, cyclopropyl, cyclobutyl, cyclopentyl, cyclohexyl, ethenyl, ethynyl, difluoromethyl, trifluoromethyl, chlorodifluoromethyl, 2,2-difluoroeth-1-yl, 3,3,3-trifluoroeth-1-yl, methoxy, ethoxy, 1-methyleth-1-yloxy, methylsulfanyl, ethylsulfanyl, trifluoromethoxy, difluoromethoxy, trifluoromethylsulfanyl, methylsulfonyl,
R¹⁷ represents hydrogen, fluoro, chloro, bromo, iodo, nitro, cyano, methyl, ethyl, difluoromethyl, trifluoromethyl, methoxy, trifluoromethoxy, difluoromethoxy
and
W represents oxygen (O).

5. The compound of the general formula (I) as claimed in claim 1 and/or the salt thereof, **characterized in that**
Q represents a group Q-1.1 to Q-2.35 as outlined below
| | | | | |
|---|---|---|---|---|
| 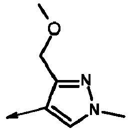 | 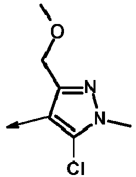 | 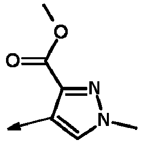 | 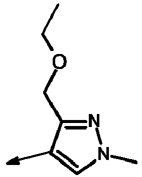 | 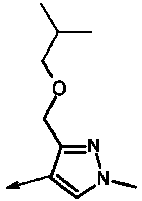 |
| Q-1.1 | Q-1.2 | Q-1.3 | Q-1.4 | Q-1.5 |
| 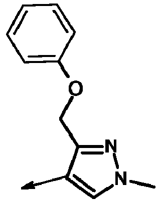 | 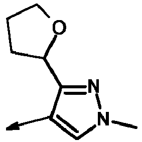 | 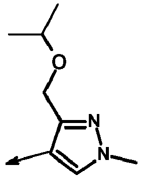 | 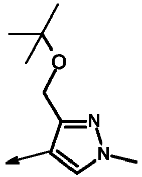 | 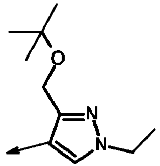 |
| Q-1.6 | Q-1.7 | Q-1.8 | Q-1.9 | Q-1.10 |
| 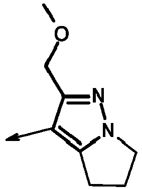 | 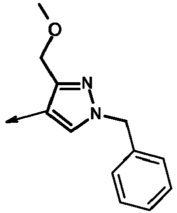 | 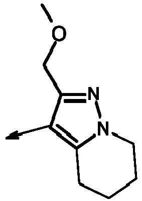 | 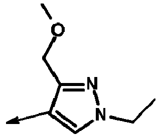 | 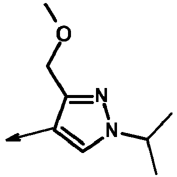 |
| Q-1.11 | Q-1.12 | Q-1.13 | Q-1.14 | Q-1.15 |
| 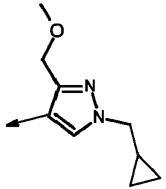 | 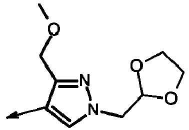 | 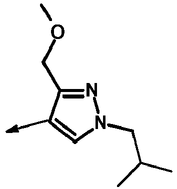 | 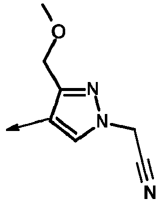 | 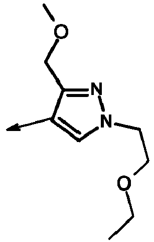 |
| Q-1.16 | Q-1.17 | Q-1.18 | Q-1.19 | Q-1.20 |
| 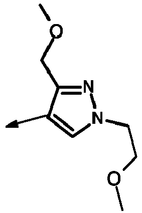 | 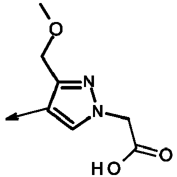 | 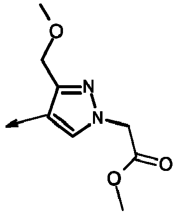 | 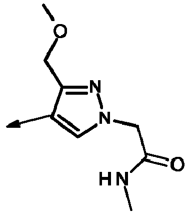 | 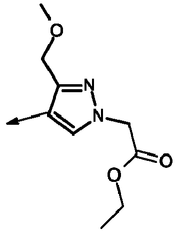 |
| Q-1.21 | Q-1.22 | Q-1.23 | Q-1.24 | Q-1.25 |
| 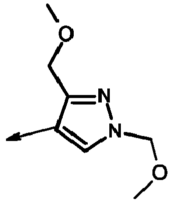 | 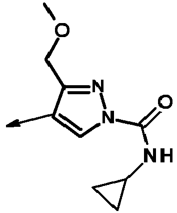 | 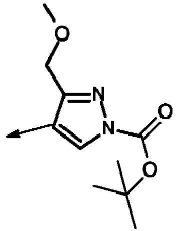 | 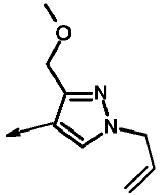 | 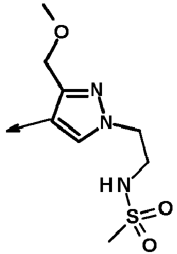 |
| Q-1.26 | Q-1.27 | Q-1.28 | Q-1.29 | Q-1.30 |
| 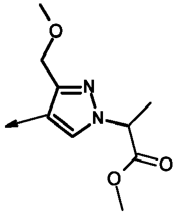 | 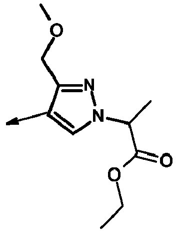 | 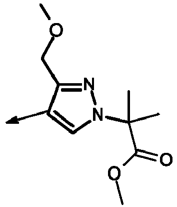 | 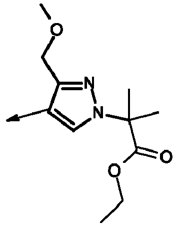 | 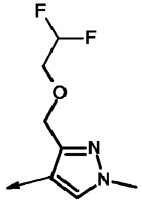 |
| Q-1.31 | Q-1.32 | Q-1.33 | Q-1.34 | Q-1.35 |
| 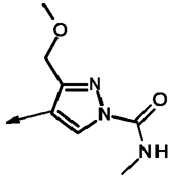 | 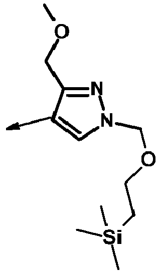 | 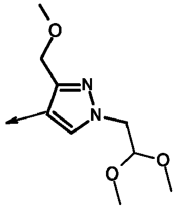 | 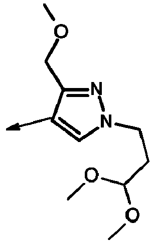 | 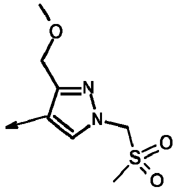 |
| Q-1.36 | Q-1.37 | Q-1.38 | Q-1.39 | Q-1.40 |
| 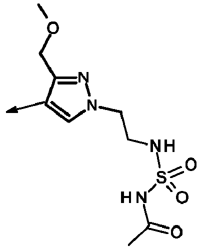 | 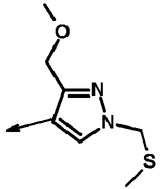 | 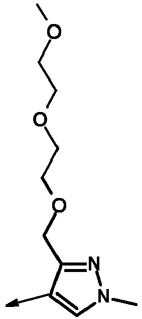 | 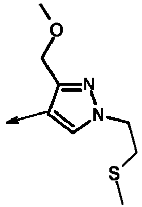 | 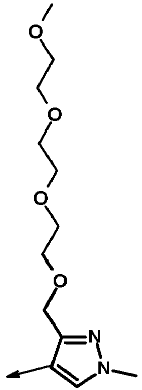 |
| Q-1.41 | Q-1.42 | Q-1.43 | Q-1.44 | Q-1.45 |
| 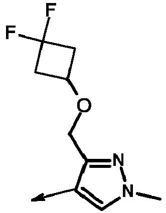 | 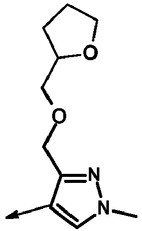 | 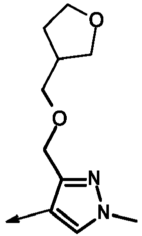 | 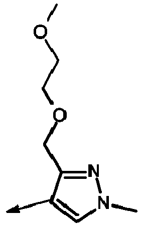 | 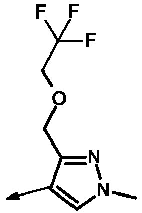 |
| Q-1.46 | Q-1.47 | Q-1.48 | Q-1.49 | Q-1.50 |
| 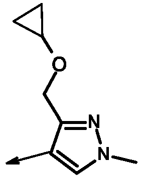 | 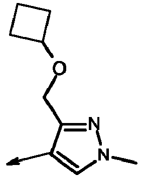 | 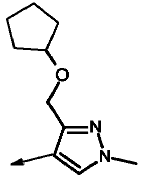 | 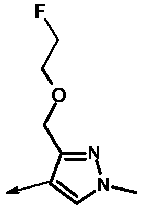 | 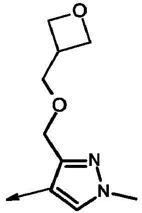 |
| Q-1.51 | Q-1.52 | Q-1.53 | Q-1.54 | Q-1.55 |
| 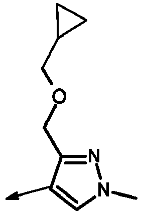 | 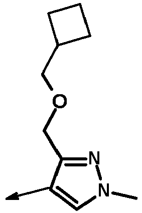 | 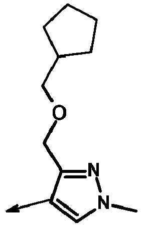 | 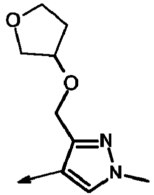 | 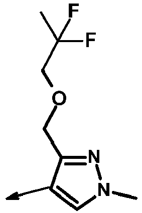 |
| Q-1.56 | Q-1.57 | Q-1.58 | Q-1.59 | Q-1.60 |
| 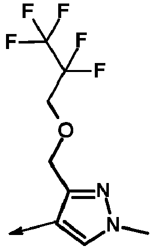 | 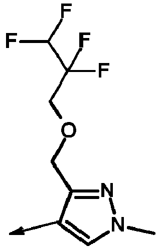 | 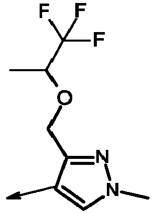 | 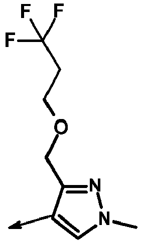 | 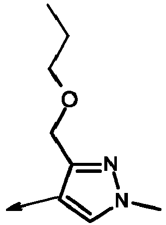 |
| Q-1.61 | Q-1.62 | Q-1.63 | Q-1.64 | Q-1.65 |
| 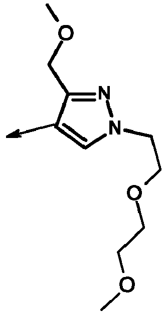 | 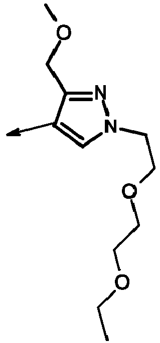 | 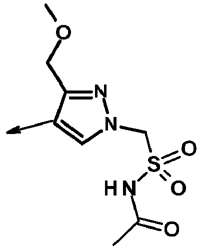 | 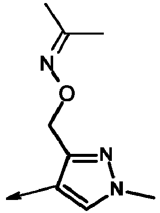 | 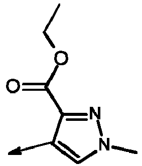 |
| Q-1.66 | Q-1.67 | Q-1.68 | Q-1.69 | Q-1.70 |
| 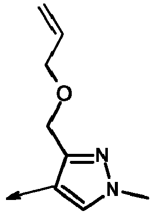 | 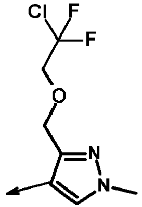 | | 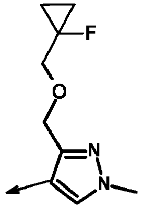 | 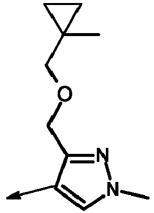 |
| Q-1.71 | Q-1.72 | | Q-1.73 | Q-1.74 |
| 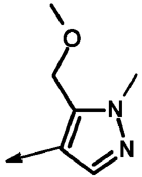 | 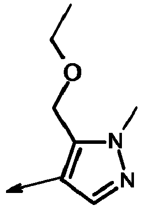 | 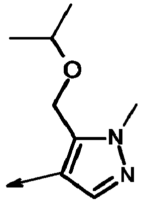 | 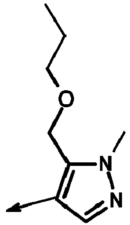 | 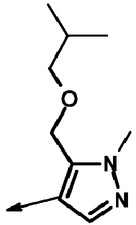 |
| Q-2.1 | Q-2.2 | Q-2.3 | Q-2.4 | Q-2.5 |
| 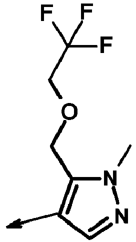 | 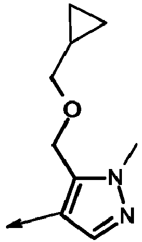 | 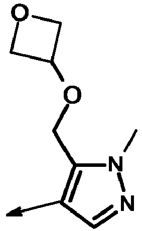 | 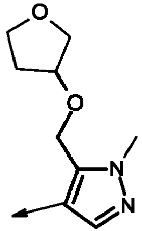 | 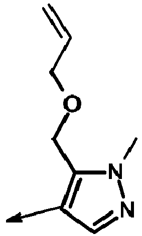 |
| Q-2.6 | Q-2.7 | Q-2.8 | Q-2.9 | Q-2.10 |
| 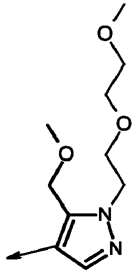 | 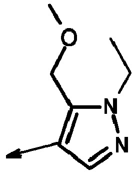 | 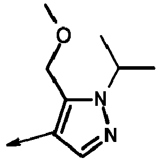 | 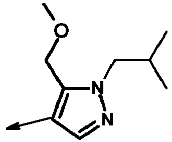 | 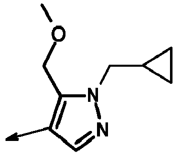 |
| Q-2.11 | Q-2.12 | Q-2.13 | Q-2.14 | Q-2.15 |
| 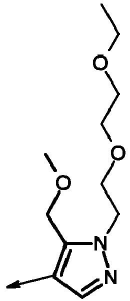 | 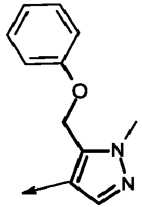 | 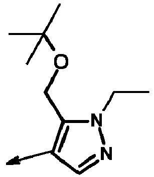 | 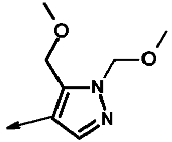 | 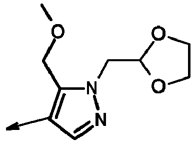 |
| Q-2.16 | Q-2.17 | Q-2.18 | Q-2.19 | Q-2.20 |
| 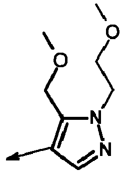 | 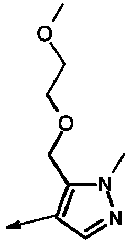 | 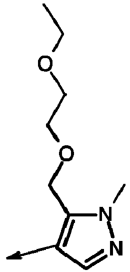 | 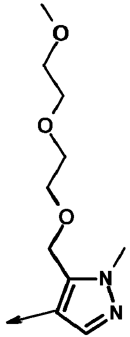 | 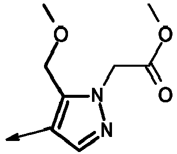 |
| Q-2.21 | Q-2.22 | Q-2.23 | Q-2.24 | Q-2.25 |
| 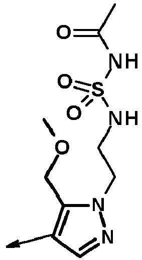 | 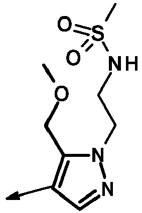 | 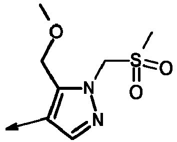 | 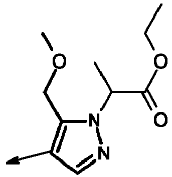 | 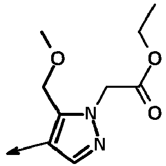 |
| Q-2.26 | Q-2.27 | Q-2.28 | Q-2.29 | Q-2.30 |
| 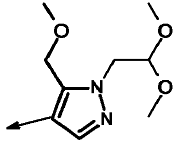 | 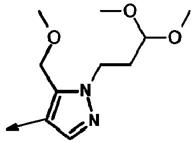 | 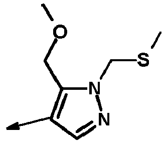 | 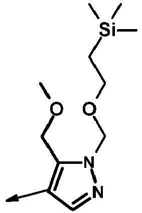 | 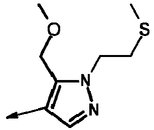 |
| Q-2.31 | Q-2.32 | Q-2.33 | Q-2.34 | Q-2.35 |
| 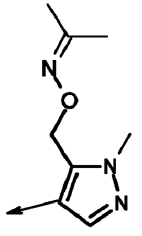 | | | | |
| | | Q-2.36 | | |
A¹, A², A³ and A⁴ are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
R¹⁷ in the moieties A³ and A⁴ is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
R¹⁷ in the moieties A¹ and A² is the same or different as defined below and together with the carbons atom to which it is bonded in each case forms a fully saturated, or partly saturated 3- to 10-membered monocyclic or bicyclic ring optionally interrupted by heteroatoms and optionally having further substitution,
X represents a direct bond or a group CR⁸R⁹, -CR⁸R⁹CH₂-, -CH₂CR⁸R⁹-,
R⁶ and R⁷ independently of one another represent hydrogen, methyl,
R⁸ and R⁹ independently of one another represent hydrogen, fluoro, methyl,
R¹⁴ and R¹⁵ independently of one another represent hydrogen, hydroxy, methyl, ethyl, difluoromethyl, 2,2-difluoroeth-1-yl, or
R¹⁴ and R¹⁵ together with the carbon atom to which they are bonded form a fully saturated or partly saturated 3- to 10-membered monocyclic or bicyclic ring optionally interrupted by heteroatoms and optionally having further substitution, or
R¹⁴ and R¹⁵ together with the carbon atom to which they are bonded form an ethylene group (C=CHz) or a carbonyl group (C=O),
R¹⁶ represents fluoro, chloro, bromo, iodo, methyl, methoxy,
R¹⁷ represents hydrogen, fluoro, chloro, bromo, methyl
and
W represents oxygen (O).

6. The compound of the general formula (I) as claimed in claim 1 and/or the salt thereof, **characterized in that**
Q represents a group Q-1.1, Q-1.2, Q-1.3, Q-1.4, Q-1.5, Q-1.6, Q-1.7, Q-1.8, Q-1.9, Q-1.10, Q-1.11, Q-1.12, Q-1.13, Q-1.14, Q-1.15, Q-1.16, Q-1.17, Q-1.19, Q-1.21, Q-1.26, Q-1.27, Q-1.28, Q-1.29, Q-1.30, Q-1.36, Q-1.37, Q-1.38, Q-1.39, Q-1.42, Q-1.50, Q-1.52, Q-1.56, Q-1.57, Q-1.59, Q-1.60, Q-1.61, Q-1.62, Q-1.63, Q-1.64, Q-1.65, Q-1.66, Q-1.72, Q-1.73, Q-2.1, Q-2.2, Q-2.3, Q-2.4, Q-2.5, Q-2.6, Q-2.7, Q-2.8, Q-2.9, Q-2.10,Q-2.12, Q-2.13, Q-2.14, Q-2.15, Q-2.21, Q-2.28, Q-2.31, Q-2.32, Q-2.34, Q-2.35, Q-2.36 as outlined in Claim 5,
A¹, A², A³ and A⁴ are the same or different and independently from one another represent N (nitrogen) or the C-R¹⁷ moiety, with a maximum of two adjacent nitrogen atoms, and where R¹⁷ in each C-R¹⁷ moiety is the same or different as defined below,
X represents a direct bond or a group CR⁸R⁹,
R⁶ and R⁷ independently of one another represent hydrogen, methyl,
R⁸ and R⁹ represent hydrogen,
R¹⁴ and R¹⁵ independently of one another represent hydrogen, hydroxy, methyl, ethyl, difluoromethyl, 2,2-difluoroeth-1-yl,
R¹⁴ and R¹⁵ together with the carbon atom to which they are bonded form a fully saturated 3-membered monocyclic ring optionally having further substitution, or
R¹⁴ and R¹⁵ together with the carbon atom to which they are bonded form an ethylene group (C=CHz) or a carbonyl group (C=O),
R¹⁶ represents fluoro, chloro, bromo, methyl, methoxy,
R¹⁷ represents hydrogen, fluoro, chloro
and
W represents oxygen (O).

7. The use of one or more compounds of the general formula (I) and/or salts thereof, as defined in any of claims 1 to 6, as herbicide and/or plant growth regulator.

8. An herbicidal and/or plant growth-regulating composition, **characterized in that** the composition comprises one or more compounds of the formula (I) and/or salts thereof as defined in any of claims 1 to 6, and one or more further substances selected from groups (i) and/or (ii), with
(i) one or more further agrochemically active substances, selected from the group consisting of insecticides, acaricides, nematicides, further herbicides, fungicides, safeners, fertilizers and/or further growth regulators,
(ii) one or more formulation auxiliaries customary in crop protection.

9. A method for controlling harmful plants or for regulating the growth of plants, **characterized in that** an effective amount
- of one or more compounds of the formula (I) and/or salts thereof, as defined in any of claims 1 to 6, or
- of a composition as claimed in claim 8,
is applied to the plants, seeds of plants, the soil in which or on which the plants grow or the area under cultivation.
